# EUROPEAN PATENT APPLICATION

(11) **EP 3 967 745 A1**
(43) Date of publication of application: **16.03.2022**
(21) Application number: 20306016.5
(22) Date of filing: 11.09.2020
(51) Int. Cl.: C12N 1/12, A01N 63/60, A01N 65/03, C12N 15/11, C12N 15/113, C12N 15/82

(54) **CHLORELLA-BASED PRODUCTION OF EXTRACELLULAR VESICLE-EMBEDDED SMALL RNAS FOR BIOCONTROL APPLICATIONS**

(71) Applicant: Immunrise, 75005 Paris (FR); Centre National de la Recherche Scientifique (CNRS), 75016 Paris (FR)
(72) Inventor: NAVARRO, Lionel, 92160 ANTONY (FR); Zervudacki, Jérôme, 92340 BOURG-LA-REINE (FR); FORTUNATO, Antonio Emidio, 93170 BAGNOLET (FR)
(74) Representative: Regimbeau

(57) **Abstract**

The invention relates to a novel method to produce small RNAs targeting virulence factors, essential genes and /or antimicrobial resistance genes of phytopathogens. More specifically, the invention involves the expression of exogenous RNA interference (RNAi) precursor(s) in *Chlorella* cells, which in turn express and release Extracellular Vesicle (EV)-embedded antimicrobial small RNAs. These EVs can be collected from the cell-free medium of *Chlorella* cultures, and further concentrated and purified for biocontrol applications. Importantly, *Chlorella* EVs protect small RNAs from ribonuclease-mediated digestion, indicating that these lipid-based particles not only act as natural vectors of small RNAs towards pathogenic cells, but also presumably limit their degradation in the environment. The invention can thus likely be used to reduce the pathogenicity and growth of a wide range of pathogens or, potentially, to enhance beneficial effects and growth of plant-associated symbiotic and commensal microbes. Furthermore, because the integrity of *Chlorella* EV-embedded antimicrobial siRNAs remains unaltered when produced in photobioreactors, and when stored frozen, this method has the potential to be further exploited for the industrialization and manufacturing of a novel generation of microalgae-based biologicals.

SEQUENCE LISTING

<110>  IMMUNRISE
       CENTRE NATIONAL DE LA RECHERCHE SCIENTIFIQUE (CNRS)

<120>  CHLORELLA-BASED PRODUCTION OF EXTRACELLULAR VESICLE-EMBEDDED
       SMALL RNAs FOR BIOCONTROL APPLICATIONS

<130>  B75971 D40243

<160>  149

<170>  PatentIn version 3.5

<210>  1
<211>  919
<212>  DNA
<213>  artificial

<220>
<223>  Sequence of the first arm of the
       cheA/gaCA/tolC/pglA/engXCA1/engXCA2 dsRNA used to concomitantly
       target cheA, gaCA, tolC, pglA, engXCA1, engXCA2 genes and regions
       from xylella fastidiosa

<400>  1
atgaacgcac ttcgtgatgc gatgagccat gctgtgcttg gctgggtaaa gcctgatcta      60

gatgaagctt tgggccaagc acgccaggag attgagttat tcgttgagaa ccctgtagac     120

actagtcgga tactttcttg cgctgattag atcatgacta ttaagatttt tctgattgat     180

gatcatactc tcgtgcgtgt tggcatgaag atgatcttat ccaatgaatt agatctcgaa     240

gtgatagggg aagcggagac aggggaggcg gctttaccac agatccgtga gctacgcccg     300

aatatgcatg atccgccgct gcctcgcctt ggccgtgacc accgccctat ttcccgtcgt     360

ttcacaagcg acaaacctat tacaagtcta cgaaatggca cgcgctgctg accctcaatt     420

aagcgcagcc gaatccactc ggctatacag cgttgaagtg catgaacctt gaccgtttcc     480

tccccctagt gtttggactg cactgttttg ccgcaatggg cggcactgct gatgtctttc     540

gagtgtcaca gctgtcctca tcagatcttt ccgagccggt atcggtgaaa acgctatggg     600

gcgaggtgca aaagctggcc tccgccatgt acggtggaac ggtgggtgca caaaatgcag     660

tgtctgatac tcactttgtt gaaccactcc gcggcgttaa ctggcgtggt ttggaaacgg     720

cgcagcacct gccgcaaggt ttaaatcaac gtccctggcg tgaagtccaa tgtcgttttc     780

caaacacttc ggcaaactca tgttggccac gtgcctatcc ttatctgcgg caccagcatt     840

cagttacagc atcagtcacg ggaaggtcgt cgatgacaaa ggcaaccaaa tccaattaag     900

gggcgtcaac tggtttggt                                                  919

<210>  2
<211>  919
<212>  DNA
<213>  artificial

<220>
<223>  Sequence of the second arm of the
      cheA/gaCA/tolC/pglA/engXCA1/engXCA2 dsRNA used to concomitantly
      target cheA, gaCA, tolC, pglA/engXCA1, engXCA2 genes and regions
      from xylella fastidiosa

<400>  2
accaaaccag ttgacgcccc ttaattggat ttggttgcct ttgtcatcga cgaccttccc      60

gtgactgatg ctgtaactga atgctggtgc cgcagataag gataggcacg tggccaacat     120

gagtttgccg aagtgtttgg aaaacgacat tggacttcac gccagggacg ttgatttaaa     180

ccttgcggca ggtgctgcgc cgtttccaaa ccacgccagt taacgccgcg gagtggttca     240

acaaagtgag tatcagacac tgcattttgt gcacccaccg ttccaccgta catggcggag     300

gccagctttt gcacctcgcc ccatagcgtt ttcaccgata ccggctcgga aagatctgat     360

gaggacagct gtgacactcg aaagacatca gcagtgccgc ccattgcggc aaaacagtgc     420

agtccaaaca ctagggggag gaaacggtca aggttcatgc acttcaacgc tgtatagccg     480

agtggattcg gctgcgctta attgagggtc agcagcgcgt gccatttcgt agacttgtaa     540

taggtttgtc gcttgtgaaa cgacgggaaa tagggcggtg gtcacggcca aggcgaggca     600

gcggcggatc atgcatattc gggcgtagct cacggatctg tggtaaagcc gcctcccctg     660

tctccgcttc ccctatcact tcgagatcta attcattgga taagatcatc ttcatgccaa     720

cacgcacgag agtatgatca tcaatcagaa aaatcttaat agtcatgatc taatcagcgc     780

aagaaagtat ccgactagtg tctacagggt tctcaacgaa taactcaatc tcctggcgtg     840

cttggcccaa agcttcatct agatcaggct ttacccagcc aagcacagca tggctcatcg     900

catcacgaag tgcgttcat                                                  919


<210>  3
<211>  1073
<212>  DNA
<213>  artificial

<220>
<223>  Sequence of the first arm of the cheA/GumH/GumD/ XpsE/ LesA/ HolC
      dsRNA used to concomitantly target cheA, GumH, GumD, Xpse, LesA,
      HolC genes and regions from xylella fastidiosa

<400>  3
atgaacgcac ttcgtgatgc gatgagccat gctgtgcttg gctgggtaaa gcctgatcta      60

gatgaagctt tgggccaagc acgccaggag attgagttat tcgttgagaa ccctgtagac     120

actagtcgga tactttcttg cgctgattag atcatgaaag tggtgcatgt tgttcgtcaa     180

tttcatccgt caattggtgg gatggaggat gtggtcttca atattgcgat gcagctgcat     240

ttgcatgctg gaatcggtgt tgatgttgtg acgttgaacc gagtatttac tcagtctgat     300

gttatgcatg cttctggcag atctgaacag tgttactgat attgcttctt caccgcggtt     360

attgtcgaaa tattctgctg ccgctgatct aatacttcgt gtttttgatc taacaatggt 420

ggtgctgtct ggtgtactga tttacagatt gttgtttgtg catgaacctt gaccgtttcc 480

tccccctagt gtttggactg cactgttttg ccgcaatggg cggcactgct gatgtctttc 540

gagtgtcaca gctgtcctca tcagatcttt ccgagccggt atcggtgaaa acgctatggg 600

gcgaggtgca aaagcgtgga aaataggaat aacgacaata cgttgttgtg cgttgttggc 660

ggaagtatga atgtaagtat ggaagcatcc atcgttgagt cgttgttgca gcgtcgttgc 720

ttgaaagaaa gtgatctgta tcgtgcgcgt cagttgcagg ccgaatccaa tgatgtatgg 780

ttctcaacga aaaccggtgc ttcttatcct gagttacttt atttttatct gtgctctgtt 840

cccctgtgtt gcagctgctt ggccttcacg tgggatggtg attaacagaa aaattattgg 900

tagctatacg cagcgtcagt ccaatggcac gcgccgactt ctatttgatt gccaaaccgc 960

gctttcttga gaaaccgctg caattagtct gcgaattagt ccgcaaagcc tacgacaccc 1020

atcagcccac actgattctg gcccgcgacc aggcccaggc cgaagcactg gat 1073

<210> 4
<211> 1073
<212> DNA
<213> artificial

<220>
<223> Sequence of the second arm of the cheA/GumH/GumD/ XpsE/ LesA/
 HolC dsRNA used to concomitantly target cheA, GumH, GumD, Xpse,
 LesA, HolC genes and regions from xylella fastidiosa

<400> 4
atccagtgct tcggcctggg cctggtcgcg ggccagaatc agtgtgggct gatgggtgtc 60

gtaggctttg cggactaatt cgcagactaa ttgcagcggt ttctcaagaa agcgcggttt 120

ggcaatcaaa tagaagtcgg cgcgtgccat tggactgacg ctgcgtatag ctaccaataa 180

tttttctgtt aatcaccatc ccacgtgaag gccaagcagc tgcaacacag gggaacagag 240

cacagataaa aataaagtaa ctcaggataa gaagcaccgg ttttcgttga gaaccataca 300

tcattggatt cggcctgcaa ctgacgcgca cgatacagat cactttcttt caagcaacga 360

cgctgcaaca acgactcaac gatggatgct tccatactta cattcatact tccgccaaca 420

acgcacaaca acgtattgtc gttattccta ttttccacgc ttttgcacct cgccccatag 480

cgttttcacc gataccggct cggaaagatc tgatgaggac agctgtgaca ctcgaaagac 540

atcagcagtg ccgcccattg cggcaaaaca gtgcagtcca aacactaggg ggaggaaacg 600

gtcaaggttc atgcacaaac aacaatctgt aaatcagtac accagacagc accaccattg 660

ttagatcaaa aacacgaagt attagatcag cggcagcaga atatttcgac aataaccgcg 720

gtgaagaagc aatatcagta acactgttca gatctgccag aagcatgcat aacatcagac 780

tgagtaaata ctcggttcaa cgtcacaaca tcaacaccga ttccagcatg caaatgcagc 840

tgcatcgcaa tattgaagac cacatcctcc atcccaccaa ttgacggatg aaattgacga 900

acaacatgca ccactttcat gatctaatca gcgcaagaaa gtatccgact agtgtctaca 960

gggttctcaa cgaataactc aatctcctgg cgtgcttggc ccaaagcttc atctagatca 1020

ggctttaccc agccaagcac agcatggctc atcgcatcac gaagtgcgtt cat 1073

<210> 5
<211> 758
<212> DNA
<213> artificial

<220>
<223> Sequence of the first arm of the LesA/GumH/GumD dsRNA used to
 concomitantly target LesA, GumH, GumD genes and regions from
 xylella fastidiosa

<400> 5
   atgatgtatg gttctcaacg aaaaccggtg cttcttatcc tgagttactt tatttttatc 60
   tgtgctctgt tcccctgtgt tgcagctgct tggccttcac gtgggatggt gattaacaga 120
   aaaattattg gtagctatac gcagcgtcag attgctgctc tgcttactga tgagccgcca 180
   tccgagcaac ccaaatgcaa cgttcgagtg gttgaaatga cgtatacaac ggttggtgtg 240
   gttggtgaac gatcatgaaa gtggtgcatg ttgttcgtca atttcatccg tcaattggtg 300
   ggatggagga tgtggtcttc aatattgcga tgcagctgca tttgcatgct ggaatcggtg 360
   ttgatgttgt gacgttgaac cgagtattta ctcagtctga tgttctactt ccatgtactg 420
   ataagtatca gggtgtgtcg atccagcgca ttggttatcg tggttcttcg cgttatccat 480
   tggcaccagg agtgttgagt atgtatgcat gcttctggca gatctgaaca gtgttactga 540
   tattgcttct tcaccgcggt tattgtcgaa atattctgct gccgctgatc taatacttcg 600
   tgtttttgat ctaacaatgg tggtgctgtc tggtgtactg atttacagat tgttgtttgg 660
   taattggatg ttaacggcca catatcgtat tgctattggt aacacgttat tgtattcggt 720
   gatttgtttt tctttttccc cgttgtatcg cagttggc 758

<210> 6
<211> 758
<212> DNA
<213> artificial

<220>
<223> Sequence of the second arm of the LesA/GumH/GumD dsRNA used to
 concomitantly target LesA, GumH, GumD genes and regions from
 xylella fastidiosa

<400> 6
gccaactgcg atacaacggg aaaaaagaaa aacaaatcac cgaatacaat aacgtgttac 60

caatagcaat acgatatgtg gccgttaaca tccaattacc aaacaacaat ctgtaaatca 120

gtacaccaga cagcaccacc attgttagat caaaaacacg aagtattaga tcagcggcag 180

cagaatattt cgacaataac cgcggtgaag aagcaatatc agtaacactg ttcagatctg 240

ccagaagcat gcatacatac tcaacactcc tggtgccaat ggataacgcg aagaaccacg 300

ataaccaatg cgctggatcg acacaccctg atacttatca gtacatggaa gtagaacatc 360

agactgagta aatactcggt tcaacgtcac aacatcaaca ccgattccag catgcaaatg 420

cagctgcatc gcaatattga agaccacatc ctccatccca ccaattgacg gatgaaattg 480

acgaacaaca tgcaccactt tcatgatcgt tcaccaacca caccaaccgt tgtatacgtc 540

atttcaacca ctcgaacgtt gcatttgggt tgctcggatg gcggctcatc agtaagcaga 600

gcagcaatct gacgctgcgt atagctacca ataatttttc tgttaatcac catcccacgt 660

gaaggccaag cagctgcaac acaggggaac agagcacaga taaaaataaa gtaactcagg 720

ataagaagca ccggttttcg ttgagaacca tacatcat 758

<210> 7
<211> 758
<212> DNA
<213> artificial

<220>
<223> Sequence of the first arm of the XpsE/HolC/LesA dsRNA used to
concomitantly target XpsE, HolC, LesA genes and regions from
xylella fastidiosa

<400> 7
gtggaaaata ggaataacga caatacgttg ttgtgcgttg ttggcggaag tatgaatgta 60

agtatggaag catccatcgt tgagtcgttg ttgcagcgtc gttgcttgaa agaaagtgat 120

ctgtatcgtg cgcgtcagtt gcaggccgaa tccggtgttg ggttgttacc tttgttgggc 180

cgtcttggtt tggtttctga gcgcgatcac gctgaagtat gtgcggaggt gatggggctg 240

ttgttgatac gatcatggca cgcgccgact tctatttgat tgccaaaccg cgctttcttg 300

agaaaccgct gcaattagtc tgcgaattag tccgcaaagc ctacgacacc catcagccca 360

cactgattct ggcccgcgac caggcccagg ccgaagcact ggatgaccta ctgtgggcgt 420

tcgacccgga cgcctacatc ccccaccaaa tcgctggcag cgacgaagac gacgacatca 480

cccccgtatt gattgccacc ccggatgcat gatgtatggt tctcaacgaa aaccggtgct 540

tcttatcctg agttacttta tttttatctg tgctctgttc ccctgtgttg cagctgcttg 600

gccttcacgt gggatggtga ttaacagaaa aattattggt agctatacgc agcgtcagat 660

tgctgctctg cttactgatg agccgccatc cgagcaaccc aaatgcaacg ttcgagtggt 720

tgaaatgacg tatacaacgg ttggtgtggt tggtgaac 758

<210> 8

<211>  758
<212>  DNA
<213>  artificial

<220>
<223>  Sequence of the second arm of the XpsE/HolC/LesA dsRNA used to
       concomitantly target XpsE, HolC, LesA  genes and regions from
       xylella fastidiosa

<400>  8
gttcaccaac cacaccaacc gttgtatacg tcatttcaac cactcgaacg ttgcatttgg      60

gttgctcgga tggcggctca tcagtaagca gagcagcaat ctgacgctgc gtatagctac     120

caataatttt tctgttaatc accatcccac gtgaaggcca agcagctgca acacagggga     180

acagagcaca gataaaaata aagtaactca ggataagaag caccggtttt cgttgagaac     240

catacatcat gcatccgggg tggcaatcaa tacgggggtg atgtcgtcgt cttcgtcgct     300

gccagcgatt tggtggggga tgtaggcgtc cgggtcgaac gcccacagta ggtcatccag     360

tgcttcggcc tgggcctggt cgcgggccag aatcagtgtg ggctgatggg tgtcgtaggc     420

tttgcggact aattcgcaga ctaattgcag cggtttctca agaaagcgcg gtttggcaat     480

caaatagaag tcggcgcgtg ccatgatcgt atcaacaaca gccccatcac ctccgcacat     540

acttcagcgt gatcgcgctc agaaaccaaa ccaagacggc ccaacaaagg taacaaccca     600

acaccggatt cggcctgcaa ctgacgcgca cgatacagat cactttcttt caagcaacga     660

cgctgcaaca acgactcaac gatggatgct tccatactta cattcatact tccgccaaca     720

acgcacaaca acgtattgtc gttattccta ttttccac                             758


<210>  9
<211>  759
<212>  DNA
<213>  artificial

<220>
<223>  Sequence of the first arm of the CheA/pglA/LesA dsRNA used to
       concomitantly target CheA, pglA, lesA  genes and regions from
       xylella fastidiosa

<400>  9
tgaacgcact tcgtgatgcg atgagccatg ctgtgcttgg ctgggtaaag cctgatctag      60

atgaagcttt gggccaagca cgccaggaga ttgagttatt cgttgagaac cctgtagaca     120

ctagtcggat actttcttgc gctgattacc tgcatcaggt gcagggtacc ttgcgaatgc     180

ttgaattata tgctcccgca atggtggccg aggaaatgga acggctagct aaggtgatgc     240

aagtcgatgg gatcatgaac cttgaccgtt tcctccccct agtgtttgga ctgcactgtt     300

ttgccgcaat gggcggcact gctgatgtct ttcgagtgtc acagctgtcc tcatcagatc     360

tttccgagcc ggtatcggtg aaaacgctat ggggcgaggt gcaacgtccc agcctaccta     420

cccacgtgtg tacggtattg cctgcgcgcc tgactcctaa acatggctcc attgacgcac     480

tcgatgccaa tccaaaggtt tccaaatgca tgatgtatgg ttctcaacga aaaccggtgc 540

ttcttatcct gagttacttt atttttatct gtgctctgtt cccctgtgtt gcagctgctt 600

ggccttcacg tgggatggtg attaacagaa aaattattgg tagctatacg cagcgtcaga 660

ttgctgctct gcttactgat gagccgccat ccgagcaacc caaatgcaac gttcgagtgg 720

ttgaaatgac gtatacaacg gttggtgtgg ttggtgaac 759

<210> 10
<211> 759
<212> DNA
<213> artificial

<220>
<223> Sequence of the second arm of the CheA/pglA/LesA dsRNA used to
concomitantly target CheA, pglA, lesA genes and regions from
xylella fastidiosa

<400> 10
gttcaccaac cacaccaacc gttgtatacg tcatttcaac cactcgaacg ttgcatttgg 60

gttgctcgga tggcggctca tcagtaagca gagcagcaat ctgacgctgc gtatagctac 120

caataatttt tctgttaatc accatcccac gtgaaggcca agcagctgca acacagggga 180

acagagcaca gataaaaata aagtaactca ggataagaag caccggtttt cgttgagaac 240

catacatcat gcatttggaa acctttggat tggcatcgag tgcgtcaatg gagccatgtt 300

taggagtcag gcgcgcaggc aataccgtac acacgtgggt aggtaggctg ggacgttgca 360

cctcgcccca tagcgttttc accgataccg gctcggaaag atctgatgag gacagctgtg 420

acactcgaaa gacatcagca gtgccgccca ttgcggcaaa acagtgcagt ccaaacacta 480

gggggaggaa acggtcaagg ttcatgatcc catcgacttg catcacctta gctagccgtt 540

ccatttcctc ggccaccatt gcgggagcat ataattcaag cattcgcaag gtaccctgca 600

cctgatgcag gtaatcagcg caagaaagta tccgactagt gtctacaggg ttctcaacga 660

ataactcaat ctcctggcgt gcttggccca aagcttcatc tagatcaggc tttacccagc 720

caagcacagc atggctcatc gcatcacgaa gtgcgttca 759

<210> 11
<211> 758
<212> DNA
<213> artificial

<220>
<223> Sequence of the first arm of the cheA/engXCA1/engXCA2 dsRNA used
to concomitantly target cheA, engXCA1, engXCA2 genes and regions
from xylella fastidiosa

<400> 11
tgaacgcact tcgtgatgcg atgagccatg ctgtgcttgg ctgggtaaag cctgatctag 60

atgaagcttt gggccaagca cgccaggaga ttgagttatt cgttgagaac cctgtagaca 120

ctagtcggat actttcttgc gctgattacc tgcatcaggt gcagggtacc ttgcgaatgc 180

ttgaattata tgctcccgca atggtggccg aggaaatgga acggctagct aaggtgatgc 240

aagtcgatgg gatcgtggcc tccgccatgt acggtggaac ggtgggtgca caaaatgcag 300

tgtctgatac tcactttgtt gaaccactcc gcggcgttaa ctggcgtggt ttggaaacgg 360

cgcagcacct gccgcaaggt ttaaatcaac gtccctggcg tgaagtactc gaccaaatgc 420

agagcctagg cattaacgca atacgcctgc ccctgtgctc ggacacccta catggcgcca 480

tgcctaccaa tctggatcta gtgcatgcat gtcgttttcc aaacacttcg gcaaactcat 540

gttggccacg tgcctatcct tatctgcggc accagcattc agttacagca tcagtcacgg 600

gaaggtcgtc gatgacaaag gcaaccaaat ccaattaagg ggcgtcaact ggtttggttt 660

tgaaaccgga gatcatgtgg taaatggact atgggcacgt aattggaaag aattcatcac 720

acagctacag ggcatgggat tcaacgcgat acgtttac 758

<210> 12
<211> 758
<212> DNA
<213> artificial

<220>
<223> Sequence of the second arm of the cheA/engXCA1/engXCA2 dsRNA used
to concomitantly target cheA, engXCA1, engXCA2 genes and regions
from xylella fastidiosa

<400> 12
gtaaacgtat cgcgttgaat cccatgccct gtagctgtgt gatgaattct ttccaattac 60

gtgcccatag tccatttacc acatgatctc cggtttcaaa accaaaccag ttgacgcccc 120

ttaattggat ttggttgcct ttgtcatcga cgaccttccc gtgactgatg ctgtaactga 180

atgctggtgc cgcagataag gataggcacg tggccaacat gagtttgccg aagtgtttgg 240

aaaacgacat gcatgcacta gatccagatt ggtaggcatg gcgccatgta gggtgtccga 300

gcacaggggc aggcgtattg cgttaatgcc taggctctgc atttggtcga gtacttcacg 360

ccagggacgt tgatttaaac cttgcggcag gtgctgcgcc gtttccaaac cacgccagtt 420

aacgccgcgg agtggttcaa caaagtgagt atcagacact gcattttgtg cacccaccgt 480

tccaccgtac atggcggagg ccacgatccc atcgacttgc atcaccttag ctagccgttc 540

catttcctcg gccaccattg cgggagcata taattcaagc attcgcaagg taccctgcac 600

ctgatgcagg taatcagcgc aagaaagtat ccgactagtg tctacagggt tctcaacgaa 660

taactcaatc tcctggcgtg cttggcccaa agcttcatct agatcaggct ttacccagcc 720

aagcacagca tggctcatcg catcacgaag tgcgttca 758

<210>  13
<211>  759
<212>  DNA
<213>  artificial

<220>
<223>  Sequence of the first arm of the gacA/pglA/engXCA2 dsRNA used to
       concomitantly target gacA, pglA, engXCA2 genes and regions from
       xylella fastidiosa

<400>  13
atgactatta agatttttct gattgatgat catactctcg tgcgtgttgg catgaagatg      60

atcttatcca atgaattaga tctcgaagtg ataggggaag cggagacagg ggaggcggct     120

ttaccacaga tccgtgagct acgcccgaat gtcgtattgt gtgacatgca tctccctggg     180

gtgagtgggc tggagattac agaaaaattg gtgaaaggga attatggtag ccgtgtaatt     240

attgtttcgg gatcatgaac cttgaccgtt tcctccccct agtgtttgga ctgcactgtt     300

ttgccgcaat gggcggcact gctgatgtct ttcgagtgtc acagctgtcc tcatcagatc     360

tttccgagcc ggtatcggtg aaaacgctat ggggcgaggt gcaacgtccc agcctaccta     420

cccacgtgtg tacggtattg cctgcgcgcc tgactcctaa acatggctcc attgacgcac     480

tcgatgccaa tccaaaggtt tccaaatgca tgtcgttttc caaacacttc ggcaaactca     540

tgttggccac gtgcctatcc ttatctgcgg caccagcatt cagttacagc atcagtcacg     600

ggaaggtcgt cgatgacaaa ggcaaccaaa tccaattaag gggcgtcaac tggtttggtt     660

ttgaaaccgg agatcatgtg gtaaatggac tatgggcacg taattggaaa gaattcatca     720

cacagctaca gggcatggga ttcaacgcga tacgtttac                            759


<210>  14
<211>  759
<212>  DNA
<213>  artificial

<220>
<223>  Sequence of the second arm of the gacA/pglA/engXCA2 dsRNA used to
       concomitantly target gacA, pglA, engXCA2 genes and regions from
       xylella fastidiosa

<400>  14
gtaaacgtat cgcgttgaat cccatgccct gtagctgtgt gatgaattct ttccaattac      60

gtgcccatag tccatttacc acatgatctc cggtttcaaa accaaaccag ttgacgcccc     120

ttaattggat ttggttgcct ttgtcatcga cgaccttccc gtgactgatg ctgtaactga     180

atgctggtgc cgcagataag gataggcacg tggccaacat gagtttgccg aagtgtttgg     240

aaaacgacat gcatttggaa acctttggat tggcatcgag tgcgtcaatg gagccatgtt     300

taggagtcag gcgcgcaggc aataccgtac acacgtgggt aggtaggctg ggacgttgca     360

cctcgcccca tagcgttttc accgataccg gctcggaaag atctgatgag gacagctgtg     420

acactcgaaa gacatcagca gtgccgccca ttgcggcaaa acagtgcagt ccaaacacta 480

gggggaggaa acggtcaagg ttcatgatcc cgaaacaata attacacggc taccataatt 540

ccctttcacc aatttttctg taatctccag cccactcacc ccagggagat gcatgtcaca 600

caatacgaca ttcgggcgta gctcacggat ctgtggtaaa gccgcctccc ctgtctccgc 660

ttcccctatc acttcgagat ctaattcatt ggataagatc atcttcatgc caacacgcac 720

gagagtatga tcatcaatca gaaaaatctt aatagtcat 759

<210> 15
<211> 759
<212> DNA
<213> artificial

<220>
<223> Sequence of the first arm of the cheA/tolC/engXCA1 dsRNA used to
      concomitantly target cheA, tolC, engXCA1 genes and regions from
      xylella fastidiosa

<400> 15
tgaacgcact tcgtgatgcg atgagccatg ctgtgcttgg ctgggtaaag cctgatctag 60

atgaagcttt gggccaagca cgccaggaga ttgagttatt cgttgagaac cctgtagaca 120

ctagtcggat actttcttgc gctgattacc tgcatcaggt gcagggtacc ttgcgaatgc 180

ttgaattata tgctcccgca atggtggccg aggaaatgga acggctagct aaggtgatgc 240

aagtcgatgg gatcatgatc cgccgctgcc tcgccttggc cgtgaccacc gccctatttc 300

ccgtcgtttc acaagcgaca aacctattac aagtctacga aatggcacgc gctgctgacc 360

ctcaattaag cgcagccgaa tccactcggc tatacagcgt tgaagggcaa gttcaggcac 420

gtgcagcgct gctgccgcag atgagcggca cagccaccct ggcacgtagc cgcactgaat 480

acgacggtat cagcggatca gccatatgcg tggcctccgc catgtacggt ggaacggtgg 540

gtgcacaaaa tgcagtgtct gatactcact ttgttgaacc actccgcggc gttaactggc 600

gtggtttgga aacggcgcag cacctgccgc aaggtttaaa tcaacgtccc tggcgtgaag 660

tactcgacca aatgcagagc ctaggcatta acgcaatacg cctgcccctg tgctcggaca 720

ccctacatgg cgccatgcct accaatctgg atctagtgc 759

<210> 16
<211> 759
<212> DNA
<213> artificial

<220>
<223> Sequence of the second arm of the cheA/tolC/engXCA1 dsRNA used to
      concomitantly target cheA, tolC, engXCA1 genes and regions from
      xylella fastidiosa

<400> 16
gcactagatc cagattggta ggcatggcgc catgtagggt gtccgagcac aggggcaggc 60

gtattgcgtt aatgcctagg ctctgcattt ggtcgagtac ttcacgccag ggacgttgat 120

ttaaaccttg cggcaggtgc tgcgccgttt ccaaaccacg ccagttaacg ccgcggagtg 180

gttcaacaaa gtgagtatca gacactgcat tttgtgcacc caccgttcca ccgtacatgg 240

cggaggccac gcatatggct gatccgctga taccgtcgta ttcagtgcgg ctacgtgcca 300

gggtggctgt gccgctcatc tgcggcagca gcgctgcacg tgcctgaact tgcccttcaa 360

cgctgtatag ccgagtggat tcggctgcgc ttaattgagg gtcagcagcg cgtgccattt 420

cgtagacttg taataggttt gtcgcttgtg aaacgacggg aaatagggcg gtggtcacgg 480

ccaaggcgag gcagcggcgg atcatgatcc catcgacttg catcacctta gctagccgtt 540

ccatttcctc ggccaccatt gcgggagcat ataattcaag cattcgcaag gtaccctgca 600

cctgatgcag gtaatcagcg caagaaagta tccgactagt gtctacaggg ttctcaacga 660

ataactcaat ctcctggcgt gcttggccca aagcttcatc tagatcaggc tttacccagc 720

caagcacagc atggctcatc gcatcacgaa gtgcgttca 759

<210> 17
<211> 799
<212> DNA
<213> artificial

<220>
<223> Sequence of the first arm of the gyrB dsRNA used to concomitantly
 target gyrase subunit B gene from xanthomonas hortorum vitians

<400> 17
atgaccgaag atcaaacacc ccgaccacac ccaacagcac ctacgattcc agcaagatca 60

ccgtgctgcg tggcctggaa gccgtccgca agcgccccgg catgtacatc ggcgatgtcc 120

atgacggcac cggcctgcat cacatggtgt tcgaggtggt cgacaactcg gtcgacgagg 180

ccctggccgg ccatgccgac gacatcgtgg tcaagatcct ggtcgatggc tcggtcgccg 240

tgtcggataa cggccgcggc gtgccggtcg acatccacaa ggaagaaggc gtgtcggcgg 300

cggaggtgat cctcaccgtg ctgcatgccg gcggcaagtt cgacgataac agttacaagg 360

tttccggcgg cctgcacggc gtgggcgtgt cggtggtcaa cgcgctgtcc gagcacctgt 420

ggctggacat ctggcgcgac ggcttccact accagcagga atacgcgctg ggtgagccgc 480

agtacccgct caagcaactc gaagcctcga ccaagcgcgg caccacgctg cgcttcaagc 540

cggccgtggc catcttcagc gacgtcgagt tccattacga catcctggcg cgccgtctgc 600

gtgagctgtc gttcctcaat tccggcgtca agatcgcgct gatcgacgaa cgtggcgaag 660

gccgccgcga cgatttccat tacgaaggcg gcatccgcag cttcgtggag catctggccc 720

agctgaagtc gccgctgcac cccaacgtga tctcggtgac cggcgagcac aacggcatcg 780

tggtggacgt ggccctgca 799

<210>  18
<211>  798
<212>  DNA
<213>  artificial

<220>
<223>  Sequence of the second arm of the gyrB dsRNA used to
       concomitantly target gyrase subunit B  gene from xanthomonas
       hortorum vitians

<400>  18
tgcagggcca cgtccaccac gatgccgttg tgctcgccgg tcaccgagat cacgttgggg      60

tgcagcggcg acttcagctg ggccagatgc tccacgaagc tgcggatgcc gccttcgtaa     120

tggaaatcgt cgcggcggcc ttcgccacgt tcgtcgatca gcgcgatctt gacgccggaa     180

ttgaggaacg acagctcacg cagacggcgc gccaggatgt cgtaatggaa ctcgacgtcg     240

ctgaagatgg ccacggccgg cttgaagcgc agcgtggtgc cgcgcttggt cgaggcttcg     300

agttgcttga gcgggtactg cggctcaccc agcgcgtatt cctgctggta gtggaagccg     360

tcgcgccaga tgtccagcca caggtgctcg gacagcgcgt tgaccaccga cacgcccacg     420

ccgtgcaggc cgccggaaac cttgtaactg ttatcgtcga acttgccgcc ggcatgcagc     480

acggtgagga tcacctccgc cgccgacacg ccttcttcct tgtggatgtc gaccggcacg     540

ccgcggccgt tatccgacac ggcgaccgag ccatcgacca ggatcttgac cacgatgtcg     600

tcggcatggc cggccagggc ctcgtcgacc gagttgtcga ccacctcgaa caccatgtga     660

tgcaggccgg tgccgtcatg gacatcgccg atgtacatgc cggggcgctt gcggacggct     720

tccaggccac gcagcacggt gatcttgctg gaatcgtagg tgctgttggg tgtggtcggg     780

ggtttgatct tcggtcat                                                   798


<210>  19
<211>  801
<212>  DNA
<213>  artificial

<220>
<223>  Sequence of the first arm of the fusA dsRNA used to concomitantly
       target fusa elongator factor G gene from xanthomonas hortorum
       vitians

<400>  19
gtggccgcaa ccaaccccta tcgagcgtta ccgcaacttc ggcatcatgg ctcacatcga      60

tgccggcaag accaccacct ccgagcgcat cctgttttac accggcgtca gtcacaagat     120

cggcgaagtc catgacggcg ccgcggtgat ggactggatg gagcaggagc aggagcgtgg     180

tatcaccatc acctccgctg ccaccaccgc gttctggtcg ggtatggaca agtccatgcc     240

gcagcatcgc ctcaacatca tcgacacccc cgggcacgtc gacttcacca tcgaggtgga     300

gcgctccttg cgcgtgctcg acggtgcggt gttcgtgctg tgcgccgtgg gtggcgtgca 360

gccgcagtcc gagaccgtgt ggcgccaggc caacaagtat tccgtgccgc gtatggcctt 420

cgtcaacaag atggaccgta ccggtgccaa cttcgacaag gtcgtcgagc agctgaaggc 480

ccgtctggga gcctacgccg tgccgatgca ggtgccgatc ggtgctgaag acggtttcga 540

gggcgtggtc gacctgctga agatgaaggc gatccattgg gatgttgcct cgcagggcac 600

caccttcgaa tacggtgaca tcccggccga tctggtcgag attgcgaccg aggcgcgttc 660

gttcatggtt gaagcggctg cagaagccag cgaagacctg atggacaagt acctcaacga 720

aggcgacctg accgaagaag agatcctggt gggtctgcgc gagcgcaccc tgaaggtcga 780

gatcgtgccg gtgttctgtg g 801

<210> 20
<211> 801
<212> DNA
<213> artificial

<220>
<223> Sequence of the second arm of the fusA dsRNA used to
 concomitantly target fusa elongator factor G gene from
 xanthomonas hortorum vitians

<400> 20
gtggccgcaa ccaaccccta tcgagcgtta ccgcaacttc ggcatcatgg ctcacatcga 60

tgccggcaag accaccacct ccgagcgcat cctgttttac accggcgtca gtcacaagat 120

cggcgaagtc catgacggcg ccgcggtgat ggactggatg gagcaggagc aggagcgtgg 180

tatcaccatc acctccgctg ccaccaccgc gttctggtcg ggtatggaca agtccatgcc 240

gcagcatcgc ctcaacatca tcgacacccc cgggcacgtc gacttcacca tcgaggtgga 300

gcgctccttg cgcgtgctcg acggtgcggt gttcgtgctg tgcgccgtgg gtggcgtgca 360

gccgcagtcc gagaccgtgt ggcgccaggc caacaagtat tccgtgccgc gtatggcctt 420

cgtcaacaag atggaccgta ccggtgccaa cttcgacaag gtcgtcgagc agctgaaggc 480

ccgtctggga gcctacgccg tgccgatgca ggtgccgatc ggtgctgaag acggtttcga 540

gggcgtggtc gacctgctga agatgaaggc gatccattgg gatgttgcct cgcagggcac 600

caccttcgaa tacggtgaca tcccggccga tctggtcgag attgcgaccg aggcgcgttc 660

gttcatggtt gaagcggctg cagaagccag cgaagacctg atggacaagt acctcaacga 720

aggcgacctg accgaagaag agatcctggt gggtctgcgc gagcgcaccc tgaaggtcga 780

gatcgtgccg gtgttctgtg g 801

<210> 21
<211> 733
<212> DNA
<213> artificial

<220>
<223>  Sequence of the first arm of the ZipA dsRNA used to concomitantly
      target ZipA gene from xanthomonas hortorum vitians

<400>  21
atgtccgaac atggccatga tccggatcgg gatcctgatc gctggactgc tgctggtcgc      60

ggccatcttc ctgtttggtc gcccgaaaaa atcgccgcaa gggcgccggg tcgacaagga     120

cgacgggcag ccgcgcgaac gccgcgagcc ggtgatttcc ggcgagcctg gggtggaggg     180

cgacccgttc aagcgcactg aaggcgccga gcagagcgaa ctcaatctgg acgatgccga     240

tgcggccggc ggcaacgatg tcggcaagcg tccgaatcag gacttcgaca agatcgtctc     300

gctgttcgtc gcggccaagg ccggacaggt gctgcgcggc gaagacgtgg ttgttgctgc     360

cgagaagacg gggttggtgt tcggccacat gaatgtcttc caccgcttgg tggaagggca     420

cccggagcgc gggccgatct tcagcatggc cagcatcctc aagcccggca gtttcgacat     480

ggccaacatc cgcgagatgc agaccccggc gatcgcattt ttcctcacgc tgccggcgcc     540

gatgactgcg ctggatgcct gggaaaaaat gctgccgacc gtgcagcgca tggccgaact     600

actcgatggc gtggtgctgg acgacagccg caatgcgctg ggccgtcaac gtgtggcgca     660

tatccgcgat gagctgcgcg cctacgatcg ccagcatcag gcgccgccgc taaccaagtc     720

gccgcgctgg tga                                                        733

<210>  22
<211>  733
<212>  DNA
<213>  artificial

<220>
<223>  Sequence of the second arm of the ZipA dsRNA used to
      concomitantly target ZipA gene from xanthomonas hortorum vitians

<400>  22
tcaccagcgc ggcgacttgg ttagcggcgg cgcctgatgc tggcgatcgt aggcgcgcag      60

ctcatcgcgg atatgcgcca cacgttgacg gcccagcgca ttgcggctgt cgtccagcac     120

cacgccatcg agtagttcgg ccatgcgctg cacggtcggc agcatttttt cccaggcatc     180

cagcgcagtc atcggcgccg gcagcgtgag gaaaaatgcg atcgccgggg tctgcatctc     240

gcggatgttg gccatgtcga aactgccggg cttgaggatg ctggccatgc tgaagatcgg     300

cccgcgctcc gggtgccctt ccaccaagcg gtggaagaca ttcatgtggc cgaacaccaa     360

ccccgtcttc tcggcagcaa caaccacgtc ttcgccgcgc agcacctgtc cggccttggc     420

cgcgacgaac agcgagacga tcttgtcgaa gtcctgattc ggacgcttgc cgacatcgtt     480

gccgccggcc gcatcggcat cgtccagatt gagttcgctc tgctcggcgc cttcagtgcg     540

cttgaacggg tcgccctcca ccccaggctc gccggaaatc accggctcgc ggcgttcgcg     600

cggctgcccg tcgtccttgt cgacccggcg cccttgcggc gattttttcg ggcgaccaaa 660

caggaagatg gccgcgacca gcagcagtcc agcgatcagg atcccgatcc ggatcatggc 720

catgttcgga cat 733

<210> 23
<211> 919
<212> DNA
<213> artificial

<220>
<223> Sequence of the first arm of the rimM/rsgA/rbfA/mreB/gyrB/ftsZ
 dsRNA used to concomitantly target rimM,rsgA,rbfA,mreB,gyrB,ftsZ
 genes from acidovorax valerianella

<400> 23
atgcctaacc tccccgttct tgagcctgcc gaattgccgc ccgacgcggt ggagatcggc 60

cgtattgccg atgcctgggg tgtgaagggc tggttcaagg tgatggcata cagtgccgac 120

cccgaagcgc tgttttcgtc caagcgctgg gatcatggct gagcgcagcg ccctgtttga 180

cggactggtg gtggccagcc atggccggca ctgccttgtc gagtcgcccg acggccagcg 240

ccgcatctgc cacccccgcg gaaagaaaag ccaggcggtg gtgggagacc atgtttttgg 300

cagatgcatg gctgagcgca gcgccctgtt tgacggactg gtggtggcca gccatggccg 360

gcactgcctt gtcgagtcgc ccgacggcca gcgccgcatc tgccaccccc gcggaaagaa 420

aagccaggcg gtggtgggag accatgttct ttggcaggtg catgttcgga gctttccgtc 480

ggtacttctc caccgacctt gccattgacc ttggcacagc caacaccctg atttttgccc 540

gcgacaaggg catcgtgctc gatgaaccct cggtcgttgc catccgccac gagggcggtc 600

cccacggaaa gaagcatgac cgctgagaac accctgcccg agccttccac cgacggcgcg 660

ggcgaagcac agcccttcaa gatcgacacc cagcaagtgg gcgccagcga aggctacggc 720

gaaggcgcga tccagatcct ggaaggcctg gaggccgtgc gcaagtccaa tgaccatcga 780

aatgatcgaa gccgaagaat tcaatcaggg cacccagatc aaggtgatcg gtgtcggcgg 840

cggcggcggc aatgccgtgg cccacatgat cgcgcgcaat gttcagggcg tggagttcgt 900

ctgcgccaat accgacgcg 919

<210> 24
<211> 919
<212> DNA
<213> artificial

<220>
<223> Sequence of the second arm of the rimM/rsgA/rbfA/mreB/gyrB/ftsZ
 dsRNA used to concomitantly target rimM,rsgA,rbfA,mreB,gyrB,ftsZ
 genes from acidovorax valerianella

<400> 24
cgcgtcggta ttggcgcaga cgaactccac gccctgaaca ttgcgcgcga tcatgtgggc 60

cacggcattg ccgccgccgc cgccgacacc gatcaccttg atctgggtgc cctgattgaa 120

ttcttcggct tcgatcattt cgatggtcat tggacttgcg cacggcctcc aggccttcca 180

ggatctggat cgcgccttcg ccgtagcctt cgctggcgcc cacttgctgg gtgtcgatct 240

tgaagggctg tgcttcgccc gcgccgtcgg tggaaggctc gggcagggtg ttctcagcgg 300

tcatgcttct ttccgtgggg accgccctcg tggcggatgg caacgaccga gggttcatcg 360

agcacgatgc ccttgtcgcg ggcaaaaatc agggtgttgg ctgtgccaag gtcaatggca 420

aggtcggtgg agaagtaccg acggaaagct ccgaacatgc acctgccaaa gaacatggtc 480

tcccaccacc gcctggcttt tctttccgcg ggggtggcag atgcggcgct ggccgtcggg 540

cgactcgaca aggcagtgcc ggccatggct ggccaccacc agtccgtcaa acagggcgct 600

gcgctcagcc atgcatctgc caaaaacatg gtctcccacc accgcctggc ttttctttcc 660

gcgggggtgg cagatgcggc gctggccgtc gggcgactcg acaaggcagt gccggccatg 720

gctggccacc accagtccgt caaacagggc gctgcgctca gccatgatcc cagcgcttgg 780

acgaaaacag cgcttcgggg tcggcactgt atgccatcac cttgaaccag cccttcacac 840

cccaggcatc ggcaatacgg ccgatctcca ccgcgtcggg cggcaattcg gcaggctcaa 900

gaacggggag gttaggcat 919

<210> 25
<211> 758
<212> DNA
<213> artificial

<220>
<223> Sequence of the first arm of the rimM/rbfA/ftsZ dsRNA used to
concomitantly target rimM, rbfA,ftsZ genes from acidovorax
valerianella

<400> 25
atgcctaacc tccccgttct tgagcctgcc gaattgccgc ccgacgcggt ggagatcggc 60

cgtattgccg atgcctgggg tgtgaagggc tggttcaagg tgatggcata cagtgccgac 120

cccgaagcgc tgttttcgtc caagcgctgg tatctgcagc ccagcgagaa gggcgccaag 180

agctttttcg aaggcaccgt ggtgctggcg gtgcgacagg cgcgcgagca ctccgacacg 240

gtcgtggcct gatcatggct gagcgcagcg ccctgtttga cggactggtg gtggccagcc 300

atggccggca ctgccttgtc gagtcgcccg acggccagcg ccgcatctgc cacccccgcg 360

gaaagaaaag ccaggcggtg gtgggagacc atgttctttg gcaggcggcc ccgcccggcc 420

agggtgatga gggcaccatc gagaaggtca aggaacgccg gaacctgttt taccggcaag 480

acgaaattcg caccaaatcc tttgatgcat gaccatcgaa atgatcgaag ccgaagaatt 540

caatcagggc acccagatca aggtgatcgg tgtcggcggc ggcggcggca atgccgtggc 600

ccacatgatc gcgcgcaatg ttcagggcgt ggagttcgtc tgcgccaata ccgacgcgca 660

agccctcacc cgcagccccg cgcaccgcac catccagctg ggcggcagcg gcctgggcgc 720

cggcagcaag cccgacaagg gccgcgacgc cgctgaag 758

<210> 26
<211> 758
<212> DNA
<213> artificial

<220>
<223> Sequence of the second arm of the rimM/rbfA/ftsZ dsRNA used to
 concomitantly target rimM, rbfA,ftsZ genes from acidovorax
 valerianella

<400> 26
cttcagcggc gtcgcggccc ttgtcgggct tgctgccggc gcccaggccg ctgccgccca 60

gctggatggt gcggtgcgcg gggctgcggg tgagggcttg cgcgtcggta ttggcgcaga 120

cgaactccac gccctgaaca ttgcgcgcga tcatgtgggc cacggcattg ccgccgccgc 180

cgccgacacc gatcaccttg atctgggtgc cctgattgaa ttcttcggct tcgatcattt 240

cgatggtcat gcatcaaagg atttggtgcg aatttcgtct tgccggtaaa acaggttccg 300

gcgttccttg accttctcga tggtgccctc atcaccctgg ccgggcgggg ccgcctgcca 360

aagaacatgg tctcccacca ccgcctggct tttctttccg cgggggtggc agatgcggcg 420

ctggccgtcg ggcgactcga caaggcagtg ccggccatgg ctggccacca ccagtccgtc 480

aaacagggcg ctgcgctcag ccatgatcag gccacgaccg tgtcggagtg ctcgcgcgcc 540

tgtcgcaccg ccagcaccac ggtgccttcg aaaaagctct tggcgccctt ctcgctgggc 600

tgcagatacc agcgcttgga cgaaaacagc gcttcggggt cggcactgta tgccatcacc 660

ttgaaccagc ccttcacacc ccaggcatcg gcaatacggc cgatctccac cgcgtcgggc 720

ggcaattcgg caggctcaag aacggggagg ttaggcat 758

<210> 27
<211> 758
<212> DNA
<213> artificial

<220>
<223> Sequence of the first arm of the RsgA/gyrB/MreB dsRNA used to
 concomitantly target RsgA, gyrB, MreB genes from acidovorax
 valerianella

<400> 27
atggctgagc gcagcgccct gtttgacgga ctggtggtgg ccagccatgg ccggcactgc 60

cttgtcgagt cgcccgacgg ccagcgccgc atctgccacc cccgcggaaa gaaaagccag 120

gcggtggtgg gagaccatgt tctttggcag gcggccccgc ccggccaggg tgatgagggc 180

accatcgaga aggtcaagga acgccggaac ctgttttacc ggcaagacga aattcgcacc 240

aaatcctttg gatcatgacc gctgagaaca ccctgcccga gccttccacc gacggcgcgg 300

gcgaagcaca gcccttcaag atcgacaccc agcaagtggg cgccagcgaa ggctacggcg 360

aaggcgcgat ccagatcctg gaaggcctgg aggccgtgcg caagcgcccc ggcatgtaca 420

tcggcgacac ctccgacggc acgggcctgc accacctcgt cttcgaagtc gtggacaact 480

ccatcgacga ggcgctggcc gggcatgcat gttcggagct ttccgtcggt acttctccac 540

cgaccttgcc attgaccttg gcacagccaa caccctgatt tttgcccgcg acaagggcat 600

cgtgctcgat gaaccctcgg tcgttgccat ccgccacgag ggcggtcccc acggaaagaa 660

agtcatccag gccgtcggcc gtgaagccaa ggccatgctg ggcaaggtgc ccggcaacat 720

cgaagccatc cgcccgatga aagacggggt gatcgcgg 758

<210> 28
<211> 758
<212> DNA
<213> artificial

<220>
<223> Sequence of the second arm of the RsgA/gyrB/MreB dsRNA used to
      concomitantly target RsgA, gyrB, MreB genes from acidovorax
      valerianella

<400> 28
ccgcgatcac cccgtctttc atcgggcgga tggcttcgat gttgccgggc accttgccca 60

gcatggcctt ggcttcacgg ccgacggcct ggatgacttt ctttccgtgg ggaccgccct 120

cgtggcggat ggcaacgacc gagggttcat cgagcacgat gcccttgtcg cgggcaaaaa 180

tcagggtgtt ggctgtgcca aggtcaatgg caaggtcggt ggagaagtac cgacggaaag 240

ctccgaacat gcatgcccgg ccagcgcctc gtcgatggag ttgtccacga cttcgaagac 300

gaggtggtgc aggcccgtgc cgtcggaggt gtcgccgatg tacatgccgg ggcgcttgcg 360

cacggcctcc aggccttcca ggatctggat cgcgccttcg ccgtagcctt cgctggcgcc 420

cacttgctgg gtgtcgatct tgaagggctg tgcttcgccc gcgccgtcgg tggaaggctc 480

gggcagggtg ttctcagcgg tcatgatcca aaggatttgg tgcgaatttc gtcttgccgg 540

taaaacaggt tccggcgttc cttgaccttc tcgatggtgc cctcatcacc ctggccgggc 600

ggggccgcct gccaaagaac atggtctccc accaccgcct ggcttttctt tccgcggggg 660

tggcagatgc ggcgctggcc gtcgggcgac tcgacaaggc agtgccggcc atggctggcc 720

accaccagtc cgtcaaacag ggcgctgcgc tcagccat 758

<210> 29
<211> 758
<212> DNA
<213> artificial

<220>
<223>  Sequence of the first arm of the RsgA/rbfA/FtsZ dsRNA used to
      concomitantly target RsgA, rbfA, FtsZ genes from acidovorax
      valerianella

<400>  29
atggctgagc gcagcgccct gtttgacgga ctggtggtgg ccagccatgg ccggcactgc      60

cttgtcgagt cgcccgacgg ccagcgccgc atctgccacc cccgcggaaa gaaaagccag     120

gcggtggtgg gagaccatgt tctttggcag gcggccccgc ccggccaggg tgatgagggc     180

accatcgaga aggtcaagga acgccggaac ctgttttacc ggcaagacga aattcgcacc     240

aaatcctttg gatcatggct gagcgcagcg ccctgtttga cggactggtg gtggccagcc     300

atggccggca ctgccttgtc gagtcgcccg acggccagcg ccgcatctgc cacccccgcg     360

gaaagaaaag ccaggcggtg gtgggagacc atgttctttg gcaggcggcc ccgcccggcc     420

agggtgatga gggcaccatc gagaaggtca aggaacgccg gaacctgttt taccggcaag     480

acgaaattcg caccaaatcc tttgatgcat gaccatcgaa atgatcgaag ccgaagaatt     540

caatcagggc acccagatca aggtgatcgg tgtcggcggc ggcggcggca atgccgtggc     600

ccacatgatc gcgcgcaatg ttcagggcgt ggagttcgtc tgcgccaata ccgacgcgca     660

agccctcacc cgcagccccg cgcaccgcac catccagctg ggcggcagcg gcctgggcgc     720

cggcagcaag cccgacaagg gccgcgacgc cgctgaag                             758


<210>  30
<211>  758
<212>  DNA
<213>  artificial

<220>
<223>  Sequence of the second arm of the RsgA/rbfA/FtsZ dsRNA used to
      concomitantly target RsgA, rbfA, FtsZ genes from acidovorax
      valerianella

<400>  30
cttcagcggc gtcgcggccc ttgtcgggct tgctgccggc gcccaggccg ctgccgccca      60

gctggatggt gcggtgcgcg gggctgcggg tgagggcttg cgcgtcggta ttggcgcaga     120

cgaactccac gccctgaaca ttgcgcgcga tcatgtgggc cacggcattg ccgccgccgc     180

cgccgacacc gatcaccttg atctgggtgc cctgattgaa ttcttcggct tcgatcattt     240

cgatggtcat gcatcaaagg atttggtgcg aatttcgtct tgccggtaaa acaggttccg     300

gcgttccttg accttctcga tggtgccctc atcaccctgg ccgggcgggg ccgcctgcca     360

aagaacatgg tctcccacca ccgcctggct tttctttccg cgggggtggc agatgcggcg     420

ctggccgtcg ggcgactcga caaggcagtg ccggccatgg ctggccacca ccagtccgtc     480

aaacagggcg ctgcgctcag ccatgatcca aaggatttgg tgcgaatttc gtcttgccgg     540

taaaacaggt tccggcgttc cttgaccttc tcgatggtgc cctcatcacc ctggccgggc     600

ggggccgcct gccaaagaac atggtctccc accaccgcct ggcttttctt tccgcggggg 660

tggcagatgc ggcgctggcc gtcgggcgac tcgacaaggc agtgccggcc atggctggcc 720

accaccagtc cgtcaaacag ggcgctgcgc tcagccat 758

<210> 31
<211> 920
<212> DNA
<213> artificial

<220>
<223> Sequence of the first arm of the mreB/ybeY/rbfA/gyrB/ftsZ/rsgA
dsRNA used to concomitantly target mreB,ybeY,rbfA,gyrB,ftsZ,rsgA
genes from acidovorax citrulli

<400> 31
atgttcggag ctttccgtcg gtacttctcc accgaccttg ccattgatct tggcacagcc 60

aacacgctga tcttcgcccg cgacaagggc atcgtgctcg acgaaccctc cgtggtcgcc 120

atccgccacg aaggcggccc ccacggcaag gatcatggcc ctgaaccagc tttccctgtc 180

cctgcagttc gcgcggtttc ccggcgtgga ggcgcaccgc gccgccctgc cccggcatgc 240

cgtcgtccgc tggatccgcc acgcgctctc gctggacgcg gagatcaccg tgcgcatcgt 300

cgatatgcat ggctgctaaa aaatcctcca cgcccaaccg cggcttcaag gtcgcggacc 360

agatccagcg cgatctgacc gagctgatcc gcgagttgaa ggacccgcgc atcggcatgg 420

tcacgctgca ggccgtggaa gtgacgcccg actacgcggt gcatgaccgc tgagaacacc 480

ctgcccgagc ccacccttcc cacgggcgac agccaagcgc agtccgtcgc agcccccgac 540

tccgtgcccg cccaggccgc cgcgcccagc gacggctacg gcgaaggcgc gatccagatc 600

ctggaaggcc tgaagcatga ccatcgaaat gatcgaagcc gaagaattca accagggcac 660

gcagatcaag gtgatcggtg tcggcggcgg cggcggcaat gccgtcgagc acatgatctc 720

gcgccaggta cagggcgtgg aattcgtatg cgccaatacc gatgcgtcca atgtctgagc 780

gctccggccc cctcgaaggc ctggtggtgg ccagccacgg ccggcactgc ctcgtcgaga 840

gccccgacgg cgcgcggcgc atctgccatc cgcgcggcaa gaagagccag gcggtggtcg 900

gcgaccgcgt gctctggcag 920

<210> 32
<211> 920
<212> DNA
<213> artificial

<220>
<223> Sequence of the second arm of the mreB/ybeY/rbfA/gyrB/ftsZ/rsgA
dsRNA used to concomitantly target mreB,ybeY,rbfA,gyrB,ftsZ,rsgA
genes from acidovorax citrulli

<400> 32

ctgccagagc acgcggtcgc cgaccaccgc ctggctcttc ttgccgcgcg gatggcagat 60

gcgccgcgcg ccgtcggggc tctcgacgag gcagtgccgg ccgtggctgg ccaccaccag 120

gccttcgagg gggccggagc gctcagacat tggacgcatc ggtattggcg catacgaatt 180

ccacgccctg tacctggcgc gagatcatgt gctcgacggc attgccgccg ccgccgccga 240

caccgatcac cttgatctgc gtgccctggt tgaattcttc ggcttcgatc atttcgatgg 300

tcatgcttca ggccttccag gatctggatc gcgccttcgc cgtagccgtc gctgggcgcg 360

gcggcctggg cgggcacgga gtcgggggct gcgacggact gcgcttggct gtcgcccgtg 420

ggaagggtgg gctcgggcag ggtgttctca gcggtcatgc accgcgtagt cgggcgtcac 480

ttccacggcc tgcagcgtga ccatgccgat gcgcgggtcc ttcaactcgc ggatcagctc 540

ggtcagatcg cgctggatct ggtccgcgac cttgaagccg cggttgggcg tggaggattt 600

tttagcagcc atgcatatcg acgatgcgca cggtgatctc cgcgtccagc gagagcgcgt 660

ggcggatcca gcggacgacg gcatgccggg gcagggcggc gcggtgcgcc tccacgccgg 720

gaaaccgcgc gaactgcagg gacagggaaa gctggttcag ggccatgatc cttgccgtgg 780

gggccgcctt cgtggcggat ggcgaccacg gagggttcgt cgagcacgat gcccttgtcg 840

cgggcgaaga tcagcgtgtt ggctgtgcca agatcaatgg caaggtcggt ggagaagtac 900

cgacggaaag ctccgaacat 920

<210> 33
<211> 758
<212> DNA
<213> artificial

<220>
<223> Sequence of the first arm of the mreB/ybeY/rbfA dsRNA used to
      concomitantly target mreB,ybeY,rbfA genes from acidovorax
      citrulli

<400> 33
atgttcggag ctttccgtcg gtacttctcc accgaccttg ccattgatct tggcacagcc 60

aacacgctga tcttcgcccg cgacaagggc atcgtgctcg acgaaccctc cgtggtcgcc 120

atccgccacg aaggcggccc ccacggcaag aaagtcatcc aggccgtcgg ccacgaggcc 180

aaggccatgc tcggcaaggt gcccggcaac atcgaggcca tccgcccgat gaaggatggc 240

gtgatcgccg gatcatggcc ctgaaccagc tttccctgtc cctgcagttc gcgcggtttc 300

ccggcgtgga ggcgcaccgc gccgccctgc cccggcatgc cgtcgtccgc tggatccgcc 360

acgcgctctc gctggacgcg gagatcaccg tgcgcatcgt cgatgccgaa gagggccagg 420

ccctgaaccg cgagtaccgg cacaaggact acgccaccaa cgtgctcacc ttcgactacg 480

cgcaggagcc cgtggtcatg gccgatgcat ggctgctaaa aaatcctcca cgcccaaccg 540

cggcttcaag gtcgcggacc agatccagcg cgatctgacc gagctgatcc gcgagttgaa 600

ggacccgcgc atcggcatgg tcacgctgca ggccgtggaa gtgacgcccg actacgcgca 660

tgcgaaggtg ttcttcagcg tgctcgtggg cgacgccgac gcgacgcagg atgcgctgaa 720

ccaggccgcg ggcttcctgc gcaacggcct gttcaagc 758

<210> 34
<211> 758
<212> DNA
<213> artificial

<220>
<223> Sequence of the second arm of the mreB/ybeY/rbfA dsRNA used to
 concomitantly target mreB,ybeY,rbfA genes from acidovorax
 citrulli

<400> 34
gcttgaacag gccgttgcgc aggaagcccg cggcctggtt cagcgcatcc tgcgtcgcgt 60

cggcgtcgcc cacgagcacg ctgaagaaca ccttcgcatg cgcgtagtcg ggcgtcactt 120

ccacggcctg cagcgtgacc atgccgatgc gcgggtcctt caactcgcgg atcagctcgg 180

tcagatcgcg ctggatctgg tccgcgacct tgaagccgcg gttgggcgtg gaggattttt 240

tagcagccat gcatcggcca tgaccacggg ctcctgcgcg tagtcgaagg tgagcacgtt 300

ggtggcgtag tccttgtgcc ggtactcgcg gttcagggcc tggccctctt cggcatcgac 360

gatgcgcacg gtgatctccg cgtccagcga gagcgcgtgg cggatccagc ggacgacggc 420

atgccggggc agggcggcgc ggtgcgcctc cacgccggga aaccgcgcga actgcaggga 480

cagggaaagc tggttcaggg ccatgatccg gcgatcacgc catccttcat cgggcggatg 540

gcctcgatgt tgccgggcac cttgccgagc atggccttgg cctcgtggcc gacggcctgg 600

atgactttct tgccgtgggg gccgccttcg tggcggatgg cgaccacgga gggttcgtcg 660

agcacgatgc ccttgtcgcg ggcgaagatc agcgtgttgg ctgtgccaag atcaatggca 720

aggtcggtgg agaagtaccg acggaaagct ccgaacat 758

<210> 35
<211> 758
<212> DNA
<213> artificial

<220>
<223> Sequence of the first arm of the rsgA/GyrB/MreB dsRNA used to
 concomitantly target RsgA, GyrB, MreB genes from acidovorax
 citrulli

<400> 35
atgtctgagc gctccggccc cctcgaaggc ctggtggtgg ccagccacgg ccggcactgc 60

ctcgtcgaga gccccgacgg cgcgcggcgc atctgccatc cgcgcggcaa gaagagccag 120

gcggtggtcg gcgaccgcgt gctctggcag gccccgccgc ccggccaggg cgacgaaggc 180

acgatcgaga aagtgctgga gcgccgcaac ctgttctacc ggcaggacga gatccgcacc 240

aaatcgttcg gatcatgacc gctgagaaca ccctgcccga gcccaccctt cccacgggcg 300

acagccaagc gcagtccgtc gcagcccccg actccgtgcc cgcccaggcc gccgcgccca 360

gcgacggcta cggcgaaggc gcgatccaga tcctggaagg cctggaggcc gtgcgcaagc 420

gccccggcat gtacatcggc gacacgtcgg acggcacggg cctgcaccac ctcgtgttcg 480

aagtggtgga caactccatc gacgatgcat gttcggagct ttccgtcggt acttctccac 540

cgaccttgcc attgatcttg gcacagccaa cacgctgatc ttcgcccgcg acaagggcat 600

cgtgctcgac gaaccctccg tggtcgccat ccgccacgaa ggcggccccc acggcaagaa 660

agtcatccag gccgtcggcc acgaggccaa ggccatgctc ggcaaggtgc ccggcaacat 720

cgaggccatc cgcccgatga aggatggcgt gatcgccg 758

<210> 36
<211> 758
<212> DNA
<213> artificial

<220>
<223> Sequence of the second arm of the rsgA/GyrB/MreB dsRNA used to
concomitantly target RsgA, GyrB, MreB genes from acidovorax
citrulli

<400> 36
cggcgatcac gccatccttc atcgggcgga tggcctcgat gttgccgggc accttgccga 60

gcatggcctt ggcctcgtgg ccgacggcct ggatgacttt cttgccgtgg gggccgcctt 120

cgtggcggat ggcgaccacg gagggttcgt cgagcacgat gcccttgtcg cgggcgaaga 180

tcagcgtgtt ggctgtgcca agatcaatgg caaggtcggt ggagaagtac cgacggaaag 240

ctccgaacat gcatcgtcga tggagttgtc caccacttcg aacacgaggt ggtgcaggcc 300

cgtgccgtcc gacgtgtcgc cgatgtacat gccggggcgc ttgcgcacgg cctccaggcc 360

ttccaggatc tggatcgcgc cttcgccgta gccgtcgctg ggcgcggcgg cctgggcggg 420

cacggagtcg ggggctgcga cggactgcgc ttggctgtcg cccgtgggaa gggtgggctc 480

gggcagggtg ttctcagcgg tcatgatccg aacgatttgg tgcggatctc gtcctgccgg 540

tagaacaggt tgcggcgctc cagcactttc tcgatcgtgc cttcgtcgcc ctggccgggc 600

ggcggggcct gccagagcac gcggtcgccg accaccgcct ggctcttctt gccgcgcgga 660

tggcagatgc gccgcgcgcc gtcggggctc tcgacgaggc agtgccggcc gtggctggcc 720

accaccaggc cttcgagggg gccggagcgc tcagacat 758

<210> 37
<211> 758
<212> DNA
<213> artificial

<220>
<223>   Sequence of the first arm of the YbeY/ RsgA/ MreB dsRNA used to
       concomitantly target YbeY, RsgA, MreB genes from acidovorax
       citrulli

<400>  37
atggccctga accagctttc cctgtccctg cagttcgcgc ggtttcccgg cgtggaggcg      60

caccgcgccg ccctgccccg gcatgccgtc gtccgctgga tccgccacgc gctctcgctg     120

gacgcggaga tcaccgtgcg catcgtcgat gccgaagagg gccaggccct gaaccgcgag     180

taccggcaca aggactacgc caccaacgtg ctcaccttcg actacgcgca ggagcccgtg     240

gtcatggccg gatcatgtct gagcgctccg gccccctcga aggcctggtg gtggccagcc     300

acggccggca ctgcctcgtc gagagccccg acggcgcgcg gcgcatctgc catccgcgcg     360

gcaagaagag ccaggcggtg gtcggcgacc gcgtgctctg gcaggccccg ccgcccggcc     420

agggcgacga aggcacgatc gagaaagtgc tggagcgccg caacctgttc taccggcagg     480

acgagatccg caccaaatcg ttcgatgcat gttcggagct ttccgtcggt acttctccac     540

cgaccttgcc attgatcttg gcacagccaa cacgctgatc ttcgcccgcg acaagggcat     600

cgtgctcgac gaaccctccg tggtcgccat ccgccacgaa ggcggccccc acggcaagaa     660

agtcatccag gccgtcggcc acgaggccaa ggccatgctc ggcaaggtgc ccggcaacat     720

cgaggccatc cgcccgatga aggatggcgt gatcgccg                             758

<210>  38
<211>  758
<212>  DNA
<213>  artificial

<220>
<223>   Sequence of the second arm of the YbeY/ RsgA/ MreB dsRNA used to
       concomitantly target YbeY, RsgA, MreB genes from acidovorax
       citrulli

<400>  38
cggcgatcac gccatccttc atcgggcgga tggcctcgat gttgccgggc accttgccga      60

gcatggcctt ggcctcgtgg ccgacggcct ggatgacttt cttgccgtgg gggccgcctt     120

cgtggcggat ggcgaccacg gagggttcgt cgagcacgat gcccttgtcg cgggcgaaga     180

tcagcgtgtt ggctgtgcca agatcaatgg caaggtcggt ggagaagtac cgacggaaag     240

ctccgaacat gcatcgaacg atttggtgcg gatctcgtcc tgccggtaga acaggttgcg     300

gcgctccagc actttctcga tcgtgccttc gtcgccctgg ccgggcggcg gggcctgcca     360

gagcacgcgg tcgccgacca ccgcctggct cttcttgccg cgcggatggc agatgcgccg     420

cgcgccgtcg gggctctcga cgaggcagtg ccggccgtgg ctggccacca ccaggccttc     480

gagggggccg gagcgctcag acatgatccg gccatgacca cgggctcctg cgcgtagtcg     540

aaggtgagca cgttggtggc gtagtccttg tgccggtact cgcggttcag ggcctggccc 600

tcttcggcat cgacgatgcg cacggtgatc tccgcgtcca gcgagagcgc gtggcggatc 660

cagcggacga cggcatgccg gggcagggcg gcgcggtgcg cctccacgcc gggaaaccgc 720

gcgaactgca gggacaggga aagctggttc agggccat 758

<210> 39
<211> 758
<212> DNA
<213> artificial

<220>
<223> Sequence of the first arm of the P1/ HC-Pro/ cp dsRNA used to
      concomitantly target P1, HC-Pro, coat protein genes from PIPVgp1
      sequene from plume pox virus

<400> 39
tcagacccag gcaaacaatt ttcggatggt ttcaccaatt catttatgca gtgcaagcta 60

cgcgaaactg atcatcagtg cacatctgac ctggacgtga aggagtgtgg ttatgtcgca 120

gcacttgtgt gccaagcgat aatcccttgc ggaaaaatca cgtgtctgca atgtgctcaa 180

aagtattctt acatgtcaca acaggaaata cgtgatagat tttcgacagt aattgagcag 240

catgagaaaa gatccacttt aaatgactta ggattctcag tactcagaca ggcaacaccc 300

cacttggtag aaaaaagtta tctccaggaa ttggagcaag cttggaaaga gttaagctgg 360

tcggaaaaat tctctgcaat cttggaatcg cagcgatggc gaaaacatat accaaaacct 420

ttcatcccaa aagacggcgc agatttagga ggcaggtacg acatctccgt tcggtcatta 480

cttggcaacc agtacaaacg cctgatgctc agacccaggc aaacaatttt cggatggttt 540

caccaattca tttatgcagt gcaagctacg cgaaactgat catcagtgca catctgacct 600

ggacgtgaag gagtgtggtt atgtcgcagc acttgtgtgc caagcgataa tcccttgcgg 660

aaaaatcacg tgtctgcaat gtgctcaaaa gtattcttac atgtcacaac aggaaatacg 720

tgatagattt tcgacagtaa ttgagcagca tgagaaaa 758

<210> 40
<211> 758
<212> DNA
<213> artificial

<220>
<223> Sequence of the second arm of the P1/ HC-Pro/ cp dsRNA used to
      concomitantly target P1, HC-Pro, coat protein genes from PIPVgp1
      sequene from plume pox virus

<400> 40
ttttctcatg ctgctcaatt actgtcgaaa atctatcacg tatttcctgt tgtgacatgt 60

aagaatactt ttgagcacat tgcagacacg tgatttttcc gcaagggatt atcgcttggc 120

acacaagtgc tgcgacataa ccacactcct tcacgtccag gtcagatgtg cactgatgat 180

cagtttcgcg tagcttgcac tgcataaatg aattggtgaa accatccgaa aattgtttgc 240

ctgggtctga gcatcaggcg tttgtactgg ttgccaagta atgaccgaac ggagatgtcg 300

tacctgcctc ctaaatctgc gccgtctttt gggatgaaag gttttggtat atgttttcgc 360

catcgctgcg attccaagat tgcagagaat ttttccgacc agcttaactc tttccaagct 420

tgctccaatt cctggagata acttttttct accaagtggg gtgttgcctg tctgagtact 480

gagaatccta agtcatttaa agtggatctt ttctcatgct gctcaattac tgtcgaaaat 540

ctatcacgta tttcctgttg tgacatgtaa gaatactttt gagcacattg cagacacgtg 600

atttttccgc aagggattat cgcttggcac acaagtgctg cgacataacc acactccttc 660

acgtccaggt cagatgtgca ctgatgatca gtttcgcgta gcttgcactg cataaatgaa 720

ttggtgaaac catccgaaaa ttgtttgcct gggtctga 758

<210> 41
<211> 750
<212> DNA
<213> artificial

<220>
<223> Sequence of the first arm of the P1 dsRNA used to concomitantly
      target P1 gene from PIPVgp1 sequene from plume pox virus

<400> 41
tcagacccag gcaaacaatt ttcggatggt ttcaccaatt catttatgca gtgcaagcta 60

cgcgaaactg atcatcagtg cacatctgac ctggacgtga aggagtgtgg ttatgtcgca 120

gcacttgtgt gccaagcgat aatcccttgc ggaaaaatca cgtgtctgca atgtgctcaa 180

aagtattctt acatgtcaca acaggaaata cgtgatagat tttcgacagt aattgagcag 240

catgagaaaa cagtgatgga taactatcca caattttcac atgttctcgc ttttctaaag 300

agatatcgtg aactaatgcg cgtggaaaat cagaattatg aagctttcaa ggatatcacg 360

cacatgatag gcgagcgtaa agaagcacct ttttcccatc tcaacaaaat caatgaatta 420

atcattaagg gtggtatgat gagcgcacaa gactatatag aagcctcgga tcatctacgc 480

gaactagcgc gatatcagaa gaatcgcacg gagaacatta ggagcggatc tataaaggct 540

ttcaggaata aaatctcatc aaaagcacat gtcaatatgc agcttatgtg tgacaatcaa 600

cttgatacta atggcaattt cgtgtgggga cagagagagt atcatgctaa acgcttcttt 660

aggaattact tcgatgtgat cgatgttagc gagggctaca gacgtcatat tgttcgtgaa 720

aatcctagag gtatccgcaa attggccatt 750

<210> 42
<211> 750
<212> DNA
<213> artificial

<220>
<223>  Sequence of the second arm of the P1 dsRNA used to concomitantly
       target P1 gene from PIPVgp1 sequene from plume pox virus

<400>  42
aatggccaat ttgcggatac ctctaggatt ttcacgaaca atatgacgtc tgtagccctc      60

gctaacatcg atcacatcga agtaattcct aaagaagcgt ttagcatgat actctctctg     120

tccccacacg aaattgccat tagtatcaag ttgattgtca cacataagct gcatattgac     180

atgtgctttt gatgagattt tattcctgaa agcctttata gatccgctcc taatgttctc     240

cgtgcgattc ttctgatatc gcgctagttc gcgtagatga tccgaggctt ctatatagtc     300

ttgtgcgctc atcataccac ccttaatgat taattcattg attttgttga gatgggaaaa     360

aggtgcttct ttacgctcgc ctatcatgtg cgtgatatcc ttgaaagctt cataattctg     420

attttccacg cgcattagtt cacgatatct ctttagaaaa gcgagaacat gtgaaaattg     480

tggatagtta tccatcactg ttttctcatg ctgctcaatt actgtcgaaa atctatcacg     540

tatttcctgt tgtgacatgt aagaatactt ttgagcacat tgcagacacg tgatttttcc     600

gcaagggatt atcgcttggc acacaagtgc tgcgacataa ccacactcct tcacgtccag     660

gtcagatgtg cactgatgat cagtttcgcg tagcttgcac tgcataaatg aattggtgaa     720

accatccgaa aattgtttgc ctgggtctga                                      750


<210>  43
<211>  750
<212>  DNA
<213>  artificial

<220>
<223>  Sequence of the first arm of the HC-Pro dsRNA used to
       concomitantly target HC-Pro gene from PIPVgp1 sequene from plume
       pox virus

<400>  43
ggtcttgaag tggataagtg tgacgaattc aaaaatgtaa agctcttgat cagaagcatt      60

tacaagccac aaatcatgga acaggtgctt aaggaagaac catatttact gctcatgagc     120

gttttgtcac ctggcgtctt gatggcgctg ttcaatagtg gttcattgga gaaagccaca     180

caatattgga tcacacgatc tcatagcttg gcagcgatca catcaatgtt atcagcactt     240

gcagccaaag tttcactcgc aagtacactg aatgcacaga tgagtgtcat tgacgaacat     300

gcagcagttc tatatgatag tgtttttgtt ggaacgcagc catacgcatc ctacatgatg     360

gcagtgaaaa ctttggaaag gatgaaagca cggacggaat ccgatcacac tttaaatgac     420

ttaggattct cagtactcag acaggcaaca ccccacttgg tagaaaaaag ttatctccag     480

gaattggagc aagcttggaa agagttaagc tggtcggaaa aattctctgc aatcttggaa     540

tcgcagcgat ggcgaaaaca tataccaaaa cctttcatcc caaaagacgg cgcagattta     600

ggaggcaggt acgacatctc cgttcggtca ttacttggca accagtacaa acgcctgaga 660

gacgtagtcc gatggaaaag agacgatgtg gtttgctata cataccagtc gatggggaag 720

ctattttgca aagccatcgg aatctcacca 750

<210> 44
<211> 750
<212> DNA
<213> artificial

<220>
<223> Sequence of the second arm of the HC-Pro dsRNA used to
      concomitantly target HC-Pro gene from PIPVgp1 sequene from plume
      pox virus

<400> 44
tggtgagatt ccgatggctt tgcaaaatag cttccccatc gactggtatg tatagcaaac 60

cacatcgtct cttttccatc ggactacgtc tctcaggcgt ttgtactggt tgccaagtaa 120

tgaccgaacg gagatgtcgt acctgcctcc taaatctgcg ccgtcttttg ggatgaaagg 180

ttttggtata tgttttcgcc atcgctgcga ttccaagatt gcagagaatt tttccgacca 240

gcttaactct ttccaagctt gctccaattc ctggagataa cttttttcta ccaagtgggg 300

tgttgcctgt ctgagtactg agaatcctaa gtcatttaaa gtgtgatcgg attccgtccg 360

tgctttcatc ctttccaaag ttttcactgc catcatgtag gatgcgtatg gctgcgttcc 420

aacaaaaaca ctatcatata gaactgctgc atgttcgtca atgacactca tctgtgcatt 480

cagtgtactt gcgagtgaaa ctttggctgc aagtgctgat aacattgatg tgatcgctgc 540

caagctatga gatcgtgtga tccaatattg tgtggctttc tccaatgaac cactattgaa 600

cagcgccatc aagacgccag gtgacaaaac gctcatgagc agtaaatatg gttcttcctt 660

aagcacctgt tccatgattt gtggcttgta aatgcttctg atcaagagct ttacattttt 720

gaattcgtca cacttatcca cttcaagacc 750

<210> 45
<211> 250
<212> DNA
<213> artificial

<220>
<223> Sequence of the first arm of the CP dsRNA used to concomitantly
      target coat protein gene from PIPVgp1 sequene from plume pox
      virus

<400> 45
cactttaaat gacttaggat tctcagtact cagacaggca acaccccact tggtagaaaa 60

aagttatctc caggaattgg agcaagcttg gaaagagtta agctggtcgg aaaaattctc 120

tgcaatcttg gaatcgcagc gatggcgaaa acatatacca aaacctttca tcccaaaaga 180

cggcgcagat ttaggaggca ggtacgacat ctccgttcgg tcattacttg gcaaccagta 240

caaacgcctg 250

<210> 46
<211> 250
<212> DNA
<213> artificial

<220>
<223> Sequence of the second arm of the CP dsRNA used to concomitantly
      target coat protein gene from PIPVgp1 sequene from plume pox
      virus

<400> 46
caggcgtttg tactggttgc caagtaatga ccgaacggag atgtcgtacc tgcctcctaa 60

atctgcgccg tcttttggga tgaaaggttt tggtatatgt tttcgccatc gctgcgattc 120

caagattgca gagaattttt ccgaccagct taactctttc caagcttgct ccaattcctg 180

gagataactt ttttctacca agtggggtgt tgcctgtctg agtactgaga atcctaagtc 240

atttaaagtg 250

<210> 47
<211> 919
<212> DNA
<213> artificial

<220>
<223> Sequence of the first arm of the gyrB/ MreB/ rbfA/ RsgA/ FliA/
      QseC dsRNA used to concomitantly target GyrB, MreB, RbfA, RsgA,
      FliA, QseC genes from erwinia carotovora

<400> 47
atgtcgaatt cttatgactc ctcaagtatc aaggtattga aagggctgga tgcggtacgt 60

aaacgcccag gtatgtatat cggcgatacg gatgacggta ccggcctgca tcacatggta 120

ttcgaggttg tggacaacgc ttcgacgaag atcatgttta agaaatttcg tggcatgttt 180

tccaacgact tgtccatcga cctgggtacc gccaataccc tgatttatgt taaagggcag 240

ggcatcatac tgaatgaacc ctctgtggtt gcgattcgtc aggatcgtgc tggttcccaa 300

aaaatgcatg gcaaaagaat tcagccgcac ccagcgcgtt gcgcaggaaa tgcaaaaaga 360

aatcgccatt atcattcagc gtgaagtaaa agatccgcgt atcggcatgg cgaccgtatc 420

cggtgtggaa gtttctcgcg atctggcgta tgccaaagtg cgtgagcaaa aagaaactgt 480

cgaaaggtca gcagcgtcgg gttagcgcaa accaccagcg ccgcctgaaa aaaacggaaa 540

gcaaagtcga atgggaagat agccaactgg gcgacgcaca ggaaggcatt atcatcagcc 600

gattcggtat gaagcgtgag cgatttgtat accgccgaag gcacaatgga taaaaattcc 660

ctttggaagc gctatgttcc actagtgcgc catgaagcct tgcgtttaca ggttcgtctc 720

cccgcgagtg ttgagcttga cgatctgcta caggctggtg gtatctccaa tgaaccgact 780

cagcctgcgc ctacgcctga ttaccggttt tacgctgctg accttgatct gctggggcgt      840

agccagcctg ctttcgtggt atcaaacacg ccataatatc aatgagctat ttgataccca      900

gcaaatgctg ttcgccaaa                                                   919

<210>  48
<211>  919
<212>  DNA
<213>  artificial

<220>
<223>  Sequence of the second arm of the gyrB/ MreB/ rbfA/ RsgA/ FliA/
       QseC dsRNA used to concomitantly target GyrB, MreB, RbfA, RsgA,
       FliA, QseC genes from erwinia carotovora

<400>  48
tttggcgaac agcatttgct gggtatcaaa tagctcattg atattatggc gtgtttgata       60

ccacgaaagc aggctggcta cgccccagca gatcaaggtc agcagcgtaa aaccggtaat      120

caggcgtagg cgcaggctga gtcggttcat tggagatacc accagcctgt agcagatcgt      180

caagctcaac actcgcgggg agacgaacct gtaaacgcaa ggcttcatgg cgcactagtg      240

gaacatagcg cttccaaagg gaatttttat ccattgtgcc ttcggcggta tacaaatcgc      300

tcacgcttca taccgaatcg gctgatgata atgccttcct gtgcgtcgcc cagttggcta      360

tcttcccatt cgactttgct ttccgttttt ttcaggcggc gctggtggtt tgcgctaacc      420

cgacgctgct gacctttcga cagtttcttt ttgctcacgc actttggcat acgccagatc      480

gcgagaaact tccacaccgg atacggtcgc catgccgata cgcggatctt ttacttcacg      540

ctgaatgata atggcgattt ctttttgcat ttcctgcgca acgcgctggg tgcggctgaa      600

ttcttttgcc atgcattttt tgggaaccag cacgatcctg acgaatcgca accacagagg      660

gttcattcag tatgatgccc tgccctttaa cataaatcag ggtattggcg gtacccaggt      720

cgatggacaa gtcgttggaa aacatgccac gaaatttctt aaacatgatc ttcgtcgaag      780

cgttgtccac aacctcgaat accatgtgat gcaggccggt accgtcatcc gtatcgccga      840

tatacatacc tgggcgttta cgtaccgcat ccagcccttt caataccttg atacttgagg      900

agtcataaga attcgacat                                                   919

<210>  49
<211>  758
<212>  DNA
<213>  artificial

<220>
<223>  Sequence of the first arm of the gyrB/ rbfA/ QseC dsRNA used to
       concomitantly target GyrB, RbfA, QseC genes from erwinia
       carotovora

<400>  49

atgtcgaatt cttatgactc ctcaagtatc aaggtattga aagggctgga tgcggtacgt 60

aaacgcccag gtatgtatat cggcgatacg gatgacggta ccggcctgca tcacatggta 120

ttcgaggttg tggacaacgc tatcgacgaa gcgctcgctg gctattgtaa agacattatc 180

atcaccatcc atgccgataa ctcggtgtcg gtgcaggatg atggccgtgg tattccgacc 240

ggcattcatg gatcatggca aaagaattca gccgcaccca gcgcgttgcg caggaaatgc 300

aaaaagaaat cgccattatc attcagcgtg aagtaaaaga tccgcgtatc ggcatggcga 360

ccgtatccgg tgtggaagtt tctcgcgatc tggcgtatgc caaagttttt gtgaccttcc 420

tgaatgataa cgagccagaa caagttaaaa cggcgctgaa agcactgcag gatgcttcag 480

gttttattcg tacactggtt ggtaatgcat gaaccgactc agcctgcgcc tacgcctgat 540

taccggtttt acgctgctga ccttgatctg ctggggcgta gccagcctgc tttcgtggta 600

tcaaacacgc cataatatca atgagctatt tgatacccag caaatgctgt tcgccaaacg 660

tctggccacc atgaatcctg atgaattgcg aatccagccg gtgtccctgc ccaaaaccaa 720

gagtctggta cataagaacc gcggcaagca ggacgatg 758

<210> 50
<211> 758
<212> DNA
<213> artificial

<220>
<223> Sequence of the second arm of the gyrB/ rbfA/ QseC dsRNA used to
      concomitantly target GyrB, RbfA, QseC genes from erwinia
      carotovora

<400> 50
catcgtcctg cttgccgcgg ttcttatgta ccagactctt ggttttgggc agggacaccg 60

gctggattcg caattcatca ggattcatgg tggccagacg tttggcgaac agcatttgct 120

gggtatcaaa tagctcattg atattatggc gtgtttgata ccacgaaagc aggctggcta 180

cgccccagca gatcaaggtc agcagcgtaa aaccggtaat caggcgtagg cgcaggctga 240

gtcggttcat gcattaccaa ccagtgtacg aataaaacct gaagcatcct gcagtgcttt 300

cagcgccgtt ttaacttgtt ctggctcgtt atcattcagg aaggtcacaa aaactttggc 360

atacgccaga tcgcgagaaa cttccacacc ggatacggtc gccatgccga tacgcggatc 420

ttttacttca cgctgaatga taatggcgat ttctttttgc atttcctgcg caacgcgctg 480

ggtgcggctg aattcttttg ccatgatcca tgaatgccgg tcggaatacc acggccatca 540

tcctgcaccg acaccgagtt atcggcatgg atggtgatga taatgtcttt acaatagcca 600

gcgagcgctt cgtcgatagc gttgtccaca acctcgaata ccatgtgatg caggccggta 660

ccgtcatccg tatcgccgat atacatacct gggcgtttac gtaccgcatc cagccctttc 720

aataccttga tacttgagga gtcataagaa ttcgacat 758

<210> 51
<211> 758
<212> DNA
<213> artificial

<220>
<223> Sequence of the first arm of the fliA/ MreB/ QseC dsRNA used to
      concomitantly target FliA, reB, QseC genes from erwinia
      carotovora

<400> 51
gtgagcgatt tgtataccgc cgaaggcaca atggataaaa attccctttg gaagcgctat      60

gttccactag tgcgccatga agccttgcgt ttacaggttc gtctccccgc gagtgttgag     120

cttgacgatc tgctacaggc tggtggtatc ggtttgctca gtgcagtcga gcgttatgat     180

tcgttgcaag gtacggcgtt tactacctat gccgttcaac gaatccgcgg ttcgatgctg     240

gatgaattac gatcatgttt aagaaatttc gtggcatgtt ttccaacgac ttgtccatcg     300

acctgggtac cgccaatacc ctgatttatg ttaaagggca gggcatcata ctgaatgaac     360

cctctgtggt tgcgattcgt caggatcgtg ctggttccca aaaaagcgtg gcagcagtag     420

gccacgacgc taagcagatg ctgggcagaa cgcctgggaa tatcgtggcg attcgcccga     480

tgaaagacgg cgttattgcc gactatgcat gaaccgactc agcctgcgcc tacgcctgat     540

taccggtttt acgctgctga ccttgatctg ctggggcgta gccagcctgc tttcgtggta     600

tcaaacacgc cataatatca atgagctatt tgatacccag caaatgctgt tcgccaaacg     660

tctggccacc atgaatcctg atgaattgcg aatccagccg gtgtccctgc ccaaaaccaa     720

gagtctggta cataagaacc gcggcaagca ggacgatg                             758


<210> 52
<211> 758
<212> DNA
<213> artificial

<220>
<223> Sequence of the second arm of the fliA/ MreB/ QseC dsRNA used to
      concomitantly target FliA, MreB, QseC genes from erwinia
      carotovora

<400> 52
catcgtcctg cttgccgcgg ttcttatgta ccagactctt ggttttgggc agggacaccg      60

gctggattcg caattcatca ggattcatgg tggccagacg tttggcgaac agcatttgct     120

gggtatcaaa tagctcattg atattatggc gtgtttgata ccacgaaagc aggctggcta     180

cgccccagca gatcaaggtc agcagcgtaa aaccggtaat caggcgtagg cgcaggctga     240

gtcggttcat gcatagtcgg caataacgcc gtctttcatc gggcgaatcg ccacgatatt     300

cccaggcgtt ctgcccagca tctgcttagc gtcgtggcct actgctgcca cgcttttttg     360

ggaaccagca cgatcctgac gaatcgcaac cacagagggt tcattcagta tgatgccctg 420

ccctttaaca taaatcaggg tattggcggt acccaggtcg atggacaagt cgttggaaaa 480

catgccacga aatttcttaa acatgatcgt aattcatcca gcatcgaacc gcggattcgt 540

tgaacggcat aggtagtaaa cgccgtacct tgcaacgaat cataacgctc gactgcactg 600

agcaaaccga taccaccagc ctgtagcaga tcgtcaagct caacactcgc ggggagacga 660

acctgtaaac gcaaggcttc atggcgcact agtggaacat agcgcttcca aagggaattt 720

ttatccattg tgccttcggc ggtatacaaa tcgctcac 758

<210> 53
<211> 758
<212> DNA
<213> artificial

<220>
<223> Sequence of the first arm of the GyrB/ MreB/ RsgA dsRNA used to
      concomitantly target GyrB, MreB, RsgA genes from erwinia
      carotovora

<400> 53
atgtcgaatt cttatgactc ctcaagtatc aaggtattga aagggctgga tgcggtacgt 60

aaacgcccag gtatgtatat cggcgatacg gatgacggta ccggcctgca tcacatggta 120

ttcgaggttg tggacaacgc tatcgacgaa gcgctcgctg gctattgtaa agacattatc 180

atcaccatcc atgccgataa ctcggtgtcg gtgcaggatg atggccgtgg tattccgacc 240

ggcattcatg gatcatgttt aagaaatttc gtggcatgtt ttccaacgac ttgtccatcg 300

acctgggtac cgccaatacc ctgatttatg ttaaagggca gggcatcata ctgaatgaac 360

cctctgtggt tgcgattcgt caggatcgtg ctggttccca aaaaagcgtg gcagcagtag 420

gccacgacgc taagcagatg ctgggcagaa cgcctgggaa tatcgtggcg attcgcccga 480

tgaaagacgg cgttattgcc gactatgcgt gagcaaaaag aaactgtcga aaggtcagca 540

gcgtcgggtt agcgcaaacc accagcgccg cctgaaaaaa acggaaagca aagtcgaatg 600

ggaagatagc caactgggcg acgcacagga aggcattatc atcagccgat tcggtatgca 660

cgccgatgtg gaagccaccg atggcgtggt gcaccgctgc aacattcgtc ggaccctttc 720

atcgctggtt actggcgatc gcgtcgtctg gcgcccag 758

<210> 54
<211> 758
<212> DNA
<213> artificial

<220>
<223> Sequence of the second arm of the GyrB/ MreB/ RsgA dsRNA used to
      concomitantly target GyrB, MreB, RsgA genes from erwinia
      carotovora

<400>  54
ctgggcgcca gacgacgcga tcgccagtaa ccagcgatga aagggtccga cgaatgttgc      60

agcggtgcac cacgccatcg gtggcttcca catcggcgtg cataccgaat cggctgatga     120

taatgccttc ctgtgcgtcg cccagttggc tatcttccca ttcgactttg ctttccgttt     180

ttttcaggcg gcgctggtgg tttgcgctaa cccgacgctg ctgacctttc gacagtttct     240

ttttgctcac gcatagtcgg caataacgcc gtctttcatc gggcgaatcg ccacgatatt     300

cccaggcgtt ctgcccagca tctgcttagc gtcgtggcct actgctgcca cgcttttttg     360

ggaaccagca cgatcctgac gaatcgcaac cacagagggt tcattcagta tgatgccctg     420

ccctttaaca taaatcaggg tattggcggt acccaggtcg atggacaagt cgttggaaaa     480

catgccacga aatttcttaa acatgatcca tgaatgccgg tcggaatacc acggccatca     540

tcctgcaccg acaccgagtt atcggcatgg atggtgatga taatgtcttt acaatagcca     600

gcgagcgctt cgtcgatagc gttgtccaca acctcgaata ccatgtgatg caggccggta     660

ccgtcatccg tatcgccgat atacatacct gggcgtttac gtaccgcatc cagccctttc     720

aataccttga tacttgagga gtcataagaa ttcgacat                             758


<210>  55
<211>  920
<212>  DNA
<213>  artificial

<220>
<223>  sequence of the first arm of
       Wp012778355/Wp015452784/Wp012778510/Wp015452939/act56857/wp012778
       668 dsRNA used to concommitantly target the sequences encoding
       these proteins from candidatus liberibacter asiaticus

<400>  55
gctatcgacc acagtaattg taaactgact aaaccaatta gttataacat tacgtgcaag      60

acttttggct ataggcatga cattctcgtc agaattaatt tcataatatt tagatggatc     120

acttgcacat tgtcttaaag tatatcgcgt gatcgtgata aaagaattag gtttgtctat     180

gttcataatg acgactatct ctggatgtgg tttggctagc agagaaaaga aaaaggtatt     240

tttgcataaa tcgaatgata cagatatcgt caataaaaga tttgggagct ctttagataa     300

agcaatgcat gtcaaaaaca aaacacttga aaaaaacaat atttttatcg aaaaaaggta     360

atataacaat attaacagcg ataatcatcc cattgattat taccttaata actatatcga     420

caacatgtgc aaatatttta taccataggg ctagcatagt gcatgaccgc aattataatt     480

tcggtatgct ttttgtttat cacctatgct atcgatttag cgcacattat gtacatacgg     540

aatcaaatgc aatctgcttt agatgctgct gttctttctg gatgtgcatc tattgtatca     600

gaccgaacaa taaagcatgg taaaaatacg atctcaatac ttttgtcaat cctgtggtga     660

aatccattcg cgttggattg gaaaatgtga ttcatgtaat gcatggaata ccatcatcga     720

ggatagttcc agaaaagaaa gcgatcaaag atttagcaaa acaatttcca atgaatattt 780

cagaaattaa caaatatttc cctcccaata ataatataga gaggaaagag atcgctgata 840

aattaatcaa aaatatctca attgttgaca aaacgatgga cgtattgcct ctgtatcatc 900

aagttcgcga attgacacaa 920

<210> 56
<211> 920
<212> DNA
<213> artificial

<220>
<223> sequence of the second arm of
Wp012778355/Wp015452784/Wp012778510/Wp015452939/act56857/wp012778
668 dsRNA used to concommitantly target the sequences encoding
these proteins from candidatus liberibacter asiaticus

<400> 56
ttgtgtcaat tcgcgaactt gatgatacag aggcaatacg tccatcgttt tgtcaacaat 60

tgagatattt ttgattaatt tatcagcgat ctctttcctc tctatattat tattgggagg 120

gaaatatttg ttaatttctg aaatattcat tggaaattgt tttgctaaat ctttgatcgc 180

tttcttttct ggaactatcc tcgatgatgg tattccatgc attacatgaa tcacattttc 240

caatccaacg cgaatggatt tcaccacagg attgacaaaa gtattgagat cgtattttta 300

ccatgcttta ttgttcggtc tgatacaata gatgcacatc cagaaagaac agcagcatct 360

aaagcagatt gcatttgatt ccgtatgtac ataatgtgcg ctaaatcgat agcataggtg 420

ataaacaaaa agcataccga aattataatt gcggtcatgc actatgctag ccctatggta 480

taaaatattt gcacatgttg tcgatatagt tattaaggta ataatcaatg ggatgattat 540

cgctgttaat attgttatat tacctttttt cgataaaaat attgtttttt tcaagtgttt 600

tgtttttgac atgcattgct ttatctaaag agctcccaaa tcttttattg acgatatctg 660

tatcattcga tttatgcaaa aatacctttt tcttttctct gctagccaaa ccacatccag 720

agatagtcgt cattatgaac atagacaaac ctaattcttt tatcacgatc acgcgatata 780

ctttaagaca atgtgcaagt gatccatcta aatattatga aattaattct gacgagaatg 840

tcatgcctat agccaaaagt cttgcacgta atgttataac taattggttt agtcagttta 900

caattactgt ggtcgatagc 920

<210> 57
<211> 758
<212> DNA
<213> artificial

<220>
<223> Sequence of the first arm of the Wp012778355/
wp015452784/WP012778510 dsRNA used to concomitantly target the

sequences encoding the proteins
Wp012778355/wp015452784/WP012778510 from candidatus liberibacter

<400> 57
gctatcgacc acagtaattg taaactgact aaaccaatta gttataacat tacgtgcaag 60

acttttggct ataggcatga cattctcgtc agaattaatt tcataatatt tagatggatc 120

acttgcacat tgtcttaaag tatatcgcgt atcttgatcc ggagaaaatc ctacagaaaa 180

tatggtaatt gcacctcttc cagttctatg atgcttatgc atgtaatagc agtcttgcaa 240

aattctatct gatctgataa aagaattagg tttgtctatg ttcataatga cgactatctc 300

tggatgtggt ttggctagca gagaaaagaa aaaggtattt ttgcataaat cgaatgatac 360

agatatcgtc aataaaagat ttgggagctc tttagataaa gcagaagatg aatttcagat 420

gcaattacag gatacgggta tagttgttag tcgtataggt gatatgatta catgttatat 480

accagtacat gtttcttttg tatcatgcat gtcaaaaaca aaacacttga aaaaaacaat 540

atttttatcg aaaaaaggta atataacaat attaacagcg ataatcatcc cattgattat 600

taccttaata actatatcga caacatgtgc aaatatttta taccataggg ctagcataga 660

agcatccgct gacgaagcct taaatcatgg aatagtcctg ctatgtaagg attctgatct 720

tacaccgcaa gacatcaccc ctcccgtatt aaaagatc 758

<210> 58
<211> 758
<212> DNA
<213> artificial

<220>
<223> Sequence of the second arm of the Wp012778355/
wp015452784/WP012778510 dsRNA used to concomitantly target the
sequences encoding the proteins
Wp012778355/wp015452784/WP012778510 from candidatus liberibacter

<400> 58
gatcttttaa tacgggaggg gtgatgtctt gcggtgtaag atcagaatcc ttacatagca 60

ggactattcc atgatttaag gcttcgtcag cggatgcttc tatgctagcc ctatggtata 120

aaatatttgc acatgttgtc gatatagtta ttaaggtaat aatcaatggg atgattatcg 180

ctgttaatat tgttatatta ccttttttcg ataaaaatat tgtttttttc aagtgttttg 240

tttttgacat gcatgataca aaagaaacat gtactggtat ataacatgta atcatatcac 300

ctatacgact aacaactata cccgtatcct gtaattgcat ctgaaattca tcttctgctt 360

tatctaaaga gctcccaaat cttttattga cgatatctgt atcattcgat ttatgcaaaa 420

ataccttttt cttttctctg ctagccaaac cacatccaga gatagtcgtc attatgaaca 480

tagacaaacc taattctttt atcagatcag atagaatttt gcaagactgc tattacatgc 540

ataagcatca tagaactgga agaggtgcaa ttaccatatt ttctgtagga ttttctccgg 600

atcaagatac gcgatatact ttaagacaat gtgcaagtga tccatctaaa tattatgaaa 660

ttaattctga cgagaatgtc atgcctatag ccaaaagtct tgcacgtaat gttataacta 720

attggtttag tcagtttaca attactgtgg tcgatagc 758

<210> 59
<211> 758
<212> DNA
<213> artificial

<220>
<223> Sequence of the first arm of the wp015452939/act56857/wp012778668
      dsRNA used to concomitantly target the sequences encoding the
      proteins wp015452939/act56857/wp012778668 from candidatus
      liberibacter asiaticus

<400> 59
atgaccgcaa ttataatttc ggtatgcttt ttgtttatca cctatgctat cgatttagcg 60

cacattatgt acatacggaa tcaaatgcaa tctgctttag atgctgctgt tctttctgga 120

tgtgcatcta ttgtatcaga ccgaacaata aaagatccga cgacaaaaaa ggatcaaaca 180

agtactatct tcaaaaaaca gatcaaaaag catctaaaac aaggatctta tataagagaa 240

aacgcaggag gatcatggta aaaatacgat ctcaatactt ttgtcaatcc tgtggtgaaa 300

tccattcgcg ttggattgga aaatgtgatt catgtaatgc atggaatacc atcatcgagg 360

atagttccag aaaagaaagc gatcaaagat ttagcaaaac aattaaacaa ggtcactcta 420

tctctctttt tatgttatca gaagaatcaa tagaagaaga atcacgcatt caaacccata 480

ttgatgaact cgaccgtgtc acagatgcat gaatatttca gaaattaaca aatatttccc 540

tcccaataat aatatagaga ggaaagagat cgctgataaa ttaatcaaaa atatctcaat 600

tgttgacaaa acgatggacg tattgcctct gtatcatcaa gttcgcgaat tgacacaaaa 660

caaagcttcc actgaacaag tcatcgatat ctcaacaaaa gtaaaagacg tcgcagtaga 720

cgtcgcagtg agcatgattc ctatttatgg cacttacc 758

<210> 60
<211> 758
<212> DNA
<213> artificial

<220>
<223> Sequence of the second arm of the
      wp015452939/act56857/wp012778668 dsRNA used to concomitantly
      target the sequences encoding the proteins
      wp015452939/act56857/wp012778668 from candidatus liberibacter

<400> 60
ggtaagtgcc ataaatagga atcatgctca ctgcgacgtc tactgcgacg tcttttactt 60

ttgttgagat atcgatgact tgttcagtgg aagctttgtt ttgtgtcaat tcgcgaactt 120

gatgatacag aggcaatacg tccatcgttt tgtcaacaat tgagatattt ttgattaatt 180

tatcagcgat ctctttcctc tctatattat tattgggagg gaaatatttg ttaatttctg 240

aaatattcat gcatctgtga cacggtcgag ttcatcaata tgggtttgaa tgcgtgattc 300

ttcttctatt gattcttctg ataacataaa aagagagata gagtgacctt gtttaattgt 360

tttgctaaat ctttgatcgc tttcttttct ggaactatcc tcgatgatgg tattccatgc 420

attacatgaa tcacattttc caatccaacg cgaatggatt tcaccacagg attgacaaaa 480

gtattgagat cgtattttta ccatgatcct cctgcgtttt ctcttatata agatccttgt 540

tttagatgct ttttgatctg ttttttgaag atagtacttg tttgatcctt ttttgtcgtc 600

ggatctttta ttgttcggtc tgatacaata gatgcacatc cagaaagaac agcagcatct 660

aaagcagatt gcatttgatt ccgtatgtac ataatgtgcg ctaaatcgat agcataggtg 720

ataaacaaaa agcataccga aattataatt gcggtcat 758

<210> 61
<211> 921
<212> DNA
<213> artificial

<220>
<223> Sequence of the first arm of the GyrB/ Hfq/ HrpR/HRPS/MreB/ RpoD
      dsRNA used to concomitantly target GyrB, Hfq, HrpR, HrpS, MreB,
      RpoD genes from pseudomonas syringae pv actinidiae

<400> 61
atgagcgaaa accaaacgta cgactcgtcc agcattaaag tgctgaaagg acttgatgcc 60

gtacgcaagc gtcccggaat gtacatcggt gatacagacg atggcagcgg tctgcaccat 120

atggtgttcg aggtggttga taactcgatc gatcatgtca aaagggcatt cgctacaaga 180

cccttacttg aacaccttgc gaaaggagaa ggtcggcgtc tcgatctacc tggtcaacgg 240

tatcaagctg cagggaacga tcgagtcttt cgaccagttc gtcattttgc tgaaaaacac 300

tgtcatgcat gaatagcgcg ttcctggata tggacttgct gttgtgcggg gaaaccggca 360

ccggcaagga cacactggcc aaccgcattc acgagttgtc cagcaggtcg ggaccctttg 420

tgggcatgaa ctgcgcagcc attcccgagt cgctggcagt gcatgagtct tgatgaaagg 480

tttgaggatg atctggacga ggagcgggtt ccgaatctgg ggatagttgc cgaaagtatt 540

tcgcaactgg gtatcgacgt gctgctgtcg ggtgagaccg gcacgggcaa agacacgatt 600

gcccgacgga ttaagcatgt tcaagaaact gcgtggcatg ttttccagtg atctatccat 660

cgacctgggc actgccaaca cccttattta cgtgcgtgag cgcggtatcg tcctgaatga 720

accctctgtc gttgccattc gtactcacgg caatcagaag agtgtcgtcc aatgtccgga 780

aaagcgcaac agcagtctcg tatcaaagag ttgatcaccc tgggtcgtga gcagaaatat 840

ctgacttacg cagaggtcaa cgaccacctg cccgaggata tttcagatcc cgagcaggtg 900

gaagacatca tccgcatgat c 921

<210> 62
<211> 921
<212> DNA
<213> artificial

<220>
<223> Sequence of the second arm of the GyrB/ Hfq/ HrpR/HRPS/MreB/ RpoD
 dsRNA used to concomitantly target GyrB, Hfq, HrpR, HrpS, MreB,
 RpoD genes from pseudomonas syringae pv actinidiae

<400> 62
gatcatgcgg atgatgtctt ccacctgctc gggatctgaa atatcctcgg gcaggtggtc 60

gttgacctct gcgtaagtca gatatttctg ctcacgaccc agggtgatca actctttgat 120

acgagactgc tgttgcgctt ttccggacat tggacgacac tcttctgatt gccgtgagta 180

cgaatggcaa cgacagaggg ttcattcagg acgataccgc gctcacgcac gtaaataagg 240

gtgttggcag tgcccaggtc gatggataga tcactggaaa acatgccacg cagtttcttg 300

aacatgctta atccgtcggg caatcgtgtc tttgcccgtg ccggtctcac ccgacagcag 360

cacgtcgata cccagttgcg aaatactttc ggcaactatc cccagattcg gaacccgctc 420

ctcgtccaga tcatcctcaa acctttcatc aagactcatg cactgccagc gactcgggaa 480

tggctgcgca gttcatgccc acaaagggtc ccgacctgct ggacaactcg tgaatgcggt 540

tggccagtgt gtccttgccg gtgccggttt ccccgcacaa cagcaagtcc atatccagga 600

acgcgctatt catgcatgac agtgtttttc agcaaaatga cgaactggtc gaaagactcg 660

atcgttccct gcagcttgat accgttgacc aggtagatcg agacgccgac cttctccttt 720

cgcaaggtgt tcaagtaagg gtcttgtagc gaatgccctt ttgacatgat cgatcgagtt 780

atcaaccacc tcgaacacca tatggtgcag accgctgcca tcgtctgtat caccgatgta 840

cattccggga cgcttgcgta cggcatcaag tcctttcagc actttaatgc tggacgagtc 900

gtacgtttgg ttttcgctca t 921

<210> 63
<211> 758
<212> DNA
<213> artificial

<220>
<223> Sequence of the first arm of the GyrB/ Hfq/ MreB dsRNA used to
 concomitantly target GyrB, Hfq, MreB genes from pseudomonas
 syringae pv actinidiae

<400> 63
   atgagcgaaa accaaacgta cgactcgtcc agcattaaag tgctgaaagg acttgatgcc 60
   gtacgcaagc gtcccggaat gtacatcggt gatacagacg atggcagcgg tctgcaccat 120
   atggtgttcg aggtggttga taactcgatc gacgaagcat tggcaggcca ttgcgacgac 180
   atcagcatca tcatccatcc agacgaatct atcaccgtgc gcgacaacgg ccgcggtatt 240
   ccggtagacg gatcatgtca aaagggcatt cgctacaaga cccttacttg aacaccttgc 300
   gaaaggagaa ggtcggcgtc tcgatctacc tggtcaacgg tatcaagctg cagggaacga 360
   tcgagtcttt cgaccagttc gtcattttgc tgaaaaacac tgtcagccag atggtctaca 420
   aacacgccat ttccaccgtc gtaccggttc gcccgattcg cctgcctagc gctaccgacg 480
   ctgacggcgc tgacgccgag ccagatgcat gttcaagaaa ctgcgtggca tgttttccag 540
   tgatctatcc atcgacctgg gcactgccaa cacccttatt tacgtgcgtg agcgcggtat 600
   cgtcctgaat gaaccctctg tcgttgccat tcgtactcac ggcaatcaga agagtgtcgt 660
   cgctgtcggt accgaagcca agcgtatgct gggccgcact ccaggcaaca ttgccgccat 720
   tcgcccaatg aaagacggcg tcatcgccga tttcagcg 758

<210> 64
<211> 758
<212> DNA
<213> artificial

<220>
<223> Sequence of the second arm of the GyrB/ Hfq/ MreB dsRNA used to
      concomitantly target GyrB, Hfq, MreB genes from pseudomonas
      syringae pv actinidiae

<400> 64
cgctgaaatc ggcgatgacg ccgtctttca ttgggcgaat ggcggcaatg ttgcctggag 60

tgcggcccag catacgcttg gcttcggtac cgacagcgac gacactcttc tgattgccgt 120

gagtacgaat ggcaacgaca gagggttcat tcaggacgat accgcgctca cgcacgtaaa 180

taagggtgtt ggcagtgccc aggtcgatgg atagatcact ggaaaacatg ccacgcagtt 240

tcttgaacat gcatctggct cggcgtcagc gccgtcagcg tcggtagcgc taggcaggcg 300

aatcgggcga accggtacga cggtggaaat ggcgtgtttg tagaccatct ggctgacagt 360

gtttttcagc aaaatgacga actggtcgaa agactcgatc gttccctgca gcttgatacc 420

gttgaccagg tagatcgaga cgccgacctt ctcctttcgc aaggtgttca agtaagggtc 480

ttgtagcgaa tgcccttttg acatgatccg tctaccggaa taccgcggcc gttgtcgcgc 540

acggtgatag attcgtctgg atggatgatg atgctgatgt cgtcgcaatg gcctgccaat 600

gcttcgtcga tcgagttatc aaccacctcg aacaccatat ggtgcagacc gctgccatcg 660

tctgtatcac cgatgtacat tccgggacgc ttgcgtacgg catcaagtcc tttcagcact 720

ttaatgctgg acgagtcgta cgtttggttt tcgctcat 758

<210>  65
<211>  758
<212>  DNA
<213>  artificial

<220>
<223>  Sequence of the first arm of the HrpR/ HrpS/Hfq dsRNA used to
       concomitantly target HrpR, HrpS, Hfq genes from pseudomonas
       syringae pv actinidiae

<400>  65
atgaatagcg cgttcctgga tatggacttg ctgttgtgcg gggaaaccgg caccggcaag      60

gacacactgg ccaaccgcat tcacgagttg tccagcaggt cgggaccctt tgtgggcatg     120

aactgcgcag ccattcccga gtcgctggca gagagccagt tattcggtgt ggtcaacggc     180

gcattcaccg gcgtatgccg ggctcgcgag ggctacatag aggcctccag tggtggcacg     240

ttgtacctgg gatcatgagt cttgatgaaa ggtttgagga tgatctggac gaggagcggg     300

ttccgaatct ggggatagtt gccgaaagta tttcgcaact gggtatcgac gtgctgctgt     360

cgggtgagac cggcacgggc aaagacacga ttgcccgacg gattcatgag atgtcaggcc     420

gcaaagggcg cctggtggcg atgaattgcg cggccattcc ggagtccctc gccgagagcg     480

agttattcgg tgtggtcagc ggtgatgcat gtcaaaaggg cattcgctac aagaccctta     540

cttgaacacc ttgcgaaagg agaaggtcgg cgtctcgatc tacctggtca acggtatcaa     600

gctgcaggga acgatcgagt ctttcgacca gttcgtcatt ttgctgaaaa acactgtcag     660

ccagatggtc tacaaacacg ccatttccac cgtcgtaccg gttcgcccga ttcgcctgcc     720

tagcgctacc gacgctgacg gcgctgacgc cgagccag                             758

<210>  66
<211>  758
<212>  DNA
<213>  artificial

<220>
<223>  Sequence of the second arm of the HrpR/ HrpS/Hfq dsRNA used to
       concomitantly target HrpR, HrpS, Hfq genes from pseudomonas
       syringae pv actinidiae

<400>  66
ctggctcggc gtcagcgccg tcagcgtcgg tagcgctagg caggcgaatc gggcgaaccg      60

gtacgacggt ggaaatggcg tgtttgtaga ccatctggct gacagtgttt ttcagcaaaa     120

tgacgaactg gtcgaaagac tcgatcgttc cctgcagctt gataccgttg accaggtaga     180

tcgagacgcc gaccttctcc tttcgcaagg tgttcaagta agggtcttgt agcgaatgcc     240

cttttgacat gcatcaccgc tgaccacacc gaataactcg ctctcggcga gggactccgg     300

aatggccgcg caattcatcg ccaccaggcg ccctttgcgg cctgacatct catgaatccg     360

tcgggcaatc gtgtctttgc ccgtgccggt ctcacccgac agcagcacgt cgatacccag     420

ttgcgaaata ctttcggcaa ctatccccag attcggaacc cgctcctcgt ccagatcatc 480

ctcaaacctt tcatcaagac tcatgatccc aggtacaacg tgccaccact ggaggcctct 540

atgtagccct cgcgagcccg gcatacgccg gtgaatgcgc cgttgaccac accgaataac 600

tggctctctg ccagcgactc gggaatggct gcgcagttca tgcccacaaa gggtcccgac 660

ctgctggaca actcgtgaat gcggttggcc agtgtgtcct tgccggtgcc ggtttccccg 720

cacaacagca agtccatatc caggaacgcg ctattcat 758

<210> 67
<211> 758
<212> DNA
<213> artificial

<220>
<223> Sequence of the first arm of the HrpR/ GyrB/ RpoD dsRNA used to
concomitantly target HrpR, GyrB, RpoD genes from pseudomonas
syringae pv actinidiae

<400> 67
atgaatagcg cgttcctgga tatggacttg ctgttgtgcg gggaaaccgg caccggcaag 60

gacacactgg ccaaccgcat tcacgagttg tccagcaggt cgggaccctt tgtgggcatg 120

aactgcgcag ccattcccga gtcgctggca gagagccagt tattcggtgt ggtcaacggc 180

gcattcaccg gcgtatgccg ggctcgcgag ggctacatag aggcctccag tggtggcacg 240

ttgtacctgg gatcatgagc gaaaaccaaa cgtacgactc gtccagcatt aaagtgctga 300

aaggacttga tgccgtacgc aagcgtcccg gaatgtacat cggtgataca gacgatggca 360

gcggtctgca ccatatggtg ttcgaggtgg ttgataactc gatcgacgaa gcattggcag 420

gccattgcga cgacatcagc atcatcatcc atccagacga atctatcacc gtgcgcgaca 480

acggccgcgg tattccggta gacgatgcat gtccggaaaa gcgcaacagc agtctcgtat 540

caaagagttg atcaccctgg gtcgtgagca gaaatatctg acttacgcag aggtcaacga 600

ccacctgccc gaggatattt cagatcccga gcaggtggaa gacatcatcc gcatgatcaa 660

cgacatgggg atcccggtac acgagagtgc tccggatgcg gacgccttga tgctggccga 720

cgccgatacc gacgaggcag ccgcagaaga agcggctg 758

<210> 68
<211> 932
<212> DNA
<213> artificial

<220>
<223> Sequence of the first arm of the
NPP1/INF1/GK4/piacwp1-1/piacwp1-2/piacwp1-3 dsRNA used to
concomitantly target NPP1, INF1, GK4, piacwp1-1, piacwp 1-2 and
piacwp1-3 genes from phytophtora infestans

<400> 68
   cgcctccaag tcctcgagac tctccaccga catgaacatc cttcaactct tcgcatcggc 60
   cgcagcagct cttactgtcg cgcatgccga tgtgatttca cacgatgcag tgatcccgtt 120
   cgcccaaccc acaaccacca ctacaacaga gcaggatcgt ttgctgaccc ttccagtcct 180
   agctccacaa cttccattgt gcaatttgct ctgttatccc cacccactcc tcctcactcc 240
   gtccacgatg aactttcgtg ctctgttcgc tgccaccgtt gctgccctcg tcggctccac 300
   ttccgccaat gcatggcgtt gtgcatccct gggtcgtcgc aaaacgcgtc ggcagccgcg 360
   agtgtagcca gcacgagcga cagcaccaat ccgttcggcc gacggctact aaccagtgtc 420
   gccagctccg cgagcgcagg tgacgatggc tacgtgcagc tcgaggtgca ggagagcgag 480
   gaattgacga atgaacttgg cgctttcttt ttgcaaatcc cggagtatag cgagctgaca 540
   cacccagcct gcctgtcgga attgtcccct ggcccacctt ttcacggagg agacagcggc 600
   gctgtctccg accgcagact aagcctggac tctgcaagca ctcagatgga gattggcagt 660
   ggcaggagag cgaggaattg aagaatgaac ttggcgcttt ctctttgcaa atcccggagt 720
   atagcgagct gacacaccca gcctgcctgt cggaattgtc ccctggccca ccttttctcc 780
   agcactcaga tggagattgg cagtggcagg agagcgagga attgacgaat gaacttggcg 840
   ctttcttttt gcaaatcccg gagtatagcg agctgacaca cccagcctgc ctgtcggaat 900
   tgtcccctgg cccacctttt cacggaggag ac 932

<210> 69
<211> 932
<212> DNA
<213> artificial

<220>
<223> Sequence of the second arm of the
NPP1/INF1/GK4/piacwp1-1/piacwp1-2/piacwp1-3 dsRNA used to
concomitantly target NPP1, INF1, GK4, piacwp1-1, piacwp 1-2 and
piacwp1-3 genes from Phytophthora infestans

<400> 69
gtctcctccg tgaaaaggtg ggccagggga caattccgac aggcaggctg ggtgtgtcag 60

ctcgctatac tccgggattt gcaaaaagaa agcgccaagt tcattcgtca attcctcgct 120

ctcctgccac tgccaatctc catctgagtg ctggagaaaa ggtgggccag gggacaattc 180

cgacaggcag gctgggtgtg tcagctcgct atactccggg atttgcaaag agaaagcgcc 240

aagttcattc ttcaattcct cgctctcctg ccactgccaa tctccatctg agtgcttgca 300

gagtccaggc ttagtctgcg gtcggagaca gcgccgctgt ctcctccgtg aaaaggtggg 360

ccaggggaca attccgacag gcaggctggg tgtgtcagct cgctatactc cgggatttgc 420

aaaaagaaag cgccaagttc attcgtcaat tcctcgctct cctgcacctc gagctgcacg 480

tagccatcgt cacctgcgct cgcggagctg gcgacactgg ttagtagccg tcggccgaac 540

ggattggtgc tgtcgctcgt gctggctaca ctcgcggctg ccgacgcgtt ttgcgacgac 600

ccagggatgc acaacgccat gcattggcgg aagtggagcc gacgagggca gcaacggtgg 660

cagcgaacag agcacgaaag ttcatcgtgg acggagtgag gaggagtggg tggggataac 720

agagcaaatt gcacaatgga agttgtggag ctaggactgg aagggtcagc aaacgatcct 780

gctctgttgt agtggtggtt gtgggttggg cgaacgggat cactgcatcg tgtgaaatca 840

catcggcatg cgcgacagta agagctgctg cggccgatgc gaagagttga aggatgttca 900

tgtcggtgga gagtctcgag gacttggagg cg 932

<210> 70
<211> 790
<212> DNA
<213> artificial

<220>
<223> Sequence of the first arm of the piacwp1-1/piacwp1-2/piacwp1-3
      dsRNA used to concomitantly target piacwp1-1, piacwp 1-2 and
      piacwp1-3 genes from Phytophthora infestans

<400> 70
aggagagcga ggaattgacg aatgaacttg gcgctttctt tttgcaaatc ccggagtata 60

gcgagctgac acacccagcc tgcctgtcgg aattgtcccc tggcccacct tttcacggag 120

gagacagcgg cgctgtctcc gcaccgcaga ctctggccca gagagcttac tggccatttt 180

acaattcgca cgctgagact caccctgaca tttctcgttc tttgcaccat ggtcttctcc 240

tctactacct gtattctgat cctggactct gcaagcactc agatggagat tggcagtggc 300

aggagagcga ggaattgaag aatgaacttg gcgctttctc tttgcaaatc ccggagtata 360

gcgagctgac acacccagcc tgcctgtcgg aattgtcccc tggcccacct tttcacggag 420

gagacagcgg cgctgtctcc gcaccgcaga ctctggccca gagagcttac tggccatttt 480

acaattcgca cgctgagact caccctgaca tttctcgttc tttgcaccat gccactcaga 540

tggagattgg cagtggcagg agagcgagga attgacgaat gaacttggcg ctttcttttt 600

gcaaatcccg gagtatagcg agctgacaca cccagcctgc ctgtcggaat tgtcccctgg 660

cccacctttt cacggaggag acagcggcgc tgtctccgca ccgcagactc tggcccagag 720

agcttactgg ccattttaca attcgcacgc tgagactcac cctgacattt ctcgttcttt 780

gcaccatggt 790

<210> 71
<211> 790
<212> DNA
<213> artificial

<220>
<223> Sequence of the second arm of the piacwp1-1/piacwp1-2/piacwp1-3
      dsRNA used to concomitantly target piacwp1-1, piacwp 1-2 and

piacwp1-3 genes from Phytophthora infestans

<400> 71
accatggtgc aaagaacgag aaatgtcagg gtgagtctca gcgtgcgaat tgtaaaatgg 60

ccagtaagct ctctgggcca gagtctgcgg tgcggagaca gcgccgctgt ctcctccgtg 120

aaaaggtggg ccaggggaca attccgacag gcaggctggg tgtgtcagct cgctatactc 180

cgggatttgc aaaaagaaag cgccaagttc attcgtcaat tcctcgctct cctgccactg 240

ccaatctcca tctgagtggc atggtgcaaa gaacgagaaa tgtcagggtg agtctcagcg 300

tgcgaattgt aaaatggcca gtaagctctc tgggccagag tctgcggtgc ggagacagcg 360

ccgctgtctc ctccgtgaaa aggtgggcca ggggacaatt ccgacaggca ggctgggtgt 420

gtcagctcgc tatactccgg gatttgcaaa gagaaagcgc caagttcatt cttcaattcc 480

tcgctctcct gccactgcca atctccatct gagtgcttgc agagtccagg atcagaatac 540

aggtagtaga ggagaagacc atggtgcaaa gaacgagaaa tgtcagggtg agtctcagcg 600

tgcgaattgt aaaatggcca gtaagctctc tgggccagag tctgcggtgc ggagacagcg 660

ccgctgtctc ctccgtgaaa aggtgggcca ggggacaatt ccgacaggca ggctgggtgt 720

gtcagctcgc tatactccgg gatttgcaaa aagaaagcgc caagttcatt cgtcaattcc 780

tcgctctcct 790


<210> 72
<211> 781
<212> DNA
<213> artificial

<220>
<223> Sequence of the first arm of the GK4/ INF1/NPP1 dsRNA used to
      concomitantly target GK4, INF1, NPP1 genes from Phytophtora
      infestans

<400> 72
atggcgttgt gcatccctgg gtcgtcgcaa aacgcgtcgg cagccgcgag tgtagccagc 60

acgagcgaca gcaccaatcc gttcggccga cggctactaa ccagtgtcgc cagctccgcg 120

agcgcaggtg acgatggcta cgtgcagctc gagaccgagt tccctggcgc catagctatc 180

tccatggtcg tgaacgcact gtccctgctt ggcttcgctt tcattctcac cacgtacctg 240

ttgttcccac ccgtgcgaaa gcgatcgttt gctgaccctt ccagtcctag ctccacaact 300

tccattgtgc aatttgctct gttatcccca cccactcctc ctcactccgt ccacgatgaa 360

ctttcgtgct ctgttcgctg ccaccgttgc tgccctcgtc ggctccactt ccgccaccac 420

gtgcaccacc tcgcagcaga ccgtagcgta cgtggcgctc gtaagcatcc tctcggacac 480

gtcgtttaat cagtgctcga cggactccgg ctactcgaat gccgcctcca agtcctcgag 540

actctccacc gacatgaaca tccttcaact cttcgcatcg gccgcagcag ctcttactgt 600

cgcgcatgcc gatgtgattt cacacgatgc agtgatcccg ttcgcccaac ccacaaccac 660

cactacaaca gagcagaagg ctggtatcaa gttcaagccc cagatccaca tctcgaacgg 720

ctgccacccg taccctgcag tggatgcaga cggcaacacc agcggcggtc taaaccctac 780

g 781

<210> 73
<211> 781
<212> DNA
<213> artificial

<220>
<223> Sequence of the second arm of the GK4/ INF1/NPP1 dsRNA used to
      concomitantly target GK4, INF1, NPP1 genes from Phytophthora
      infestans

<400> 73
cgtagggttt agaccgccgc tggtgttgcc gtctgcatcc actgcagggt acgggtggca 60

gccgttcgag atgtggatct ggggcttgaa cttgatacca gccttctgct ctgttgtagt 120

ggtggttgtg ggttgggcga acgggatcac tgcatcgtgt gaaatcacat cggcatgcgc 180

gacagtaaga gctgctgcgg ccgatgcgaa gagttgaagg atgttcatgt cggtggagag 240

tctcgaggac ttggaggcgg cattcgagta gccggagtcc gtcgagcact gattaaacga 300

cgtgtccgag aggatgctta cgagcgccac gtacgctacg gtctgctgcg aggtggtgca 360

cgtggtggcg gaagtggagc cgacgagggc agcaacggtg gcagcgaaca gagcacgaaa 420

gttcatcgtg gacggagtga ggaggagtgg gtggggataa cagagcaaat tgcacaatgg 480

aagttgtgga gctaggactg gaagggtcag caaacgatcg ctttcgcacg ggtgggaaca 540

acaggtacgt ggtgagaatg aaagcgaagc caagcaggga cagtgcgttc acgaccatgg 600

agatagctat ggcgccaggg aactcggtct cgagctgcac gtagccatcg tcacctgcgc 660

tcgcggagct ggcgacactg gttagtagcc gtcggccgaa cggattggtg ctgtcgctcg 720

tgctggctac actcgcggct gccgacgcgt tttgcgacga cccagggatg cacaacgcca 780

t 781

<210> 74
<211> 802
<212> DNA
<213> artificial

<220>
<223> Sequence of the first arm of the GK4/ INF1/piacwp1.1 dsRNA used
      to concomitantly target GK4, INF1, piacwp1.1 genes from
      Phytophthora infestans

<400> 74
atggcgttgt gcatccctgg gtcgtcgcaa aacgcgtcgg cagccgcgag tgtagccagc 60

acgagcgaca gcaccaatcc gttcggccga cggctactaa ccagtgtcgc cagctccgcg 120

agcgcaggtg acgatggcta cgtgcagctc gagaccgagt tccctggcgc catagctatc 180

tccatggtcg tgaacgcact gtccctgctt ggcttcgctt tcattctcac cacgtacctg 240

ttgttcccac ccgtgcgaaa gcgatcgttt gctgaccctt ccagtcctag ctccacaact 300

tccattgtgc aatttgctct gttatcccca cccactcctc ctcactccgt ccacgatgaa 360

ctttcgtgct ctgttcgctg ccaccgttgc tgccctcgtc ggctccactt ccgccaccac 420

gtgcaccacc tcgcagcaga ccgtagcgta cgtggcgctc gtaagcatcc tctcggacac 480

gtcgtttaat cagtgctcga cggactccgg ctactcgaat gcaggagagc gaggaattga 540

cgaatgaact tggcgctttc tttttgcaaa tcccggagta tagcgagctg acacacccag 600

cctgcctgtc ggaattgtcc cctggcccac cttttcacgg aggagacagc ggcgctgtct 660

ccgcaccgca gactctggcc cagagagctt actggccatt ttacaattcg cacgctgaga 720

ctcaccctga catttctcgt tctttgcacc atggtcttct cctctactac ctgtattctg 780

ggtgtggcgg cgctcgcgtc gg 802

<210> 75
<211> 802
<212> DNA
<213> artificial

<220>
<223> Sequence of the second arm of the GK4/ INF1/piacwp1.1 dsRNA used
to concomitantly target GK4, INF1, piacwp1.1 genes from
Phytophthora infestans

<400> 75
ccgacgcgag cgccgccaca cccagaatac aggtagtaga ggagaagacc atggtgcaaa 60

gaacgagaaa tgtcagggtg agtctcagcg tgcgaattgt aaaatggcca gtaagctctc 120

tgggccagag tctgcggtgc ggagacagcg ccgctgtctc ctccgtgaaa aggtgggcca 180

ggggacaatt ccgacaggca ggctgggtgt gtcagctcgc tatactccgg gatttgcaaa 240

aagaaagcgc caagttcatt cgtcaattcc tcgctctcct gcattcgagt agccggagtc 300

cgtcgagcac tgattaaacg acgtgtccga gaggatgctt acgagcgcca cgtacgctac 360

ggtctgctgc gaggtggtgc acgtggtggc ggaagtggag ccgacgaggg cagcaacggt 420

ggcagcgaac agagcacgaa agttcatcgt ggacggagtg aggaggagtg ggtggggata 480

acagagcaaa ttgcacaatg gaagttgtgg agctaggact ggaagggtca gcaaacgatc 540

gctttcgcac gggtgggaac aacaggtacg tggtgagaat gaaagcgaag ccaagcaggg 600

acagtgcgtt cacgaccatg gagatagcta tggcgccagg gaactcggtc tcgagctgca 660

cgtagccatc gtcacctgcg ctcgcggagc tggcgacact ggttagtagc cgtcggccga 720

acggattggt gctgtcgctc gtgctggcta cactcgcggc tgccgacgcg ttttgcgacg 780

acccagggat gcacaacgcc at                                              802

<210>  76
<211>  1079
<212>  DNA
<213>  artificial

<220>
<223>  Sequence of the first arm of the TOR/ CGF1/ DCL1/DCL2/ LTF1/HBF1
       dsRNA used to concomitantly target TOR/ CGF1/ DCL1/DCL2/
       LTF1/HBF1  genes from Botrytis cinerea

<400>  76
atgtcacaac aatctaatca agcatttgat gctttgaatc gcattgtggc agatttaaga      60

tcacggagtg atgaacagcg aaagagagcg gccctggagt taagagagtt gcttgaggtt     120

gcttctagag actttcccca ggaacgatac acggaatttt ccaatattgt aagtggaaag     180

attagatcat gcatactcca tccatcaata tcttgctcgc tgtgggcctt ctcttctcta     240

ccccattcac atcagctctt actattcctt cgattgacaa gaagtctatc gctggcacag     300

gcatagaatc cattgagact cgtgatattc ctgctctcat caatcgtcac cacaccgatg     360

cgagtttaca tgttttatat atcttcaagc tttctacatt gggaaagatc ttgggcatat     420

ttcaaccgcc cgcaggagca caagcgaatg acgtctcgca gatgacgaga gacgcagcag     480

cagcaaaaag tctctaccat tggcgttggg tttctcctat cggatgaaac gctgggggtg     540

tgcgactgcc tgtaccgaca ttctcagcat ttcttatcga aattgagaat gtcgtacaaa     600

ggtaaaaaaa aaaaaatgac aaatgaaagt ccataaattc ccaaaatgga caactattcg     660

cggaaacaaa agaccccggt cgcaaaaaca aacgcctcat cttaaccacg actctctatc     720

tctcaaacaa gcatggaggg tgcggaaagt ggcagtaggt caagtggcat cctctccatc     780

atgaaccgtg aacatatcga gcgcataaat accgacacga gatatatgca atcaaatcca     840

atggctgcac caccgactgc cagtcctgcg tccgctcact actcaaacgg tgcaccatac     900

tccgcaggat ggccctccag agcgcacaac gtaacaatgg aaacttttcg aatttttctg     960

aaattctcgt cgatttcgac aaagaacgtc ctaagctcct tcttccggta tcattcaaag    1020

aagattttat gaaaaaaaaa acaaagtttg taatgttcta cgctcacgat caacctttg     1079

<210>  77
<211>  1079
<212>  DNA
<213>  artificial

<220>
<223>  Sequence of the second arm of the TOR/ CGF1/ DCL1/DCL2/ LTF1/HBF1
       dsRNA used to concomitantly target TOR, CGF1, DCL1, DCL2, LTF1,
       HBF1 genes from Botrytis cinerea

<400>  77
caaaggttga tcgtgagcgt agaacattac aaactttgtt ttttttttca taaaatcttc      60

tttgaatgat accggaagaa ggagcttagg acgttctttg tcgaaatcga cgagaatttc 120

agaaaaattc gaaaagtttc cattgttacg ttgtgcgctc tggagggcca tcctgcggag 180

tatggtgcac cgtttgagta gtgagcggac gcaggactgg cagtcggtgg tgcagccatt 240

ggatttgatt gcatatatct cgtgtcggta tttatgcgct cgatatgttc acggttcatg 300

atggagagga tgccacttga cctactgcca ctttccgcac cctccatgct tgtttgagag 360

atagagagtc gtggttaaga tgaggcgttt gtttttgcga ccggggtctt ttgtttccgc 420

gaatagttgt ccattttggg aatttatgga ctttcatttg tcattttttt ttttttacct 480

ttgtacgaca ttctcaattt cgataagaaa tgctgagaat gtcggtacag gcagtcgcac 540

acccccagcg tttcatccga taggagaaac ccaacgccaa tggtagagac tttttgctgc 600

tgctgcgtct ctcgtcatct gcgagacgtc attcgcttgt gctcctgcgg gcggttgaaa 660

tatgcccaag atctttccca atgtagaaag cttgaagata tataaaacat gtaaactcgc 720

atcggtgtgg tgacgattga tgagagcagg aatatcacga gtctcaatgg attctatgcc 780

tgtgccagcg atagacttct tgtcaatcga aggaatagta agagctgatg tgaatggggt 840

agagaagaga aggcccacag cgagcaagat attgatggat ggagtatgca tgatctaatc 900

tttccactta caatattgga aaattccgtg tatcgttcct ggggaaagtc tctagaagca 960

acctcaagca actctcttaa ctccagggcc gctctctttc gctgttcatc actccgtgat 1020

cttaaatctg ccacaatgcg attcaaagca tcaaatgctt gattagattg ttgtgacat 1079

<210> 78
<211> 797
<212> DNA
<213> artificial

<220>
<223> Sequence of the first arm of the TOR/ DCL1/DCL2 dsRNA used to
concomitantly target TOR, DCL1, DCL2 genes from Botrytis cinerea

<400> 78
atgtcacaac aatctaatca agcatttgat gctttgaatc gcattgtggc agatttaaga 60

tcacggagtg atgaacagcg aaagagagcg gccctggagt taagagagtt gcttgaggtt 120

gcttctagag actttcccca ggaacgatac acggaatttt ccaatattgt aagtggaaag 180

attaccagcc tcatcggtca ccatggagat gttgagcgaa ctggtggtat atatgctatc 240

gatgcgctgg tcgattttga cggtgatcga gtttacatgt tttatatatc ttcaagcttt 300

ctacattggg aaagatcttg ggcatatttc aaccgcccgc aggagcacaa gcgaatgacg 360

tctcgcagat gacgagagac gcagcagcag caaaaagtct ctaccattgg cgttgggttt 420

ctcctatcgg atgaaacgct gggggtgagg ggacgttcac ttcttctgaa taacacaacc 480

agataacaat aacaagaatt caatttctcc cgcctctcac ctcccaatgc gactgcctgt 540

accgacattc tcagcatttc ttatcgaaat tgagaatgtc gtacaaaggt aaaaaaaaaa 600

aaatgacaaa tgaaagtcca taaattccca aaatggacaa ctattcgcgg aaacaaaaga 660

ccccggtcgc aaaaacaaac gcctcatctt aaccacgact ctctatctct caaacccaca 720

tcgctcttcc acaattcctc attcccagtt agggttttta gtctaacgaa tcattttcgc 780

tctccggctt ctttcga 797

<210> 79
<211> 797
<212> DNA
<213> artificial

<220>
<223> Sequence of the second arm of the TOR/ DCL1/DCL2 dsRNA used to
      concomitantly target TOR, DCL1, DCL2 genes from Botrytis cinerea

<400> 79
tcgaaagaag ccggagagcg aaaatgattc gttagactaa aaaccctaac tgggaatgag 60

gaattgtgga agagcgatgt gggtttgaga gatagagagt cgtggttaag atgaggcgtt 120

tgtttttgcg accggggtct tttgtttccg cgaatagttg tccattttgg gaatttatgg 180

actttcattt gtcatttttt tttttttacc tttgtacgac attctcaatt tcgataagaa 240

atgctgagaa tgtcggtaca ggcagtcgca ttgggaggtg agaggcggga gaaattgaat 300

tcttgttatt gttatctggt tgtgttattc agaagaagtg aacgtcccct cacccccagc 360

gtttcatccg ataggagaaa cccaacgcca atggtagaga ctttttgctg ctgctgcgtc 420

tctcgtcatc tgcgagacgt cattcgcttg tgctcctgcg ggcggttgaa atatgcccaa 480

gatctttccc aatgtagaaa gcttgaagat atataaaaca tgtaaactcg atcaccgtca 540

aaatcgacca gcgcatcgat agcatatata ccaccagttc gctcaacatc tccatggtga 600

ccgatgaggc tggtaatctt tccacttaca atattggaaa attccgtgta tcgttcctgg 660

ggaaagtctc tagaagcaac ctcaagcaac tctcttaact ccagggccgc tctctttcgc 720

tgttcatcac tccgtgatct taaatctgcc acaatgcgat tcaaagcatc aaatgcttga 780

ttagattgtt gtgacat 797

<210> 80
<211> 811
<212> DNA
<213> artificial

<220>
<223> Sequence of the first arm of the CGF1/HBF1/LTF1 dsRNA used to
      concomitantly target CGF1, HBF1, LTF1 genes from Botrytis cinerea

<400> 80
atgcatactc catccatcaa tatcttgctc gctgtgggcc ttctcttctc taccccattc 60

acatcagctc ttactattcc ttcgattgac aagaagtcta tcgctggcac aggcatagaa 120

tccattgaga ctcgtgatat tcctgctctc atcaatcgtc accacaccga ggcccagatc 180

gccgctaagg cgggaaaggc aaaaggaaaa ggacagcgcg atgttttaga gagagagccg 240

catcacactg aagcacagat tgcagcaaag aaagcaaaag gatcgagcgc acaacgtaac 300

aatggaaact tttcgaattt ttctgaaatt ctcgtcgatt tcgacaaaga acgtcctaag 360

ctccttcttc cggtatcatt caaagaagat tttatgaaaa aaaaaacaaa gtttgtaatg 420

ttctacgctc acgatcaacc tttgacttca acggattgcc atcacataca caaaattctc 480

ctggcacatt atcaacgatt cattcaagtc taccgtcaac tcgtagacag ctaaaagctg 540

taaaatgcat ggagggtgcg gaaagtggca gtaggtcaag tggcatcctc tccatcatga 600

accgtgaaca tatcgagcgc ataaataccg acacgagata tatgcaatca aatccaatgg 660

ctgcaccacc gactgccagt cctgcgtccg ctcactactc aaacggtgca ccatactccg 720

caggatggcc cagtacttct catggcggtt taatatctcc tccagagtcg cggaggacgt 780

ccaacgacaa agcgcacccg catccattgc a 811

<210> 81
<211> 811
<212> DNA
<213> artificial

<220>
<223> Sequence of the second arm of the CGF1/HBF1/LTF1 dsRNA used to
concomitantly target CGF1, HBF1, LTF1 genes from Botrytis cinerea

<400> 81
tgcaatggat gcgggtgcgc tttgtcgttg gacgtcctcc gcgactctgg aggagatatt 60

aaaccgccat gagaagtact gggccatcct gcggagtatg gtgcaccgtt tgagtagtga 120

gcggacgcag gactggcagt cggtggtgca gccattggat ttgattgcat atatctcgtg 180

tcggtattta tgcgctcgat atgttcacgg ttcatgatgg agaggatgcc acttgaccta 240

ctgccacttt ccgcaccctc catgcatttt acagctttta gctgtctacg agttgacggt 300

agacttgaat gaatcgttga taatgtgcca ggagaatttt gtgtatgtga tggcaatccg 360

ttgaagtcaa aggttgatcg tgagcgtaga acattacaaa ctttgttttt tttttcataa 420

aatcttcttt gaatgatacc ggaagaagga gcttaggacg ttctttgtcg aaatcgacga 480

gaatttcaga aaaattcgaa aagtttccat tgttacgttg tgcgctcgat ccttttgctt 540

tctttgctgc aatctgtgct tcagtgtgat gcggctctct ctctaaaaca tcgcgctgtc 600

cttttccttt tgcctttccc gccttagcgg cgatctgggc ctcggtgtgg tgacgattga 660

tgagagcagg aatatcacga gtctcaatgg attctatgcc tgtgccagcg atagacttct 720

tgtcaatcga aggaatagta agagctgatg tgaatggggt agagaagaga aggcccacag 780

cgagcaagat attgatggat ggagtatgca t 811

<210> 82
<211> 771
<212> DNA
<213> artificial

<220>
<223> Sequence of the first arm of the DCL1/ HBF1/TOR dsRNA used to
concomitantly target DCL1, HBF1, TOR genes from Botrytis cinerea

<400> 82
gagtttacat gttttatata tcttcaagct ttctacattg ggaaagatct tgggcatatt 60

tcaaccgccc gcaggagcac aagcgaatga cgtctcgcag atgacgagag acgcagcagc 120

agcaaaaagt ctctaccatt ggcgttgggt ttctcctatc ggatgaaacg ctgggggtga 180

ggggacgttc acttcttctg aataacacaa ccagataaca ataacaagaa ttcaatttct 240

cccgcctctc acctcccaac agatcgagcg cacaacgtaa caatggaaac ttttcgaatt 300

tttctgaaat tctcgtcgat ttcgacaaag aacgtcctaa gctccttctt ccggtatcat 360

tcaaagaaga ttttatgaaa aaaaaaacaa agtttgtaat gttctacgct cacgatcaac 420

ctttgacttc aacggattgc catcacatac acaaaattct cctggcacat tatcaacgat 480

tcattcaagt ctaccgtcaa ctcgtatgca tgtcacaaca atctaatcaa gcatttgatg 540

ctttgaatcg cattgtggca gatttaagat cacggagtga tgaacagcga aagagagcgg 600

ccctggagtt aagagagttg cttgaggttg cttctagaga ctttccccag gaacgataca 660

cggaattttc caatattgta agtggaaaga ttaccagcct catcggtcac catggagatg 720

ttgagcgaac tggtggtata tatgctatcg atgcgctggt cgattttgac g 771

<210> 83
<211> 771
<212> DNA
<213> artificial

<220>
<223> Sequence of the second arm of the DCL1/ HBF1/TOR dsRNA used to
concomitantly target DCL1, HBF1, TOR genes from Botrytis cinerea

<400> 83
cgtcaaaatc gaccagcgca tcgatagcat atataccacc agttcgctca acatctccat 60

ggtgaccgat gaggctggta atctttccac ttacaatatt ggaaaattcc gtgtatcgtt 120

cctggggaaa gtctctagaa gcaacctcaa gcaactctct taactccagg gccgctctct 180

ttcgctgttc atcactccgt gatcttaaat ctgccacaat gcgattcaaa gcatcaaatg 240

cttgattaga ttgttgtgac atgcatacga gttgacggta gacttgaatg aatcgttgat 300

aatgtgccag gagaattttg tgtatgtgat ggcaatccgt tgaagtcaaa ggttgatcgt 360

gagcgtagaa cattacaaac tttgtttttt ttttcataaa atcttctttg aatgataccg 420

gaagaaggag cttaggacgt tctttgtcga aatcgacgag aatttcagaa aaattcgaaa 480

agtttccatt gttacgttgt gcgctcgatc tgttgggagg tgagaggcgg gagaaattga 540

attcttgtta ttgttatctg gttgtgttat tcagaagaag tgaacgtccc ctcaccccca 600

gcgtttcatc cgataggaga aacccaacgc caatggtaga gactttttgc tgctgctgcg 660

tctctcgtca tctgcgagac gtcattcgct tgtgctcctg cgggcggttg aaatatgccc 720

aagatctttc ccaatgtaga aagcttgaag atatataaaa catgtaaact c 771

<210> 84
<211> 947
<212> DNA
<213> artificial

<220>
<223>
Sequence of the first arm of the dsRNA used to concomitantly
target CclA/ ACS1/ ACS2/ ELP1/ ELP2/ MOB2 genes from
Colletotrichum higginsianum

<400> 84
   atggcgtccg actcgggtac tccccctcct ccttgggagc cgactctgag cggttccctt 60
   cctgcctcaa ccgcgatccc gttgaagcgc gccgccctcg aggaggattt tactccctct 120
   gtaccatctc ctctcaatcc agacgtccga tcaacaccga tctgaccaaa agccgtgatt 180
   ggtggtggca cgaggaggtc gagaagtggc ctgcctactt cccccccgag cgtgtcaact 240
   ccgaggaccc cttgttcctc ctctacacct ccggctctac cggtaagccc aaaggtgtca 300
   tgcacaccac cggagatgca tgggcgactt acaccctcaa gacgaggtcc tgcgccgttc 360
   cctcgaggac cccgaggggt tctgggccca ccaggccgag catctgcact ggcacaagaa 420
   accctcgtcc gcattgaaag tctccaaaaa gacgctcaag ggcggcatca gtgcatgcag 480
   atctccgaac tcttctccat cctggctgtc gtcggcgttg ccgccgccct ccctcaggct 540
   accccgacca cgcccactcc caccagcgcg tcggccgcgg ccacaatttc ggccacctcc 600
   tcgccgacct gctcccccgg cccggtcata agcatgcagt tctccatctt caccgtcctt 660
   gccgctgctg ccagcttcgc tgttgctctc cctgtctgtg acgccactac cacggccact 720
   actaccagcg ccgctgcgaa cccctcccct actaccagcg gtgctgcgaa cccctcccct 780
   acttgctcca atggacgacc agggtagctt gaccgacgga gacagcatcc tcaactccta 840
   ccatctccct cggcccatgc ctatctggct caactccaac tatggcaagc atattgtcaa 900
   gggcaacttc atgaccctca gcgcgagacc caagacggtc gagcagg 947

<210> 85
<211> 947
<212> DNA
<213> artificial

<220>
<223>  Sequence of the second arm of the dsRNA used to concomitantly
       target CclA/ ACS1/ ACS2/ ELP1/ ELP2/ MOB2 genes from
       Colletotrichum higginsianum

<400>  85
cctgctcgac cgtcttgggt ctcgcgctga gggtcatgaa gttgcccttg acaatatgct      60

tgccatagtt ggagttgagc cagataggca tgggccgagg gagatggtag gagttgagga     120

tgctgtctcc gtcggtcaag ctaccctggt cgtccattgg agcaagtagg ggaggggttc     180

gcagcaccgc tggtagtagg ggaggggttc gcagcggcgc tggtagtagt ggccgtggta     240

gtggcgtcac agacagggag agcaacagcg aagctggcag cagcggcaag gacggtgaag     300

atggagaact gcatgcttat gaccgggccg ggggagcagg tcggcgagga ggtggccgaa     360

attgtggccg cggccgacgc gctggtggga gtgggcgtgg tcggggtagc ctgagggagg     420

gcggcggcaa cgccgacgac agccaggatg gagaagagtt cggagatctg catgcactga     480

tgccgccctt gagcgtcttt ttggagactt tcaatgcgga cgagggtttc ttgtgccagt     540

gcagatgctc ggcctggtgg gcccagaacc cctcggggtc ctcgagggaa cggcgcagga     600

cctcgtcttg agggtgtaag tcgcccatgc atctccggtg gtgtgcatga cacctttggg     660

cttaccggta gagccggagg tgtagaggag gaacaagggg tcctcggagt tgacacgctc     720

gggggggaag taggcaggcc acttctcgac ctcctcgtgc caccaccaat cacggctttt     780

ggtcagatcg gtgttgatcg gacgtctgga ttgagaggag atggtacaga gggagtaaaa     840

tcctcctcga gggcggcgcg cttcaacggg atcgcggttg aggcaggaag ggaaccgctc     900

agagtcggct cccaaggagg agggggagta cccgagtcgg acgccat                    947

<210>  86
<211>  792
<212>  DNA
<213>  artificial

<220>
<223>  Sequence of the first arm of the CclA/ ACS1/ ACS2 dsRNA used to
       concomitantly target CclA/ ACS1/ ACS2 genes from Colletotrichum
       higginsianum

<400>  86
atggcgtccg actcgggtac tccccctcct ccttgggagc cgactctgag cggttccctt      60

cctgcctcaa ccgcgatccc gttgaagcgc gccgccctcg aggaggattt tactccctct     120

gtaccatctc ctctcaatcc agacgtccga tcaacaccga aacctcaagg cgtcgacgac     180

atgcctgcta agcgcaacaa aaaggagtcg ctgaagaagc gcgagtccaa gggcgctttc     240

ggcggcgaca gcaaccgatc atgaccaaaa gccgtgattg gtggtggcac gaggaggtcg     300

agaagtggcc tgcctacttc ccccccgagc gtgtcaactc cgaggacccc ttgttcctcc      360

tctacacctc cggctctacc ggtaagccca aaggtgtcat gcacaccacc ggaggctacc 420

tggtcggcgc cgccgccacc ggcaagtacg ttttcgacat ccacgacggc gaccgctact 480

tttgtggtgg tgacgttggc tggattaccg gtcacaccta cgttatgcat gggcgactta 540

caccctcaag acgaggtcct gcgccgttcc ctcgaggacc ccgaggggtt ctgggcccac 600

caggccgagc atctgcactg gcacaagaaa ccctcgtccg cattgaaagt ctccaaaaag 660

acgctcaagg gcggcatcac acacgacaca tgggaatggt tcccggacgg cgagatctcg 720

acctgcttca actgcgtcga ccgccacgtc cacgacggca aaggcgatgc cccggcgatc 780

ttctacgaca gc 792

<210> 87
<211> 792
<212> DNA
<213> artificial

<220>
<223> Sequence of the second arm of the CclA/ ACS1/ ACS2 dsRNA used to
 concomitantly target CclA/ ACS1/ ACS2 genes from Colletotrichum
 higginsianum

<400> 87
gctgtcgtag aagatcgccg gggcatcgcc tttgccgtcg tggacgtggc ggtcgacgca 60

gttgaagcag gtcgagatct cgccgtccgg gaaccattcc catgtgtcgt gtgtgatgcc 120

gcccttgagc gtctttttgg agactttcaa tgcggacgag ggtttcttgt gccagtgcag 180

atgctcggcc tggtgggccc agaacccctc ggggtcctcg agggaacggc gcaggacctc 240

gtcttgaggg tgtaagtcgc ccatgcataa cgtaggtgtg accggtaatc cagccaacgt 300

caccaccaca aaagtagcgg tcgccgtcgt ggatgtcgaa aacgtacttg ccggtggcgg 360

cggcgccgac caggtagcct ccggtggtgt gcatgacacc tttgggctta ccggtagagc 420

cggaggtgta gaggaggaac aaggggtcct cggagttgac acgctcgggg gggaagtagg 480

caggccactt ctcgacctcc tcgtgccacc accaatcacg gcttttggtc atgatcggtt 540

gctgtcgccg ccgaaagcgc ccttggactc gcgcttcttc agcgactcct ttttgttgcg 600

cttagcaggc atgtcgtcga cgccttgagg tttcggtgtt gatcggacgt ctggattgag 660

aggagatggt acagagggag taaaatcctc ctcgaggcgg gcgcgcttca acgggatcgc 720

ggttgaggca ggaagggaac cgctcagagt cggctcccaa ggaggagggg gagtacccga 780

gtcggacgcc at 792

<210> 88
<211> 792
<212> DNA
<213> artificial

<220>

<223>
      Sequence of the first arm of the  ELP1/ ELP2/ MOB2 dsRNA used to
      concomitantly target ELP1/ ELP2/ MOB2 genes from Colletotrichum
      higginsianum

<400>  88
atgcagatct ccgaactctt ctccatcctg gctgtcgtcg gcgttgccgc cgccctccct      60

caggctaccc cgaccacgcc cactcccacc agcgcgtcgg ccgcggccac aatttcggcc     120

acctcctcgc cgacctgctc ccccggcccg gtcattgact acaccgtcgt ctcgggcgac     180

accctgacca tcatctcgca gagattcagc tccggcatct gcaacatcgc cgacgcgagc     240

ggcatcacca accccaactg atcatgcagt tctccatctt caccgtcctt gccgctgctg     300

ccagcttcgc tgttgctctc cctgtctgtg acgccactac cacggccact actaccagcg     360

ccgctgcgaa cccctcccct actaccagcg gtgctgcgaa cccctcccct acttgcggca     420

agcttggaga tttccacaag accaccgtta aggctggcca gaccttgacg accatcgccg     480

agcgcttcca ctccggtatc tgtgacatcg cctggcagaa tgcatggacg accagggtag     540

cttgaccgac ggagacagca tcctcaactc ctaccatctc cctcggccca tgcctatctg     600

gctcaactcc aactatggca agcatattgt caagggcaac ttcatgaccc tcagcgcgag     660

acccaagacg gtcgagcagg gcgagtggat cgctcaccag gtcgtcgagc actacagaaa     720

cctctggaat tttgttcgtg ttctccacga gaaggaggag gatggctcga ctatctgcaa     780

tgccaccacc tg                                                         792


<210>  89
<211>  792
<212>  DNA
<213>  artificial

<220>
<223>  Sequence of the second arm of the ELP1/ ELP2/ MOB2 dsRNA used to
      concomitantly target ELP1, ELP2, and MOB2 genes from
      Colletotrichum higginsianum

<400>  89
caggtggtgg cattgcagat agtcgagcca tcctcctcct tctcgtggag aacacgaaca      60

aaattccaga ggtttctgta gtgctcgacg acctggtgag cgatccactc gccctgctcg     120

accgtcttgg gtctcgcgct gagggtcatg aagttgccct tgacaatatg cttgccatag     180

ttggagttga gccagatagg catgggccga gggagatggt aggagttgag gatgctgtct     240

ccgtcggtca agctaccctg gtcgtccatg cattctgcca ggcgatgtca cagataccgg     300

agtggaagcg ctcggcgatg gtcgtcaagg tctggccagc cttaacggtg gtcttgtgga     360

aatctccaag cttgccgcaa gtaggggagg ggttcgcagc accgctggta gtaggggagg     420

ggttcgcagc ggcgctggta gtagtggccg tggtagtggc gtcacagaca gggagagcaa     480

cagcgaagct ggcagcagcg gcaaggacgg tgaagatgga gaactgcatg atcagttggg     540

gttggtgatg ccgctcgcgt cggcgatgtt gcagatgccg gagctgaatc tctgcgagat 600

gatggtcagg gtgtcgcccg agacgacggt gtagtcaatg accgggccgg gggagcaggt 660

cggcgaggag gtggccgaaa ttgtggccgc ggccgacgcg ctggtgggag tgggcgtggt 720

cggggtagcc tgagggaggg cggcggcaac gccgacgaca gccaggatgg agaagagttc 780

ggagatctgc at 792

<210> 90
<211> 500
<212> DNA
<213> artificial

<220>
<223> Sequence of the first arm of the CFA6/HRPL dsRNA used to
 concomitantly target HrpL and Cfa6 genes of Pto DC3000

<400> 90
atgaacaagc gctacggcaa caacttgccg ggatcctgtt gcgcgctgag caaccggatc 60

gactggacgt caccttcgcg cagcacgcca tctggcaaaa aggccgccgt taatcgtttc 120

caatatttct gcaggagtct gcccgtgacc accttcaccg tgcccctgat tctgtcggat 180

gcaagcccca cggcactcgc cgctaccgcg caacgtctgc gcgccgagca cgagacgcgc 240

tccttcgccg ctgatgactg acatcaccgt gcccttccag ctcggaatgc acttcgtctt 300

cccagctttc ctgatacggc tgacgataca ttttgcggaa gtgattgcgg atcaggttca 360

gcgcgatgcc acacagccag gtctgcggtt tgctggcatg ttgaaacttg tgctcgttac 420

gcagggcttc aagaaacacg cactggagaa tgtcatccac atcatcaggg ttcatcaccc 480

gcttttggat aaacgccctg 500

<210> 91
<211> 500
<212> DNA
<213> artificial

<220>
<223> Sequence of the second arm of the CFA6/HRPL dsRNA used to
 concomitantly target HrpL and Cfa6 genes of Pto DC3000

<400> 91
cagggcgttt atccaaaagc gggtgatgaa ccctgatgat gtggatgaca ttctccagtg 60

cgtgtttctt gaagccctgc gtaacgagca caagtttcaa catgccagca aaccgcagac 120

ctggctgtgt ggcatcgcgc tgaacctgat ccgcaatcac ttccgcaaaa tgtatcgtca 180

gccgtatcag gaaagctggg aagacgaagt gcattccgag ctggaagggc acggtgatgt 240

cagtcatcag cggcgaagga gcgcgtctcg tgctcggcgc gcagacgttg cgcggtagcg 300

gcgagtgccg tggggcttgc atccgacaga atcaggggca cggtgaaggt ggtcacgggc 360

agactcctgc agaaatattg gaaacgatta acggcggcct ttttgccaga tggcgtgctg 420

cgcgaaggtg acgtccagtc gatccggttg ctcagcgcgc aacaggatcc cggcaagttg 480

ttgccgtagc gcttgttcat 500

<210> 92
<211> 348
<212> DNA
<213> artificial

<220>
<223> Sequence of the first arm of the HRPL dsRNA used to target the
HrpL gene of Pto DC3000

<400> 92
ctgatgactg acatcaccgt gcccttccag ctcggaatgc acttcgtctt cccagctttc 60

ctgatacggc tgacgataca ttttgcggaa gtgattgcgg atcaggttca gcgcgatgcc 120

acacagccag gtctgcggtt tgctggcatg ttgaaacttg tgctcgttac gcagggcttc 180

aagaaacacg cactggagaa tgtcatccac atcatcaggg ttcatcaccc gcttttggat 240

aaacgccctg agcatctgaa tctgatcggc cgtcatttgg cgaataccgg cggacgaaga 300

tggctgacgg ggctgggttg agtcgaggat cacaatcttc tgaaacat 348

<210> 93
<211> 348
<212> DNA
<213> artificial

<220>
<223> Sequence of the second arm of the HRPL dsRNA used to target the
HrpL gene of Pto DC3000

<400> 93
atgtttcaga agattgtgat cctcgactca acccagcccc gtcagccatc ttcgtccgcc 60

ggtattcgcc aaatgacggc cgatcagatt cagatgctca gggcgtttat ccaaaagcgg 120

gtgatgaacc ctgatgatgt ggatgacatt ctccagtgcg tgtttcttga agccctgcgt 180

aacgagcaca agtttcaaca tgccagcaaa ccgcagacct ggctgtgtgg catcgcgctg 240

aacctgatcc gcaatcactt ccgcaaaatg tatcgtcagc cgtatcagga aagctgggaa 300

gacgaagtgc attccgagct ggaagggcac ggtgatgtca gtcatcag 348

<210> 94
<211> 400
<212> DNA
<213> artificial

<220>
<223> Sequence of the first arm of the AvrPto/AvrPtoB dsRNA used to
target the AvrPto and AvrPtoB genes of Pto DC3000

<400> 94

atgggaaata tatgtgtcgg cggatccagg atggcccatc aggtgaactc cccagaccga 60

gttagtaaca actcgggtga cgaagataac gtaacgtcca gtcaactgct gagcgtcaga 120

catcaacttg cggagtctgc tggtgtacca agagatcagc atgaatttgt tagtaaccaa 180

gcacctcaaa gcctgagaaa atggcgggta tcaatagagc gggaccatcg ggcgcttatt 240

ttgttggcca cacagacccc gagccagtat cggggcaagc acacggatcc ggcagcggcg 300

ccagctcctc gaacagtccg caggttcagc cgcgaccctc gaatactccc ccgtcgaacg 360

cgcccgcacc gccgccaacc ggacgtgaga ggctttcacg 400

<210> 95
<211> 400
<212> DNA
<213> artificial

<220>
<223> Sequence of the second arm of the AvrPto/AvrPtoB dsRNA used to
      target the AvrPto and AvrPtoB genes of Pto DC3000

<400> 95
cgtgaaagcc tctcacgtcc ggttggcggc ggtgcgggcg cgttcgacgg gggagtattc 60

gagggtcgcg gctgaacctg cggactgttc gaggagctgg cgccgctgcc ggatccgtgt 120

gcttgccccg atactggctc ggggtctgtg tggccaacaa aataagcgcc cgatggtccc 180

gctctattga tacccgccat tttctcaggc tttgaggtgc ttggttacta acaaattcat 240

gctgatctct tggtacacca gcagactccg caagttgatg tctgacgctc agcagttgac 300

tggacgttac gttatcttcg tcacccgagt tgttactaac tcggtctggg gagttcacct 360

gatgggccat cctggatccg ccgacacata tatttcccat 400

<210> 96
<211> 752
<212> DNA
<213> artificial

<220>
<223> Sequence of the first arm of the CYP51 dsRNA used to target the
      FgCYP51A, FgCYP51B and FgCYP51C genes of Fusarium graminearum

<400> 96
cagcaagttt gacgagtccc tggccgctct ctaccacgac ctcgatatgg gcttcacccc 60

catcaacttc atgcttcact gggcccctct cccctggaac cgtaagcgcg accacgccca 120

gcgcactgtt gccaagatct acatggacac tatcaaggag cgccgcgcca agggcaacaa 180

cgaatccgag catgacatga tgaagcacct tatgaactct cggtccattg acaatccccg 240

tctttggtag cgatgtcgta tacgattgtc ccaactcgaa gctcatggaa caaaagaagt 300

ttgtcaagtt tggccttacg caaaaagcac tcgagtcaca cgtccagtta atcgagcgag 360

aggttcttga ctacgtcgaa actgatccat ccttttctgg cagaactagc accatcgatg 420

tccccaaggc aatggctgag ataacaatct ttactgcctc acgttctttg cagggtgagg 480

aagttcggag aaaactcact gccgagtttg ctgcattgga agcaccgtac aatatggcat 540

cgacccgtac gcttttttct tcgactgcag agataaatac ggcgactgct ttacctttat 600

tctccttggc aaatcaacga ctgtctttct tggtcccaag ggcaatgact ttatcctcaa 660

cggcaaacac gccgatctca acgccgagga cgtttatggg aaacttacca cgcccgtgtt 720

tggtgaggag gttgtttatg actgctccaa tg 752

<210> 97
<211> 752
<212> DNA
<213> artificial

<220>
<223> Sequence of the second arm of the CYP51 dsRNA used to target the
      FgCYP51A, FgCYP51B and FgCYP51C genes of Fusarium graminearum

<400> 97
cattggagca gtcataaaca acctcctcac caaacacggg cgtggtaagt ttcccataaa 60

cgtcctcggc gttgagatcg gcgtgtttgc cgttgaggat aaagtcattg cccttgggac 120

caagaaagac agtcgttgat ttgccaagga gaataaaggt aaagcagtcg ccgtatttat 180

ctctgcagtc gaagaaaaaa gcgtacgggt cgatgccata ttgtacggtg cttccaatgc 240

agcaaactcg gcagtgagtt ttctccgaac ttcctcaccc tgcaaagaac gtgaggcagt 300

aaagattgtt atctcagcca ttgccttggg gacatcgatg gtgctagttc tgccagaaaa 360

ggatggatca gtttcgacgt agtcaagaac ctctcgctcg attaactgga cgtgtgactc 420

gagtgctttt tgcgtaaggc caaacttgac aaacttcttt tgttccatga gcttcgagtt 480

gggacaatcg tatacgacat cgctaccaaa gacggggatt gtcaatggac cgagagttca 540

taaggtgctt catcatgtca tgctcggatt cgttgttgcc cttggcgcgg cgctccttga 600

tagtgtccat gtagatcttg gcaacagtgc gctgggcgtg gtcgcgctta cggttccagg 660

ggagaggggc ccagtgaagc atgaagttga tgggggtgaa gcccatatcg aggtcgtggt 720

agagagcggc cagggactcg tcaaacttgc tg 752

<210> 98
<211> 667
<212> DNA
<213> artificial

<220>
<223> Sequence of the first arm of the HRPG/HRPB/HRCC dsRNA used to
      target concomitantly the HrpG, HrpB and HrcC genes of Ralstonia
      solanacearum

<400> 98
cgcatgcctg gccgtgcaga acgtggaatg tgtccggttc gacgatgccc tgacgctgtt 60

gcgtgcacgg cgtaccgagg cattcagtct gctgctgatc gatgcccagc agttccgcag 120

cgcgggacag ctggtgctgt cctggcgcga gtgcaacgcc gacatgtgct ggccgacgct 180

ggtgttcggc cagttcgcag tgaagaagcg gaagaaggct tccgcctggc gcagcgcctg 240

atccgccatt cggatgacgc gcgatcgcta cgccgcggcg gcgagctgct tctcgcgcat 300

ggccgaagac gatggcgcga cctggaccca gcaggtcgag ggcctgatcg gcccgcggct 360

ggctggccac catcgatctg atcatctacg ttttcgccgt gcaggccggc atccgctgct 420

ccaaccgcct gctcgagcat gcgttctggc aatcggccga aatgggcatc tcgacgcagg 480

tcgagggcag cgtgacgggc tcgttcaacg agacgccgca gaagtttctc gaccgcatgg 540

ccggcacgtt cggctttgcg tggtactacg acggcgcggt gctgcgcgtg accagcgcca 600

acgaagcgca gagcgccacc atcgcgctga cccgcgcctc gaccgcgcag gtcaagcggg 660

cgctgac 667

<210> 99
<211> 667
<212> DNA
<213> artificial

<220>
<223> Sequence of the second arm of the HRPG/HRPB/HRCC dsRNA used to
      target concomitantly the HrpG, HrpB and HrcC genes of Ralstonia
      solanacearum

<400> 99
gtcagcgccc gcttgacctg cgcggtcgag gcgcgggtca gcgcgatggt ggcgctctgc 60

gcttcgttgg cgctggtcac gcgcagcacc gcgccgtcgt agtaccacgc aaagccgaac 120

gtgccggcca tgcggtcgag aaacttctgc ggcgtctcgt tgaacgagcc cgtcacgctg 180

ccctcgacct gcgtcgagat gcccatttcg gccgattgcc agaacgcatg ctcgagcagg 240

cggttggagc agcggatgcc ggcctgcacg gcgaaaacgt agatgatcag atcgatggtg 300

gccagccagc cgcgggccga tcaggccctc gacctgctgg gtccaggtcg cgccatcgtc 360

ttcggccatg cgcgagaagc agctcgccgc cgcggcgtag cgatcgcgcg tcatccgaat 420

ggcggatcag gcgctgcgcc aggcggaagc cttcttccgc ttcttcactg cgaactggcc 480

gaacaccagc gtcggccagc acatgtcggc gttgcactcg cgccaggaca gcaccagctg 540

tcccgcgctg cggaactgct gggcatcgat cagcagcaga ctgaatgcct cggtacgccg 600

tgcacgcaac agcgtcaggg catcgtcgaa ccggacacat tccacgttct gcacggccag 660

gcatgcg 667

<210> 100
<211> 705
<212> DNA

<213>  artificial

<220>
<223>  Sequence of the first arm of the HRPB/HRCC/TssB/XpsR dsRNA used
       to target concomitantly the HrpB, HrcC, XpsR and TssB genes of
       Ralstonia solanacearum

<400>  100
gcgcgatcgc tacgccgcgg cggcgagctg cttctcgcgc atggccgaag acgatggcgc      60

gacctggacc cagcaggtcg agggcctgat cggcccgcgg ctggctggcc accatcgatc     120

tgatcatcta cgttttcgcc gtgcaggccg gcatccgctg ctccaaccgc ctgctcgagc     180

atgcgttctg gcaatcggcc gaaatgggca tctcgacgca ggtcgagggc agcgtgacgg     240

gctcgttcaa cgagacgccg cagaagtttc tcgaccgcat ggccggcacg ttcggctttg     300

cgtggtacta cgacggcgcg gtgctgcgcg tgaccagcgc caacgaagcg cagagcgcca     360

ccatcgcgct gacccgcgcc ggccgcgcgt gcggagaccc atcggcggcc gcagagcgaa     420

tgcgactgga agcattactt tgcggacctg ctctatgcgc cgaacggcgc cgagttcaag     480

ctcagcctgt ttccgctgcc cgcgcagcag atcggcgaca cgccctggtc gcgggcattc     540

cgcgggcagc cggcgatgct tgacaaccaa tccgccgcgc tcgcgcagag cgcaccggca     600

gccgagtccg tcaacctgct cgacgagatc gtcgagcaga gccgcgtcgc caagtcggac     660

gccgagcacg cgcgcgccaa ggacatcatc ggcgagctgg tcaac                     705


<210>  101
<211>  705
<212>  DNA
<213>  artificial

<220>
<223>  Sequence of the second arm of the HRPB/HRCC/TssB/XpsR dsRNA used
       to target concomitantly the HrpB, HrcC, XpsR and TssB genes of
       Ralstonia solanacearum

<400>  101
gttgaccagc tcgccgatga tgtccttggc gcgcgcgtgc tcggcgtccg acttggcgac      60

gcggctctgc tcgacgatct cgtcgagcag gttgacggac tcggctgccg gtgcgctctg     120

cgcgagcgcg gcggattggt tgtcaagcat cgccggctgc ccgcggaatg cccgcgacca     180

gggcgtgtcg ccgatctgct gcgcgggcag cggaaacagg ctgagcttga actcggcgcc     240

gttcggcgca tagagcaggt ccgcaaagta atgcttccag tcgcattcgc tctgcggccg     300

ccgatgggtc tccgcacgcg cggccggcgc gggtcagcgc gatggtggcg ctctgcgctt     360

cgttggcgct ggtcacgcgc agcaccgcgc cgtcgtagta ccacgcaaag ccgaacgtgc     420

cggccatgcg gtcgagaaac ttctgcggcg tctcgttgaa cgagcccgtc acgctgccct     480

cgacctgcgt cgagatgccc atttcggccg attgccagaa cgcatgctcg agcaggcggt     540

tggagcagcg gatgccggcc tgcacggcga aaacgtagat gatcagatcg atggtggcca     600

gccagccgcg ggccgatcag gccctcgacc tgctgggtcc aggtcgcgcc atcgtcttcg 660

gccatgcgcg agaagcagct cgccgccgcg gcgtagcgat cgcgc 705

<210> 102
<211> 633
<212> DNA
<213> artificial

<220>
<223> Sequence of the first arm of the HRPG/HRPX/RsmA dsRNA used to
 target concomitantly the HrpG, HrpX, and RsmA genes of
 Xanthomonas campestris pv. campestris

<400> 102
gtgatggacg ccgctgcgaa tgaccgccaa ggatcggcat tcgtactgac gcaggacgcg 60

cggctggcat cacagatcaa cgccagcctg gcgactctgg tcccagaggt caccatcttt 120

tcagacgaac tcgaattgct gcgctgcctg cgccactcgc cgtgcgagct tctggttttc 180

gatgcccatt gcgtggcatc ggacgacagt atgatccttt cgacctactt cgcagcgatc 240

tctgcgttgt cttacgcaga ccgtcttccg atctatacga gcaggatgct tgttggtgct 300

tggccacagg gtctgcagca cctccagcaa cgtcgcgagg ggcaggcagc tccggatggc 360

agcgacgacg aagtcgcatt gctgggcgcc agcgccgatg ccttgttgat tctggagtat 420

atgttgatcc tcactcgccg agtaggcgaa accctgatga tcggcgactt ggtcaccgtg 480

accgtgctcg gcgtcaaggg aaatcaggtg cgcattggca ttgacgcgcc taaggatgtt 540

gccgtgcatc gcgaagaaat ctatcagcgc atccagcgcg gtgacgagcc ggttgcgtcc 600

ggtgcgcatc acaacgacga ttgttcggac tga 633

<210> 103
<211> 633
<212> DNA
<213> artificial

<220>
<223> Sequence of the second arm of the HRPG/HRPX/RsmA dsRNA used to
 target concomitantly the HrpG, HrpX, and RsmA genes of
 Xanthomonas campestris pv. campestris

<400> 103
tcagtccgaa caatcgtcgt tgtgatgcgc accggacgca accggctcgt caccgcgctg 60

gatgcgctga tagatttctt cgcgatgcac ggcaacatcc ttaggcgcgt caatgccaat 120

gcgcacctga tttcccttga cgccgagcac ggtcacggtg accaagtcgc cgatcatcag 180

ggtttcgcct actcggcgag tgaggatcaa catatactcc agaatcaaca aggcatcggc 240

gctggcgccc agcaatgcga cttcgtcgtc gctgccatcc ggagctgcct gcccctcgcg 300

acgttgctgg aggtgctgca gaccctgtgg ccaagcacca acaagcatcc tgctcgtata 360

gatcggaaga cggtctgcgt aagacaacgc agagatcgct gcgaagtagg tcgaaaggat      420

catactgtcg tccgatgcca cgcaatgggc atcgaaaacc agaagctcgc acggcgagtg      480

gcgcaggcag cgcagcaatt cgagttcgtc tgaaaagatg gtgacctctg ggaccagagt      540

cgccaggctg gcgttgatct gtgatgccag ccgcgcgtcc tgcgtcagta cgaatgccga      600

tccttggcgg tcattcgcag cggcgtccat cac                                   633

<210>  104
<211>  718
<212>  DNA
<213>  artificial

<220>
<223>  Sequence of the first arm of the NadHb/NadHd/NadHe dsRNA used to
       target concomitantly the NadHb, NadHd and NadHe genes of
       Xanthomonas campestris pv. campestris (and likely other
       Xanthomonas species)

<400>  104
aacagaagaa gggagcgctg gaatgggagt gattcagacc ctggatcgtc tgatgaccaa       60

cccgatgccg gaaggccggg tcgaagacat cctgcgcccg gaaggcgaaa acccgttgct      120

cgaaaagggc tacgtgacca ccagcgtcga tgcgctgttg aactgggcgc gtacaggttc      180

gatgtggccg atgacctttg gtctggcctg ctgtgcagtc gagatgatgc agatcgtgag      240

tgagtaccgc caggcaaccg atgccttcgc gagcaatcct gtggaaagca agcaggaaat      300

ccgcaattac acgatgaact tcggcccgca gcatcctgcg gcgcacggcg tattgcgcct      360

gatcctggaa atggacggcg aaaccgtggt gcgtgccgac ccgcatatcg gcctgctgca      420

ccgtggcacc gagaagctgg ccgagtccaa gccgttcaac cagtcggttc cgtacagcat      480

cgacgggtaa tttcgaagcg gcgcgcgacg tcgatccgca ggtagtgctg agcgacaaga      540

cgcgcgcgca catcgatcat tggctgagca agttcccgcc cgaccgcaag cgttcggccg      600

tgttgcaggg tctgcatgcc gcgcaggaac agaaccaggg ttggttgacc gacgagctga      660

tcgtgggcgt ggccaagtat ctggagctgc cgccggtgtg ggcctacgaa gtggggct        718

<210>  105
<211>  718
<212>  DNA
<213>  artificial

<220>
<223>  Sequence of the second arm of the NadHb/NadHd/NadHe dsRNA used to
       target concomitantly the NadHb, NadHd and NadHe genes of
       Xanthomonas campestris pv. campestris (and likely other
       Xanthomonas species).

<400>  105
tcagccactt cgtaggccca caccggcggc agctccagat acttggccac gcccacgatc       60

agctcgtcgg tcaaccaacc ctggttctgt tcctgcgcgg catgcagacc ctgcaacacg      120

gccgaacgct tgcggtcggg cgggaacttg ctcagccaat gatcgatgtg cgcgcgcgtc 180

ttgtcgctca gcactacctg cggatcgacg tcgcgcgccg cttcgaaatt acccgtcgat 240

gctgtacgga accgactggt tgaacggctt ggactcggcc agcttctcgg tgccacggtg 300

cagcaggccg atatgcgggt cggcacgcac cacggtttcg ccgtccattt ccaggatcag 360

gcgcaatacg ccgtgcgccg caggatgctg cgggccgaag ttcatcgtgt aattgcggat 420

ttcctgcttg ctttccacag gattgctcgc gaaggcatcg gttgcctggc ggtactcact 480

cacgatctgc atcatctcga ctgcacagca ggccagacca aaggtcatcg gccacatcga 540

acctgtacgc gcccagttca acagcgcatc gacgctggtg gtcacgtagc ccttttcgag 600

caacgggttt tcgccttccg ggcgcaggat gtcttcgacc cggccttccg gcatcgggtt 660

ggtcatcaga cgatccaggg tctgaatcac tcccattcca gcgctccctt cttcctgc 718

<210> 106
<211> 677
<212> DNA
<213> artificial

<220>
<223> Sequence of the first arm of the DnaA/DnaE1/DnaE2 dsRNA used to
      target concomitantly the DnaA, DnaE1 and DnaE2 genes of
      Xanthomonas campestris pv. campestris (and likely other species)

<400> 106
aacaatgctt ggccccgctg tctggaacgt ctcgaagctg aattcccgcc cgaggatgtc 60

cacacctggt tgaaacccct gcaggccgaa gatcgcggcg acagcatcgt gctgtacgcg 120

ccgaacgcct tcattgttga gcaggtccgc gagcgatacc tgccgcgcat ccgcgagttg 180

ctggcatatt tcgccggcaa tggcgaggtg gcgctggcgg tcggctcccg tcgatcgcgc 240

gaagccttcc tcaagccgtt ggcccaggtg gtcaatgtcg tcgaacggcg tcagacattg 300

ccggtactgg cgaacttgct ggtgcaggtg aacaacggcc agctgtcgct gacggggacc 360

gacctggaag tcgaaatgat ctcgcgcacc atggtcgagg acgcccagga cggcgaaacc 420

acgatcccgg cgcgcaagct gttcgacatc ctggcatatg tccacttccc gcttcgtcca 480

tctgcacgtc cacaccgagt tctcgttggc ggattccacc atccgcgtgc ccgagaaacc 540

ggatcaggct gacccgaaaa aagccaagca ggccaacctg ctgagccgcg cggtcgaact 600

cgacttgccc gcgctggcgg tcaccgacct gaacaacctg ttcgccctgg tcaagttcta 660

caaggccgcc gaaggct 677

<210> 107
<211> 677
<212> DNA
<213> artificial

<220>
<223>  Sequence of the second arm of the DnaA/DnaE1/DnaE2 dsRNA used to
      target concomitantly the DnaA, DnaE1 and DnaE2 genes of
      Xanthomonas campestris pv. campestris (and likely other
      Xanthomonas species)

<400>  107
tcagttcggc ggccttgtag aacttgacca gggcgaacag gttgttcagg tcggtgaccg      60

ccagcgcggg caagtcgagt tcgaccgcgc ggctcagcag gttggcctgc ttggcttttt     120

tcgggtcagc ctgatccggt ttctcgggca cgcggatggt ggaatccgcc aacgagaact     180

cggtgtggac gtgcagatgg acgaagcggg aagtggacat atgccaggat gtcgaacagc     240

ttgcgcgccg ggatcgtggt ttcgccgtcc tgggcgtcct cgaccatggt gcgcgagatc     300

atttcgactt ccaggtcggt ccccgtcagc gacagctggc cgttgttcac ctgcaccagc     360

aagttcgcca gtaccggcaa tgtctgacgc cgttcgacga cattgaccac ctgggccaac     420

ggcttgagga aggcttcgcg cgatcgacgg gagccgaccg ccagcgccac ctcgccattg     480

ccggcgaaat atgccagcaa ctcgcggatg cgcggcaggt atcgctcgcg gacctgctca     540

acaatgaagg cgttcggcgc gtacagcacg atgctgtcgc cgcgatcttc ggcctgcagg     600

ggtttcaacc aggtgtggac atcctcgggc gggaattcag cttcgagacg ttccagacag     660

cggggccaag catctgc                                                    677


<210>  108
<211>  884
<212>  DNA
<213>  artificial

<220>
<223>  Sequence of the first arm of the Br1A2/stuA/F1bc/pks1/PPT1 dsRNA
      used to concomitantly target Br1A2/stuA/F1bc/pks1/PPT1 genes from
      zymoseptoria tritici

<400>  108
atgccaccac cgcagccacg atccgtgcag cccgaaacat tcctcctgga ccaggatgca      60

cagcagagct tgcctcaaga ttcagtggtc gcgctgcagc aggtggacaa tctgaaatac     120

tttctcatat ctgctcctgt ggactggaga caagactcgt actgcaggag agatcagttc     180

cttcgaccag accggacaag tcgcgccgca tggtatgaag cctagggtca ctgcgacctt     240

gtgggaagat gagggtagct tgtgcttcca ggtagaagcg aacggcgtgt gcgtcgcgcg     300

gcgcgaagac aaccacatga tcaatggcac gaaactgttg aacgatgcat ggcaaccatg     360

gccgtcgaca accgccaaca attcagcagc atgggtggct acgacgcaaa gttctacccg     420

cacccgccgc agttcacgaa tccttggacg tctggggctt ccaacccaag tcaaatgtac     480

gcgacatcca tgccacatac atcaggtggc agcgatgcgc gggtgcatgt cgaacatact     540

tctcttcggc gatcaaaccg ccgaacaata cccgctcctc catcgaatcg ttctccgaaa     600

ggacaatgtc ttgctgctca gctttgtcga gcgttgcgct cttgctctcc gcgaagagac 660

tcgcaccctg tcccgaagcc aacgcgaagt gatgccggac taagcatggc ccaacaactg 720

cagtcgctgt cagacagcct gccgagtgtc tcgactctgg tcaatggaat aaccaacgcc 780

ttccacaatg ccacatctct ggtcagtggc gctccgcacg ctggcgaaat tacctgctgg 840

ctgatcgata ctcgaaagct gtggcctgga accaaaatct ggga 884

<210> 109
<211> 884
<212> DNA
<213> artificial

<220>
<223> Sequence of the second arm of the Br1A2/stuA/F1bc/pks1PPT1 dsRNA
used to concomitantly target Br1A2/stuA/F1bc/pks1/PPT1 genes from
zymoseptoria tritici

<400> 109
tcccagattt tggttccagg ccacagcttt cgagtatcga tcagccagca ggtaatttcg 60

ccagcgtgcg gagcgccact gaccagagat gtggcattgt ggaaggcgtt ggttattcca 120

ttgaccagag tcgagacact cggcaggctg tctgacagcg actgcagttg ttgggccatg 180

cttagtccgg catcacttcg cgttggcttc gggacagggt gcgagtctct tcgcggagag 240

caagagcgca acgctcgaca aagctgagca gcaagacatt gtcctttcgg agaacgattc 300

gatggaggag cgggtattgt tcggcggttt gatcgccgaa gagaagtatg ttcgacatgc 360

acccgcgcat cgctgccacc tgatgtatgt ggcatggatg tcgcgtacat ttgacttggg 420

ttggaagccc cagacgtcca aggattcgtg aactgcggcg ggtgcgggta gaactttgcg 480

tcgtagccac ccatgctgct gaattgttgg cggttgtcga cggccatggt tgccatgcat 540

cgttcaacag tttcgtgcca ttgatcatgt ggttgtcttc gcgccgcgcg acgcacacgc 600

cgttcgcttc tacctggaag cacaagctac cctcatcttc ccacaaggtc gcagtgaccc 660

taggcttcat accatgcggc gcgacttgtc cggtctggtc gaaggaactg atctctcctg 720

cagtacgagt cttgtctcca gtccacagga gcagatatga gaaagtattt cagattgtcc 780

acctgctgca gcgcgaccac tgaatcttga ggcaagctct gctgtgcatc ctggtccagg 840

aggaatgttt cgggctgcac ggatcgtggc tgcggtggtg gcat 884

<210> 110
<211> 796
<212> DNA
<213> artificial

<220>
<223> Sequence of the first arm of the Br1A2/stuA/F1bc dsRNA used to
concomitantly target Br1A2/stuA/F1bc/ genes from zymoseptoria
tritici

<400>  110
atgccaccac cgcagccacg atccgtgcag cccgaaacat tcctcctgga ccaggatgca      60

cagcagagct tgcctcaaga ttcagtggtc gcgctgcagc aggtggacaa tctgaaatac     120

tttctcatat ctgctcctgt ggactggaga caagactcgt actgcaggag atttctcttg     180

ccaaccggcg aatacgtgtc ttgcgtgtta tggaacaatc tcttccacat ctccgggacc     240

gacattgtcc gatgcttgtc gttccgtttc gatcgttcct tcgaccagac cggacaagtc     300

gcgccgcatg gtatgaagcc taggtcactg gcgaccttgt gggaagatga gggtagcttg     360

tgcttccagg tagaagcgaa cggcgtgtgc gtcgcgcggc gcgaagacaa ccacatgatc     420

aatggcacga aactgttgaa cgttgccggg atgacacgcg gtcggcgcga tggcatcttg     480

aagagcgaga agactcgcca tgtggtcaag attggtccga tgcacctcaa aggtgatgct     540

ggcaaccatg gccgtcgaca accgccaaca attcagcagc atgggtggct acgacgcaaa     600

gttctacccg cacccgccgc agttcacgaa tccttggacg tctggggctt ccaacccaag     660

tcaaatgtac gcgacatcca tgccacatac atcaggtggc agcgatgcgc ggtacgtcca     720

ggcgcccaat gtttctgctg catacggtgg tagcatccca gctcccgcta tggcatcagg     780

tatagatcta ctggac                                                     796


<210>  111
<211>  796
<212>  DNA
<213>  artificial

<220>
<223>  Sequence of the second arm of the Br1A2/stuA/F1bc dsRNA used to
       concomitantly target Br1A2/stuA/F1bc/ genes from zymoseptoria
       tritici

<400>  111
gtccagtaga tctatacctg atgccatagc gggagctggg atgctaccac cgtatgcagc      60

agaaacattg ggcgcctgga cgtaccgcgc atcgctgcca cctgatgtat gtggcatgga     120

tgtcgcgtac atttgacttg ggttggaagc cccagacgtc caaggattcg tgaactgcgg     180

cgggtgcggg tagaactttg cgtcgtagcc acccatgctg ctgaattgtt ggcggttgtc     240

gacggccatg gttgccagca tcacctttga ggtgcatcgg accaatcttg accacatggc     300

gagtcttctc gctcttcaag atgccatcgc gccgaccgcg tgtcatcccg gcaacgttca     360

acagtttcgt gccattgatc atgtggttgt cttcgcgccg cgcgacgcac acgccgttcg     420

cttctacctg gaagcacaag ctaccctcat cttcccacaa ggtcgcagtg accctaggct     480

tcataccatg cggcgcgact tgtccggtct ggtcgaagga acgatcgaaa cggaacgaca     540

agcatcggac aatgtcggtc ccggagatgt ggaagagatt gttccataac acgcaagaca     600

cgtattcgcc ggttggcaag agaaatctcc tgcagtacga gtcttgtctc cagtccacag     660

gagcagatat gagaaagtat ttcagattgt ccacctgctg cagcgcgacc actgaatctt 720

gaggcaagct ctgctgtgca tcctggtcca ggaggaatgt ttcgggctgc acggatcgtg 780

gctgcggtgg tggcat 796

<210> 112
<211> 782
<212> DNA
<213> artificial

<220>
<223> Sequence of the first arm of the stuA/pks1/PPT1 dsRNA used to
concomitantly target stuA/ /pks1/PPT1 genes from zymoseptoria
tritici

<400> 112
agttccttcg accagaccgg acaagtcgcg ccgcatggta tgaagcctag ggtcactgcg 60

accttgtggg aagatgaggg tagcttgtgc ttccaggtag aagcgaacgg cgtgtgcgtc 120

gcgcggcgcg aagacaacca catgatcaat ggcacgaaac tgttgaacgt tgccgggatg 180

acacgcggtc ggcgcgatgg catcttgaag agcgagaaga ctcgccatgt ggtcaagatt 240

ggtccgatgc acctcaaaga tcatgtcgaa catacttctc ttcggcgatc aaaccgccga 300

acaatacccg ctcctccatc gaatcgttct ccgaaaggac aatgtcttgc tgctcagctt 360

tgtcgagcgt tgcgctcttg ctctccgcga agagactcgc accctgtccc gaagccaacg 420

cgaagtgatg ccggacttcc tcaccctcag caatctgatc gaggcatact accagaaggg 480

tgaaaaagtc ctcatgttgg aatcagcatt tcttacgatt gcatgctggc ccaacaactg 540

cagtcgctgt cagacagcct gccgagtgtc tcgactctgg tcaatggaat aaccaacgcc 600

ttccacaatg ccacatctct ggtcagtggc gctccgcacg ctggcgaaat tacctgctgg 660

ctgatcgata ctcgaaagct gtggcctgga accaaaatct gggatgctcc tggcgcagca 720

gaagccatgg gtttgatttc tgtggccgag cagaatgcca tctcgacgaa gatgttcatt 780

ca 782

<210> 113
<211> 782
<212> DNA
<213> artificial

<220>
<223> Sequence of the second arm of the stuA/pks1/PPT1 dsRNA used to
concomitantly target stuA/ /pks1/PPT1 genes from zymoseptoria
tritici

<400> 113
tgaatgaaca tcttcgtcga gatggcattc tgctcggcca cagaaatcaa acccatggct 60

tctgctgcgc caggagcatc ccagattttg gttccaggcc acagctttcg agtatcgatc 120

agccagcagg taatttcgcc agcgtgcgga gcgccactga ccagagatgt ggcattgtgg 180

aaggcgttgg ttattccatt gaccagagtc gagacactcg gcaggctgtc tgacagcgac 240

tgcagttgtt gggccagcat gcaatcgtaa gaaatgctga ttccaacatg aggacttttt 300

cacccttctg gtagtatgcc tcgatcagat tgctgagggt gaggaagtcc ggcatcactt 360

cgcgttggct tcgggacagg gtgcgagtct cttcgcggag agcaagagcg caacgctcga 420

caaagctgag cagcaagaca ttgtcctttc ggagaacgat tcgatggagg agcgggtatt 480

gttcggcggt ttgatcgccg aagagaagta tgttcgacat gatctttgag gtgcatcgga 540

ccaatcttga ccacatggcg agtcttctcg ctcttcaaga tgccatcgcg ccgaccgcgt 600

gtcatcccgg caacgttcaa cagtttcgtg ccattgatca tgtggttgtc ttcgcgccgc 660

gcgacgcaca cgccgttcgc ttctacctgg aagcacaagc taccctcatc ttcccacaag 720

gtcgcagtga ccctaggctt cataccatgc ggcgcgactt gtccggtctg gtcgaaggaa 780

ct 782

<210> 114
<211> 795
<212> DNA
<213> artificial

<220>
<223> Sequence of the first arm of the chitinase/ elicitin/glucanase
 inhibitor, transglutaminase elicitor/ secretory protein dsRNA
 used to concomitantly target genes producing the protein defined
 by PITG_17947, PITG_10772, PITG_13671, PITG_16956, PITG_00891

<400> 114
atgaaattcg tcggggttat cgcgtcaagt cttctggttg tgccttctgc ggtctctggt 60

gacgctgata gctcgagttt cgcgctcttc ttcgatcagg atcgtttcca ggaggttttc 120

ccggacgctg tggagctcta caacttcaac ggtcttgtgg gatcatggcc aagttcactc 180

tcgctgtttc gacgcttctt gtcgcgtcgg ctgccgtctc ggcccacgag gggcacgacc 240

actcgtcgtc gacccacgac gaacatgagc actcaggctc tgccgcttca ggcaccgttg 300

gttcggagtg ctcgatgccc atgaagatca tctctgctgt cgccgcctcc tcaattgcgc 360

ttggagccgt ctctgccact actgaccatg tcagccgaat gctggttctt ggtggagcag 420

ttgttcccag cggcacgaag acgtacacga ccggaatccg ccctaccatt gatggcggtg 480

ccgtcgagaa gattacaaag ccttcctccg ttccaggttg acgcgctatc ccttccggat 540

ctctagatcg actctctttg caacatggta ttctcgccca gcacctatct cgtctccgct 600

gccgtcgctg tcgtggcgct gcagatgcag caagcgtgga gcagcgactt gtttattcaa 660

caatgcaacc ttatttgcta gtagcgacat tactgggtgc tgccacgacg gcaaatgcaa 720

ttccagtgca cttcaacaat cactgcaatg ctgcaattga gctgtacaac aacacataca 780

tcgaggtgat caatc                                                    795

<210>  115
<211>  795
<212>  DNA
<213>  artificial

<220>
<223>  Sequence of the second arm of the chitinase/ elicitin/glucanase
       inhibitor, transglutaminase elicitor/ secretory protein dsRNA
       used to concomitantly target genes producing the protein defined
       by PITG_17947, PITG_10772, PITG_13671, PITG_16956, PITG_00891

<400>  115
gattgatcac ctcgatgtat gtgttgttgt acagctcaat tgcagcattg cagtgattgt      60

tgaagtgcac tggaattgca tttgccgtcg tggcagcacc cagtaatgtc gctactagca     120

aataaggttg cattgttgaa taaacaagtc gctgctccac gcttgctgca tctgcagcgc     180

cacgacagcg acggcagcgg agacgagata ggtgctgggc gagaatacca tgttgcaaag     240

agagtcgatc tagagatccg gaagggatag cgcgtcaacc tggaacggag gaaggctttg     300

taatcttctc gacggcaccg ccatcaatgg tagggcggat tccggtcgtg tacgtcttcg     360

tgccgctggg aacaactgct ccaccaagaa ccagcattcg gctgacatgg tcagtagtgg     420

cagagacggc tccaagcgca attgaggagg cggcgacagc agagatgatc ttcatgggca     480

tcgagcactc cgaaccaacg gtgcctgaag cggcagagcc tgagtgctca tgttcgtcgt     540

gggtcgacga cgagtggtcg tgcccctcgt gggccgagac ggcagccgac gcgacaagaa     600

gcgtcgaaac agcgagagtg aacttggcca tgatcccaca agaccgttga agttgtagag     660

ctccacagcg tccgggaaaa cctcctggaa acgatcctga tcgaagaaga gcgcgaaact     720

cgagctatca gcgtcaccag agaccgcaga aggcacaacc agaagacttg acgcgataac     780

cccgacgaat ttcat                                                    795

<210>  116
<211>  781
<212>  DNA
<213>  artificial

<220>
<223>  Sequence of the first arm of the chitinase/ elicitin/glucanase
       inhibitor, dsRNA used to concomitantly target genes producing the
       protein defined by PITG_17947, PITG_10772, PITG_13671 from
       plasmopora viticola

<400>  116
atgaaattcg tcggggttat cgcgtcaagt cttctggttg tgccttctgc ggtctctggt      60

gacgctgata gctcgagttt cgcgctcttc ttcgatcagg atcgtttcca ggaggttttc     120

ccggacgctg tggagctcta caacttcaac ggtcttgtgg atgcggccag caagtacagc     180

gaattcgcta atacgggcaa cgacgacaat gacaagcgtg agctggcagc gttcctggct 240

caaacagctc acgagtgcgg atcatggcca agttcactct cgctgtttcg acgcttcttg 300

tcgcgtcggc tgccgtctcg gcccacgagg ggcacgacca ctcgtcgtcg acccacgacg 360

aacatgagca ctcaggctct gccgcttcag gcaccgttgg ttcggagtgc tcggccgacg 420

tgagcgaggc gttcatcgcg actgtcgaca acagcaccta cttcgaaacg tgcgcagaag 480

gcatgacctt caacatcacg tcggtcttcg acgtactcaa atgcgccatg aagatcatct 540

ctgctgtcgc cgcctcctca attgcgcttg gagccgtctc tgccactact gaccatgtca 600

gccgaatgct ggttcttggt ggagcagttg ttcccagcgg cacgaagacg tacacgaccg 660

gaatccgccc taccattgat ggcgacaatt tttgcggagg atcgctgatc tccccaacgc 720

atgttcttac gacgacggcg tgtcttcgtg gtatcaagcc gccgaattgg gtgtctgtgg 780

g 781

<210> 117
<211> 781
<212> DNA
<213> artificial

<220>
<223> Sequence of the second arm of the chitinase/ elicitin/glucanase
      inhibitor, dsRNA used to concomitantly target genes producing the
      protein defined by PITG_17947, PITG_10772, PITG_13671 from
      plasmopora viticola

<400> 117
cccacagaca cccaattcgg cggcttgata ccacgaagac acgccgtcgt cgtaagaaca 60

tgcgttgggg agatcagcga tcctccgcaa aaattgtcgc catcaatggt agggcggatt 120

ccggtcgtgt acgtcttcgt gccgctggga acaactgctc caccaagaac cagcattcgg 180

ctgacatggt cagtagtggc agagacggct ccaagcgcaa ttgaggaggc ggcgacagca 240

gagatgatct tcatggcgca tttgagtacg tcgaagaccg acgtgatgtt gaaggtcatg 300

ccttctgcgc acgtttcgaa gtaggtgctg ttgtcgacag tcgcgatgaa cgcctcgctc 360

acgtcggccg agcactccga accaacggtg cctgaagcgg cagagcctga gtgctcatgt 420

tcgtcgtggg tcgacgacga gtggtcgtgc ccctcgtggg ccgagacggc agccgacgcg 480

acaagaagcg tcgaaacagc gagagtgaac ttggccatga tccgcactcg tgagctgttt 540

gagccaggaa cgctgccagc tcacgcttgt cattgtcgtc gttgcccgta ttagcgaatt 600

cgctgtactt gctggccgca tccacaagac cgttgaagtt gtagagctcc acagcgtccg 660

ggaaaacctc ctggaaacga tcctgatcga agaagagcgc gaaactcgag ctatcagcgt 720

caccagagac cgcagaaggc acaaccagaa gacttgacgc gataaccccg acgaatttca 780

t 781

<210>  118
<211>  767
<212>  DNA
<213>  artificial

<220>
<223>  Sequence of the first arm of the glucanase inhibitor,
       transglutaminase elicitor/ secretory protein dsRNA used to
       concomitantly target genes producing the protein defined by
       PITG_13671, PITG_16956, PITG_00891 from plasmopora viticola

<400>  118
gccatgaaga tcatctctgc tgtcgccgcc tcctcaattg cgcttggagc cgtctctgcc      60

actactgacc atgtcagccg aatgctggtt cttggtggag cagttgttcc cagcggcacg     120

aagacgtaca cgaccggaat ccgccctacc attgatggcg acaattttgg cggaggatcg      180

ctgatctccc caacgcatgt tcttacgacg acggcgtgtc ttcgtggtat caagccgccg     240

aattgggtgt ctgtgggcac gatctcgtcg agaagattac aaagccttcc tccgttccag     300

gttgacgcgc tatcccttcc ggatctctag atcgactctc tttgcaacat ggtattctcg     360

cccagcacct atctcgtctc cgctgccgtc gctgtcgtgg cgctgcagat gcagcacgct     420

tctgccgcgt cgctgtacta cggagtgacc accgtgtcaa agacggccga tacgatctcc     480

aaatcgtcac catttgctgg tggcgaagtt cctgaccagg attatgcgtg gagcagcgac     540

ttgtttattc aacaatgcaa ccttatttgc tagtagcgac attactgggt gctgccacga     600

cggcaaatgc aattccagtg cacttcaaca atcactgcaa tgctgcaatt gagctgtaca     660

acaacacata catcgaggtg atcaatccag gcagcaccac tactcgcaat cttgaggagg     720

gattcagtgg gatgtttcgg aacggatgga atccccaagc aacacgt                   767

<210>  119
<211>  767
<212>  DNA
<213>  artificial

<220>
<223>  Sequence of the second arm of the glucanase inhibitor,
       transglutaminase elicitor/ secretory protein dsRNA used to
       concomitantly target genes producing the protein defined by
       PITG_13671, PITG_16956, PITG_00891 from plasmopora viticola

<400>  119
acgtgttgct tggggattcc atccgttccg aaacatccca ctgaatccct cctcaagatt      60

gcgagtagtg gtgctgcctg gattgatcac ctcgatgtat gtgttgttgt acagctcaat     120

tgcagcattg cagtgattgt tgaagtgcac tggaattgca tttgccgtcg tggcagcacc     180

cagtaatgtc gctactagca aataaggttg cattgttgaa taaacaagtc gctgctccac     240

gcataatcct ggtcaggaac ttcgccacca gcaaatggtg acgatttgga gatcgtatcg     300

gccgtctttg acacggtggt cactccgtag tacagcgacg cggcagaagc gtgctgcatc     360

tgcagcgcca cgacagcgac ggcagcggag acgagatagg tgctgggcga gaataccatg 420

ttgcaaagag agtcgatcta gagatccgga agggatagcg cgtcaacctg gaacggagga 480

aggctttgta atcttctcga cgagatcgtg cccacagaca cccaattcgg cggcttgata 540

ccacgaagac acgccgtcgt cgtaagaaca tgcgttgggg agatcagcga tcctccgcaa 600

aaattgtcgc catcaatggt agggcggatt ccggtcgtgt acgtcttcgt gccgctggga 660

acaactgctc caccaagaac cagcattcgg ctgacatggt cagtagtggc agagacggct 720

ccaagcgcaa ttgaggaggc ggcgacagca gagatgatct tcatggc 767

<210> 120
<211> 934
<212> DNA
<213> artificial

<220>
<223> Sequence of the first arm of the GyrB/ FusA/ MreB/HrpG/PhoP/FhaB
 dsRNA used to concomitantly target GyrB/ FusA/
 MreB/HrpG/PhoP/FhaB genes from xanthomonas citri pv fuscan

<400> 120
tgaccgacga acaaaacacc ccgccaacac ccaacggcac ctacgactcc agcaagatca 60

ccgtgctgcg tggcctggaa gccgttcgca agcgccccgg catgtatatc ggcgacgtcc 120

atgacggcac cggcctgcat cacatggtgt gatcgtggcc cgcaccaccc ctatcgagcg 180

ttaccgcaac ttcggcatca tggcccacat tgatgccggc aagaccacca cgtccgaacg 240

catcctgttc tacaccggtg tgagtcacaa gatcggcgaa gtccatgacg gcgctgcagt 300

gatgatgcat gttcaagaaa ctccgcggca tgttctccaa cgacctgtcc atcgatttgg 360

gcacggccaa tacgctgatc tacgtgcgcg gacagggcat cgtgctgaac gagccgtcgg 420

tggttgcggt gcgtcaggat cgcgcgatcg gcggcacgcg gtgcatgaac gaccactctc 480

cccccaacgc cggatcggtg ttcctgttga cgcaggatgc gcgcctggtc tcgcaggtca 540

acgccagcct cgcaccgctt gcgcgcaatg tctcgacctt ttccgatgaa ctggagcttt 600

tgcggacgct gcggcattca agcatgcgta tccttttggt cgaagacgaa gctccgctgc 660

gcgaaaccct tgcggcgcgc ctgaagcgcg aaggctttgc ggtcgacgct gcacaggacg 720

gcgaagaagg cctctacatg ggccgtgaag tgccatttga tgtcggcatc atcgtccaat 780

ggacaacgaa gcgatgcgcg tgcgtggtta cctggccgag cagggcaagc gtgcgcttgc 840

actgttgctg tgctgcacga ccacctggac atcgtttcca gcatgggcgc aggttgcgcc 900

aaccgccaac cctgcagggc agacgcccgg tcag 934

<210> 121
<211> 934
<212> DNA

<213>  artificial

<220>
<223>  Sequence of the second arm of the GyrB/ FusA/ MreB/HrpG/PhoP/FhaB
       dsRNA used to concomitantly target GyrB/ FusA/
       MreB/HrpG/PhoP/FhaB genes from xanthomonas citri pv fuscan

<400>  121
   ctgaccgggc gtctgccctg cagggttggc ggttggcgca acctgcgccc atgctggaaa        60
   cgatgtccag gtggtcgtgc agcacagcaa cagtgcaagc gcacgcttgc cctgctcggc       120
   caggtaacca cgcacgcgca tcgcttcgtt gtccattgga cgatgatgcc gacatcaaat       180
   ggcacttcac ggcccatgta gaggccttct tcgccgtcct gtgcagcgtc gaccgcaaag       240
   ccttcgcgct tcaggcgcgc cgcaagggtt tcgcgcagcg gagcttcgtc ttcgaccaaa       300
   aggatacgca tgcttgaatg ccgcagcgtc cgcaaaagct ccagttcatc ggaaaaggtc       360
   gagacattgc gcgcaagcgg tgcgaggctg gcgttgacct gcgagaccag gcgcgcatcc       420
   tgcgtcaaca ggaacaccga tccggcgttg gggggagagt ggtcgttcat gcaccgcgtg       480
   ccgccgatcg cgcgatcctg acgcaccgca accaccgacg gctcgttcag cacgatgccc       540
   tgtccgcgca cgtagatcag cgtattggcc gtgcccaaat cgatggacag gtcgttggag       600
   aacatgccgc ggagtttctt gaacatgcat catcactgca gcgccgtcat ggacttcgcc       660
   gatcttgtga ctcacaccgg tgtagaacag gatgcgttcg gacgtggtgg tcttgccggc       720
   atcaatgtgg gccatgatgc cgaagttgcg gtaacgctcg ataggggtgg tgcgggccac        780
   gatcacacca tgtgatgcag gccggtgccg tcatggacgt cgccgatata catgccgggg       840
   cgcttgcgaa cggcttccag gccacgcagc acggtgatct tgctggagtc gtaggtgccg       900
   ttgggtgttg gcggggtgtt ttgttcgtcg gtca                                   934


<210>  122
<211>  793
<212>  DNA
<213>  artificial

<220>
<223>  Sequence of the first arm of the GyrB/ RbfA/MreB dsRNA used to
       concomitantly target GyrB/ RbfA/ MreB genes from xanthomonas
       citri pv fuscan

<400>  122
atgaccgacg aacaaaacac cccgccaaca cccaacggca cctacgactc cagcaagatc        60

accgtgctgc gtggcctgga agccgttcgc aagcgccccg gcatgtatat cggcgacgtc       120

catgacggca ccggcctgca tcacatggtg ttcgaggtgg tcgacaactc ggtcgacgaa       180

gcccttgccg ggcatgccga cgacatcgtg gtaaagatca tggccgatgg ctcggtggca       240

gtttccgaca acgggcgcgg cgtgccggat catgccaacc aaatcattcc atcgcaccga       300

ccgcgtatct gcgcaggtgc gtcgcgacct cggcacgatc gtgcacgcgg ccgtgcgcga       360

ccatggcctg ccgtcggtca gcgtgtcaga cgtcgaaatc agtcgcgacc tggcgcacgc 420

caaggtcttc gtcaccgcgc tgcagcagga gcgttcggcc gaagcggtca aggggctcaa 480

ggaaatcgcc ggccagctgc gtacgcagtt ggcgcgcgcg aatgcatgtt caagaaactc 540

cgcggcatgt tctccaacga cctgtccatc gatttgggca cggccaatac gctgatctac 600

gtgcgcggac agggcatcgt gctgaacgag ccgtcggtgg ttgcggtgcg tcaggatcgc 660

gcgatcggcg gcacgcgctc ggtcgctgcc gtcggtgcgg aggccaagca gatgctcggc 720

cgtacccccg gccacatcac caccattcgc ccgatgaagg acggcgtcat cgccgacttc 780

acctacaccg aag 793

<210> 123
<211> 793
<212> DNA
<213> artificial

<220>
<223> Sequence of the second arm of the GyrB/ RbfA/MreB dsRNA used to
      concomitantly target GyrB/ RbfA/ MreB genes from xanthomonas
      citri pv fuscan

<400> 123
cttcggtgta ggtgaagtcg gcgatgacgc cgtccttcat cgggcgaatg gtggtgatgt 60

ggccgggggt acggccgagc atctgcttgg cctccgcacc gacggcagcg accgagcgcg 120

tgccgccgat cgcgcgatcc tgacgcaccg caaccaccga cggctcgttc agcacgatgc 180

cctgtccgcg cacgtagatc agcgtattgg ccgtgcccaa atcgatggac aggtcgttgg 240

agaacatgcc gcggagtttc ttgaacatgc attcgcgcgc gccaactgcg tacgcagctg 300

gccggcgatt tccttgagcc ccttgaccgc ttcggccgaa cgctcctgct gcagcgcggt 360

gacgaagacc ttggcgtgcg ccaggtcgcg actgatttcg acgtctgaca cgctgaccga 420

cggcaggcca tggtcgcgca cggccgcgtg cacgatcgtg ccgaggtcgc gacgcacctg 480

cgcagatacg cggtcggtgc gatggaatga tttggttggc atgatccggc acgccgcgcc 540

cgttgtcgga aactgccacc gagccatcgg ccatgatctt taccacgatg tcgtcggcat 600

gcccggcaag ggcttcgtcg accgagttgt cgaccacctc gaacaccatg tgatgcaggc 660

cggtgccgtc atggacgtcg ccgatataca tgccggggcg cttgcgaacg gcttccaggc 720

cacgcagcac ggtgatcttg ctggagtcgt aggtgccgtt gggtgttggc ggggtgtttt 780

gttcgtcggt cat 793

<210> 124
<211> 779
<212> DNA
<213> artificial

<220>
<223>  Sequence of the first arm of the HrpG/PhoP/ FtsZ dsRNA used to
      concomitantly target HrpG/ PhoP/ FtsZ genes from xanthomonas
      citri pv fuscan

<400>  124
atgaacgacc actctccccc caacgccgga tcggtgttcc tgttgacgca ggatgcgcgc      60

ctggtctcgc aggtcaacgc cagcctcgca ccgcttgcgc gcaatgtctc gaccttttcc     120

gatgaactgg agcttttgcg gacgctgcgg cattcgccgt gcgagctgct gatcttcgat     180

gccagctgtg tcgccgccga tgacagttca ctgctggcct ggcagcgctg ccatagcggc     240

cagcccagat catgcgtatc cttttggtcg aagacgaagc tccgctgcgc gaaacccttg     300

cggcgcgcct gaagcgcgaa ggctttgcgg tcgacgctgc acaggacggc gaagaaggcc     360

tctacatggg ccgtgaagtg ccatttgatg tcggcatcat cgaccttggc ttgcccaaga     420

tgtcggggat ggagctgatc aaggccttgc gcgatgaggg caagaagttt ccggtgctga     480

tcctgaccgc gcgctcgagc tggcaggaca atgcatggca catttcgaac tgattgaaaa     540

aatggctccc aacgcggtca tcaaggtggt tggcgtcggc ggcggcggtg gcaatgccgt     600

cgcgcacatg gtcaacacca acgtcgatgg cgtggaattc atcaccgcca acaccgactc     660

gcaggcgatc aagaactgcg gcgccaagct gcagctgcag ctcggcacca acgtcaccaa     720

gggcctgggc gcaggcgcca atccggaagt cggccgtcag gctgcgctgg aagatcgcg      779

<210>  125
<211>  779
<212>  DNA
<213>  artificial

<220>
<223>  Sequence of the second arm of the HrpG/PhoP/ FtsZ dsRNA used to
      concomitantly target HrpG/ PhoP/ FtsZ genes from xanthomonas
      citri pv fuscan

<400>  125
cgcgatcttc cagcgcagcc tgacggccga cttccggatt ggcgcctgcg cccaggccct      60

tggtgacgtt ggtgccgagc tgcagctgca gcttggcgcc gcagttcttg atcgcctgcg     120

agtcggtgtt ggcggtgatg aattccacgc catcgacgtt ggtgttgacc atgtgcgcga     180

cggcattgcc accgccgccg ccgacgccaa ccaccttgat gaccgcgttg ggagccattt     240

tttcaatcag ttcgaaatgt gccatgcatt gtcctgccag ctcgagcgcg cggtcaggat     300

cagcaccgga aacttcttgc cctcatcgcg caaggccttg atcagctcca tccccgacat     360

cttgggcaag ccaaggtcga tgatgccgac atcaaatggc acttcacggc ccatgtagag     420

gccttcttcg ccgtcctgtg cagcgtcgac cgcaaagcct tcgcgcttca ggcgcgccgc     480

aagggtttcg cgcagcggag cttcgtcttc gaccaaaagg atacgcatga tctgggctgg     540

ccgctatggc agcgctgcca ggccagcagt gaactgtcat cggcggcgac acagctggca     600

tcgaagatca gcagctcgca cggcgaatgc cgcagcgtcc gcaaaagctc cagttcatcg 660

gaaaaggtcg agacattgcg cgcaagcggt gcgaggctgg cgttgacctg cgagaccagg 720

cgcgcatcct gcgtcaacag gaacaccgat ccggcgttgg ggggagagtg gtcgttcat 779

<210> 126
<211> 798
<212> DNA
<213> artificial

<220>
<223> Sequence of the first arm of the FhaB/FusA/MreB dsRNA used to
      concomitantly target HrpG/ PhoP/ FtsZ genes from xanthomonas
      citri pv fuscan

<400> 126
atggacaacg aagcgatgcg cgtgcgtggt tacctggccg agcagggcaa gcgtgcgctt 60

gcactgttgc tgtgctgcac gaccacctgg acatcgtttc cagcatgggc gcaggttgcg 120

ccaaccgcca accctgcagg gcagacgccc ggtcagcagg tcgctgccaa tggtgtgccg 180

gtggtggaca tcgtcgcgcc caatgcacgc ggcatttcgc acaaccgcta cagcaacttc 240

aatgtcggcc cgaatgggct gatcctcgat cgtggcccgc accaccccta tcgagcgtta 300

ccgcaacttc ggcatcatgg cccacattga tgccggcaag accaccacgt ccgaacgcat 360

cctgttctac accggtgtga gtcacaagat cggcgaagtc catgacggcg ctgcagtgat 420

ggactggatg gagcaggagc aggagcgtgg tatcaccatc acctccgctg cgaccaccgc 480

gttctggtcg ggtatggaca agtccatgcc gcagcaccgc ttcaacatca tcgacacatg 540

catgttcaag aaactccgcg gcatgttctc caacgacctg tccatcgatt tgggcacggc 600

caatacgctg atctacgtgc gcggacaggg catcgtgctg aacgagccgt cggtggttgc 660

ggtgcgtcag gatcgcgcga tcggcggcac gcgctcggtc gctgccgtcg gtgcggaggc 720

caagcagatg ctcggccgta cccccggcca catcaccacc attcgcccga tgaaggacgg 780

cgtcatcgcc gacttcac 798

<210> 127
<211> 798
<212> DNA
<213> artificial

<220>
<223> Sequence of the second arm of the FhaB/FusA/MreB dsRNA used to
      concomitantly target FhaB/FusA/ MreB genes from xanthomonas citri
      pv fuscan

<400> 127
   gtgaagtcgg cgatgacgcc gtccttcatc gggcgaatgg tggtgatgtg gccgggggta 60
   cggccgagca tctgcttggc ctccgcaccg acggcagcga ccgagcgcgt gccgccgatc 120
   gcgcgatcct gacgcaccgc aaccaccgac ggctcgttca gcacgatgcc ctgtccgcgc 180
   acgtagatca gcgtattggc cgtgcccaaa tcgatggaca ggtcgttgga gaacatgccg 240
   cggagtttct tgaacatgca tgtgtcgatg atgttgaagc ggtgctgcgg catggacttg 300
   tccatacccg accagaacgc ggtggtcgca gcggaggtga tggtgatacc acgctcctgc 360
   tcctgctcca tccagtccat cactgcagcg ccgtcatgga cttcgccgat cttgtgactc 420
   acaccggtgt agaacaggat gcgttcggac gtggtggtct tgccggcatc aatgtgggcc 480
   atgatgccga agttgcggta acgctcgata ggggtggtgc gggccacgat cgaggatcag 540
   cccattcggg ccgacattga agttgctgta gcggttgtgc gaaatgccgc gtgcattggg 600
   cgcgacgatg tccaccaccg gcacaccatt ggcagcgacc tgctgaccgg gcgtctgccc 660
   tgcagggttg gcggttggcg caacctgcgc ccatgctgga aacgatgtcc aggtggtcgt 720
   gcagcacagc aacagtgcaa gcgcacgctt gccctgctcg gccaggtaac cacgcacgcg 780
   catcgcttcg ttgtccat 798

<210> 128
<211> 784
<212> DNA
<213> artificial

<220>
<223> Sequence of the first arm of the GyrB/ FusA/ZipA dsRNA used to
      concomitantly target GyrB, FusA, ZipA genes from xanthomonas
      hortorum pv vitians

<400> 128
atgaccgaag atcaaacacc ccgaccacac ccaacagcac ctacgattcc agcaagatca 60

ccgtgctgcg tggcctggaa gccgtccgca agcgccccgg catgtacatc ggcgatgtcc 120

atgacggcac cggcctgcat cacatggtgt tcgaggtggt cgacaactcg gtcgacgagg 180

ccctggccgg ccatgccgac gacatcgtgg tcaagatcct ggtcgatggc tcggtcgccg 240

tgtcggataa cggccgcgga tcgtggccgc aaccaacccc tatcgagcgt taccgcaact 300

tcggcatcat ggctcacatc gatgccggca agaccaccac ctccgagcgc atcctgtttt 360

acaccggcgt cagtcacaag atcggcgaag tccatgacgg cgccgcggtg atggactgga 420

tggagcagga gcaggagcgt ggtatcacca tcacctccgc tgccaccacc gcgttctggt 480

cgggtatgga caagtccatg ccgcagcatc gcctcaacat catgcatgtc cgaacatggc 540

catgatccgg atcgggatcc tgatcgctgg actgctgctg gtcgcggcca tcttcctgtt 600

tggtcgcccg aaaaaatcgc cgcaagggcg ccgggtcgac aaggacgacg ggcagccgcg 660

cgaacgccgc gagccggtga tttccggcga gcctggggtg gagggcgacc cgttcaagcg 720

cactgaaggc gccgagcaga gcgaactcaa tctggacgat gccgatgcgg ccggcggcaa 780

cgat 784

<210> 129
<211> 784
<212> DNA
<213> artificial

<220>
<223> Sequence of the second arm of the GyrB/ FusA/ZipA dsRNA used to
      concomitantly target GyrB, FusA, ZipA genes from xanthomonas
      hortorum pv vitians

<400> 129
atcgttgccg ccggccgcat cggcatcgtc cagattgagt tcgctctgct cggcgccttc      60

agtgcgcttg aacgggtcgc cctccacccc aggctcgccg gaaatcaccg gctcgcggcg     120

ttcgcgcggc tgcccgtcgt ccttgtcgac ccggcgccct tgcggcgatt ttttcgggcg     180

accaaacagg aagatggccg cgaccagcag cagtccagcg atcaggatcc cgatccggat     240

catggccatg ttcggacatg catgatgttg aggcgatgct gcggcatgga cttgtccata     300

cccgaccaga acgcggtggt ggcagcggag gtgatggtga taccacgctc ctgctcctgc     360

tccatccagt ccatcaccgc ggcgccgtca tggacttcgc cgatcttgtg actgacgccg     420

gtgtaaaaca ggatgcgctc ggaggtggtg gtcttgccgg catcgatgtg agccatgatg     480

ccgaagttgc ggtaacgctc gataggggtt ggttgcggcc acgatccgcg gccgttatcc     540

gacacggcga ccgagccatc gaccaggatc ttgaccacga tgtcgtcggc atggccggcc     600

agggcctcgt cgaccgagtt gtcgaccacc tcgaacacca tgtgatgcag gccggtgccg     660

tcatggacat cgccgatgta catgccgggg cgcttgcgga cggcttccag gccacgcagc     720

acggtgatct tgctggaatc gtaggtgctg ttgggtgtgg tcggggtgtt tgatcttcgg     780

tcat                                                                  784


<210> 130
<211> 663
<212> DNA
<213> artificial

<220>
<223> Sequence of the first arm of the avrPTO/avrPTOB/HopT1-1 dsRNA
      used to concomitantly target avrPTO/avrPTOB/HopT1-1 genes from
      pseudomonas syringae pto DC3000

<400> 130
atgggaaata tatgtgtcgg cggatccagg atggcccatc aggtgaactc cccagaccga      60

gttagtaaca actcgggtga cgaagataac gtaacgtcca gtcaactgct gagcgtcaga     120

catcaacttg cggagtctgc tggtgtacca agagatcagc atgaatttgt tagtaaccaa     180

gcacctcaaa gcctgagaaa atggcgggta tcaatagagc gggaccatcg ggcgcttatt     240

ttgttggcca cacagacccc gagccagtat cggggcaagc acacggatcc ggcagcggcg     300

ccagctcctc gaacagtccg caggttcagc cgcgaccctc gaatactccc ccgtcgaacg 360

cgcccgcacc gccgccaacc ggacgtgaga ggctttcacg atgcatgaaa acagtcagca 420

atcactcgat acccagtaca aatctcgtcg tggatgcggg aacggaaact tcggcgcaga 480

aatcccagcc ggtttgcagc gaaatccagc gtaacagcaa gatcgaaaaa gcagtcatcg 540

aacacattgc cgaccacccg gcagcgaaaa tgacaataag cgcgctggtt gacacgttga 600

cagacgtttt tgtcagggct catggggagg ttaaggggtg ggccgaaatc gtccaggcag 660

tct 663

<210> 131
<211> 663
<212> DNA
<213> artificial

<220>
<223> Sequence of the second arm of the avrPTO/avrPTOB/HopT1-1 dsRNA
 used to concomitantly target avrPTO/avrPTOB/HopT1-1 genes from
 pseudomonas syringae pto DC3000

<400> 131
agactgcctg gacgatttcg gcccacccct taacctcccc atgagccctg acaaaaacgt 60

ctgtcaacgt gtcaaccagc gcgcttattg tcattttcgc tgccgggtgg tcggcaatgt 120

gttcgatgac tgctttttcg atcttgctgt tacgctggat ttcgctgcaa accggctggg 180

atttctgcgc cgaagtttcc gttcccgcat ccacgacgag atttgtactg ggtatcgagt 240

gattgctgac tgttttcatg catcgtgaaa gcctctcacg tccggttggc ggcggtgcgg 300

gcgcgttcga cgggggagta ttcgagggtc gcggctgaac ctgcggactg ttcgaggagc 360

tggcgccgct gccggatccg tgtgcttgcc ccgatactgg ctcggggtct gtgtggccaa 420

caaaataagc gcccgatggt cccgctctat tgatacccgc cattttctca ggctttgagg 480

tgcttggtta ctaacaaatt catgctgatc tcttggtaca ccagcagact ccgcaagttg 540

atgtctgacg ctcagcagtt gactggacgt tacgttatct tcgtcacccg agttgttact 600

aactcggtct ggggagttca cctgatgggc catcctggat ccgccgacac atatatttcc 660

cat 663

<210> 132
<211> 668
<212> DNA
<213> artificial

<220>
<223> Sequence of the first arm of the rpoB/rpoC/fusA dsRNA used to
 concomitantly target the rpoB, RpoC and fusA genes from Pto
 DC3000

<400> 132
cgtatccgca aggactttag caagttgccg gacgtaatgg atgtgccgta tctcttggcc 60

atccagctgg attcgtatcg cgaattcctg caggcgggag cgaccaaaga tcagttccgc 120

gacgtcggtc tgcatgcagc cttcaaatcc gttttcccga tcatcagcta ctccggcaat 180

gctgcgctgg agtatgtagg ttatcgcttg ggcgagatct tgaaagacct actgaatttg 240

ctgaaaaacc agggtcaagt cgaagagttc gacgcaatcc gtatcggact tgcatcgcct 300

gagatgatcc gctcttggtc gttcggtgaa gttaaaaagc cggaaaccat caactaccgt 360

acgttcaagc ctgagcgtga cggtctgttc tgcgccaaga tctttggtcc ggtaaaagat 420

tacgagtgcc tgtgcggtaa gtacaagatg catggctcgt actactccga ttagccgtta 480

ccgtaacatc ggtatcgtcg ctcacgtgga tgctggtaaa accaccacca ccgagcgcgt 540

ccttttttac accggcaaaa gccacaaaat gggcgaggtg catgatggcg ccgcgaccac 600

ggactggatg gttcaggagc aggagcgtgg tattaccatt acttctgctg ctatcactgc 660

cttttggc 668

<210> 133
<211> 668
<212> DNA
<213> artificial

<220>
<223> Sequence of the second arm of the rpoB/rpoC/fusA dsRNA used to
      concomitantly target the rpoB, RpoC and fusA genes from Pto
      DC3000

<400> 133
gccaaaaggc agtgatagca gcagaagtaa tggtaatacc acgctcctgc tcctgaacca 60

tccagtccgt ggtcgcggcg ccatcatgca cctcgcccat tttgtggctt ttgccggtgt 120

aaaaaaggac gcgctcggtg gtggtggttt taccagcatc cacgtgagcg acgataccga 180

tgttacggta acggctaatc ggagtagtac gagccatgca tcttgtactt accgcacagg 240

cactcgtaat cttttaccgg accaaagatc ttggcgcaga acagaccgtc acgctcaggc 300

ttgaacgtac ggtagttgat ggtttccggc tttttaactt caccgaacga ccaagagcgg 360

atcatctcag gcgatgcaag tccgatacgg attgcgtcga actcttcgac ttgaccctgg 420

tttttcagca aattcagtag gtctttcaag atctcgccca agcgataacc tacatactcc 480

agcgcagcat tgccggagta gctgatgatc gggaaaacgg atttgaaggc tgcatgcaga 540

ccgacgtcgc ggaactgatc tttggtcgct cccgcctgca ggaattcgcg atacgaatcc 600

agctggatgg ccaagagata cggcacatcc attacgtccg gcaacttgct aaagtccttg 660

cggatacg 668

<210> 134
<211> 673
<212> DNA

<213>  artificial

<220>
<223>  Sequence of the first arm of the secE/rpoA/rplQ dsRNA used to
       concomitantly target the secE, RpoA and rplQ genes from Pto
       DC3000

<400>  134
tgaatcccaa ggctgaagca tcagactctc gctttgattt gctgaaatgg ctcctggtag        60

tcattttggt agtcgtgggt gttgtcggta atcagtatta ctctgctgag ccgatcctgt       120

accgtgttct cgctctcctt gtgattgccg cagcagctgc atttgtagcg ctgcagactg       180

gcaagggcaa agctttcttc gttttggcga aagaagcgcg tgcagagatg atctgcagat       240

ttcggtaaat gagttcctga caccccgcca cattgatgtg caggttgtca gtccaacccg       300

cgccaaaatc actctcgagc ctctcgagcg tggtttcggc cataccctgg gcaacgcgct       360

tcgccgcatt ttgttgtcct ccatgcccgg ctgtgcagta gtcgaggccg agattgacgg       420

tgtactccat gagtacagcg ccatcgaagg tgtaatgcat gcgtcatcgt aaaagtggac       480

gtcacctgag ccgtaccagc tctcaccgta aagccatgtt tcaaaacatg gcggtgtcgc       540

tgttcgagca cgagctgatc aaaactaccc tgccgaaagc caaggaactg cgccgcgttg       600

ccgagccgct gatcaccctg gccaaggaag acagtgttgc taaccgtcgt ctggctttcg       660

accgtactcg ttc                                                          673

<210>  135
<211>  673
<212>  DNA
<213>  artificial

<220>
<223>  Sequence of the second arm of the secE/rpoA/rplQ dsRNA used to
       concomitantly target the secE, RpoA and rplQ genes from Pto
       DC3000

<400>  135
gaacgagtac ggtcgaaagc cagacgacgg ttagcaacac tgtcttcctt ggccagggtg        60

atcagcggct cggcaacgcg gcgcagttcc ttggctttcg gcagggtagt tttgatcagc       120

tcgtgctcga acagcgacac cgccatgttt tgaaacatgg ctttacggtg agagctggta       180

cggctcaggt gacgtccact tttacgatga cgcatgcatt acaccttcga tggcgctgta       240

ctcatggagt acaccgtcaa tctcggcctc gactactgca cagccgggca tggaggacaa       300

caaaatgcgg cgaagcgcgt tgcccagggt atggccgaaa ccacgctcga gaggctcgag       360

agtgattttg gcgcgggttg gactgacaac ctgcacatca atgtggcggg gtgtcaggaa       420

ctcatttacc gaaatctgca gatcatctct gcacgcgctt ctttcgccaa aacgaagaaa       480

gctttgccct tgccagtctg cagcgctaca aatgcagctg ctgcggcaat cacaaggaga       540

gcgagaacac ggtacaggat cggctcagca gagtaatact gattaccgac aacacccacg       600

actaccaaaa tgactaccag gagccatttc agcaaatcaa agcgagagtc tgatgcttca 660

gccttgggat tca 673

<210> 136
<211> 217
<212> DNA
<213> artificial

<220>
<223> Sequence of the first arm of the fusA dsRNA used to target the
      fusA gene from Pto DC3000

<400> 136
   atggctcgta ctactccgat tagccgttac cgtaacatcg gtatcgtcgc tcacgtggat 60
   gctggtaaaa ccaccaccac cgagcgcgtc cttttttaca ccggcaaaag ccacaaaatg 120
   ggcgaggtgc atgatggcgc cgcgaccacg gactggatgg ttcaggagca ggagcgtggt 180
   attaccatta cttctgctgc tatcactgcc ttttggc 217

<210> 137
<211> 217
<212> DNA
<213> artificial

<220>
<223> Sequence of the second arm of the fusA dsRNA used to target the
      fusA gene from Pto DC3000

<400> 137
gccaaaaggc agtgatagca gcagaagtaa tggtaatacc acgctcctgc tcctgaacca 60

tccagtccgt ggtcgcggcg ccatcatgca cctcgcccat tttgtggctt ttgccggtgt 120

aaaaaaggac gcgctcggtg gtggtggttt taccagcatc cacgtgagcg acgataccga 180

tgttacggta acggctaatc ggagtagtac gagccat 217

<210> 138
<211> 221
<212> DNA
<213> artificial

<220>
<223> Sequence of the first arm of the gyrB dsRNA used to target the
      gyrB gene from Pto DC3000

<400> 138
agcgaaaacc aaacgtacga ctcgtccagc attaaagtgc tgaaaggact tgatgccgta 60

cgcaagcgtc ccggaatgta catcggtgat acagacgatg gcagcggtct gcaccatatg 120

gtgttcgagg tggttgataa ctcgatcgac gaagcattgg caggccattg cgacgacatc 180

agcatcatca tccatccaga cgaatctatc accgtgcgcg a 221

<210>  139
<211>  221
<212>  DNA
<213>  artificial

<220>
<223>  Sequence of second arm of the gyrB dsRNA used to target the gyrB
       gene from Pto DC3000

<400>  139
tcgcgcacgg tgatagattc gtctggatgg atgatgatgc tgatgtcgtc gcaatggcct      60

gccaatgctt cgtcgatcga gttatcaacc acctcgaaca ccatatggtg cagaccgctg     120

ccatcgtctg tatcaccgat gtacattccg ggacgcttgc gtacggcatc aagtcctttc     180

agcactttaa tgctggacga gtcgtacgtt tggttttcgc t                         221


<210>  140
<211>  442
<212>  DNA
<213>  artificial

<220>
<223>  Sequence of the first arm of the fusA/gyrB dsRNA used to
       concomitantly target the fusA and gyrB genes from Pto DC3000

<400>  140
atggctcgta ctactccgat tagccgttac cgtaacatcg gtatcgtcgc tcacgtggat      60

gctggtaaaa ccaccaccac cgagcgcgtc cttttttaca ccggcaaaag ccacaaaatg     120

ggcgaggtgc atgatggcgc cgcgaccacg gactggatgg ttcaggagca ggagcgtggt     180

attaccatta cttctgctgc tatcactgcc ttttggcgat cagcgaaaac caaacgtacg     240

actcgtccag cattaaagtg ctgaaaggac ttgatgccgt acgcaagcgt cccggaatgt     300

acatcggtga tacagacgat ggcagcggtc tgcaccatat ggtgttcgag gtggttgata     360

actcgatcga cgaagcattg gcaggccatt gcgacgacat cagcatcatc atccatccag     420

acgaatctat caccgtgcgc ga                                              442


<210>  141
<211>  442
<212>  DNA
<213>  artificial

<220>
<223>  Sequence of the second arm of the fusA/gyrB dsRNA used to
       concomitantly target the fusA and gyrB genes from Pto DC3000

<400>  141
tcgcgcacgg tgatagattc gtctggatgg atgatgatgc tgatgtcgtc gcaatggcct      60

gccaatgctt cgtcgatcga gttatcaacc acctcgaaca ccatatggtg cagaccgctg     120

ccatcgtctg tatcaccgat gtacattccg ggacgcttgc gtacggcatc aagtcctttc     180

agcactttaa tgctggacga gtcgtacgtt tggttttcgc tgatcgccaa aaggcagtga     240

tagcagcaga agtaatggta ataccacgct cctgctcctg aaccatccag tccgtggtcg 300

cggcgccatc atgcacctcg cccattttgt ggcttttgcc ggtgtaaaaa aggacgcgct 360

cggtggtggt ggttttacca gcatccacgt gagcgacgat accgatgtta cggtaacggc 420

taatcggagt agtacgagcc at 442

<210> 142
<211> 229
<212> DNA
<213> artificial

<220>
<223> Sequence of the first arm of the secE dsRNA used to target the
      secE gene from Pto DC3000

<400> 142
tgaatcccaa ggctgaagca tcagactctc gctttgattt gctgaaatgg ctcctggtag 60

tcattttggt agtcgtgggt gttgtcggta atcagtatta ctctgctgag ccgatcctgt 120

accgtgttct cgctctcctt gtgattgccg cagcagctgc atttgtagcg ctgcagactg 180

gcaagggcaa agctttcttc gttttggcga aagaagcgcg tgcagagat 229

<210> 143
<211> 229
<212> DNA
<213> artificial

<220>
<223> Sequence of the second arm of the secE dsRNA used to target the
      secE gene from Pto DC3000

<400> 143
atctctgcac gcgcttcttt cgccaaaacg aagaaagctt tgcccttgcc agtctgcagc 60

gctacaaatg cagctgctgc ggcaatcaca aggagagcga gaacacggta caggatcggc 120

tcagcagagt aatactgatt accgacaaca cccacgacta ccaaaatgac taccaggagc 180

catttcagca aatcaaagcg agagtctgat gcttcagcct tgggattca 229

<210> 144
<211> 475
<212> DNA
<213> artificial

<220>
<223> Sequence of the first arm of the gyrB/hrpL dsRNA used to
      concomitantly target the gyrB and hrpL genes from Pto DC3000

<400> 144
agcgaaaacc aaacgtacga ctcgtccagc attaaagtgc tgaaaggact tgatgccgta 60

cgcaagcgtc ccggaatgta catcggtgat acagacgatg gcagcggtct gcaccatatg 120

gtgttcgagg tggttgataa ctcgatcgac gaagcattgg caggccattg cgacgacatc 180

agcatcatca tccatccaga cgaatctatc accgtgcgcg agatcctgat gactgacatc      240

accgtgccct tccagctcgg aatgcacttc gtcttcccag ctttcctgat acggctgacg      300

atacattttg cggaagtgat tgcggatcag gttcagcgcg atgccacaca gccaggtctg      360

cggtttgctg gcatgttgaa acttgtgctc gttacgcagg gcttcaagaa acacgcactg      420

gagaatgtca tccacatcat cagggttcat cacccgcttt tggataaacg ccctg           475


<210>  145
<211>  475
<212>  DNA
<213>  artificial

<220>
<223>  Sequence of the second arm of the gyrB/hrpL dsRNA used to
       concomitantly target the gyrB and hrpL genes from Pto DC3000

<400>  145
cagggcgttt atccaaaagc gggtgatgaa ccctgatgat gtggatgaca ttctccagtg       60

cgtgtttctt gaagccctgc gtaacgagca caagtttcaa catgccagca aaccgcagac      120

ctggctgtgt ggcatcgcgc tgaacctgat ccgcaatcac ttccgcaaaa tgtatcgtca      180

gccgtatcag gaaagctggg aagacgaagt gcattccgag ctggaagggc acggtgatgt      240

cagtcatcag gatctcgcgc acggtgatag attcgtctgg atggatgatg atgctgatgt      300

cgtcgcaatg gcctgccaat gcttcgtcga tcgagttatc aaccacctcg aacaccatat      360

ggtgcagacc gctgccatcg tctgtatcac cgatgtacat tccgggacgc ttgcgtacgg      420

catcaagtcc tttcagcact ttaatgctgg acgagtcgta cgtttggttt tcgct           475


<210>  146
<211>  673
<212>  DNA
<213>  artificial

<220>
<223>  Sequence of the first arm of the dnaA/dnaN/gyrB dsRNA used to
       concomitantly target the dnaA, dnaN and gyrB genes from Pto
       DC3000

<400>  146
gtgtcagtgg aactttggca gcagtgcgtg gagcttttgc gcgacgagct gccggcccag       60

caattcaaca cttggatccg tccgctacag gtcgaagccg aaggcgacga gttgcgtgtg      120

tacgcaccca accgctttgt tctcgattgg gtcaatgaaa aatacctcgg ccgtttgctt      180

gagcttctcg gcgaacgtgg tcaaggcatg gcgcccgctg atcatgcatt tcaccattca      240

acgcgaagcc ctgttgaaac ccctgcaatt ggtcgccggc gtggtagaac gccgccagac      300

cttgccggtt ctttccaatg tgcttctggt cgtgcaaggc cagcagctgt cgctgaccgg      360

tactgacctg gaagtcgagc tggttggccg tgttcagctg gaagagcctg ccgaacctgg      420

ggaaatcacc gttcccgcgc gcaagcttat gcagcgaaaa ccaaacgtac gactcgtcca 480

gcattaaagt gctgaaagga cttgatgccg tacgcaagcg tcccggaatg tacatcggtg 540

atacagacga tggcagcggt ctgcaccata tggtgttcga ggtggttgat aactcgatcg 600

acgaagcatt ggcaggccat tgcgacgaca tcagcatcat catccatcca gacgaatcta 660

tcaccgtgcg cga 673

<210> 147
<211> 673
<212> DNA
<213> artificial

<220>
<223> Sequence of the second arm of the dnaA/dnaN/gyrB dsRNA used to
 concomitantly target the dnaA, dnaN and gyrB genes from Pto
 DC3000

<400> 147
tcgcgcacgg tgatagattc gtctggatgg atgatgatgc tgatgtcgtc gcaatggcct 60

gccaatgctt cgtcgatcga gttatcaacc acctcgaaca ccatatggtg cagaccgctg 120

ccatcgtctg tatcaccgat gtacattccg ggacgcttgc gtacggcatc aagtcctttc 180

agcactttaa tgctggacga gtcgtacgtt tggttttcgc tgcataagct tgcgcgcggg 240

aacggtgatt tccccaggtt cggcaggctc ttccagctga acacggccaa ccagctcgac 300

ttccaggtca gtaccggtca gcgacagctg ctggccttgc acgaccagaa gcacattgga 360

aagaaccggc aaggtctggc ggcgttctac cacgccggcg accaattgca ggggtttcaa 420

cagggcttcg cgttgaatgg tgaaatgcat gatcagcggg cgccatgcct tgaccacgtt 480

cgccgagaag ctcaagcaaa cggccgaggt atttttcatt gacccaatcg agaacaaagc 540

ggttgggtgc gtacacacgc aactcgtcgc cttcggcttc gacctgtagc ggacggatcc 600

aagtgttgaa ttgctgggcc ggcagctcgt cgcgcaaaag ctccacgcac tgctgccaaa 660

gttccactga cac 673

<210> 148
<211> 758
<212> DNA
<213> artificial

<220>
<223> Sequence of the second arm of the HrpR/ GyrB/ RpoD dsRNA used to
 concomitantly target HrpR, GyrB, RpoD genes from pseudomonas
 syringae pv actinidiae

<400> 148
cagccgcttc ttctgcggct gcctcgtcgg tatcggcgtc ggccagcatc aaggcgtccg 60

catccggagc actctcgtgt accgggatcc ccatgtcgtt gatcatgcgg atgatgtctt 120

ccacctgctc gggatctgaa atatcctcgg gcaggtggtc gttgacctct gcgtaagtca 180

gatatttctg ctcacgaccc agggtgatca actctttgat acgagactgc tgttgcgctt 240

ttccggacat gcatcgtcta ccggaatacc gcggccgttg tcgcgcacgg tgatagattc 300

gtctggatgg atgatgatgc tgatgtcgtc gcaatggcct gccaatgctt cgtcgatcga 360

gttatcaacc acctcgaaca ccatatggtg cagaccgctg ccatcgtctg tatcaccgat 420

gtacattccg ggacgcttgc gtacggcatc aagtcctttc agcactttaa tgctggacga 480

gtcgtacgtt tggttttcgc tcatgatccc aggtacaacg tgccaccact ggaggcctct 540

atgtagccct cgcgagcccg gcatacgccg gtgaatgcgc cgttgaccac accgaataac 600

tggctctctg ccagcgactc gggaatggct gcgcagttca tgcccacaaa gggtcccgac 660

ctgctggaca actcgtgaat gcggttggcc agtgtgtcct tgccggtgcc ggtttccccg 720

cacaacagca agtccatatc caggaacgcg ctattcat 758

<210> 149
<211> 1410
<212> DNA
<213> artificial

<220>
<223> Sequence of a specific intron sequence from the Petunia Chalcone
 synthase gene CHSA

<400> 149
   ggcgcgccca atcgatgatt taaatgtgta agaatttctt atgttacatt attacattca 60
   acgttttatc ttaattggct cttcatttga ttgaaatttg acaattattt cttgtttttt 120
   tttttgtcac actctttttg ggttggggtg gccgacgaat tgtgggaagg tagaaagagg 180
   ggaggacttt tgttatactc cattagtaat tactgtttcc gtttcaattt atgtgacaat 240
   atttcctttt tagtcggttc caaaagaaaa tgtcagcatt ataaacaatt taattttgaa 300
   attacaattt tgccattaat aaaatgattt acaaccacaa aagtatctat gagcctgttt 360
   gggtgggctt ataagcagct tattttaagt ggcttataag tcaaaaagtg acattttttg 420
   agaagttaga aaatcctaac ttctcaaaaa gtagctttta agccacttat gacttataag 480
   tccaaaaatt tttaagttac caaacatata ttaatgggtt tataagctta taagccactt 540
   ttaagctcac ccaaacgggt tctatgtctc actttagact acaaatttta aaagtcttca 600
   tttatttctt aatctccgtg gcgagtaaaa ctataacaca taaagtgaaa cggagggaat 660
   aagatggagt cataaactaa tccaaatcta tactctctcc gttaatttgt tttttagttt 720
   gatttggtac attaataaaa cagatttttc gaaggttata aacacagaca gatgtttccc 780
   agcgagctag caaaattcca agatttctgt cgaaaattcg tgtgtttcta gctagtactt 840
   gatgttatct ttaacctttt agtaattttt tgtccttttc tttctatttt tcatcttaca 900
   atgaattatg agcaagttcc ttaagtagca tcacacgtga gatgtttttt atgatattga 960
   ctaaatccaa tctttaccat tccttaacta gtaaaataca acacatgtta attgatacat 1020
   tgcttaacac tgaggttaga aaattttaga aattagttgt ccaaatgctt tgaaattaga 1080
   aatctttaat cccttatttt tttttaaaat gttttttctc actccaaaga aagagaaact 1140
   gacatgaaag ctcaaaagat catgaatctt actaactttg tggaactaaa tgtacatcag 1200
   aatgtttctg acatgtgaaa atgaaagctc ttaattttct tcttttattt attgagggtt 1260
   tttgcatgct atgcattcaa tttgagtact ttaaagcacc tataaacact tacttacact 1320
   tgccttggag tttatgtttt agtgtttctt tcacatcttt tttggtcaat ttgcaggtat 1380
   ttggatccta ggtgagtcta gaggcgcgcc 1410

## Description

### SUMMARY OF THE INVENTION

The invention relates to a novel method to produce small RNAs targeting virulence factors, essential genes and /or antimicrobial resistance genes of phytopathogens. More specifically, the invention involves the expression of exogenous RNA interference (RNAi) precursor(s) in *Chlorella* cells, which in turn express and release Extracellular Vesicle (EV)-embedded antimicrobial small RNAs. These EVs can be collected from the cell-free medium of *Chlorella* cultures, and further concentrated and purified for biocontrol applications. Importantly, *Chlorella* EVs protect small RNAs from ribonuclease-mediated digestion, indicating that these lipid-based particles not only act as natural vectors of small RNAs towards pathogenic cells, but also presumably limit their degradation in the environment. The invention can thus likely be used to reduce the pathogenicity and growth of a wide range of pathogens or, potentially, to enhance beneficial effects and growth of plant-associated symbiotic and commensal microbes. Furthermore, because the integrity of *Chlorella* EV-embedded antimicrobial siRNAs remains unaltered when produced in photobioreactors, and when stored frozen, this method has the potential to be further exploited for the industrialization and manufacturing of a novel generation of microalgae-based biologicals.

### PRIOR ART DESCRIPTION

### RNA silencing controls plant-pathogen interactions

In plants, mobile small RNAs can trigger non-cell autonomous silencing in adjacent cells as well as in distal tissues (Melynk *et al*., 2011). They are notably important to prime antiviral defense ahead of the infection front (Melynk *et al.,* 2011). Non-cell autonomous silencing is also critical for the translocation of silencing signals between plant cells and their interacting non-viral pathogenic, parasitic or symbiotic organisms - excluding bacteria, which have not been shown to be targeted by this process (Baulcombe, 2015). This natural cross-kingdom regulatory mechanism has been notably recently characterized in plant-fungal interactions (Weiberg *et al*., 2015, Koch *et al.,* 2014, Zhang *et al.,* 2016, Wang *et al*., 2016, Weiberg *et al.,* 2013, Cai *et al.,* 2018). For instance, specific plant miRNAs were found to be exported into the hyphae of the fungal pathogen *Verticillium dahliae* to trigger silencing of virulence factors (Zhang *et al*., 2016, Cai *et al*., 2018). On the other hand, endogenous *B. cinerea* small RNAs can be exported into plant cells to silence plant defense genes (Weiberg *et al*., 2013), highlighting bi-directional cross-kingdom RNAi between plant and fungal pathogens. Although very little is known about the mechanisms of small RNA/dsRNA trafficking between host cells and fungal cells, the presence of numerous vesicles in the extrahaustorial matrix suggests that they may transfer silencing signals between the two organisms (Micali *et al*., 2011). Consistent with this hypothesis, two recent studies provide convincing evidence that plant extracellular vesicles (EVs) are essential to deliver antifungal small RNAs into *B. cinerea* cells as well as antioomycete small RNAs into *Phytophthora capsici* cells (Cai *et al.,* 2018, Koch *et al.,* 2013).

### Cross-kingdom RNAi can be exploited to confer protection against pathogens possessing a canonical RNA silencing machinery

The biological relevance of cross-kingdom RNAi has been initially demonstrated by expressing dsRNAs bearing homologies to vital or pathogenicity factors from a given parasite or pest provided that they possess a canonical RNAi machinery (e.g. functional DCL and AGO proteins). So far, this Host-Induced Gene Silencing (HIGS) technology has been successfully used to protect plants from invasion and predation of insects, nematodes, oomycetes, fungi, bacteria and parasitic plants (Singla-Rastogi et al., 2019; Singla-Rastogi, Navarro, PCT/EP2019/072169, PCT/EP2019/072170; WO 2012/155112, WO 2012/155109, CA 2 799 453, EP 2 405 013, US 2013/177539, Wang *et al.,* 2016, Koch *et al.,* 2016, Wang *et al.,* 2017). For example, HIGS confers full protection against *Fusarium graminearum* and *B. cinerea* and this phenomenon is fully recapitulated by spraying relevant exogenous dsRNAs or siRNAs onto wild type plants prior fungal infections (Wang *et al.,* 2016, Koch *et al.,* 2016, Wang *et al.,* 2017). The latter phenomenon is referred to as Spray-Induced Gene Silencing (SIGS) and is reminiscent of 'environmental RNAi', a process involving the uptake of RNAs from the environment initially described in *Caenorhabditis elegans* and in some insects (Wang *et al*., 2016, Wang *et al.,* 2017, Lacombe *et al.,* 2010). HIGS/SIGS is thus considered as a powerful complement, or even sometimes an alternative, to conventional breeding or genetic engineering designed to introduce disease resistance genes in agriculturally relevant crops (Jones *et al*., 2014, Mansfield *et al*., 2012, Escobar *et al*., 2001). Furthermore, this technology provides a more durable and environmentally friendly plant protection solution that will likely contribute to a reduced use of agrochemicals, which can have, in some instances, significant impact on human health and on the environment.

However, for the industrialization and manufacturing of such RNA-based biologicals, there is a need to establish methods to produce, at a high-throughput level and in a cost-effective manner, vectorized small RNAs conferring efficient protection towards phytopathogens.

The present invention fulfills this need. As a matter of fact, it is herein proposed a method to produce high amounts of EV-embedded small RNAs that can be effective on a wide range of phytopathogen(s), and that can be applied on various plant tissues, prior to and / or after an infection. It can also be delivered into woody plants by trunk injection or petiole absorption and systemically transported towards the targeted vascular phytopathogen(s). This approach will therefore have major agricultural applications in disease management through the use of a novel generation of microalgae-based biologicals.

Furthermore, as EV-embedded small RNAs were found resistant to ribonuclease-mediated digestion, it is anticipated that this method will be suitable to protect the integrity of antimicrobial small RNAs in the environment, before being internalized by the targeted pathogen(s), in which they will trigger the desired anti-virulence and/or anti-survival effects.

In addition, because *Chlorella* can be engineered to produce siRNA populations, bearing sequence homologies to large portions of virulence factors, essential genes and/or antimicrobial resistance genes of the targeted pathogen(s), the proposed approach should confer durable resistance. This is a major distinction from synthetic pesticides, which often become ineffective within a few years due to pathogen escape mutations.

Finally, it can be anticipated that the herein described technology will also be useful to control the expression of genes from beneficial microbes in order to enhance their multiplication and / or their beneficial effects towards host plants.

### DETAILLED DESCRIPTION OF THE INVENTION

### Overview of the results and the invention

In the results below, the Inventors herein provide the first evidence that *Chlorella* cells can produce extracellular vesicles (EVs), including exosome-like particles. They also demonstrate for the first time that *Chlorella* can be engineered to produce biologically active antibacterial small RNAs embedded into EVs. More specifically, by transforming *C*. *vulgaris* with an inverted repeat transgene bearing sequence homology with key virulence factors from a phytopathogenic bacterium, they show that *Chlorella* EVs are competent in delivering effective siRNAs in bacterial cells, resulting in the dampening of pathogenicity. Furthermore, they show that *Chlorella* EVs efficiently protect these antibacterial siRNAs from digestion mediated by the non-specific micrococcal nuclease. These data therefore highlight the tremendous potential of *Chlorella* EVs as vehicles of small RNAs towards bacterial pathogens. Furthermore, because plant EVs are known to deliver effective antimicrobial small RNAs in phytopathogenic fungi and oomycetes (Cai *et al*., 2018; Hou *et al*., 2019), it is anticipated that *Chlorella* EVs will be employed to transfer active small RNAs in a wide range of phytopathogens including bacterial, fungal and oomycetal organisms.

A pre-requisite for the industrialization of *Chlorella* EV-embedded small RNA products is to demonstrate that they can maintain a full integrity when produced in photobioreactors (PBRs). To address this issue, the inventors have first grown a *Chlorella* reference line producing antibacterial siRNAs in a PBR of one liter, collected the corresponding extracellular medium, which was further stored frozen. The extracellular medium was subsequently thawed and subjected to ultrafiltration and ultracentrifugation, to recover purified EVs. Importantly, these *Chlorella* EVs were found to exhibit a normal size distribution. Furthermore, comparable results were obtained when the same *Chlorella* transgenic line was grown in a PBR of 150 liters, except that in those conditions, the yield of recovered EV particles was ∼20 times enhanced compared than the ones collected from flasks or 1L PBRs. These findings indicate that the integrity of *Chlorella* EV-embedded siRNAs remain unaltered when produced in PBRs, despite being stored frozen. The Microalgae-Induced Gene Silencing (MIGS) technology is thus suitable for high yield production of EV-embedded antimicrobial small RNAs in PBRs.

Another pre-requisite for the industrialization of *Chlorella* EV-embedded antimicrobial small RNAs, is to verify -in a rapid, reliable and cost-effective manner- the efficacy of each batch produced from PBRs. To address this issue, the inventors have designed and engineered bacterially expressed small RNA reporter systems, which rely on the differential fluorescence or bioluminescence signal detection in the presence of effective *Chlorella* EV-embedded antimicrobial small RNAs. These quantitative reporters can be easily generated and manipulated to ensure that each batch produced is highly active prior product manufacturing.

Based on all these discoveries, the present Inventors propose to use this MIGS technology to rapidly produce *Chlorella* EV-embedded small RNAs directed against any phytopathogen(s) of interest. More precisely, they propose a method to produce high yields of *Chlorella* EV-embedded small RNAs targeting one or multiple target pathogen(s), i) by expressing iRNA molecules (precursors of siRNAs and miRNAs) in *Chlorella* cells, ii) collecting the EVs released by said *Chlorella* cells, iii) verifying the efficacy of *Chlorella* EV-embedded siRNAs prior product manufacturing, and iv) delivering the concentrated or purified EV products on plants (*i.e.* on leaf surface, seeds or in the vasculature, notably through trunk injection or petiole absorption in the case of woody plants). It is noteworthy that during such production pipeline, both the extracellular medium carrying the effective EVs, or purified EVs, can be stored frozen without any negative impact on the integrity of these EV-based anti-infective agents.

It is therefore anticipated that the MIGS technology will be extensively used for biocontrol applications against a wide spectrum of phytopathogens.

*The following features and advantages of the methods of the invention are worth to be mentioned.*
1) *Chlorella* is an ideal biological system for the production of endogenous and heterologous molecules:
   *Chlorella* belongs to a group of green microalgae (Chlorophyta, Trebuxiophyceae) able to adapt and grow in a variety of conditions. *Chlorella* is easy to maintain in laboratory conditions, possess a simple life cycle, a haploid genome and metabolic pathways similar to higher plants (Blanc et al., 2010). It also possesses the capacity to grow in auto-, hetero- or mixo-trophic conditions with high growth rates (Zuniga et al., 2016). The metabolic flexibility, the ease of maintenance and growth are features that enable *Chlorella* to be exploited as industrial production scaffold in PBRs for a variety of molecules of interest. In particular, *Chlorella* cells can be easily transformed with a disarmed *Agrobacterium tumefaciens* (Cha et al., 2012), and stable transformed transgenic lines can be selected within a 2 months period. This exceptional rapid selection process positions *Chlorella* as an ideal biological system to produce within a short timeframe any construct of interest. This feature is notably valuable in the context of outbreak situations, as *Chlorella* can be exploited to rapidly produce vectorized siRNAs against virulence factors, essential genes and/or antimicrobial resistance genes from any plant pathogen(s) of interest (which can nowadays be sequenced within a few days).
   To summarize, the possibility to rapidly transform *Chlorella* with a transgene of interest, and to obtain large volumes of *Chlorella* extracellular media from PBRs, shows that this green microalga is suitable for the industrial production of dedicated EV-embedded antimicrobial small RNAs.
2) The MIGS technology is highly versatile and sequence-specific.
   The MIGS technology relies on the stable expression of inverted repeat, artificial miRNAs or sense-antisense, transgenes in *Chlorella,* which will be processed into siRNAs or miRNAs by the endogenous Dicer-like enzyme and further internalized into EVs. The MIGS technology can also rely on the production of RNAi precursors from recombinant viruses that can infect *Chlorella* cells and likewise generate high yields of siRNA populations through Virus-Induced Gene Silencing (VIGS), as previously described in plants. These transgene- and viral-based RNAi precursors can notably be designed in such a way that they will target one or multiple genes of interest and trigger their selective silencing. This feature is particularly valuable for controlling the replication of one or multiple plant pathogens, while having no side effects on the cultivated plants, their associated-commensal microbes, or the animals that feed on those plants, including humans.
3) The MIGS technology can be used to produce antimicrobial siRNA populations, likely conferring durable disease resistance.
   *Chlorella* can be employed to produce siRNA populations targeting up to 1500 bp long regions from a single gene or up to a dozen genes. *Chlorella* is thus well-suited to produce siRNAs covering large portion of microbial gene(s), thereby maximizing the chance of detecting a potent silencing effect towards the targeted microbial gene(s). Furthermore, by targeting long sequence regions, the microbe will unlikely be able to mutate all along the targeted region, thereby resulting in long-lasting protection effects against the targeted plant pathogen(s). The MIGS technology is thus expected to overcome the recurrent problem of pathogen-directed escaping mutations and is therefore expected to confer durable disease resistance.
4) The MIGS technology is effective against prokaryotic cells, which is not the case of other platforms producing long dsRNAs.

The present inventors have previously reported that the exogenous application of siRNAs can trigger gene silencing in bacterial pathogens (Singla-Rastogi et al., 2019; Singla-Rastogi, Navarro, PCT/EP2019/072169, PCT/EP2019/072170). By contrast, long dsRNAs were not active in this process, suggesting that they are either not taken-up by, or not active in, bacterial cells. This is a major distinction from environmental RNAi previously reported in nematodes and plant herbivores, which exclusively relies on long dsRNAs (Bolognesi et al., 2012; Ivashuta et al., 2015; Whangbo et al., 2008), or in fungi and oomycetes, which is dependent on both small RNA and long dsRNA species (Koch et al., 2016; Wang et al., 2016). By producing siRNA species, the MIGS technology has therefore the potential of being exploited as a new production scaffold for antibacterial agents, which is not the case of other industrial biological systems currently exploited to produce long dsRNAs as fungicides, insecticides or nematicides for agricultural applications. The MIGS technology can also be employed to selectively target genes from symbiotic or commensal bacteria to enhance their beneficial effects for host plants. MIGS is therefore a unique RNAi production scaffold technology that can be exploited towards prokaryotic cells, and likely many other unrelated microbes and pests.

### Definitions

As used herein, the term "functional interfering RNA" (functional iRNA) refers to a RNA molecule capable of inducing the process of sequence-specific silencing of at least one gene(s). In particular, said functional interfering RNA molecule can be either i) a small interfering RNA, well-known in the art as small or short interfering RNA (siRNA) molecule (simplex or duplex), or a precursor thereof, or ii) a microRNA (miRNA) molecule (simplex or duplex) or a precursor thereof.

iRNA is a conserved gene regulatory mechanism that promotes antiviral resistance in plants, flies, worms and mammals (Guo *et al*., 2019). The core mechanism of antiviral silencing involves the recognition and processing of viral double-stranded RNAs (dsRNAs) by the RNAse III enzyme DICER leading to the production of 20-25 nt long short interfering RNA (siRNA) duplexes. These siRNA duplexes subsequently bind to a central component of the RNA Induced Silencing Complex (RISC), namely the Argonaute (AGO) protein, and one strand, the guide, remains bound to AGO to silence post-transcriptionally complementary viral transcripts. Recent studies have shown that plant and/or animal endogenous small RNAs can additionally directly target virulence or essential genes from fungi, bacterial and oomycete pathogens, supporting a broad role for RNAi in trans-kingdom gene regulation during host-pathogen interactions (Guo *et al.,* 2019; Cai *et al*., 2018).

The term "precursor of siRNA" or "siRNA precursor" herein refers to a RNA molecule which can be, directly or indirectly, processed into siRNA duplex(es) in *Chlorella* cells (or *Chlorella* extracts). Examples of siRNA precursors that can be directly processed include long double-stranded RNA (long dsRNA), while examples of siRNA precursors that can be indirectly processed include long single-stranded RNA (long ssRNA) that can be used as template for the production of processable long dsRNAs.

The term "precursor of miRNA" or "miRNA precursor" herein refers to an RNA molecule which can be processed into miRNA duplex(es) in *Chlorella* cells (or *Chlorella* extracts). Examples of miRNA precursors include primary miRNA precursors (pri-miRNAs) and pre-miRNAs, comprising a hairpin loop.

Of note, plasmids or vectors and other DNA constructs or viral vectors encoding said precursor molecules are also encompassed in the definition of "functional interfering iRNA".

For targeting multiple genes, the method of the invention can use i) a mixture of several different iRNAs which altogether target multiple genes of interest or ii) a chimeric iRNA targeting several different genes of interest or iii) a mixture of any of these chimeric iRNAs.

In one particular embodiment, the method / use of the invention comprises the introduction of one or several long functional iRNAs into *Chlorella* cells as precursors, and these cells will produce the small RNAs (such as siRNAs or miRNAs) that can be further formulated and used to prevent pathogenic infections.

These long functional iRNAs can be long single-stranded RNA molecules (named hereafter as "long ssRNAs"). These long ssRNA may be introduced in a *Chlorella* cell, converted into long dsRNA molecules, and further processed into siRNAs by the *Chlorella* DCL enzyme. Alternatively, long ssRNA may be produced by a RNA virus that can infect *Chlorella* cells and further converted into long dsRNA molecules during viral replication (as replicative intermediates). The resulting viral dsRNA is subsequently processed into siRNAs by the *Chlorella* DCL enzyme.

As used herein, the term "long ssRNA" designates single-stranded structures containing a single-strand of at least 50 bases, more preferably of 80 to 3000 bases. Long ssRNAs may contain 80 to 3000 bases when produced by a *Chlorella* transgene, but preferably contain 80 to 1500 bases when produced by a recombinant RNA virus.

These long functional iRNAs can also be double-stranded RNA molecules (named hereafter as "long dsRNAs"). These long dsRNAs act as miRNA or siRNA precursors and can be processed into miRNAs or siRNAs in *Chlorella* cells, thanks to the DCL proteins encoded by *Chlorella* genomes (see EXAMPLE 3).

As used herein, the term "long dsRNA" designates double-stranded structures containing a first (sense strand) and a second (antisense) strand of at least 50 base pairs, more preferably of 80 to 3000 base pairs.

The results of the present inventors show that, in *Chlorella* cells, long dsRNAs can be efficiently processed into effective small RNAs (EXAMPLE 3). Such long dsRNAs are advantageously chimeric dsRNA, *i.e.,* they bear sequence homologies to multiple genes.

The long functional iRNA used in the method of the invention is preferably a long dsRNA that is cleavable by the DCL enzyme in *Chlorella* cells so as to generate miRNAs or siRNAs in *Chlorella* cells.

These long dsRNAs can be generated from a hairpin structure, through sense-antisense transcription constructs, through an artificial sense transcript construct further used as a substrate for the production of long dsRNAs, or through VIGS. More precisely, they may comprise bulges, loops or wobble base pairs to modulate the activity of the dsRNA molecule so as to mediate efficient RNA interference in the target cells. The complementary sense and antisense regions of these long dsRNA molecules may be connected by means of nucleic acid based or non-nucleic acid based linker(s). These long dsRNA may also comprise one duplex structure and one loop structure to form a symmetric or asymmetric hairpin secondary structure.

In a particular embodiment, the precursor of the invention can simultaneously target several *Xylella fastidiosa* gene regions of ∼150-250 bp and have for example the following sequences: IR*-cheA*/*gaCA*/*tolC*/*pglAlengXCA1*/*engXCA2* = SEQ ID NO: 1-2; IR-*cheA*/*GumH*/*GumD*/*XpsE*/*LesA*/*HolC* = SEQ ID NO: 3-4; IR-*LesA*/*gumH*/*gumD =* SEQ ID NO: 5-6; IR-*XpsE*/*HolC*/*LesA* = SEQ ID NO: 7-8; IR*-cheA*/*pglA*/*LesA* = SEQ ID NO: 9-10; IR-*cheA*/*engXCA1*/*engXCA2=* SEQ ID NO: 11-12; IR*-gacA*/*pglA*/*engXCA2* = SEQ ID NO:13-14 and IR*-cheA*/*tolC*/*engXCA1* = SEQ ID NO: 15-16.

In another particular embodiment (see EXAMPLE 7), the precursor of the invention can target essential genes from *Candidatus Liberibacter asiaticus* and have the following sequences: IR-*wp015452784*/*WP012778510*/*wp015452939*/*act56857*/*wp012778668*= SEQ ID NO: 55-56; IR-*wp012778355*/*wp015452784*/*WP012778510* = SEQ ID NO: 57-58 and IR-*wp015452939*/*act56857*/*wp012778668* = SEQ ID NO: 59-60.

In another particular embodiment, the precursor of the invention can target key virulence genes from *Erwinia carotovora* with the following sequences: IR-*GyrB*/*MreB*/*rbfA*/*RsgA*/*FliA*/*QseC* = SEQ ID NO: 47-48; IR-*GyrB*/*rbfA*/*QseC =* SEQ ID NO: 49-50; IR*-fliA*/*MreB*/*QseC* =SEQ ID NO: 51-52 and IR*-GyrB*/*MreB*/*RsgA* = SEQ ID NO: 53-54.

In another particular embodiment, the precursor of the invention can target key genes from *Pseudomonas syringae pv. actinidiae* and have the following sequences: IR-*GyrB*/*Hfq*/*HrpR*/*HRPS*/*MreB*/*RpoD* = SEQ ID NO:61-62; IR*-GyrB*/*Hfq*/*MreB* = SEQ ID NO:63-64; IR*-HrpR*/*HrpS*/*Hfq =* SEQ ID NO:65-66 and IR*-HrpR*/*GyrB*/*RpoD* = SEQ ID NO:67/148.

In another particular embodiment, the precursor of the invention can target essential genes of *Pseudomonas syringae pv. tomato* strain *DC3000* have the following sequences IR-*CFA6*/*HRPL* = SEQ ID NO:90-91; IR*-HRPL* = SEQ ID NO:92-93; IR*-FusA* = SEQ ID NO:136-137; *IR-GyrB* = SEQ ID NO:138-139; IR*-FusA*/*GyrB* = SEQ ID NO:140-141; IR-*AvrPto*/*AvrPtoB =* SEQ ID NO:94-95; IR*-AvrPto*/*AvrPtoB*/*HopT1-1* = SEQ ID NO: 130-131; IR*-rpoB*/*rpoC*/*FusA* = SEQ ID NO:132-133 ; IR*-secE*/*rpoA*/*rplQ =* SEQ ID NO:134-135 ; IR-*secE =* SEQ ID NO:142-143; IR*-GyrB*/*hrpL* = SEQ ID NO:144-145 and IR*-dnaA*/*dnaN*/*gyrB* = SEQ ID NO:146-147.

In another particular embodiment, the precursor of the invention can target virulence genes of *Ralstonia solanacearum* with the construct IR-HRPH/HRPB/HRCC = SEQ ID NO:99-98; IR-*HRPB*/*HRCC*/*TssB*/*XpsR-* SEQ ID NO: 100-101.

In another particular embodiment, the precursor of the invention can target virulence genes of *Xanthomonas campestris pv. campestris* with the construct IR-*HRPG*/*HRPX*/*RsmA* = SEQ ID NO:102-103; IR-*NadHb*/*NadHd*/*NadHe* = SEQ ID NO:104-105 and IR*-DnaA*/*DnaE1*/*DnaE2* = SEQ ID NO:106-107.

In another particular embodiment, the precursor of the invention can target essential genes from *Xanthomonas hortorum pv. vitians* and have the following sequences: *IR-GyrB* = SEQ ID NO:17-18; *IR-FusA* = SEQ ID NO:19-20; IR*-ZipA* = SEQ ID NO:21-22 and IR-*GyrB*/*FusA*/*ZipA* = SEQ ID NO:128-129.

In another particular embodiment, the precursor of the invention can target essential genes from *Xanthomonas citri pv. fucan* and have the following sequences: IR-*GyrB*/*FusA*/*MreB*/*HrpG*/*PhoP*/*FhaB* = SEQ ID NO:120-121; IR*-GyrB*/*RbfA*/*MreB* = SEQ ID NO:122-123; IR-*HrpG*/*PhoP*/*FtsZ* = SEQ ID NO:124-125; IR*-FhaB*/*FusA*/*MreB* = SEQ ID NO:126-127 and IR*-GyrB*/*FusA*/*ZipA* = SEQ ID NO:128-129.

In another particular embodiment, the precursor of the invention can target essential genes from *Acidovorax valerianella* and have the following sequences: IR-*rimM*/*rsgA*/*rbfA*/*MreB*/*gyrB*/*FtsZ* = SEQ ID NO:23-24; IR-*rimM*/*rbfA*/*FtsZ =* SEQ ID NO:25-*26;* IR-*RsgA*/*gyrB*/*MreB =* SEQ ID NO:27-28 and IR-*RsgA*/*rbfA*/*FtsZ* = SEQ ID NO:29-30;

In another particular embodiment, the precursor of the invention can essential genes from *Acidovorax citrulli* and have the following sequences: IR-*MreB*/*ybeY*/*rbfA*/*gyrB*/*FtsZ*/*rsgA* = SEQ ID NO:31-32; IR-*MreB*/*ybeY*/*rbfA* = SEQ ID NO:33-34; IR*-rsgA*/*gyrB*/*MreB* = SEQ ID NO:35-36 and IR-*YbeY*/*RsgA*/*MreB* = SEQ ID NO:37-38.

In another particular embodiment, the precursor of the invention can target essential genes from *Botrytis cinerea* and have the following sequences: IR-*TOR*/*CGF1*/*DCL1*/*DCL2*/*LTF1*/*HBF1* = SEQ ID NO:76-77; IR-*TOR*/*DCL1*/*DCL2* = SEQ ID NO:78-79; IR*-CGF1*/*HBF1*/*LTF1* = SEQ ID NO:80-81 and IR*-DCL1*/*HBF1*/*TOR* = SEQ ID NO:82-83;

In another particular embodiment, the precursor of the invention can target essential genes from *Zymoseptoria tritici* and have the following sequences: IR*-Br1A2*/*StuA*/*F1bc*/*PKS1*/*PPT1* = SEQ ID NO:108-109; IR-*Br1A2*/*StuA*/*F1bc* = SEQ ID NO:110-111 and IR-*StuA*/*PKS*/*PPT1* = SEQ ID NO:112-113.

In another particular embodiment, the precursor of the invention can target essential genes from *Phytophthora infestans* and have the following sequences: IR-*NPP1*/*INF1*/*GK4*/*piacwp1-1*/*piacwp1-2*/*piacwp1-3* = SEQ ID NO:68-69; IR*-piacwp1-1*/*piacwp1-2*/*piacwp1-3* = SEQ ID NO:70-71; IR*-GK4*/*INF1*/*NPP1* = SEQ ID NO:72-73 and IR-*GK4*/*INF1*/*piacwp1-1* = SEQ ID NO:74-75.

In another particular embodiment, the precursor of the invention can target essential genes from *Plasmopara viticola* and have the following sequences: IR-*PITG_17947*/*PITG_10772*/*PITG_13671*/*PITG_16956*/*PITG_00891* = SEQ ID NO:114-115; IR-*PITG_17947*/*PITG_10772*/*PITG_13671* = SEQ ID NO:116-117 and IR-*PITG_13671*/*PITG_16956*/*PITG_00891* = SEQ ID NO:118-119.

In another particular embodiment, the precursor of the invention can target viral genes from *Plum Pox Virus (PPV)* and have the following sequences: IR*-P1*/*HC-Pro*/*CP* = SEQ ID NO:39-40; IR*-P1* = SEQ ID NO:41-42; IR*-HC-Pro* = SEQ ID NO:43-44 and *IR-CP* = SEQ ID NO:45-46.

In another particular embodiment, the precursor of the invention can target essential genes from *Colletotrichum species* and have the following sequences: IR-*CclA*/*ACS1*/*ACS2*/*ELP1*/*ELP2*/*MOB2* = SEQ ID NO:84-85; IR-*CclA*/*ACS1*/*ACS2* = SEQ ID NO:86-87 and IR*-ELP1*/*ELP2*/*MOB2* = SEQ ID NO:88-89;

In another particular embodiment, the precursor of the invention can target essential genes from *Fusarium graminearum* and have the following sequence: IR*-CYP51A*/*CYP51B*/*CYP51C* = SEQ ID NO:96-97.

The present invention targets the use of any of these siRNA precursors of SEQ ID NO:1-148 to produce a population of functional small iRNAs in *Chlorella* cells.

As demonstrated in the examples of the present application, the introduction of dsRNA into *Chlorella* cells triggers the production of small RNA molecules that are embedded into EVs and therefore protected from ribonuclease-mediated digestion (EXAMPLE 4). More precisely, the *Chlorella* cells of the invention are able to produce functional small iRNAs such as siRNAs or miRNAs. These small RNAs have a short size, which is less than 50 base pairs, preferably comprised between 15 and 30 base pairs.

In one particularly preferred embodiment, the functional interfering small RNA of the invention is a "siRNA", which designates either a "siRNA duplex" or a "siRNA simplex".

More specifically, the term "siRNA duplex" designates double-stranded structures or duplex molecules containing a first (sense strand) and a second (antisense) strand of at least 15 base pairs, preferably of at least 19 base pairs; preferably, said antisense strand comprises a region of at least 15 contiguous nucleotides that are complementary to a transcript of the targeted gene. These siRNA duplexes can be produced from long dsRNA precursors that are processed by the *Chlorella* DCL enzyme.

As used herein, the term "siRNA simplex" or "mature siRNA" designates simplex molecules (also known as "single-stranded" molecules) that originate from the siRNA duplex but have been matured in the RISC machinery of a microalgae cell and are loaded in the *Chlorella* AGO protein and / or associated with other RNA-binding proteins. They have a short size, which is less than 50 bases, preferably between 15 and 30 bases.

In another embodiment, the functional iRNA of the invention is a "miRNA", which designates either a "miRNA duplex" or a "miRNA simplex". In a preferred embodiment, the iRNAs of the invention are double-stranded miRNAs.

More specifically, the term "miRNA duplex" designates double-stranded structures or duplex molecules containing a first (sense strand) and a second (antisense) strand of at least 15 base pairs, preferably of at least 19 base pairs; preferably, said antisense strand comprises a region of at least 15 contiguous nucleotides that are complementary to a transcript of the targeted gene. These miRNA duplexes may also contain bulges. These miRNA duplexes can be produced from miRNA precursors that are processed by the *Chlorella* DCL enzyme. As the duplex siRNAs, they have short size, which is less than 50 base pairs, preferably comprised between 15 and 30 base pairs.

As used herein, the term "miRNA simplex" or "mature miRNA" designates simplex molecules (also known as "single-stranded" molecules) that originate from the miRNA duplex but have been matured in the RISC machinery of a microalgae cell and are loaded in the *Chlorella* AGO protein and / or associated with other RNA-binding proteins.

Methods to design iRNAs such as long dsRNAs that can be converted into siRNA/miRNA are available in the art and can be used to obtain the sequence of the precursors of the invention.

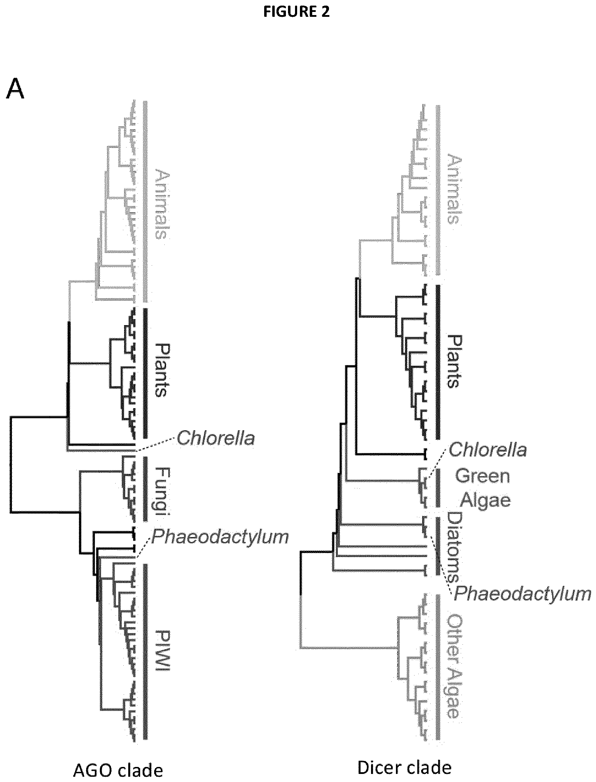

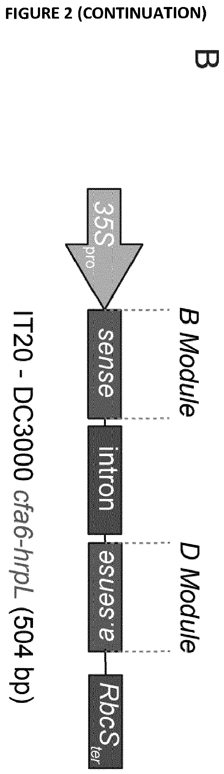

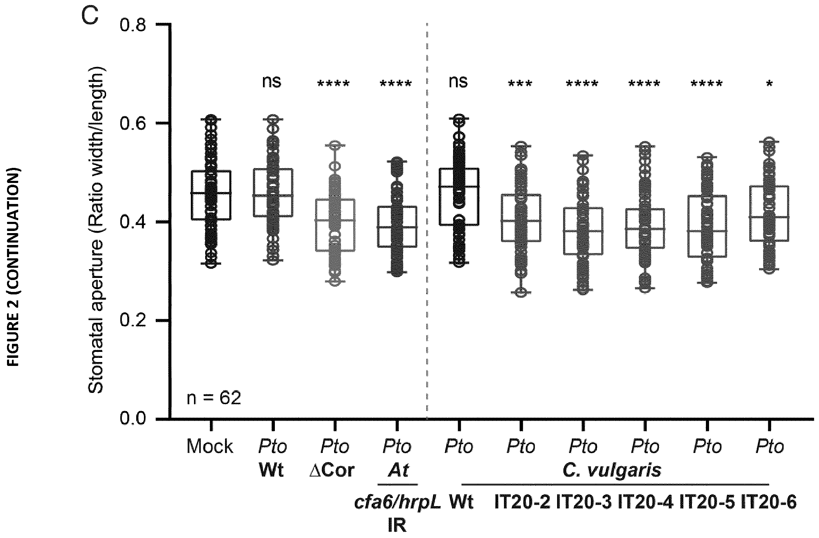

The inventors herein show (EXAMPLES 2-6) that it is possible to use long double-stranded inverted repeat constructs in order to (i) transform *Chlorella* cells efficiently and (ii) have them produce functional small iRNAs that can dampen pathogenicity, said small iRNAs being embedded into EVs, which protect these RNA entities from ribonuclease-mediated digestion (see Figures 2-4).

As used herein, the term "sequence homology" refers to sequences that have sequence similarity, *i.e.,* a sufficient degree of identity or correspondence between nucleic acid sequences. In the context of the invention, two nucleotide sequences share "sequence homology" when at least about 80%, alternatively at least about 81%, alternatively at least about 82%, alternatively at least about 83%, alternatively at least about 84%, alternatively at least about 85%, alternatively at least about 86%, alternatively at least about 87%, alternatively at least about 88%, alternatively at least about 89%, alternatively at least about 90%, alternatively at least about 91%, alternatively at least about 92%, alternatively at least about 93%, alternatively at least about 94%, alternatively at least about 95%, alternatively at least about 96%, alternatively at least about 97%, alternatively at least about 98%, alternatively at least about 99% of the nucleotides are similar.

Conversely, nucleotide sequences that have "no sequence homology" are nucleotide sequences that have a degree of identity of less than about 10%, alternatively of less than about 5%, alternatively of less than 2%.

Preferably, the similar or homologous nucleotide sequences are identified by using the algorithm of Needleman and Wunsch. Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. By "equivalent program" is intended any sequence comparison program that, for any two sequences in question, generates an alignment having identical nucleotide residue matches and an identical percent sequence identity when compared to the corresponding alignment generated by GAP Version 10.

### Production methods of the invention

In a first aspect, the present invention is drawn to a method for producing functional interfering small RNAs, said method comprising at least the steps of:
a) transforming *Chlorella* cells with a siRNA or miRNA precursor comprising a fragment of at least one target gene, and
b) cultivating said *Chlorella* cells in appropriate conditions so that they express said precursor and release EV-embedded functional small iRNAs targeting said gene fragment.

The terms "interfering small RNA" and "siRNA or miRNA precursor" have been defined above, in the definition section.

In a preferred embodiment, said siRNA or miRNA precursor is a long single- or double-stranded RNA molecule. In a more preferred embodiment, said siRNA or miRNA precursor is a long double-stranded RNA molecule, said molecule comprising a fragment of at least one target gene, or a complementary sequence thereof.

As explained above, *Chlorella* cells can be transformed by large nucleotide constructs. More precisely, the targeted fragment contained in the said precursor can have a large size, e.g., up to 3000 bp.

The "fragment" contained in the precursor of the invention can in fact contain one or several portion(s) of one single gene, or several portion of several genes (see the EXAMPLES 9 and 10 below). After transformation, the *Chlorella* cells will then produce siRNA populations targeting one or various portions from a single gene or from several genes. This is a clear advantage over other iRNA producer cells, as covering large portions of microbial/parasitic gene(s) maximizes the chance of triggering an effective silencing effect towards the targeted gene(s) and reduces the chance that the microbe/parasite acquire resistance against the small iRNA population (to do so, it will have to mutate all along the small RNA targeted portions), thereby resulting in long-lasting protection effects against the targeted pathogen(s). It is also possible to design and use a precursor that contains one or more portions of genes from several pathogens (they will be called "chimeric precursors", see below).

In a particular embodiment, the fragment of the target gene(s) contained in the precursor of the invention comprises between 50 and 3000 bp, preferably between 100 bp and 2000 bp, more preferably between 500 bp and 1500 bp.

Particular "target genes" are described below, in the appropriate sections.

*Chlorella* is a genus of single-celled green algae belonging to the division Chlorophyta. It is spherical in shape, about 2 to 10 µm in diameter, and is without flagella. It contains the green photosynthetic pigments chlorophyll-a and -b in its chloroplast. In ideal conditions it multiplies rapidly, requiring only carbon dioxide, water, light, and a small amount of minerals to grow. Due to the elevated protein, vitamin, mineral and pigment content, various *Chlorella* cells are currently used as food complement for humans and livestock.

The *Chlorella* cells used in the method of the invention can be of any *Chlorella* species. In particular, they can be any cells that are currently used as food complement for humans and livestock (Safi et al, 2014). In a particular embodiment, they can belong to the species: *Chlorella ellipsoidea*, *Chlorella pyrenoidosa, Chlorella sorokiniana, Chlorella vulgaris* or *Chlorella variabilis.*

In a preferred embodiment, the *Chlorella* cells used in the method of the invention are from the *vulgaris* genus. As the other *Chlorella* cells, C. *vulgaris* cells are able to adapt and grow in a variety of conditions. They are easy to maintain in laboratory conditions, possess a simple life cycle, a haploid genome and metabolic pathways similar to higher plants. They also possess the capacity to grow in auto-, hetero- or mixo-trophic conditions with high growth rates (de Andrade et al, 2017). The metabolic flexibility, the ease of maintenance and growth are features that enable C. *vulgaris* to be exploited as industrial production scaffold in photobioreactors (PBRs) for a variety of molecules of interest (Lin et al., 2013; Blanc et al., 2010).

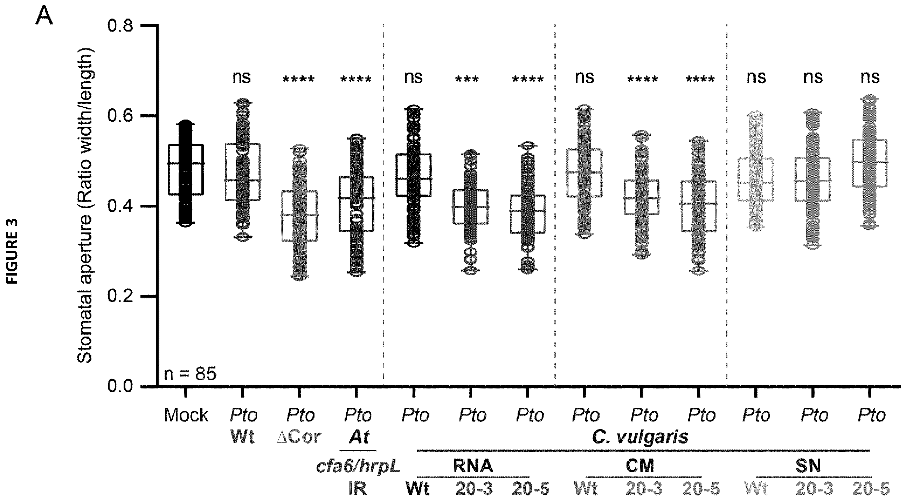

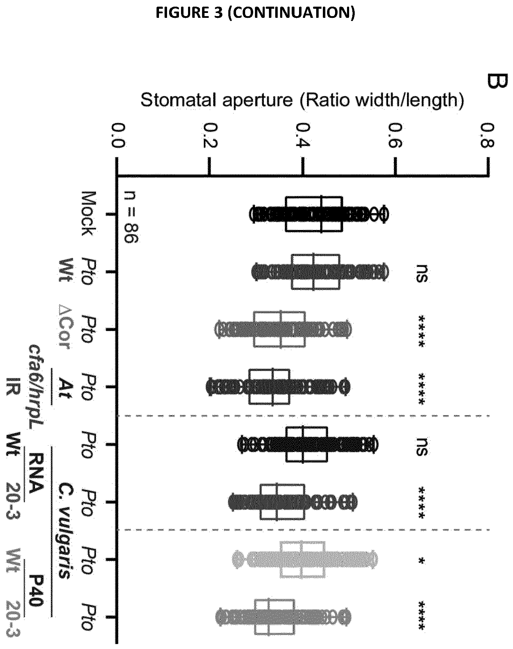

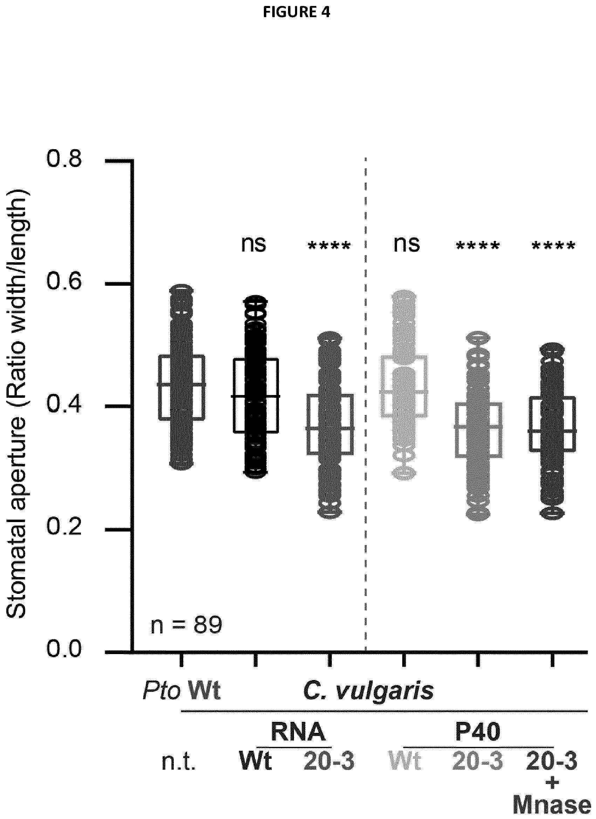

In a first step of the method of the invention, the siRNA or miRNA precursor of the invention is introduced in the selected *Chlorella* cells. Said siRNA or miRNA precursor will be processed into siRNA or miRNA duplexes by using the *Chlorella* DCL enzyme and other small RNA processing factors. Said small RNAs duplexes and / or mature small RNA guides (*i.e.* loaded into AGOs) are thereafter released in the extracellular medium, or at the surface of the *Chlorella* cells, embedded into Extracellular Vesicles. As demonstrated in the examples below (EXAMPLES 4 and 5 and figures 3 and 4), the virulence of bacterial cells is decreased when placed in contact with *Chlorella* EVs containing siRNAs.

The term "introduced" in the context of inserting a nucleic acid into a cell, means "transfection" or "transformation" or "transduction" and includes reference to the incorporation of a nucleic acid into a eukaryotic cell where the nucleic acid may be stably incorporated into the genome of the cell (*e.g.,* chromosome, plasmid), or transiently expressed (*e.g.,* transient delivery of a gene construct via *Agrobacterium tumefaciens,* an infection with a recombinant virus).

The expression of the iRNAs of the invention in the host *Chlorella* cell may be transient or stable. Stable expression refers in particular to the preparation of transgenic *Chlorella* cell lineages using conventional techniques.

By way of non-limitative examples, step a) of the method of the invention can be performed by using electroporation, projectile bombardment, PEG-mediated protoplast transformation, virus-mediated transformation, conjugation, *Agrobacterium-mediated* transformation, and the like. These transformation methods are described e.g. in Kim et al,. 2002; Cha et al,. 2012; Lin et al,. 2013; Yang et al,. 2015; Bai et al,. 2013; Niu et al,. 2011; Chien et al,. 2012; Run et al,. 2016.

In a preferred embodiment, step a) of the method of the invention involves the delivery of the gene construct into *Chlorella* cells by means of *Agrobacterium tumefaciens.* This technic is well-known and do not need to be explained (Cha et al,. 2012 ; Lin et al,. 2013).

In a particular embodiment, the method of the invention comprises introducing into *Chlorella* cells one or several dsRNAs targeting one or multiple genes of different parasites, such as viruses, fungi, oomycetes, bacteria, insects or nematodes. In this embodiment, the EVs are directed to an essential gene, to a virulence gene or an antimicrobial/antiparasitic resistance gene of several pathogens or parasites.

Such methods are useful for concomitant prevention or treatment of diseases caused on plants by several pathogens and/or parasites. They can be carried out using chimeric EVs carrying sequence homologies with different pathogenic/parasitic genes, or a cocktail of EVs that have been produced separately, some containing iRNAs bearing homologies to genes of one pathogen/parasites and others containing iRNAs bearing homologies to genes from other pathogens/parasites.

In a second step, the transformed *Chlorella* cells containing the precursors of the invention are cultivated so as to express said precursor and release EV-embedded functional small iRNAs targeting said gene fragment.

In this step, one can use classical conditions well-known in the art for cultivating *Chlorella* cells (Kim et al,. 2002; Cha et al,. 2012; Lin et al,. 2013; Yang et al,. 2015; Bai et al,. 2013; Niu et al,. 2011; Chien et al,. 2012; Run et al,. 2016). A particular cultivating method is described in EXAMPLE 1 below.

In one preferred embodiment, the small RNAs of the invention are isolated as free RNA molecules. These RNA molecules can be used directly for phytotherapeutic purposes (see EXAMPLES 4 and 5).

In this embodiment, the methods of the invention further comprise the step of recovering the expressed small iRNAs from the cultivated *Chlorella* cells.

Isolating intact RNA contained within *Chlorella* cells requires four steps: 1) Disruption of the *Chlorella* cells; 2) Inactivation of endogenous ribonuclease (RNase) activity; 3) Denaturation of nucleoprotein complexes; and 4) Removal of contaminating DNA and proteins. The most important step is the immediate inactivation of endogenous RNases that are released from membrane-bound organelles when cells are disrupted. RNA purification methods typically use silica membrane-based, resin-based and magnetic options for nucleic acid binding and incorporate DNase treatment to remove contaminating genomic DNA. Purified RNA is then eluted from the solid support.

RNA is notoriously susceptible to degradation and RNases are ubiquitous. Many commercially available RNA purification methods include specific chemicals to inactivate RNases present in cell or tissue lysates and may also include RNase inhibitors to safeguard against RNA degradation throughout the procedure. Any of these methods can be used to recover the small RNAs of the invention.

In another preferred embodiment, the small RNAs are not used as free RNA molecules, but they are embedded into extracellular vesicles (EVs). The present inventors have indeed shown that *Chlorella* cells can produce EVs which are in a size range that is similar to the one of plant exosomes, and that these EVs can be taken-up by plant cells, where they can deliver their small iRNA content and have effective silencing effect (see EXAMPLES 5 and 6). These *Chlorella* derived iRNA-containing EVs can be used for biocontrol purposes, as mammalian and plant-derived EVs are.

In this particularly preferred embodiment, the method of the invention further comprises the step of recovering the Extracellular Vesicles (EV) released by *Chlorella* cells in the extracellular medium.

In the context of the invention, recovering EVs can be done by any conventional means described in the art. Isolation and purification means are for example discussed in Colao et al., 2018. Downstream processing for efficient purification can be used to enrich EVs from cell culture media, e.g., by size-exclusion (based on typica diameters), sedimentation force or flotation density, precipitation-based methods and affinity-based capture. While differential ultracentrifugation can be used, other purification methods will be preferred, such as filtration or chromatic separation. Tangential-flow filtration is more promising, due to tight and reproducible size distributions and the ease with which processes can be scaled. Immunoaffinity methods can also be adjusted to the particular EVs of the invention.

### EVs obtained by the method of the invention

Extracellular Vesicles (EVs) are nanosized, membrane-bound vesicles that are released into the extracellular space and transport cargoes towards recipient cells. Mammalian EVs are in part composed of exosomes, which are formed by the fusion between multivesicular bodies (MVBs) and the plasma membrane, in which MVBs release vesicles whose diameters range from 40 to 150 nanometers (O' Brien *et al.,* 2020). During the last decade, mammalian exosomes have been extensively characterized as vehicles of miRNAs. Interestingly, emerging evidence indicates that plant-derived EVs can also operate as carriers of miRNAs or siRNAs (Wang *et al*., 2013; Zhang *et al*., 2016; Cai et al., 2018; Hou et al., 2019).

In another aspect, the present invention is drawn to the Extracellular Vesicles (EVs) obtained by the method of the invention, as disclosed above. These EV contain a population of functional small iRNAs targeting one or several region(s) in the target gene(s) of interest.

In a preferred embodiment, the EVs of the invention preferably contain a population of functional small iRNAs that targets several regions in one or several viral gene(s). Accordingly, these EVs can be used as anti-viral agents.

For example, these anti-viral EVs can contain a population of functional small iRNAs that targets one or several regions of one or several viral gene(s) that are critical for the replication or the pathogenicity of the Plum pox virus, responsible of the Sharka disease.

In another preferred embodiment, these EVs of the invention preferably contain a population of functional small iRNAs that targets several regions in one or several bacterial gene(s). Accordingly, these EVs can be used as anti-bacterial agents.

For example, these anti-bacterial EVs can contain population of functional small iRNAs that targets one or several regions of one or several bacterial gene(s) that are critical for the replication or the pathogenicity of *Xylella fastidiosa*, *Candidatus liberibacter, Pseudomonas syringae pv. actinidiae, Pseudomonas syringae pv. tomato* strain DC3000, *Xanthomonas campestris pv. campestris, Ralstonia solanacearum, Erwinia carotovora*, *Xanthomonas hortorum, Acidovorax vallerianellae, Acidovorax citrulli.*

In another preferred embodiment, these EVs of the invention preferably contain a population of functional small iRNAs that targets several regions in one or several fungal gene(s). Accordingly, these EVs can be used as anti-fungal agents.

For example, these anti-fungal EVs can contain population of functional small iRNAs that targets one or several regions of one or several bacterial gene(s) that are critical for the replication or the pathogenicity of *Fusarium graminearum, Botrytis cinerea, Colletrotrichum species, Zynoseptoria tritici.*

In another preferred embodiment, these EVs of the invention preferably contain a population of functional small iRNAs that targets several regions in one or several oomycetal gene(s). Accordingly, these EVs can be used as anti-oomycetal agents.

For example, these anti-oomycetal EVs can contain population of functional small iRNAs that targets one or several regions of one or several bacterial gene(s) that are critical for the replication or the pathogenicity of *Phytophthora infestans, Plasmopara viticola.*

As explained above, purification of EVs can be performed by various methods. While differential ultracentrifugation can be used, other purification methods will be preferred for industrial purposes, such as filtration, chromatic separation, or affinity-purification methods.

### Phytotherapeutic compositions of the invention

It is noteworthy that the small RNAs of the invention contained within the natural Extracellular Vesicles (EVs) of the invention are protected from the action of RNases (EXAMPLE 6). iRNA-containing EVs can therefore be used efficiently and long lastingly in phytotherapeutic compositions as a tool to kill or dampen the infection of target pathogens.

In a further aspect, the present invention is thus drawn to phytotherapeutic compositions containing, as active principle, the small RNAs-embedded EVs of the invention. More precisely, they contain an effective amount of the *Chlorella-derived* EVs as defined above.

By "effective amount", it is herein meant an amount that has been shown to have an antipathogenic effect (e.g., an antibacterial or antiviral effect) in a test such as described in the examples below. This amount is preferably comprised between 0.05 and 100 pM of EVs containing effective small RNAs.

The phytotherapeutic compositions of the invention can be formulated in a physiological or agronomical acceptable carrier, excipient or diluent. Such carriers can be any material that the plant to be treated can tolerate. Furthermore, the carrier must be such that the composition remains effective at controlling the infection, and not toxic for animals or insects that feed on the treated plants. Examples of such carriers include water, saline, Ringer's solution, dextrose or other sugar solutions, Hank's solution, and other aqueous physiologically balanced salt solutions, phosphate buffer, bicarbonate buffer and Tris buffer.

The compositions of the invention can be supplied in a concentrated form, such as a concentrated aqueous solution. It may even be supplied in frozen form or in freeze-dried or lyophilized powder form. This latter may be more stable for long term storage and may be de-frosted and / or reconstituted with a suitable diluent immediately prior to use.

These compositions may furthermore contain a surface-active agent, an inert carrier, a preservative, a humectant, a feeding stimulant, an attractant, an encapsulating agent, a binder, an emulsifier, a dye, a UV protectant, a buffer, a flow agent or fertilizers, micronutrient donors, or other preparations that influence plant growth. One or more agrochemicals including, but not limited to, herbicides, insecticides, fungicides, bactericides, nematicides, molluscicides, acaricides, plant growth regulators, harvest aids, and fertilizers, can be combined with carriers, surfactants or adjuvants customarily employed in the art of formulation or other components to facilitate product handling and application for particular target bacteria. Suitable carriers and adjuvants can be solid or liquid and correspond to the substances ordinarily employed in formulation technology, e.g., natural or regenerated mineral substances, solvents, dispersants, wetting agents, tackifiers, binders, or fertilizers.

The compositions of the invention can be solid slow release formulations, surfactant diatomaceous earth formulations for pesticidal use in the form of dry spreadable granules, water-insoluble lipospheres formed of a solid hydrophobic core having a layer of a phospholipid embedded on the surface of the core, microcapsules, etc.

The nature of the excipients and the physical form of the composition may vary depending on the nature of the plant part that is desired to treat.

### Phytotherapeutic methods and uses of the invention

In another aspect of the invention, the present invention is drawn to phytotherapeutic methods involving the use of the EVs of the invention. These EVs can be used for treating any parasitic infection and/or infectious disease in a plant.

Said parasitic infection and/or infectious disease can be caused e.g., by a virus, a bacterium, a fungus, an oomycete, or any other pathogens or parasites.

The EVs of the invention can target a gene of said pathogen and/or a gene of the diseased host cultivated plants, if this/these gene(s) is/are known to facilitate the infection or to act as negative regulator(s) of defense.

In one aspect, the present invention relates to a method for treating a target plant against a pathogenic or parasitic infection, said method comprising the step of applying the EVs of the invention on a part of said plant.

This method is useful to avoid the contamination of plants and ensure their adequate growth and high yield of production.

In another aspect, the present invention therefore relates to an EV-based biocontrol method for treating plants against a pathogen or parasite infection, said method comprising the step of delivering the EVs of the invention, or a composition comprising these EVs on plant tissues, seeds, fruits, vegetables, prior to and / or after infections. Such delivery can also be performed by trunk injection or petiole absorption in the case of woody plants, as previously showed for synthetic siRNAs (Dalakouras et al., 2018). This infection can be due to any pathogen, such as bacteria, virus, fungus, oomycetes, or other parasites associated with plant organisms.

All these pathogens are described below.

Another aspect of the invention relates to the use of EVs of the invention, as defined above, as a phytotherapeutic agent. Preferably, said EVs are used for treating a disease caused by a pathogenic bacterium in plants or for preventing a bacterial infection in plants.

In one embodiment, these phytotherapeutic EVs or compositions containing thereof contain siRNA duplex or miRNA duplex molecules, as defined above. In yet another embodiment, these EVs targets bacterial genes and genes of other non-bacterial pathogens or parasites, as defined above, for concomitant prevention or treatment of diseases caused by bacterial pathogens and other pathogens/parasites in plants. All the embodiments proposed above for the EVs, iRNAs, the vectors, and the transformation methods are herewith encompassed and do not need to be repeated.

The EVs of the present invention can be applied to the crop area, plant, reproductive organs, fruits, seed and roots to be infected or that is already infected.

Methods of applying the EVs or a composition that contains the EVs of the invention include, but are not limited to, petiole absorption, trunk injection, foliar application, seed coating, and soil application. The number of applications and the rate of application depend on the intensity of the infection.

Specifically, the compositions of the invention can be applied to the plants by, for example, spraying, atomizing, dusting, scattering, coating or pouring, introducing into or on the soil, introducing into irrigation water, by seed treatment or general application or dusting at the time when the bacterial infection has begun or before the bacterial infection as a protective measure.

The invention also relates to the use of said phytotherapeutic composition for controlling, inhibiting or preventing the growth or pathogenicity of any pathogen on target plants.

As explained in details below, the composition of the invention can more precisely be used for:
- Treating and / or preventing and / or controlling a pathogen pathogenicity,
- Treating and / or preventing and / or controlling a pathogen growth,
- Reducing the antimicrobial resistance of pathogens,
- Enhancing the growth or beneficial effects of symbiotic or some commensal microbes,
thereby increasing plant yields.

The EVs of the invention are useful for silencing genes in any microbes: pathogenic or non-pathogenic bacteria; Gram-positive or Gram-negative bacteria, virus, fungi, oomycetes, or other organisms associated with plants. Examples of these different target pathogens are now disclosed.

### Pathogenic bacteria

In a preferred embodiment, said pathogen is a plant pathogenic bacterium.

Non-limitative examples of plant pathogenic bacteria, which can be targeted using the EVs of the invention include: *Ralstonia solanacearum, Xanthomonas oryzae* pathovars, *Xanthomonas campestris* pathovars, *Xanthomonas axonopodis* pathovars, *Xanthomonas euvesicatoria* pathovars, *Xanthomonas hostorum* pathovars, *Pseudomonas syringae* pathovars, *Pseudomonas viridiflava* pathovars, *Pseudomonas savastonoi* pathovars, *Candidatus liberibacter asiaticus, Candidatus liberibacter solanacearum, Acidovorax citrulli, Acidovorax avenae* pathovars, *Pectobacterium atrosepticum* pathovars, *Pectobacterium carotovorum* pathovars, *Pectobacterium sp., Agrobacterium tumefaciens, Dickeya (dadantii and solani), Erwinia amylovora, Clavibacter michiganensis (michiganensis and sepedonicus), Xylella fastidiosa, Pectobacterium (carotovorum and atrosepticum), Streptomyces scabies, Phytoplasma sp., Spiroplasma sp.* (Zhang *et al*., 2009)

If ornamental plants are treated, the following bacteria can be targeted: *Pseudomonas cichorii,* known to infect Chrysanthemum, Geranium, and Impatiens; *Xanthomonas campestris pv. Pelargoni*, known to infect Geranium; *Rhodococcus fascians*, known to infect *Chrysanthemum morifolium*, Pelargonium, Phlox, and possibly Rhododendron; *Ralstonia solanacearum,* known to infect Geranium, Anthurium spp, Rose tree, Curcumas, and Anthuriums; *Xanthomonas axonopodis, Xanthomonas hortorum*, known to infect Geranium, Begonia, Anthurium, and Hibiscus rosa-sinensis; *Pectobacterium carotovorum,* known to infect Amaryllis, Begonia, Calla, Cyclamen, Dracaena and Impatiens.

### Beneficial bacteria

In a particular embodiment, the EVs of the invention contain functional iRNA(s) targeting one or multiple genes of beneficial bacteria often referred to as Plant-growth-promoting rhizobacteria (PGPR). The purpose of this particular embodiment is to promote the beneficial effects of said PGPR. In this particular embodiment, the targeted bacterial genes are factors that, when silenced, promote the replication of the targeted bacterial cells or a pathway that is beneficial for the host and that positively regulate the production of a beneficial compound (*e.g*. a phytohormone), secondary metabolites that (i) alter the survival/pathogenicity of surrounding phytopathogens, (ii) activate plant defense responses (*e.g.* Induced Systemic Resistance), (iii) facilitate the uptake of nutrients from the environment (*e.g*. by enhancing the production of bacterial factors that are essential for Rhizobium-legume symbiosis), (iv) enhance the tolerance of the host organism to abiotic stress conditions *etc.* Silencing of such bacterial targeted genes would thus lead to an increased growth rate of the host organism and / or several other possible beneficial effects for the host organism.

In such an embodiment, the iRNAs contained in the EVs of the invention should have sequence homologies with beneficial bacterial genes but no sequence homology to pathogenic bacterial genomes, with the host genome or with other genomes of host colonizers and / or mammals that feed on the host organism.

Non-limitative examples of beneficial bacteria which can be targeted with the method of the invention include: Bacillus (*e.g. Bacillus subtilis*)*,* Pseudomonas (*e.g. Pseudomonas putida, Pseudomonas stuzeri, Pseudomonas fluorescens, Pseudomonas protegens, Pseudomonas brassicacearum*)*,* Rhizobia (*Rhizobium meliloti*)*,* Burkholderia (*e.g. Burkholderia phytofirmans*)*,* Azospirillum (*e.g. Azospirillum lipoferum*)*,* Gluconacetobacter (*e.g. Gluconacetobacter diazotrophicus*)*,* Serratia (*e.g. Serratia proteamaculans*)*,* Stenotrophomonas (*e.g. Stenotrophomonas maltophilia*)*,* Enterobacter (*e.g. Enterobacter cloacae*)*.*

### Pathogenic fungi or oomycetes

In a particular embodiment, the EVs of the invention contain functional iRNA(s) targeting one or multiple genes of pathogenic fungi or oomycetes.

Said fungi or oomyctes can for example be chosen in the group consisting of: *Acrocalymma, Acrocalymma medicaginis, Fusarium, Fusarium affine, Fusarium arthrosporioides*, *Fusarium crookwellense, Fusarium culmorum, Fusarium graminearum, Fusarium incarnatum*, *Fusarium solani, Fusarium langsethiae, Fusarium mangiferae*, *Fusarium oxysporum f.sp. albedinis, Fusarium oxysporum f.sp. asparagi, Fusarium oxysporum f.sp. batatas, Fusarium oxysporum f.sp. betae, Fusarium oxysporum f.sp. cannabis, Fusarium oxysporum f.sp. carthami, Fusarium oxysporum f.sp. cattleyae*, *Fusarium oxysporum f.sp. ciceris, Fusarium oxysporum f.sp. coffea*, *Fusarium oxysporum f.sp. cubense, Fusarium oxysporum f.sp. cyclaminis, Fusarium oxysporum f.sp. dianthi, Fusarium oxysporum f.sp. lentis, Fusarium oxysporum f.sp. lini, Fusarium oxysporum f.sp. lycopersici, Fusarium oxysporum f.sp. medicaginis, Fusarium oxysporum f.sp. pisi, Fusarium oxysporum f.sp. radicis-lycopersici, Fusarium oxysporum f.sp. spinacia, Fusarium oxysporum, Fusarium pallidoroseum, Fusarium patch, Fusarium proliferatum, Fusarium redolens, Fusarium sacchari, Fusarium solani, Fusarium subglutinans, Fusarium sulphureum, Fusarium wilt, Botrytis, Botrytis allii, Botrytis anthophila, Botrytis cinerea, Botrytis fabae, Botrytis narcissicola, Alternaria, Alternaria alternata, Alternaria brassicae, Alternaria brassicicola, Alternaria carthami, Alternaria cinerariae, Alternaria dauci, Alternaria dianthi, Alternaria dianthicola, Alternaria euphorbiicola, Alternaria helianthi, Alternaria helianthicola, Alternaria japonica, Alternaria leucanthemi, Alternaria limicola, Alternaria linicola, Alternaria padwickii, Alternaria panax, Alternaria radicina, Alternaria raphani, Alternaria saponariae, Alternaria senecionis, Alternaria solani, Alternaria tenuissima, Alternaria triticina, Alternaria zinniae, Erisyphe, Erisyphe necator, Erysiphe betae, Erysiphe brunneopunctata, Erysiphe cichoracearum, Erysiphe cruciferarum, Erysiphe graminis f. sp. Avenae, Erysiphe graminis f.sp. tritici, Erysiphe heraclei, Erysiphe pisi, Claviceps, Claviceps fusiformis, Claviceps purpurea, Claviceps sorghi, Claviceps zizaniae, Gaeumannomyces, Gaeumannomyces graminis, Leptosphaeria, Leptosphaeria nodorum, Leptosphaeria acuta, Leptosphaeria cannabina, Leptosphaeria coniothyrium, Leptosphaeria libanotis, Leptosphaeria lindquistii, Leptosphaeria maculans, Leptosphaeria musarum, Leptosphaeria pratensis, Leptosphaeria sacchari, Leptosphaeria woroninii, Microdochium, Microdochium spp. Microdochium bolleyi, Microdochium dimerum, Microdochium panattonianum, Microdochium phragmitis, Mycosphaerella, Mycosphaerella arachidis, Mycosphaerella areola, Mycosphaerella berkeleyi, Mycosphaerella bolleana, Mycosphaerella brassicicola, Mycosphaerella caricae, Mycosphaerella caryigena, Mycosphaerella cerasella, Mycosphaerella coffeicola, Mycosphaerella confusa, Mycosphaerella cruenta, Mycosphaerella dendroides, Mycosphaerella eumusae, Mycosphaerella gossypina, Mycosphaerella graminicola, Mycosphaerella henningsii, Mycosphaerella horii, Mycosphaerella juglandis, Mycosphaerella lageniformis, Mycosphaerella linicola, Mycosphaerella louisianae, Mycosphaerella musae, Mycosphaerella musicola, Mycosphaerellapalmicola, Mycosphaerella pinodes, Mycosphaerella pistaciarum, Mycosphaerella pistacina, Mycosphaerella platanifolia, Mycosphaerella polymorpha, Mycosphaerella pomi, Mycosphaerella punctiformis, Mycosphaerella pyri, Oculimacula, Oculimacula acuformis, Oculimacula yallundae,Blumeria, Blumeria graminis, Pyrenophora, Pyrenophora avenae, Pyrenophora chaetomioides, Pyrenophora graminea, Pyrenophora seminiperda, Pyrenophora teres, Pyrenophora teres f maculata, Pyrenophora teresf teres, Pyrenophora tritici-repentis,Ramularia, Ramularia collo-cygni, Ramularia beticola, Ramularia coryli, Ramularia cyclaminicola, Ramularia macrospora, Ramularia menthicola, Ramularia necator, Ramularia primulae, Ramularia spinaciae, Ramularia subtilis, Ramularia tenella, Ramularia vallisumbrosae, Rhynchosporium, Rhynchosporium secalis, Cochliobolus, Cochliobolus, Cochliobolus carbonum, Cochliobolus cymbopogonis, Cochliobolus hawaiiensis, Cochliobolus heterostrophus, Cochliobolus lunatus, Cochliobolus miyabeanus, Cochliobolus ravenelii, Cochliobolus sativus, Cochliobolus setariae, Cochliobolus spicifer, Cochliobolus stenospilus, Cochliobolus tuberculatus, Cochliobolus victoriae, Microdochium, Microdochium oryzae, Pyricularia, Pyricularia oryzae, Sarocladium, Sarocladium oryzae, Ustilaginoides, Ustilaginoides virens, Cercospora, Cercospora, Cercospora apii, Cercospora apii f.sp. clerodendri, Cercospora apiicola, Cercospora arachidicola, Cercospora asparagi, Cercospora atrofiliformis, Cercospora beticola, Cercospora brachypus, Cercospora brassicicola, Cercospora brunkii, Cercospora cannabis, Cercospora cantuariensis, Cercospora capsici, Cercospora carotae, Cercospora corylina, Cercospora fuchsiae, Cercospora fusca, Cercospora fusimaculans, Cercospora gerberae, Cercospora halstedii, Cercospora handelii, Cercospora hayi, Cercospora hydrangeae, Cercospora kikuchii, Cercospora lentis, Cercospora liquidambaris, Cercospora longipes, Cercospora longissima, Cercospora mamaonis, Cercospora mangiferae*, *Cercospora medicaginis, Cercospora melongenae, Cercospora minuta, Cercospora nicotianae, Cercospora odontoglossi, Cercospora papayae, Cercospora penniseti, Cercospora pisa-sativae, Cercospora platanicola, Cercospora puderii, Cercospora pulcherrima, Cercospora rhapidicola, Cercospora rosicola, Cercospora sojina, Cercospora solani, Cercospora solani-tuberosi, Cercospora sorghi, Cercospora theae, Cercospora tuberculans, Cercospora vexans, Cercospora vicosae, Cercospora zeae-maydis, Cercospora zebrina, Cercospora zonata, Corynespora, Corynespora cassiicola, Phakospora, Phakospora pachyrhizi, Phakopsora gossypii, Colletotrichum, Colletotrichum acutatum, Colletotrichum arachidis, Colletotrichum capsici, Colletotrichum cereale, Colletotrichum crassipes, Colletotrichum dematium, Colletotrichum dematium f spinaciae, Colletotrichum derridis, Colletotrichum destructivum, Colletotrichum glycines, Colletotrichum gossypii, Colletotrichum higginsianum, Colletotrichum kahawae, Colletotrichum lindemuthianum, Colletotrichum lini, Colletotrichum mangenotii, Colletotrichum musae, Colletotrichum nigrum, Colletotrichum orbiculare, Colletotrichum pisi, Colletotrichum sublineolum, Colletotrichum trichellum, Colletotrichum trifolii, Colletotrichum truncatum, Pythium spp., Diplodia, Diplodia allocellula, Diplodia laelio-cattleyae, Diplodia manihoti, Diplodia paraphysaria, Diplodia seriata, Diplodia theae-sinensis, Monilia, Monilinia azaleae, Monilinia fructicola, Monilinia fructigena, Monilinia laxa, Monilinia oxycocci, Pezzicula, Pezzicula alba, Pezzicula malicorticis, Zymoseptoria, Zymoseptoria tritici, Phytophthora, Phytophthora infestans, Guignardia, Guignardia bidwelli, Guignardia camelliae, Guignardia fulvida, Guignardia mangiferae*, *Guignardia musae, Guignardia philoprina, Plasmopara, Plasmopara viticola, Puccinia, Puccinia angustata, Puccinia arachidis, Puccinia aristidae, Puccinia asparagi, Puccinia cacabata, Puccinia campanulae, Puccinia carthami, Puccinia coronata, Puccinia dioicae, Puccinia erianthi, Puccinia extensicola, Puccinia helianthi, Puccinia hordei, Puccinia jaceae, Puccinia kuehnii, Puccinia malvacearum, Puccinia mariae-wilsoniae, Puccinia melanocephala, Puccinia menthae, Puccinia oxalidis, Puccinia pelargonii-zonalis, Puccinia pittieriana, Puccinia poarum, Puccinia purpurea, Puccinia recondita, Puccinia schedonnardii, Puccinia sessilis, Puccinia striiformis, Puccinia striiformis, Puccinia subnitens, Puccinia substriata, Puccinia verruca, Puccinia xanthii, Rhizoctonia, Rhizoctonia solani, Rhizoctonia oryzae, Rhizoctonia cerealis, Rhizoctonia leguminicola, Rhizoctonia rubi, Sclerotinia, Sclerotinia borealis, Sclerotinia bulborum, Sclerotinia minor, Sclerotinia ricini, Sclerotinia sclerotiorum, Sclerotinia spermophila, Sclerotinia trifoliorum, Septoria, Septoria ampelina, Septoria azaleae, Septoria bataticola, Septoria campanulae, Septoria cannabis, Septoria cucurbitacearum, Septoria darrowii, Septoria dianthi, Septoria eumusae, Septoria glycines, Septoria helianthi, Septoria humuli, Septoria hydrangeae, Septoria lactucae, Septoria lycopersici, Septoria lycopersici, Septoria menthae, Septoria passerinii, Septoria pisi, Septoria rhododendri, Septoria secalis, Septoria selenophomoides, Venturia, Venturia inaequalis. Venturia carpophila, Acrodontium, Acrodontium simplex, Acrophialophora, Acrophialophora fusispora, Acrosporium, Acrosporium tingitaninum, Aecidium, Aecidium aechmantherae, Aecidium amaryllidis, Aecidium breyniae, Aecidium campanulastri, Aecidium cannabis, Aecidium cantensis, Aecidium caspicum, Aecidium foeniculi, Aecidium narcissi, Ahmadiago, Albonectria, Albonectria rigidiuscula, Allodus podophylli, Amphobotrys ricini, Anguillosporella vermiformis, Anthostomella pullulans, Antrodia albida, Antrodia serialiformis, Antrodia serialis, Apiospora montagnei, Appendiculella, Armillaria heimii, Armillaria sinapina, Armillaria socialis, Armillaria tabescens, Arthrocladiella, Arthuriomyces peckianus, Ascochyta asparagina, Ascochyta bohemica, Ascochyta caricae, Ascochyta doronici, Ascochyta fabae f.sp. lentis, Ascochyta graminea, Ascochyta hordei, Ascochyta humuli, Ascochyta pisi, Ascochyta prasadii, Ascochyta sorghi, Ascochyta spinaciae, Ascochyta tarda, Ascochyta tritici, Ascospora ruborum, Aspergillus aculeatus, Aspergillus fischerianus, Aspergillus niger, Asperisporium caricae, Asteridiella, Asteroma caryae, Athelia arachnoidea, Athelia rolfsii, Aurantiporus fissilis, Aureobasidium pullulans, Bambusiomyces, Banana freckle, Bayoud disease, Beniowskia sphaeroidea, Bionectria ochroleuca, Bipolaris, Bipolaris cactivora, Bipolaris cookei, Bipolaris incurvata, Bipolaris sacchari, Biscogniauxia capnodes, Biscogniauxia marginata, Bjerkandera adusta, Black sigatoka, Blakeslea trispora, Botryodiplodia oncidii, Botryodiplodia ulmicola, Botryosphaeria cocogena, Botryosphaeria dothidea, Botryosphaeria marconii, Botryosphaeria obtusa, Botryosphaeria rhodina, Botryosphaeria ribis, Botryosphaeria stevensii, Botryosporium pulchrum, Botryotinia, Botryotinia fuckeliana, Botryotinia polyblastis, Boxwood blight, Brachybasidiaceae, Brasiliomyces malachrae, Briosia ampelophaga, Brown ring patch, Buckeye rot of tomato, Bulbomicrosphaera, Cadophora malorum, Caespitotheca, Calonectria ilicicola, Calonectria indusiata, Calonectria kyotensis, Calonectria pyrochroa, Calonectria quinqueseptata, Camarotella acrocomiae, Camarotella costaricensis, Canna rust, Capitorostrum cocoes, Capnodium footii, Capnodium mangiferum, Capnodium ramosum, Capnodium theae, Cephalosporium gramineum, Ceratobasidium cereale, Ceratobasidium cornigerum, Ceratobasidium noxium, Ceratobasidium ramicola, Ceratobasidium setariae, Ceratobasidium stevensii, Ceratocystis, Ceratocystis adiposa, Ceratocystis coerulescens, Ceratocystis fimbriata, Ceratocystis moniliformis, Ceratocystis oblonga, Ceratocystis obpyriformis, Ceratocystis paradoxa, Ceratocystis pilifera, Ceratocystis pluriannulata, Ceratocystis polyconidia, Ceratocystis tanganyicensis, Ceratocystis zombamontana, Ceratorhiza hydrophila, Ceratospermopsis, Cercoseptoria ocellata, Cercosporella rubi, Ceriporia spissa, Ceriporia xylostromatoides, Cerrena unicolor, Ceuthospora lauri, Choanephora, Choanephora cucurbitarum, Choanephora infundibulifera, Chrysanthemum white rust, Chrysomyxa cassandrae, Chrysomyxa himalensis, Chrysomyxa ledi, Chrysomyxa ledi var. rhododendri, Chrysomyxa ledicola, Chrysomyxa nagodhii, Chrysomyxa neoglandulosi, Chrysomyxa piperiana, Chrysomyxa pirolata, Chrysomyxa pyrolae, Chrysomyxa reticulata, Chrysomyxa roanensis, Chrysomyxa succinea, Cladosporium arthropodii, Cladosporium cladosporioides, Cladosporium cladosporioides f.sp. pisicola, Cladosporium cucumerinum, Cladosporium herbarum, Cladosporium musae, Cladosporium oncobae, Climacodon pulcherrimus, Climacodon septentrionalis, Clitocybe parasitica, Clonostachys rosea f rosea, Clypeoporthe iliau, Coleosporium helianthi, Coleosporium ipomoeae, Coleosporium madiae, Coleosporium pacificum, Coleosporium tussilaginis, Conidiosporomyces, Coniella castaneicola, Coniella diplodiella, Coniella fragariae, Coniothecium chomatosporum, Coniothyrium celtidis-australis, Coniothyrium henriquesii, Coniothyrium rosarum, Coniothyrium wernsdorffiae, Coprinopsis psychromorbida, Cordana johnstonii, Cordana musae, Coriolopsis floccosa, Corn grey leaf spot, Corticium invisum, Corticium penicillatum, Corticium theae, Coryneopsis rubi, Coryneum rhododendri, Covered smut, Crinipellis sarmentosa, Cronartium ribicola, Cryphonectriaceae, Cryptobasidiaceae, Cryptocline cyclaminis, Cryptomeliola, Cryptosporella umbrina, Cryptosporiopsis tarraconensis, Cryptosporium minimum, Curvularia lunata, Curvularia caricae-papayae, Curvularia penniseti, Curvularia senegalensis, Curvularia trifolii, Cyclaneusma needle cast, Cylindrocarpon ianthothele var. ianthothele, Cylindrocarpon magnusianum, Cylindrocarpon musae, Cylindrocladiella camelliae, Cylindrocladiella parva, Cylindrocladium clavatum, Cylindrocladium lanceolatum, Cylindrocladium peruvianum, Cylindrocladium pteridis, Cylindrosporium cannabinum, Cylindrosporium juglandis, Cylindrosporium rubi, Cymadothea trifolii, Cytospora palmarum, Cytospora personata, Cytospora sacchari, Cytospora sacculus, Cytospora terebinthi, Cytosporina ludibunda, Dactuliophora elongata, Davidiella dianthi, Davidiella tassiana, Deightoniella papuana, Deightoniella torulosa, Dendrophoma marconii, Dendrophora erumpens, Denticularia mangiferae, Dermea pseudotsugae, Diaporthaceae, Diaporthe, Diaporthe arctii, Diaporthe dulcamarae, Diaporthe eres, Diaporthe helianthi, Diaporthe lagunensis, Diaporthe lokoyae, Diaporthe melonis, Diaporthe orthoceras, Diaporthe perniciosa, Diaporthe phaseolorum, Diaporthe phaseolorum var. caulivora, Diaporthe phaseolorum var. phaseolorum, Diaporthe phaseolorum var. soja, Diaporthe rudis, Diaporthe tanakae, Diaporthe toxica, Dicarpella dryina, Didymella applanata, Didymella bryoniae, Didymella fabae, Didymella lycopersici, Didymosphaeria arachidicola, Didymosphaeria taiwanensis, Dilophospora alopecuri, Dimeriella sacchari, Diplocarpon mespili, Diplocarpon rosae, Discosia artocreas, Discostroma corticola, Distocercospora livistonae, Dothiorella brevicollis, Dothiorella dominicana, Dothiorella dulcispinae, Dothiorella gregaria, Drechslera avenacea, Drechslera campanulata, Drechslera dematioidea, Drechslera gigantea, Drechslera glycines, Drechslera musae-sapientium, Drechslera teres f. maculata, Drechslera wirreganensis, Eballistra lineata, Eballistra oryzae, Eballistraceae, Echinodontium ryvardenii, Echinodontium tinctorium, Ectendomeliola, Elsinoë ampelina, Elsinoë batatas, Elsinoë brasiliensis, Elsinoë leucospila, Elsinoë randii, Elsinoë rosarum, Elsinoë sacchari, Elsinoë theae, Elsinoë veneta, Endomeliola, Endothia radicalis, Endothiella gyrosa, Entorrhizomycetes, Entyloma ageratinae, Entyloma dahliae, Entyloma ellisii, Epicoccum nigrum, Eremothecium coryli, Eremothecium gossypii, Erysiphales, Exobasidiaceae, Exobasidium burtii, Exobasidium reticulatum, Exobasidium vaccinii var. japonicum, Exobasidium vaccinii-uliginosi, Exobasidium vexans,xxophiala alcalophila, Exophiala angulospora, Exophiala attenuata, Exophiala calicioides, Exophiala castellanii, Exophiala dermatitidis, Exophiala dopicola, Exophiala exophialae, Exophiala heteromorpha, Exophiala hongkongensis, Exophiala jeanselmei, Exophiala lecanii-corni, Exophiala mansonii, Exophiala mesophila, Exophiala moniliae, Exophiala negronii, Exophiala phaeomuriformis, Exophiala pisciphila, Exophiala psychrophila, Exophiala salmonis, Exophiala spinifera, Fomes lamaënsis, Fomitopsis rosea, Fusicladium pisicola, Fusicoccum aesculi, Fusicoccum amygdali, Fusicoccum quercus, Galactomyces candidum, Ganoderma brownii, Ganoderma lobatum, Ganoderma megaloma, Ganoderma meredithiae, Ganoderma orbiforme, Ganoderma philippii, Ganoderma sessile, Ganoderma tornatum, Ganoderma zonatum, Geastrumia polystigmatis, Georgefischeriaceae, Georgefischeriales, Geosmithia pallida, Geotrichum candidum, Geotrichum klebahnii, Gibberella acuminata, Gibberella avenacea, Gibberella baccata, Gibberella cyanogena, Gibberella fujikuroi, Gibberella intricans, Gibberella pulicaris, Gibberella stilboides, Gibberella tricincta, Gibberella xylarioides, Gibberella zeae, Gibellina cerealis, Gilbertella persicaria, Gjaerumiaceae, Gliocladiopsis tenuis, Gliocladium vermoeseni, Gloeocercospora sorghi, Gloeocystidiellum porosum, Gloeophyllum mexicanum, Gloeophyllum trabeum, Gloeoporus dichrous, Gloeosporium cattleyae, Gloeosporium theae-sinensis, Glomerella cingulata, Glomerella graminicola, Glomerella tucumanensis, Gnomonia caryae*, *Gnomonia comari, Gnomonia dispora, Gnomonia iliau, Gnomonia rubi, Golovinomyces cichoracearum, Graphiola phoenicis, Graphiolaceae, Graphium rigidum, Graphium rubrum, Graphyllium pentamerum, Grovesinia pyramidalis, Gymnoconia nitens, Gymnopus dryophilus, Gymnosporangium kernianum, Gymnosporangium libocedri, Gymnosporangium nelsonii, Gymnosporangium yamadae, Haematonectria haematococca, Hansenula subpelliculosa, Hapalosphaeria deformans, Haplobasidion musae, Helicobasidium compactum, Helicobasidium longisporum, Helicobasidium purpureum, Helicoma muelleri, Helminthosporium cookei, Helminthosporium solani, Hendersonia creberrima, Hendersonia theicola, Hericium coralloides, Heterobasidion irregulare, Heterobasidion occidentale, Hexagonia hydnoides, Hymenula affinis, Hyphodermella corrugata, Hyphodontia aspera, Hyphodontia sambuci, Hypoxylon tinctor, Inonotus arizonicus, Inonotus cuticularis, Inonotus dryophilus, Inonotus hispidus, Inonotus ludovicianus, Irpex destruens, Irpex lacteus, Kabatiella caulivora, Karnal bunt, Koa wilt, Kretzschmaria zonata, Kuehneola uredinis, Kutilakesa pironii, Laetiporus ailaoshanensis, Laetiporus baudonii, Laetiporus caribensis, Laetiporus conifericola, Laetiporus cremeiporus, Laetiporus gilbertsonii, Laetiporus huroniensis, Laetiporus montanus, Laetiporus portentosus, Laetiporus zonatus, Laxitextum bicolor, Leandria momordicae, Lentinus tigrinus, Lenzites betulina, Lenzites elegans, Leohumicola atra, Leohumicola incrustata, Leohumicola levissima, Leptodontidium elatius, Leptographium microsporum, Leptosphaerulina crassiasca, Leptosphaerulina trifolii, Leptothyrium nervisedum, Leptotrochila medicaginis, Leucocytospora leucostoma, Leucostoma auerswaldii, Leucostoma canker, Leucostoma kunzei, Leucostoma persoonii, Leveillula compositarum, Leveillula leguminosarum, Leveillula taurica, Limacinula tenuis, Linochora graminis, Loose smut, Lopharia crassa, Lophodermium aucupariae, Lophodermium schweinitzii, Macrophoma mangiferae, Macrophoma theicola, Macrosporium cocos, Magnaporthe grisea, Magnaporthe salvinii, Magnaporthiopsis, Mamianiella coryli, Marasmiellus cocophilus, Marasmiellus stenophyllus, Marasmius crinis-equi, Marasmius sacchari, Marasmius semiustus, Marasmius stenophyllus, Marasmius tenuissimus, Massarina walkeri, Mauginiella scaettae, Melampsora lini, Melampsora occidentalis, Melanconis carthusiana, Melanconium juglandinum, Meliola mangiferae*, *Meliola zangii, Meruliopsis ambigua, Microascus brevicaulis, Microbotryum silenes-dioicae, Microbotryum violaceum, Microsphaera coryli, Microsphaera diffusa, Microsphaera ellisii, Microsphaera euphorbiae, Microsphaera hommae, Microsphaera penicillata, Microsphaera vaccinii, Microsphaera verruculosa, Microstroma juglandis, Moesziomyces bullatus, Moniliophthora roreri, Monilochaetes infuscans, Monochaetia coryli, Monochaetia mali, Monographella albescens, Monographella cucumerina, Monographella nivalis, Monosporascus cannonballus, Monosporascus eutypoides, Monostichella coryli, Mucor circinelloides, Mucor hiemalis, Mucor mucedo, Mucor paronychius, Mucor piriformis, Mucor racemosus, Mycena citricolor, Mycocentrospora acerina, Mycoleptodiscus terrestris, Didymella rabiei, Mycosphaerella recutita, Mycosphaerella rosicola, Mycosphaerella rubi, Mycosphaerella stigmina-platani, Mycosphaerella striatiformans, Mycovellosiella concors, Passalora fulva, Mycovellosiella koepkei, Mycovellosiella vaginae, Myriogenospora aciculispora, Myrothecium roridum, Myrothecium verrucaria, Naevala perexigua, Naohidemyces vaccinii, Nectria cinnabarina, Nectria ditissima, Nectria foliicola, Nectria mammoidea, Nectria mauritiicola, Nectria peziza, Nectria pseudotrichia, Nectria radicicola, Nectria ramulariae, Nectriella pironii, Nemania diffusa, Nemania serpens, Neocosmospora vasinfecta, Neodeightonia phoenicum, Neoerysiphe galeopsidis, Neofabraea perennans, Neofusicoccum mangiferae, Oidiopsis gossypii, Oidium arachidis, Oidium caricae-papayae, Oidium indicum, Oidium mangiferae, Oidium manihotis, Olpidium brassicae, Omphalia tralucida, Ophiobolus anguillides, Ophiobolus cannabinus, Ophioirenina, Ovulinia azaleae, Oxyporus corticola, Ozonium texanum, Peltasterfructicola, Penicillium expansum, Penicillium funiculosum, Peniophora, Periconia circinata, Periconiella cocoes, Peridermium californicum, Pestalosphaeria concentrica, Pestalotia longiseta, Pestalotia rhododendri, Pestalotiopsis, Pestalotiopsis adusta, Pestalotiopsis arachidis, Pestalotiopsis disseminata, Pestalotiopsis guepini, Pestalotiopsis leprogena, Pestalotiopsis longiseta, Pestalotiopsis mangiferae, Pestalotiopsis palmarum, Pestalotiopsis sydowiana, Pestalotiopsis theae, Peyronellaea curtisii, Phacidiopycnis padwickii, Phaeochoropsis mucosa, Phaeocytostroma iliau, Phaeocytostroma sacchari, Phaeoisariopsis bataticola, Phaeoramularia heterospora, Phaeoramularia indica, Phaeoramularia manihotis, Phaeoseptoria musae, Phaeosphaerella mangiferae*, *Phaeosphaerella theae, Phaeosphaeria avenaria, Phaeosphaeria herpotrichoides, Phaeosphaeria microscopica, Phaeosphaeria nodorum, Phaeosphaeriopsis obtusispora, Phaeotrichoconis crotalariae, Phialophora asteris, Phialophora cinerescens, Phialophora gregata, Phialophora tracheiphila, Phoma clematidina, Phoma costaricensis, Phoma cucurbitacearum, Phoma destructiva, Phoma draconis, Phoma exigua, Phoma exigua, Phoma exigua var. foveata, Phoma exigua, Phoma glomerata, Phoma glycinicola, Phoma herbarum, Phoma insidiosa, Phoma medicaginis, Phoma microspora, Phoma narcissi, Phoma nebulosa, Phoma oncidii-sphacelati, Phoma pinodella, Phoma sclerotioides, Phoma strasseri, Phomopsis asparagi, Phomopsis asparagicola, Phomopsis cannabina, Phomopsis coffeae, Phomopsis ganjae, Phomopsis javanica, Phomopsis longicolla, Phomopsis mangiferae, Phomopsis prunorum, Phomopsis sclerotioides, Phomopsis theae, Phragmidium mucronatum, Phragmidium rosae-pimpinellifoliae, Phragmidium rubi-idaei, Phragmidium violaceum, Phyllachora banksiae, Phyllachora cannabis, Phyllachora graminis, Phyllachora gratissima, Phyllachora musicola, Phyllachora pomigena, Phyllachora sacchari, Phyllactinia, Phyllosticta alliariaefoliae Phyllosticta arachidis-hypogaeae, Phyllosticta batatas, Phyllosticta capitalensis, Phyllosticta carpogena, Phyllosticta coffeicola, Phyllosticta concentrica, Phyllosticta coryli, Phyllosticta cucurbitacearum, Phyllosticta cyclaminella, Phyllosticta erratica, Phyllosticta hawaiiensis, Phyllosticta lentisci, Phyllosticta manihotis, Phyllosticta micropuncta, Phyllosticta mortonii, Phyllosticta nicotianae, Phyllosticta palmetto, Phyllosticta penicillariae, Phyllosticta perseae, Phyllosticta pseudocapsici, Phyllosticta sojaecola, Phyllosticta theae, Phyllosticta theicola, Phymatotrichopsis omnivora, Physalospora disrupta, Physalospora perseae, Physoderma alfalfae, Physoderma leproides, Physoderma trifolii, Physopella ampelopsidis, Pileolaria terebinthi, Piricaudiopsis punicae, Piricaudiopsis rhaphidophorae, Piricaudiopsis rosae, Plenodomus destruens, Plenodomus meliloti, Pleosphaerulina sojicola, Pleospora alfalfae, Pleospora betae, Pleospora herbarum, Pleospora lycopersici, Pleospora tarda, Pleospora theae, Pleuroceras, Podosphaera, Podosphaera fuliginea, Podosphaera fusca, Podosphaera leucotricha, Podosphaera macularis, Podosphaera pannosa, Polyscytalum pustulans, Poria hypobrunnea, Postia tephroleuca, Powdery mildew, Pseudocercospora arecacearum, Pseudocercospora cannabina, Pseudocercospora fuligena, Pseudocercosporella herpotrichoides, Pseudocercospora gunnerae, Pseudocercospora pandoreae, Pseudocercospora puderi, Pseudocercospora rhapisicola, Pseudocercospora theae, Pseudocercospora vitis, Pseudocercosporella capsellae, Pseudocochliobolus eragrostidis, Pseudoepicoccum cocos, Pseudopeziza jonesii, Pseudopeziza medicaginis, Pseudopeziza trifolii, Pseudoseptoria donacis, Pucciniaceae, Pucciniastrum americanum, Pucciniastrum arcticum, Pucciniastrum epilobii, Pucciniastrum hydrangeae, Pycnostysanus azaleae, Pyrenochaeta lycopersici, Pyrenochaeta terrestris, Pyrenopeziza brassicae, Ramichloridium musae, Ramulispora sorghi, Ramulispora sorghicola, Rhinocladium corticola, Rhizophydium graminis, Rhizopus arrhizus, Rhizopus circinans, Rhizopus microsporus, Rhizopus oryzae, Rhytisma punctatum, Rhytisma vitis, Rigidoporus vinctus, Rosellinia arcuata, Rosellinia bunodes, Rosellinia necatrix, Rosellinia pepo, Saccharicola taiwanensis, Schiffnerula cannabis, Schizophyllum commune, Schizopora flavipora, Schizothyrium pomi, Sclerophthora macrospora, Sclerotium cinnamomi, Sclerotium delphinii, Scytinostroma galactinum, Seimatosporium mariae, Seimatosporium rhododendri, Selenophoma linicola, Septobasidium bogoriense, Septobasidium euryae-groffii, Septobasidium gaoligongense, Septobasidium pilosum, Septobasidium polygoni, Septobasidium pseudopedicellatum, Septobasidium theae, Septocyta ruborum, Serpula lacrymans, Setosphaeria rostrata, Setosphaeria turcica, Spencermartinsia pretoriensis, Sphaceloma arachidis, Sphaceloma menthae, Sphaceloma perseae, Sphaceloma poinsettiae, Sphaceloma sacchari, Sphaceloma theae, Sphacelotheca reiliana, Sphaerotheca castagnei, Sphaerulina oryzina, Sphaerulina rehmiana, Sphaerulina rubi, Sphenospora kevorkianii, Spilocaea oleaginea, Sporisorium cruentum, Sporisorium ehrenbergii, Sporisorium scitamineum, Sporisorium sorghi, Sporonema phacidioides, Stagonospora avenae, Stagonospora meliloti, Stagonospora recedens, Stagonospora sacchari, Stagonospora tainanensis, Stagonosporopsis, Stegocintractia junci, Stemphylium, Stemphylium alfalfae, Stemphylium bolickii, Stemphylium cannabinum, Stemphylium globuliferum, Stemphylium lycopersici, Stemphylium sarciniforme, Stemphylium solani, Stemphylium vesicarium, Stenella anthuriicola, Stigmatomycosis, Stigmina carpophila, Stigmina palmivora, Stigmina platani-racemosae, Stromatinia cepivora, Sydowiella depressula, Sydowiellaceae, Synchytrium endobioticum, Tapesia acuformis, Tapesia yallundae, Taphrina coryli, Taphrina potentillae, Thanatephorus cucumeris, Thecaphora solani, Thielaviopsis, Thielaviopsis basicola, Thielaviopsis ceramica, Thyrostroma compactum, Tiarosporella urbis-rosarum, Tilletia barclayana, Tilletia caries, Tilletia controversa, Tilletia laevis, Tilletia tritici, Tilletia walkeri, Tilletiariaceae, Togniniaceae, Tranzschelia pruni-spinosae, Trichoderma koningii, Trichoderma paucisporum, Trichoderma songyi, Trichoderma theobromicola, Trichoderma viride, Tubercularia lateritia, Tunstallia aculeata, Typhula blight, Typhula idahoensis, Typhula incarnata, Typhula ishikariensis, Typhula variabilis, Ulocladium consortiale, Uncinula, Uredo behnickiana, Uredo kriegeriana, Uredo musae, Uredo nigropuncta, Uredo rangelii, Urocystis agropyri, Urocystis brassicae, Urocystis occulta, Uromyces apiosporus, Uromyces appendiculatus, Uromyces beticola, Uromyces ciceris-arietini, Uromyces dianthi, Uromyces euphorbiae, Uromyces graminis, Uromyces inconspicuus, Uromyces lineolatus, Uromyces musae, Uromyces oblongus, Uromyces pisi-sativi, Uromyces proëminens, Uromyces medicaginis, Uromyces trifolii-repentis, Uromyces viciae-fabae, Urophlyctis leproides, Urophlyctis trifolii, Ustilaginales, Ustilago avenae, Ustilago esculenta, Ustilago hordei, Ustilago maydis, Ustilago nigra, Ustilago nuda, Ustilago scitaminea, Ustilago tritici, Vankya ornithogali, Velvet blight, Veronaea musae, Verticillium albo-atrum, Verticillium alfalfae, Verticillium dahliae, Verticillium isaacii, Verticillium klebahnii, Verticillium longisporum, Verticillium nonalfalfae, Verticillium theobromae, Verticillium wilt, Verticillium zaregamsianum, Waitea circinata, Westea, Wheat leaf rust, Wheat mildew, Wuestneiopsis georgiana, Xeromphalina fraxinophila, Zopfia rhizophila, Zygosaccharomyces bailii, Zygosaccharomyces florentinus,* and *Zythiostroma.*

Particular target genes of some of these fungi (*Botrytis cinerea, Colletotrichum higginsianum, Fusarium graminearum, Zymoseptoria tritici*) are disclosed in EXAMPLE 7 below.

Particular target genes of some of these oomyctes (*Phytphtora infestans, plasmopara viticola)* are disclosed in EXAMPLE 7 below.

### Beneficial fungi

In a particular embodiment, the EVs of the invention contain functional iRNA(s) targeting one or multiple genes of beneficial fungi. Beneficial fungi include classical arbuscular mycorrhizal fungi but also other commensal fungi including the recently characterized *Colletotrichum tofieldiae* (Hiruma et al., 2016).

### Targeted virus

In a particular embodiment, the EVs of the invention contain functional iRNA(s) targeting one or multiple genes of a virus. In a particular embodiment, said virus is chosen in the group consisting of: *Tobacco mosaic virus, Tomato spotted wilt virus, Tomato yellow leaf curl virus, Cucumber mosaic virus, Potato virus Y, Cauliflower mosaic virus, African cassava mosaic virus, Plum pox virus, Brome mosaic virus and Potato virus X, Citrus tristeza virus, Barley yellow dwarf virus, Potato leafroll virus* and *Tomato bushy stunt virus.*

Particular target genes of one of these viruses (*Plum Pox Virus*) are disclosed in EXAMPLE 7 below.

### Chimeric EVs of the invention

For protecting plants against diseases caused by several bacterial pathogens, the method of the invention advantageously uses functional EVs carrying sequence homologies with more than one plant pathogen or pest (hereafter referred to as "chimeric EVs").

In this embodiment, the small RNAs contained in the EVs of the invention can target several genes of several pathogens or parasites. These "chimeric EVs" are not specific of one pathogen or pest but can affect the growth of several pathogens (e.g., a bacterium and a virus, or of two different bacteria, or of three different viruses, *etc*.).

All the embodiments proposed above for the EVs, the iRNAs, the vectors, and the transformation methods are herewith encompassed and do not need to be repeated.

When the EVs of the invention target several pathogens or parasites, the phytotherapeutic composition of the invention can concomitantly treat or prevent diseases caused by these different pathogens / parasites.

In a preferred embodiment, the EVs of the invention contain chimeric iRNAs inhibiting at least one gene encoding a virulence factor or an essential gene of bacterial cells as defined above, together with at least one other gene encoding a virulence factor or an essential gene of other pathogens or parasites known to be sensitive to host-induced gene silencing. It can be also a gene required for the biosynthesis of toxic secondary metabolites from non-bacterial pathogens or parasites.

In another preferred embodiment, the phytotherapeutic applications of the invention uses: (i) EVs containing one or more iRNAs targeting a widespread sequence region of an essential or virulence gene that is conserved in a large set of pathogens or (ii) EVs containing one or more iRNAs targeting genes that are essential or virulence factors from unrelated pathogens. Such particular embodiment confers broad-spectrum protection towards multiple pathogens.

The EVs of the invention are useful for silencing any gene(s) in any microbes. Examples of useful target genes are now disclosed.

### Bacterial target genes

For anti-bacterial applications, the EVs of the invention should contain effective small RNAs having a sufficient sequence homology with at least one bacterial gene in order to induce sequence-specific silencing of said at least one gene. In addition, to prevent unwanted off-target effects, the sequence homology of the dsRNAs, miRNAs or small RNA species contained in said EVs with the eukaryotic host genome or other genomes of beneficial bacteria, host colonizers and / or mammals that feed on the host organism should be quasi inexistent (if not absent).

According to the invention, the term "bacterial gene" refers to any gene in bacteria including (natural) protein-coding genes or non-coding genes, present naturally in bacteria and artificial genes introduced in bacteria by recombinant DNA technology. Said target bacterial genes are either specific to a given bacterial species or conserved across multiple bacterial species. Preferably, it shares no homology with any gene of the eukaryotic host genome, host colonizers and / or mammals that feed on the host organism. This avoids collateral effects on the plant host, beneficial bacteria associated with the host, host colonizers and / or animals that feed on the host organism.

In a preferred embodiment, said at least one bacterial gene is a bacterial virulence factor or an essential gene for bacteria or an antibiotic resistance gene.

As used herein, the term "essential gene for bacteria" refers to any bacterial gene that is essential for bacterial cell viability. These genes are absolutely required to maintain bacteria alive, provided that all nutrients are available. It is thought that the absolutely required number of essential genes for bacteria is about 250-500 in number. The identification of such essential genes from unrelated bacteria is now becoming relatively easily accessible through the use of transposon sequencing approaches. These essential genes encode proteins to maintain a central metabolism, replicate DNA, ensure proper cell division, translate genes into proteins, maintain a basic cellular structure, and mediate transport processes into and out of the cell (Zhang *et al.,* 2009). This is the case of *GyrB* and *FusA.*

As used therein, the term "virulence gene" refers to any bacterial gene that has been shown to play a critical role for at least one of the following activity: pathogenicity, disease development, colonization of a specific host tissues (*e.g.* vascular tissues) or host cell environment (*e.g.* the apoplast), suppression of plant defense responses, modulation of plant hormone signaling and / or biosynthesis to facilitate multiplication and / or disease development, interference with conserved host regulatory processes to facilitate multiplication and / or disease development, *etc.* All these activities help the bacteria to grow and / or promote disease symptoms in the host, although they are not essential for their survival *in vitro.* Well-known virulence factors are: adhesins, phytotoxins *(e.g.* coronatine, syringoline A), degrading enzymes *(e.g.* cellulases, cellobiosidases, lipases, xylanases, endoglucanases, polygalacturonases), factors required for the assembly of type I/II/III/IV or VI secretion systems, effector proteins, transcription factors required to promote the expression of *Hrp* genes upon contact with plant cells, machineries required for the proper expression of virulence factors (*e.g*. quorum sensing, two-component systems), post-transcriptional factors controlling the stability/translation of mRNAs from virulence factor genes.

Well-known bacterial essential genes or virulence factors are provided in the following tables, for several different phytopathogenic bacteria:
• For most bacteria: central factors required for survival and/or virulence such as *FtsZ, FtsA, cheA, gacA, tolC, pglA, engXCA1, engXCA2, GumH, GumD, XpsE, LesA, HolC, Wp012778355, wp015452784, WP012778510, wp015452939, act56857, wp012778668, GyrB, MreB, RbfA, RsgA, FliA, QseC, Hfq, HrpR, HrpS, RpoD, HrpL, Cfa6, fusA, gyrB, rpoB, RpoC, secE, RpoA, dnaA, dnaN, HrpG, HrpB, HrcC, XpsR, TssB, HrpG, HrpX, RsmA, NadHb, NadHd and NadHe, DnaA, DnaE1, DnaE2, ZipA, PhoP, FhaB, PhoP, rim, ybeY, rplQ.*
• For the bacteria *Pseudomonas syringae* pv. *phaseolicola* (strains *Pph 1448A; Pph 1302A),* known to infect common bean plants *Phaseolus vulgaris* and cause the halo blight disease:

| Essential gene | Description | Function |
|---|---|---|
| *cheA2* | Chemotaxis sensor histidine kinase CheA | Chemotaxis |
| *cheZ* | Chemotaxis protein CheZ | Chemotaxis |
| *flhB* | Flagellar biosynthetic protein FlhB | Flagellum |
| *fliO* | Flagellar protein FliO | Flagellum |
| *fliN* | Flagellar motor switch protein FliN | Flagellum |
| *fliM* | Flagellar motor switch protein FliM | Flagellum |
| *fliK* | Flagellar hook-length control protein FliK | Flagellum |
| *fleS* | Flagellar sensor histidine kinase FleS | Flagellum |
| *flgJ* | Peptidoglycan hydrolase FlgJ | Flagellum |
| *flgE* | Flagellar hook protein FlgE | Flagellum |
| *flgD* | Basal-body rod modification protein FlgD | Flagellum |
| *FlgA* | Flagellar basal-body P-ring formation protein, putative | Flagellum |
| *MotA* | MotA family motility protein | Flagellum |
| *plsC* | hdtS protein (100%) | quorum-sensing molecule |
| *impL* | OmpA domain protein (98%) | Outer membrane protein and pathogenicity; might be involved in T6SS |
| *mdoG1* | periplasmic glucans biosynthesis protein MdoG | |
| *CobQ* | cobyric acid synthase | cobalamin (vitamin B12) biosynthesis |
| *carA* | carbamoyl-phosphate synthase, small subunit | pyrimidine biosynthesis |
| *pyrB* | aspartate carbamoyltransferase | pyrimidine biosynthesis |
| *carB* | carbamoyl-phosphate synthase, large subunit | pyrimidine biosynthesis |
| *purH* | bifunctional purine biosynthesis protein PurH | purine biosynthesis |
| *pyrD* | dihydroorotate dehydrogenase | pyrimidine biosynthesis |
| | cobalamin synthesis protein/P47K family protein | |

• For the bacteria *Pseudomonas syringae* pv. *actinidiae,* known to infect kiwi plants and fruits (Actinidia spp.) and cause the kiwifruit canker:

| Essential gene | Description |
|---|---|
| *fliR* | Flagellar biosynthesis protein |
| *flhA* | Flagellar biosynthesis protein |
| *flgC* | Flagellar basal-body rod protein |
| *flgH* | Flagellar biosynthesis protein |
| *FlgL* | flagellar hook-associated protein |
| | Flagellar basal-body rod modification protein |

• For the bacteria *Pectobacterium carotovorum,* known to infect the Chinese cabbage and cause the soft rot disease:

| Essential gene | Description |
|---|---|
| *pyrD* | Dihydroorotate dehydrogenase |
| *purH* | Bifunctional phosphoribosylaminoimidazole carboxamide formyltransferase/IMP cyclohydrolase |
| *purD* | Phosphoribosylamine-glycine ligase |
| *leuA* | 2-Isopropylmalate synthase |
| *serB* | Phosphoserine phosphatase |
| *expI* | Synthesis of N-(3-oxohexanoyl)-I-homoserine lactone |
| *expR* | Quorum-sensing transcriptional regulator |
| *flgA* | Flagellar basal body P-ring biosynthesis protein |
| *fliA* | Flagellar biosynthesis sigma factor |
| *flhB* | Flagellar biosynthesis protein |
| *qseC* | Sensor protein QseC bacterial adrenergic receptor |
| *tolC* | Outer membrane channel protein |
| PCC21_023220 | Putative DNA-binding protein |

• For the bacteria *Ralstonia solanacearum,* known to infect for instance tomato, potato, tobacco, banana and soybean and cause the bacterial wilt:

| Essential gene | Description |
|---|---|
| *in Tomato* | |
| RSc2735 (phcB) | Regulatory protein |
| RSc2748 (phcA) | A LysR family transcriptional regulator, virulence genes transcriptional regulator |
| RSp0852 (hrpG) | Transcription regulator protein |
| RSc2827 (pilD) | Probable type 4 prepilin peptidase; bifunctional: leader peptidase and N-methyltransferase transmembrane protein |
| RSc3111 (gspL) | Probable general secretory pathway L transmembrane protein |
| RSc3114 (gspD) | Probable general secretory pathway D transmembrane protein |
| RSp1007 | Putative acetyl transferase protein |
| RSc0571 (pgk) | Probable phosphoglycerate kinase protein |
| RSc1591 (hpal1) | Putative 2,4-dihydroxyhept-2-ene-1,7-dioic acid aldolase oxidoreductase protein |
| RSp1010 | hypothetical protein |
| RSc3115 (gspE) | Probable general secretion pathway protein E |
| | |

| in Arabidopsis | |
|---|---|
| RSc0903 (serC) | Probable phosphoserine aminotransferase (PSAT) protein |
| RSc1981 (trpA) | Probable tryptophane synthase alpha chain protein |
| RSc0454 | Putative Fe-S oxidoreductase; FAD/FMN-containing dehydrogenase oxidoreductase protein |
| RSc0411 | Probable transmembrane protein |
| RSc0353 (gpmA) | Probable 2,3-bisphosphoglycerate-dependent phosphoglycerate mutase |
| RSc1956 (murl) | Probable glutamate racemase protein |
| RSc0565 (rfaF) | Probable ADP-heptose--lipopolysaccharide heptosyltransferase II protein |
| RSc1326 | Probable aspartate aminotransferase protein |
| RSc2464 (clpA) | Probable ATP-dependent protease (ATP-binding specificity subunit) protein |
| RSc0734 (tolA) | Probable TolA-related transport transmembrance protein |
| RSc0736 (pal) | Probable peptidoglycan-associated lipoprotein precursor |
| RSc0732 (tolQ) | Probable TolQ-related transport transmembrane protein |
| RSc2684 (proC) | Probable oxidoreductase pyrroline-5-carboxylate reductase signal peptide protein |
| RSc1206 | Probable lipoprotein NIpD |
| RSc0500 (ruvB) | Probable holiday junction DNA helicase RuvB protein |

• For the bacteria *Xanthomonas oryzae* pv. *oryzicola,* known to infect rice (*Oryza sativa*) and cause the bacterial leaf streak of rice:

| Essential gene | Description |
|---|---|
| XOCORF_4443 (HrcU) | Type III secretion protein HrcU |
| XOCORF_4444 (HcrV) | Type III secretion protein HrcV |
| XOCORF_4434 (HrcC) | Type III secretion protein HrcC |
| XOCORF_3864 (wxocB) | Putative glycosyltransferase |
| XOCORF_2264 (rpfG) | Two-component system response regulator RpfG |
| XOCORF_2899 (pilY1) | RpfG |
| XOCORF_3636 (pilQ) | Fimbrial assembly protein |
| XOCORf_3640 (pilM) | Type IV pilus assembly protein |
| XOCORF_1181 (pilZ) | Type IV pilus assembly protein |
| XOCORF_1487 (pilT) | Twitching mobility protein |
| XOCORF_3589 (pgk) | Phosphoglycerate kinase |
| XOCORF_3592 (gapA) | Glyceraldehyde-3-phosphate dehydrogenase, type I |
| XOCORF_1172 | 3-Oxoacyl-[acyl-carrier-protein] synthase III |
| XOCORF_2877 | P protein |
| | |
| | |
| XOCORF_3805 (XpsE) | General secretion pathway protein E (XpsE) |
| XOCORF_4457 (HrpF) | |
| XOCORF_3672 (ThiE) | Thiamine-phosphate pyrophosphorylase (ThiE) |
| XOCORF_3439 (HrpX) | |
| XOCORF_3794 (XpsD) | General secretion pathway protein D (XpsD) |
| XOCORF_4434 (HrcC-) | HrcC |
| XOCORF_1450 | Conserved hypothetical protein |
| XOCORF_1282 | Dipeptidyl carboxypeptidase I |

• For the bacteria *Xanthomonas campestris* pv. *campestris,* known to infect all the *Brassicaceae* (cabbage, broccoli, cauliflower, kale, turnip, oilseed rape, mustard, radish, *etc*.) and cause the crucifer black rot disease:

| Essential gene | Description |
|---|---|
| B109F06 (pilC) | Fimbrial assembly protein |
| B205G02 (pilB) | Pilus biogenesis protein |
| B202G01 (iroN) | TonB-dependent receptor |
| B302H04 (wxcM) | Bifunctional acetyl transferase / isomerase |
| A404F11 (wrcC) | Glycosyltransferase |
| C302F11 (wxcA) | Glycosyltransferase |
| A105E01 (nagA) | N-acetylglucosamine-6-phosphate deacetylase |
| C110C10 (rmlA) | Glucose-1-phosphate thymidylyltransferase |
| A202H06 (xpsM) | General secretion pathway protein M |
| C406A04 (xpsK) | General secretion pathway protein K |
| B502F09 (xpsF) | General secretion pathway protein F |
| B405E02 (xpsE) | General secretion pathway protein E |
| B401G05 (virB8) | VirB8 protein |
| C109G03 (engXCA) | Cellulase |
| B101F11 (rpfG) | Response regulator |
| B203D06 | Transducer protein car |
| A308G04 (trpE) | Anthranilate synthase component I |
| B605H08 (trpD) | Anthranilate synthase component II |
| B504A10 (metA) | Homoserine O-acetyltransferase |
| A506A08 (hisF) | Cyclase |
| C206E04 | Probable restriction modification system specificity subunit |
| C111B03 (cypC) | Fatty acid alpha hydroxylase |
| A507C08 (fadB) | P-hydroxycinnamoyl CoA hydratase / lyase |
| B302H05 (uptB) | Maleylacetoacetate isomerase |
| C307G11 (catB) | Catalase (precursor) |
| B104E02 (icd) | Isocitrate dehydrogenase |
| B106G06 (ppsA) | Phosphoenolpyruvate synthase |

| Essential gene) | Description |
|---|---|
| B401E10 | Conserved hypothetical protein |
| C205C01 | Conserved hypothetical proteins |
| C301C03 | Conserved hypothetical protein |
| B407F08 | Conserved hypothetical proteins |
| B410B02 | Conserved hypothetical proteins |
| B606A8 | Inserted between XC0132 and XC0133 |
| B310E10 | Inserted between XC0350 and XC0351 |
| B203A9 | Inserted between XC0408 and XC0409 (bfeA) |
| A505G9 | Inserted between XC0509 (purD) and XC0510 (purH) |
| A301D7 | Inserted between XC2898 and XC2899 (btuB) |
| B306H2 | Inserted between XC3602 and XC3603 (alkB) |
| A105E4 | Inserted between XC3604 (etf-qo) and XC3605 |
| C401H6 | Inserted between XC3604 (etf-qo) and XC3605 |
| B203C8 | Inserted between XC4037 (catB) and XC4038 (dinG) |

• For the bacteria *Xanthomonas axonopodis pathovars (e.g.* pv. *citri* and pv. *manihotis),* known to infect the citrus tree and cassava respectively and cause the citrus canker and the cassava bacterial blight respectively:

| Essential gene | Description |
|---|---|
| XAC1211 (KatE) | monofunctional catalase |
| Xac1815 (XacFhaB) | putative hemagglutinin transporter protein |
| XAC3600 (wzt) | LPS biosynthesis |
| | |
| | |
| AvrBs2 | Type three secretion effector |
| XopX | Type three secretion effector |
| XopZ | Type three secretion effector |
| XopAO1 | Type three secretion effector |
| XopN | Type three secretion effector |
| XopQ | Type three secretion effector |

• For the bacteria *Erwinia amylovora*, known to infect the apple and pear tree and cause the fire blight disease:

| Essential gene |
|---|
| A 7/74SmSp-A20 |
| Ea7/74SmSp-A29 |
| Ea7/74Sm-A33 |
| Ea7174SmSp-A38 |
| Ea7/74Sm-A41 |
| Ea7/74SmSp-A56 |
| Ea7/74SmSp-A64 |
| Ea7/74SmSp-A72 |
| Ea7/74SmSp-A75 |
| Ea7/74SmSp-A76 |
| Ea7/74SmSp-A83 |

• For the bacteria *Dickeya dadantii,* known to infect potato, tomato, eggplant, chicory and cause the soft rot disease:

| Essential gene | Description |
|---|---|
| Dda3937_00335 (glpD) | glycerol-3-phosphate dehydrogenase |
| Dda3937_03379 (purL) | phosphoribosylformyl-glycineamide synthetase |
| Dda3937_03564 (opgG) | Glucans biosynthesis protein G precursor |
| Dda3937_00244 (purH) | phosphoribosylaminoimidazolecarboxamide formyltransferase/IMP cyclohydrolase |
| Dda3937_00532 (hflK) | FtsH protease regulator |
| Dda3937_02515 (purM) | phosphoribosylaminoimidazole synthetase |
| Dda3937_02627 | 4-hydroxythreonine-4-phosphate dehydrogenase |
| Dda3937_00004 (guaB) | IMP dehydrogenase |
| Dda3937_03563 (opgH) | Glucans biosynthesis glucosyltransferase H |
| Dda3937_01284 (pyrB) | aspartate carbamoyltransferase |
| Dda3937_03924 (rffG) | dTDP-glucose 4,6-dehydratase |
| Dda3937_01389 (carB) | carbamoyl-phosphate synthase large subunit |
| Dda3937_03299 (acrA) | MexE family multidrug efflux RND transporter periplasmic adaptator subunit |
| Dda3937_03300 (acrB) | Multidrug efflux system protein |
| Dda3937_03258 (pyrE) | orotate phosphoribosyltransferase |
| Dda3937_02336 (nlpl) | lipoprotein |
| Dda3937_02506 (nlpB) | outer membrane protein assembly factor BamC |
| Dda3937_04018 (pta) | phosphate acetyltransferase |
| Dda3937_03554 (pyrC) | dihydro-orotase |
| Dda3937_04573 (IpxM) | acyl (myristate) transferase |
| Dda3937_01116 (glnG) | Nitrogen regulation protein NR(I), Two-component system |
| Dda3937_02099 (purF) | amidophosphoribosyltransferase |

• For the bacteria *Xylellafastidiosa,* known to infect grapevine and olive trees and cause the pierce's disease on grapevine, the citrus variegated chlorosis, or the olive quick decline syndrome:

| Essential gene | Description |
|---|---|
| rpfC | two-component regulatory protein |
| (PD1709) MopB | major outer membrane protein |
| LesA | putative lipase/esterase |
| PD1703 (LipA) | lipase |
| PD0928 (Zot) | Zonula occludens toxin |
| PD0986 | hemagglutinin-like protein |
| gumD | Exopolysaccharides biosynthesis gene |
| gumH | Exopolysaccharides biosynthesis gene |
| xhpT | response regulators |
| PD0528 (XatA) | autotransporter protein |
| PD0279 (cgsA) | cyclic di-GMP synthase A |
| PD0062 (FimA) | Fimbrial adhesions protein |
| PD0058 (FimF) | Fimbrial adhesions protein |
| PD0731 (XadA) | encoding Xanthomonas adhesin-like protein A |
| PD1792 (HxfB) | hemagglutins |

• For the bacteria *Candidatus Liberibacter solanacearum,* known to infect potato and to cause Zebra Chip disease, and *Candidatus Liberibacter asiaticus* (or *americanuslafricanus*), known to infect citrus and to cause Citrus greening disease:

| Candidatus Liberibacter solanacearum | |
|---|---|
| Essential gene* | Description |
| WP_013462130 | VWA domain-containing protein |
| WP_076969829 | unknown protein |
| WP_013461676 | endonuclease/exonuclease/phosphatase family protein |
| WP_076969883 | unknown protein |
| WP_013462162 | OmpA family protein |
| WP_080550991 | TadE/TadG family protein |
| WP_076969829 | unknown protein |
| WP_076970537 | unknown protein |
| WP_034443047 | unknown protein |
| WP _034441878 | unknown protein |
| WP_076970544 | VWA domain-containing protein |
| WP_076969422 | unknown protein |
| WP_103846864 | VWA domain-containing protein |
| ONI59292 | serralysin |
| WP_013462515 | unknown protein |
| WP_045960760 | unknown protein |
| WP_0134622S9 | unknown protein |
| WP_076969829 | unknown protein |
| WP_013461834 | Na+/H+ antiporter NhaA |
| WP_013461833 | ferredoxin-NADP+ reductase protein |
| * Genes denoted as WP_XXXXXXXXX correspond to "protein_id" of the gene in Candidatus Liberibacter solanacearum CLso-ZC1, complete genome with Genbank accession number NC_014774.1 of April 17, 2017 | |

| Candidatus Liberibacter asiaticus | |
|---|---|
| | |
| WP_012778355 | unknown protein |
| WP_012778427 | unknown protein |
| WP_012778510 | unknown protein |
| WP_012778517 | unknown protein |
| ACT56857 | Serralysin |
| WP_012778607 | VWA domain-containing protein |
| WP_012778668 | unknown protein |
| WP _015452668 | VWA domain-containing protein |
| WP_015452678 | unknown protein |
| WP_015452727 | unknown protein |
| WP_ 015824938 | unknown protein |
| WP_015824940 | unknown protein |
| WP _015452765 | VWA domain-containing protein |
| WP _015452784 | OmpA family protein |
| WP_015452797 | unknown protein |
| WP_015452S37 | unknown protein |
| WP_015824957 | unknown protein |
| WP _015452939 | VWA domain-containing protein |
| ACT56835 | Na+/H+ antiporter NhaA |
| ACT56837 | ferredoxin-NADP+ reductase protein |
| * Genes denoted as WP_XXXXXXXXX correspond to "protein_id" of the gene in Candidatus Liberibacter asiaticus str. psy62, complete genome with Genbank accession number NC_012985.3 of March 30, 2017 | |

Any of these genes can be the target of the EVs of the invention.

In beneficial bacteria, the bacterial genes to be targeted are for examples: genes required for phage production that negatively regulate bacterial survival (e.g. phage baseplate assembly protein GpV), *NolA* and *NodD2* genes from *Bradyrhizobium japonicum* that are known to reduce the expression of *nod* genes at high population densities and therefore to decrease Nod production, a bacterial signal that is essential for symbiotic invasion (knocking-down these genes from inoculant strains should thus result in competitive nodulation), the small non-coding RNA *spot42* encoded by the spot forty-two (*spf*) gene that controls carbohydrate metabolism and uptake (knocking-down this gene from a given bacterium should result in an increased bacterial titer).

In vascular bacteria, the bacterial genes to be targeted are for example type III secretion genes.

When the targeted bacteria are phytopathogenic bacteria, said essential or virulence bacterial genes can be structural genes of secretion systems including the type III secretion system (*e.g*. *HrcC*),
- genes that are required for the proper function of the type VI secretion system (*e.g. TssB*),
- genes encoding master regulators of bacterial effector expression (*e.g. HrpL, HrpG, HrpX*),
- genes encoding factors required for the biosynthesis of phytotoxins (*e.g. Cfa6,* which is important for coronatine biosynthesis in some *Pseudomonas syringae* pathovars),
- genes required for the biosynthesis of virulence compounds (*e.g. XpsR,* which is important for the biosynthesis of exopolysaccharide EPS1, two-component system *RavS*/*RavR*).

The EVs of the invention contain small RNAs that share advantageously sequence homologies with any of these essential genes or virulence genes from the targeted bacterial pathogen species.

In a preferred embodiment, said virulence factor gene or bacterial viability gene is therefore chosen in the group consisting of: *AvrPto, AvrPtoB, HopT1-1, FtsZ, FtsA, cheA, gacA, tolC, pglA, engXCA1, engXCA2, GumH, GumD, XpsE, LesA, HolC, Wp012778355, wp015452784, WP012778510, wp015452939, act56857, wp012778668, GyrB, MreB, RbfA, RsgA, FliA, QseC, Hfq, HrpR, HrpS, RpoD, HrpL, Cfa6, fusA, gyrB, rpoB, RpoC, secE, RpoA, dnaA, dnaN, HrpG, HrpB, HrcC, XpsR, TssB, HrpG, HrpX, RsmA, NadHb, NadHd and NadHe, DnaA, DnaE1, DnaE2, ZipA, PhoP, FhaB, PhoP, rim, ybeY,* and *rplQ.*

In anti-bacterial applications, the EVs of the invention have advantageously sequence homologies with essential genes for the viability or virulence genes from bacterial pathogen species but no sequence homology with commensal bacteria genomes. Such advantageous embodiment of the method avoids collateral effects on the commensal bacteria present in the host.

Particular useful sequences targeting some of these bacterial genes are provided in the EXAMPLE 7 below and in the enclosed sequence listing, notably in SEQ ID NO:1-16 (IR constructs targeting *Xylella fastidiosa* genes), SEQ ID NO:55-60 (IR constructs that target *Candidatus Liberibacter asiaticus* genes), SEQ ID NO:47-54 (IR constructs that target *Erwinia carotovora* genes), SEQ ID NO: 61-67 and 148 (IR constructs targeting *Pseudomonas syringae pv. actinidiae* genes), SEQ ID NO:90-95 and SEQ ID NO:130-145 (IR constructs targeting genes from *Pseudomonas syringae pv. tomato* strain *DC3000),* SEQ ID NO:98-101 (IR constructs targeting genes from *Ralstonia solanacearum),* SEQ ID NO:102-107 (IR constructs targeting genes from *Xanthomonas campestris pv. Campestris),* SEQ ID NO: 17-22 and SEQ ID NO: 128-129 (IR constructs targeting genes from *Xanthomonas hortorum pv. Vitians),* SEQ ID NO: 120-129 (IR constructs targeting genes *from Xanthomonas citri pv. Fucan),* SEQ ID NO:23-30 (IR constructs target genes from *Acidovorax valerianella),* and in SEQ ID NO:31-38 (IR constructs targeting genes from *Acidovorax citrulli).*

### Viral target genes

For anti-viral applications, the EVs of the invention should contain effective small RNAs having a sufficient sequence homology with at least one viral gene in order to induce sequence-specific silencing of said at least one gene. Said viral gene can for example be chosen in the group consisting of: *P1, HC-Pro, CP, RdR, MP, N, 5'LTR, 3'LTR, 5'UTR, 3'UTR, P3, 6kl, CI, 6k2, NIa, Nib, VAP, pro-pol, viroplasmin, CPm, HEL, HSP70h, HSP70 L1, L2, MT, CP-RTD, VSR, VPg, P2, P3, P4, P5, Rapl, p33, p92, p41, p19, p22, AV2, AC4, TrAP,* and *Ben.*

Particular useful sequences targeting viral regions are provided in EXAMPLE 7 below and in the enclosed sequence listing, notably in SEQ ID NO:39-46 (IR constructs targeting genes from *Plum Pox Virus (PPV)).*

### Fungal and oomycete target genes

For anti-fungal applications, the EVs of the invention should contain effective small RNAs having a sufficient sequence homology with at least one fungal gene in order to induce sequence-specific silencing of said at least one gene. Said fungal gene can for example be chosen in the group consisting of: *CYP51A, CYP51B, CYP51C, TOR, CGF1, DCL1, DCL2, LTF1, HBF1, CclA, ACS1, ToxA, ACS2, ELP1, ELP2, MOB2, BrlA2, stuA, F1bc, pks1 , PPT1, NPP1, INF1, GK4, piacwp1-1, piacwp 1-2, piacwp1-3, PITG_17947*, *PITG_10772*, *PITG_13671*, *PITG_16956*, *PITG_00891.*

Particular useful sequences targeting fungal or oomycetes regions are provided in EXAMPLE 7 below and in the enclosed sequence listing, notably in SEQ ID NO:96-97 (IR constructs targeting genes from *Fusarium graminearum),* SEQ ID NO:76-83 (IR constructs target genes from *Botrytis cinereal),* SEQ ID NO: 84-89 (IR constructs targeting genes from *Colletotrichum species),* SEQ ID NO: 108-113 (IR constructs targeting genes from *Zymoseptoria tritici),* SEQ ID NO:68-75 (IR constructs targeting genes from *Phytophthora infestans),* and in SEQ ID NO: 114-119 (IR constructs targeting genes from *Plasmopara viticola).*

### Kit of part with compounds having biocide effects

The EVs of the invention may be applied simultaneously or in succession with other compounds.

In a preferred embodiment, the phytotherapeutic composition of the invention contains, in addition to the EVs of the invention, a biocide compound. This is particularly appropriate when the silencing element of the invention inhibits the expression of a gene that triggers the resistance to said bactericidal compound.

In this case, the composition of the invention may be supplied as a "kit of parts", comprising the EVs of the invention and the corresponding biocide compound in a separate container.

Said kit-of-part preferably contains the phytotherapeutic composition of the invention containing *Chlorella-derived* EVs carrying small RNAs and the corresponding biocide compound.

The invention also relates to the use of said combination product, for inhibiting or preventing the growth or pathogenicity of pathogen(s) on target plants.

In other terms, the present invention relates to a method for treating target plants against pathogen infection, said method comprising the step of introducing into a cell of said target plant a long dsRNA molecule targeting at least one antibacterial resistance gene, and delivering to said plant the corresponding biocide compound.

The invention also relates to a method for treating target plants against pathogen infection, said method comprising the step of delivering the EVs of the invention, or a composition containing same, as well as the corresponding biocide compound, on target plant tissues prior to and / or after bacterial infection.

In this embodiment, the EVs of the invention are preferably applied prior to the biocide compound, for example few hours before, typically, two hours before.

### Transgenic Chlorella cells of the invention

The *Chlorella* cells transformed with the iRNAs of the invention and able to generate the EVs of the invention are hereafter designated as "transgenic *Chlorella* cells of the invention" or "recombinant *Chlorella* cells of the invention" or "host cells of the invention".

These producer cells can be of any *Chlorella* species. In particular, they can be any cells that are currently used as food complement for humans and livestock. In a particular embodiment, they can belong to the species: *Chlorella ellipsoidea, Chlorella pyrenoidosa, Chlorella sorokiniana, Chlorella vulgaris* or *Chlorella variabilis.*

In another aspect, the present invention relates to an isolated *Chlorella* cell or to a transgenic *Chlorella* stably or transiently expressing at least one functional iRNA of the invention. It also relates to an isolated *Chlorella* cell containing a DNA or a viral vector containing the precursor of the invention. Said *Chlorella* cell may be a genetically modified cell obtained by transformation with said DNA construct or vector.

Examples of transformation processes are *Agrobacterium-mediated* transformation or shot-gun-mediated transformation, as described above.

All the embodiments proposed above for the *Chlorella* cells, the iRNAs, the precursor, the vectors, and the transformation methods are herewith encompassed and do not need to be repeated.

Methods to generate such transgenic *Chlorella* are disclosed in the example part below (EXAMPLE 1). They contain the steps of:
i) transforming a *Chlorella* cell with a DNA vector expressing at least one long functional interfering RNA of the invention, as explained above, or,
ii) infecting a *Chlorella* cell with a virus, preferably selected from RNA viruses able to infect *Chlorella* cells, engineered to express at least one functional interfering RNA of the invention from their genomes,
for a sufficient time (typically 3 to 8 days) for the *Chlorella* cell to stably or transiently express a significant amount of small RNAs.

By "significant amount", it is herein meant an amount that has been shown to have an antimicrobial/antiparasitic effect in a test such as described above. This significant amount is preferably comprised between 0.05 to 100 pM of EVs containing the effective small RNAs of the invention.

In particular, said transgenic *Chlorella* is capable of triggering host-induced gene silencing of a pathogen (e.g., a virus or a bacterium), and contains an expressible iRNA, capable of down-regulating or suppressing the expression of at least one gene of said pathogen.

In another aspect, the present invention relates to a target transgenic *Chlorella* stably or transiently expressing the small RNAs described above. In one embodiment, said target transgenic *Chlorella* contains the precursors of the invention, described above.

More precisely, the invention relates to recombinant *Chlorella* cells expressing a siRNA or miRNA precursor comprising a fragment of at least one target gene, said *Chlorella* cells releasing EV-embedded functional small iRNAs targeting said gene fragment.

In one preferred embodiment, said at least one target gene is an oomycete gene, a viral gene, a bacterial gene, or a fungus gene or a gene of any other pathogens or parasites.

In one preferred embodiment, said *Chlorella* cells are chosen from: *Chlorella ellipsoidea, Chlorella pyrenoidosa, Chlorella sorokiniana, Chlorella vulgaris* or *Chlorella variabilis.*

### Platform of the invention

In a final aspect, the invention relates to a versatile platform for producing high throughput amount of functional EV-embedded interfering small RNAs, said platform using the recombinant *Chlorella* cells as defined above.

By "versatile", it is meant that this platform is able to adapt or be adapted to many different functions or activities, generating modulators of a number of different pathogens, in a rapid manner.

This platform is called "MIGS platform". It is useful for producing high amounts of siRNA populations targeting up to 1500 bp long regions in up to a dozen gene. These siRNAs are thus effective against any pathogens, in particular essential genes, which cannot easily accumulate pathogen escape mutations. They are embedded in extracellular vesicles (EVs) that protect small RNAs from ribonuclease-mediated digestion. All the advantages of this platform have been highlighted above.

The inventors are generating reporters for rapidly evaluating the biological activity of each P40 fraction batch produced from transformed *Chlorella* reference lines. More precisely, they engineered bacteria (here the *Escherichia coli* K12 strain) to express a reporter system that exhibits a differential siRNA targeted reporter gene expression when EV-embedded siRNAs are internalized and active in bacterial cells.

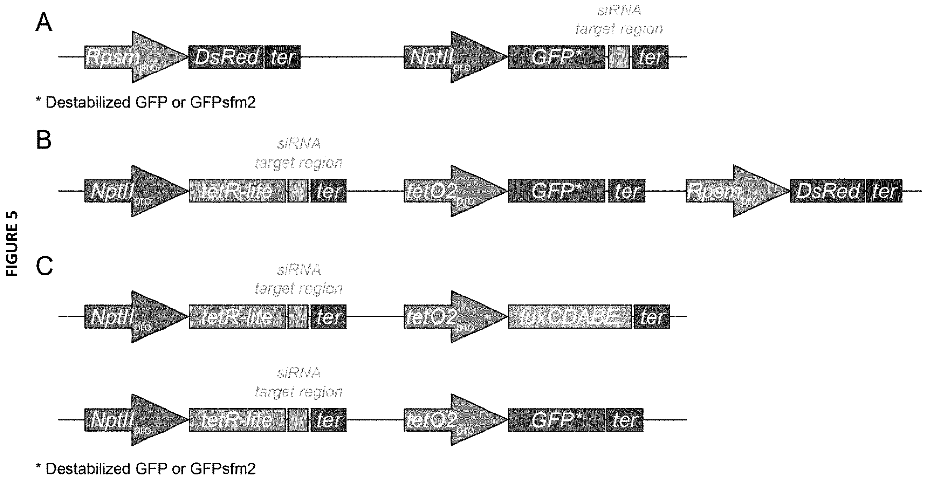

Three different reporter systems are herein proposed:
A first reporter system family relies on the plasmid-based expression of a cassette composed of a first construct constitutively expressing a non-targeted *DsRed* reporter that is used as an internal control for normalization, and a second construct carrying a destabilized *GFP* reporter, containing in its downstream region (or upstream region) the antimicrobial siRNA target region of interest (Figure 5). When expressed in bacteria (e.g. *E. coli*), this system will result in a specific decrease in *GFP* expression and fluorescence signal upon internalization of a given effective EV-embedded siRNA population.

A second dual reporter system family is based on the plasmid expression of a tripartite cassette composed of a first construct expressing a short-lived variant of the transcriptional repressor, namely TetR-lite, carrying in its downstream region the antimicrobial siRNA target region of interest, a second construct composed of an intermediate stability variant of the *GFP* (Andersen et al., 1998; Elowitz & Leibler, 2000), whose transcriptional activity is controlled by the tetO2 (or tetO1) operator, and a third construct expressing a non-targeted *DsRed* reporter, which serves as internal control for normalization (Figure 5). In the absence of EV-embedded small RNA, TetR-lite proteins should be constitutively produced in bacteria and in turn shut-down the expression of the *GFP,* resulting in an absence of GFP fluorescence signal (only the fluorescence of the DsRed reporter should be detected). By contrast, when a given siRNA population is internalized and active in bacterial cells, the silencing of *TetR-lite* results in the derepression of the *GFP* expression, leading to the detection of GFP fluorescence signal.

A third family of reporter system relies on the plasmid-based expression of a bipartite cassette composed of a first construct expressing a short-lived variant of the transcriptional repressor, namely TetR-lite, carrying in its downstream region (or upstream region) the antimicrobial siRNA target region of interest and a second construct composed of the *GFP* (Andersen et al., 1998; Elowitz & Leibler, 2000), or a bioluminescence reporter (*e.g.* the *Photorhabdus luminescens* operon *luxCDABE* (Meighen, 1991), whose transcriptional activity is controlled by the tetO2 (or tetO1) operator. When a given siRNA population is internalized and active in bacterial cells, the silencing of *TetR-lite* results in the derepression of the *GFP* or *luxCDABE* operon expression, leading to the detection of GFP fluorescence or bioluminescence signals. Of note, other systems than TetR-tetO2 could also be used for the same purpose, such as the *lacI-lite*/*lacO* or *cl-lite*/*λPR* systems.

These reporter systems are also part of the invention and will be instrumental to validate the biological activities or EV-embedded siRNA batches prior product manufacturing.

In addition to the above arrangements, the invention also comprises other arrangements, which will emerge from the description that follows, which refers to exemplary embodiments of the subject of the present invention, with reference to the attached drawings and Table of sequences in which:

| **SEQ ID NO:** | **Name/details** |
|---|---|
| **1** | Sequence of the first arm of the *cheA*/*gaCA*/*tolC*/*pglA*/*engXCA1*/*engXCA2* dsRNA used to concomitantly target *cheA, gaCA*, *tolC, pglA*, *engXCA1*, *engXCA2* genes and regions from *Xylella fastidiosa* |
| **2** | Sequence of the second arm of the *cheA*/*gaCA*/*tolC*/*pglA*/*engXCA1*/*engXCA2* dsRNA used to concomitantly target *cheA, gaCA*, *tolC, pglA*, *engXCA1*, *engXCA2* genes and regions from *Xylella fastidiosa* |
| **3** | Sequence of the first arm of the *cheA*/*GumH*/*GumD*/*XpsE*/*LesA*/*HolC* dsRNA used to concomitantly target *cheA*, *GumH*, *GumD*, *Xpse*, *LesA, HolC* genes and regions from *Xylella fastidiosa* |
| **4** | Sequence of the second arm of the *cheA*/*GumH*/*GumD*/*XpsE*/*LesA*/*HolC* dsRNA used to concomitantly target *cheA*, *GumH*, *GumD*, *Xpse*, *LesA, HolC* genes and regions from *Xylella fastidiosa* |
| **5** | Sequence of the first arm of the *LesA*/*GumH*/*GumD* dsRNA used to concomitantly target *LesA, GumH*, *GumD* genes and regions from *Xylella fastidiosa* |
| **6** | Sequence of the second arm of the *LesA*/*GumH*/*GumD* dsRNA used to concomitantly target *LesA, GumH*, *GumD* genes and regions from *Xylella fastidiosa* |
| **7** | Sequence of the first arm of the *XpsE*/*HolC*/*LesA* dsRNA used to concomitantly target *XpsE, HolC, LesA* genes and regions from *Xylella fastidiosa* |
| **8** | Sequence of the second arm of the *XpsE*/*HolC*/*LesA* dsRNA used to concomitantly target *XpsE, HolC, LesA* genes and regions from *Xylella fastidiosa* |
| **9** | Sequence of the first arm of the *CheA*/*pglA*/*LesA* dsRNA used to concomitantly target *CheA, pglA*, *lesA* genes and regions from *Xylella fastidiosa* |
| **10** | Sequence of the second arm of the *CheA*/*pglA*/*LesA* dsRNA used to concomitantly target *CheA, pglA*, *lesA* genes and regions from *Xylella fastidiosa* |
| **11** | Sequence of the first arm of the *cheA*/*engXCA1*/*engXCA2* dsRNA used to concomitantly target *cheA*, *engXCA1*, *engXCA2* genes and regions from *Xylella fastidiosa* |
| **12** | Sequence of the second arm of the *cheA*/*engXCA1*/*engXCA2* dsRNA used to concomitantly target *cheA*, *engXCA1*, *engXCA2* genes and regions from *Xylella fastidiosa* |
| **13** | Sequence of the first arm of the *gacA*/*pglA*/*engXCA2* dsRNA used to concomitantly target *gacA, pglA*, *engXCA2* genes and regions from *Xylella fastidiosa* |
| **14** | Sequence of the second arm of the *gacA*/*pglA*/*engXCA2* dsRNA used to concomitantly target *gacA*, *pglA*, *engXCA2* genes and regions from *Xylella fastidiosa* |
| **15** | Sequence of the first arm of the *cheA*/*tolC*/*engXCA1* dsRNA used to concomitantly target *cheA*, *tolC*, *engXCA1* genes and regions from *Xylella fastidiosa* |
| **16** | Sequence of the second arm of the *cheA*/*tolC*/*engXCA1* dsRNA used to concomitantly target *cheA*, *tolC*, *engXCA1* genes and regions from *Xylella fastidiosa* |
| **17** | Sequence of the first arm of the *gyrB* dsRNA used to target the gyrase subunit B gene from *Xanthomonas hortorum vitians* |
| **18** | Sequence of the second arm of the *gyrB* dsRNA used to target the gyrase subunit B gene from *Xanthomonas hortorum vitians* |
| **19** | Sequence of the first arm of the *fusA* dsRNA used to target the fusA elongator factor G gene from *Xanthomonas hortorum vitians* |
| **20** | Sequence of the second arm of the *fusA* dsRNA used to target the fusA elongator factor G gene from *Xanthomonas hortorum vitians* |
| **21** | Sequence of the first arm of the *ZipA* dsRNA used to target the *ZipA* gene from *Xanthomonas hortorum vitians* |
| **22** | Sequence of the second arm of the *ZipA* dsRNA used to target the *ZipA* gene from *Xanthomonas hortorum vitians* |
| **23** | Sequence of the first arm of the *rimM*/*rsgA*/*rbfA*/*mreB*/*gyrB*/*ftsZ* dsRNA used to concomitantly target *rimM*, *rsgA*, *rbfA*, *mreB*, *gyrB*, *ftsZ* genes from *Acidovorax valerianella* |
| **24** | Sequence of the second arm of the *rimM*/*rsgA*/*rbfA*/*mreB*/*gyrB*/*ftsZ* dsRNA used to concomitantly target *rimM*, *rsgA*, *rbfA*, *mreB*, *gyrB, ftsZ* genes from *Acidovorax valerianella* |
| **25** | Sequence of the first arm of the *rimM*/*rbfA*/*ftsZ* dsRNA used to concomitantly target *rimM, rbfA*, *ftsZ* genes from *Acidovorax valerianella* |
| **26** | Sequence of the second arm of the *rimM*/*rbfA*/*ftsZ* dsRNA used to concomitantly target *rimM, rbfA*, *ftsZ* genes from *Acidovorax valerianella* |
| **27** | Sequence of the first arm of the *RsgA*/*gyrB*/*MreB* dsRNA used to concomitantly target *RsgA, gyrB, MreB* genes from *Acidovorax valerianella* |
| **28** | Sequence of the second arm of the *RsgA*/*gyrB*/*MreB* dsRNA used to concomitantly target *RsgA, gyrB, MreB* genes from *Acidovorax valerianella* |
| **29** | Sequence of the first arm of the *RsgA*/*rbfA*/*FtsZ* dsRNA used to concomitantly target *RsgA, rbfA*, *FtsZ* genes from *Acidovorax valerianella* |
| **30** | Sequence of the second arm of the *RsgA*/*rbfA*/*FtsZ* dsRNA used to concomitantly target *RsgA, rbfA*, *FtsZ* genes from *Acidovorax valeianella* |
| **31** | Sequence of the first arm of the *mreB*/*ybeY*/*rbfA*/*gyrB*/*ftsZ*/*rsgA* dsRNA used to concomitantly target the *mreB*, *ybeY*, *rbfA*, *gyrB*, *ftsZ*, *rsgA* genes from *Acidovorax citrulli* |
| **32** | Sequence of the second arm of the *mreB*/*ybeY*/*rbfA*/*gyrB*/*ftsZ*/*rsgA* dsRNA used to concomitantly target the *mreB*, *ybeY*, *rbfA*, *zvrB*, *ftsZ*, *rsgA* genes from *Acidovorax citrulli* |
| **33** | Sequence of the first arm of the *mreB*/*ybeY*/*rbfA* dsRNA used to concomitantly target the *mreB, ybeY, rbfA* genes from *Acidovorax citrulli* |
| **34** | Sequence of the second arm of the *mreB*/*ybeY*/*rbfA* dsRNA used to concomitantly target the *mreb, ybeY, rbfA* genes from *Acidovorax citrulli* |
| **35** | Sequence of the first arm of the *rsgA*/*GyrB*/*MreB* dsRNA used to concomitantly target the *RsgA, GyrB, MreB* genes from *Acidovorax citrulli* |
| **36** | Sequence of the second arm of the *rsgA*/*GyrB*/*MreB* dsRNA used to concomitantly target the *RsgA, GyrB, MreB* genes from *Acidovorax citrulli* |
| **37** | Sequence of the first arm of the *YbeY*/*RsgA*/*MreB* dsRNA used to concomitantly target the *YbeY*, *RsgA*, *MreB* genes from *Acidovorax citrulli* |
| **38** | Sequence of the second arm of the *YbeY*/*RsgA*/*MreB* dsRNA used to concomitantly target the *YbeY*, *RsgA*, *MreB* genes from *Acidovorax citrulli* |
| **39** | Sequence of the first arm of the *P1*/*HC-Pro*/*CP* dsRNA used to concomitantly target the *P1, HC-Pro, coat protein* genes from the PIPVgp1 sequence of the *Plum pox virus* |
| **40** | Sequence of the second arm of the *P1*/*HC-Pro*/*CP* dsRNA used to concomitantly target the *P1, HC-Pro, coat protein* genes from the PIPVgp1 sequence of the *Plum pox virus* |
| **41** | Sequence of the first arm of the P1 dsRNA used to target the P1 gene from the PIPVgp1 sequence of the *Plum pox virus* |
| **42** | Sequence of the second arm of the *P1* dsRNA used to target the P1 gene from the PIPVgp 1 sequence of the *Plum pox virus* |
| **43** | Sequence of the first arm of the *HC-Pro* dsRNA used to target the *HC-Pro* gene from the PIPVgp1 sequence of the *Plum pox virus* |
| **44** | Sequence of the second arm of the HC-Pro dsRNA used to target the *HC-Pro* gene from the PIPVgp1 sequence of the *Plum pox virus* |
| **45** | Sequence of the first arm of the CP dsRNA used to target the coat protein gene from the PIPVgp1 sequence of the *Plum pox virus* |
| **46** | Sequence of the second arm of the CP dsRNA used to target the coat protein gene from the PIPVgp1 sequence of the *Plum pox virus* |
| **47** | Sequence of the first arm of the *gyrB*/*MreB*/*rbfA*/*RsgA*/*FliA*/*QseC* dsRNA used to |
| | concomitantly target the *GyrB, MreB, RbfA, RsgA, FliA*, *QseC* genes from *Erwinia carotovora* |
| **48** | Sequence of the second arm of the *gyrB*/*MreB*/*rbfA*/*RsgA*/*FliA*/*QseC* dsRNA used to concomitantly target the *GyrB, MreB, RbfA, RsgA, FliA*, *QseC* genes from *Erwinia carotovora* |
| **49** | Sequence of the first arm of the *gyrB*/*rbfA*/*QseC* dsRNA used to concomitantly target the *GyrB, RbfA, QseC* genes from *Erwinia carotovora* |
| **50** | Sequence of the second arm of the *gyrB*/ *rbfA*/*QseC* dsRNA used to concomitantly target the *GyrB, RbfA, QseC* genes from *Erwinia carotovora* |
| **51** | Sequence of the first arm of the *fliA*/*MreB*/*QseC* dsRNA used to concomitantly target the *FliA, MreB, QseC* genes from *Erwinia carotovora* |
| **52** | Sequence of the second arm of the *fliA*/*MreB*/*QseC* dsRNA used to concomitantly target the *FliA*, *MreB*, *QseC* genes from *Erwinia carotovora* |
| **53** | Sequence of the first arm of the *GyrB*/*MreB*/*RsgA* dsRNA used to concomitantly target the *GyrB, MreB*, *RsgA* genes from *Erwinia carotovora* |
| **54** | Sequence of the second arm of the *GyrB*/*MreB*/*RsgA* dsRNA used to concomitantly target the *GyrB, MreB*, *RsgA* genes from *Erwinia carotovora* |
| **55** | Sequence of the first arm of the Wp012778355/wp015452784/WP012778510/wp015452939/act56857/wp012778668dsRNA used to concomitantly target the sequences encoding the proteins Wp012778355/wp015452784/WP012778510/wp015452939/act56857/wp012778668 from *Candidatus liberibacter asiaticus* |
| **56** | Sequence of the second arm of the Wp012778355/wp015452784/WP012778510/wp015452939/act56857/wp012778668 dsRNA used to concomitantly target the sequences encoding the proteins Wp012778355/wp015452784/WP012778510/wp015452939/act56857/wp012778668 from *Candidatus liberibacter asiaticus* |
| **57** | Sequence of the first arm of the Wp012778355/ wp015452784/WP012778510 dsRNA used to concomitantly target the sequences encoding the proteins Wp012778355/wp015452784/WP012778510 from *Candidatus liberibacter asiaticus* |
| **58** | Sequence of the first arm of the Wp012778355/ wp015452784/WP012778510 dsRNA used to concomitantly target the sequences encoding the proteins Wp012778355/wp015452784/WP012778510 from *Candidatus liberibacter asiaticus* |
| **59** | Sequence of the first arm of the wp015452939/act56857/wp012778668 dsRNA used to concomitantly target the sequences encoding the proteins wp015452939/act56857/wp012778668 from *Candidatus liberibacter asiaticus* |
| **60** | Sequence of the second arm of the wp015452939/act56857/wp012778668 dsRNA used to concomitantly target the sequences encoding the proteins wp015452939/act56857/wp012778668 from *Candidatus liberibacter asiaticus* |
| **61** | Sequence of the first arm of the *GyrB*/*Hfq*/*HrpR*/*HRPS*/*MreB*/*RpoD* dsRNA used to concomitantly target the *GyrB, Hfq*, *HrpR*, *HrpS*, *MreB*, *RpoD* genes from *Pseudomonas syringae pv actinidiae* |
| **62** | Sequence of the second arm of the *GyrB*/*Hfq*/*HrpR*/*HRPS*/*MreB*/*RpoD* dsRNA used to concomitantly target the *GyrB, Hfq*, *HrpR*, *HrpS*, *MreB*, *RpoD* genes from *Pseudomonas syringae pv actinidiae* |
| **63** | Sequence of the first arm of the *GyrB*/*Hfq*/*MreB* dsRNA used to concomitantly target the *GyrB, Hfq, MreB* genes from *Pseudomonas syringae pv actinidiae* |
| **64** | Sequence of the second arm of the *GyrB*/*Hfq*/*MreB* dsRNA used to concomitantly target the *GyrB, Hfq, MreB* genes from *Pseudomonas syringae pv actinidiae* |
| **65** | Sequence of the first arm of the *HrpR*/*HrpS*/*Hfq* dsRNA used to concomitantly target the *HrpR, HrpS, Hfq* genes from *Pseudomonas syringae pv actinidiae* |
| **66** | Sequence of the second arm of the *HrpR*/*HrpS*/*Hfq* dsRNA used to concomitantly target the *HrpR*, *HrpS*, *Hfq* genes from *Pseudomonas syringae pv actinidiae* |
| **67** | Sequence of the first arm of the *HrpR*/ *GyrB*/*RpoD* dsRNA used to concomitantly target the *HrpR, GyrB, RpoD* genes from *Pseudomonas syringae pv actinidiae* |
| **68** | Sequence of the first arm of the *NPP1*/*INF1*/*GK4*/*piacwp1-1*/*piacwp1-2*/*piacwp1-3* dsRNA used to concomitantly target the *NPP1, INF1, GK4, piacwp1-1, piacwp 1-2 and piacwpl-3* genes from *Phytophtora infestans* |
| **69** | Sequence of the second arm of the *NPP1*/*INF1*/*GK4*/*piacwp1-1*/*piacwp1-2*/*piacwp1-3* dsRNA used to concomitantly target the *NPP1, INF1, GK4, piacwp1-1, piacwp 1-2 and piacwp1-3* genes from *Phytophtora infestans* |
| **70** | Sequence of the first arm of the *piacwp1-1*/*piacwp1-2*/*piacwp1-3* dsRNA used to concomitantly target the *piacwp1-1, piacwp 1-2 and piacwp1-3* genes from *Phytophthora infestans* |
| **71** | Sequence of the second arm of the *piacwp1-1*/*piacwp1-2*/*piacwp1-3* dsRNA used to concomitantly target the *piacwp1-1, piacwp 1-2 and piacwp1-3* genes from *Phytophthora infestans* |
| **72** | Sequence of the first arm of the *GK4*/*INF1*/*NPP1* dsRNA used to concomitantly target the *GK4, INF1, NPP1* genes from *Phytophtora infestans* |
| **73** | Sequence of the second arm of the *GK4*/*INF1*/*NPP1* dsRNA used to concomitantly target the *GK4, INF1, NPP1* genes from *Phytophtora infestans* |
| **74** | Sequence of the first arm of the *GK4*/*INF1*/*piacwp1.1* dsRNA used to concomitantly target the *GK4, INF1, piacwp1.1* genes from *Phytophthora infestans* |
| **75** | Sequence of the second arm of the *GK4*/*INF1*/*piacwp1.1* dsRNA used to concomitantly target the *GK4, INF1, piacwp1. 1* genes from *Phytophthora infestans* |
| **76** | Sequence of the first arm of the *TOR*/ *CGF1*/ *DCL1*/*DCL2*/*LTF1*/*HBF1* dsRNA used to concomitantly target the *TOR*/*CGF1l DCL1*/*DCL2*/*LTF1*/*HBF1* genes from *Botrytis cinerea* |
| **77** | Sequence of the second arm of the *TOR*/*CGF1*/ *DCL1*/*DCL2*/*LTF1*/*HBF1* dsRNA used to concomitantly target the *TOR*/*CGF1l DCL1*/*DCL2*/*LTF1*/*HBF1* genes from *Botrytis cinerea* |
| **78** | Sequence of the first arm of the *TOR*/*DCL1*/*DCL2* dsRNA used to concomitantly target the *TOR, DCL1, DCL2* genes from *Botrytis cinerea* |
| **79** | Sequence of the second arm of the *TOR*/*DCL1*/*DCL2* dsRNA used to concomitantly target the *TOR, DCL1, DCL2* genes from *Botrytis cinerea* |
| **80** | Sequence of the first arm of the *CGF1*/*HBF1*/*LTF1* dsRNA used to concomitantly target the *CGF1, HBF1, LTF1* genes from *Botrytis cinerea* |
| **81** | Sequence of the first arm of the *CGF1*/*HBF1*/*LTF1* dsRNA used to concomitantly target the *CGF1, HBF1, LTF1* genes from *Botrytis cinerea* |
| **82** | Sequence of the first arm of the *DCL1*/*HBF1*/*TOR* dsRNA used to concomitantly target the *DCL1, HBF1, TOR* genes from *Botrytis cinerea* |
| **83** | Sequence of the second arm of the *DCL1*/*HBF1*/*TOR* dsRNA used to concomitantly target the *DCL1, HBF1, TOR* genes from *Botrytis cinerea* |
| **84** | Sequence of the first arm of the *CclA*/*ACS1*/*ACS2*/*ELP1*/*ELP2*/*MOB2* dsRNA used to concomitantly target the *CclA*/*ACS1*/*ACS2*/*ELP1*/*ELP2*/*MOB2* genes from *Colletotrichum higginsianum* |
| **85** | Sequence of the second arm of the *CclA*/*ACS1*/*ACS2*/*ELP1*/*ELP2*/*MOB2* dsRNA used to concomitantly target the *CclA*/*ACS1*/*ACS2*/*ELP1*/*ELP2*/*MOB2* genes from *Colletotrichum higginsianum* |
| **86** | Sequence of the first arm of the *CclA*/*ACS1*/*ACS2* dsRNA used to concomitantly target the *CclA*/*ACS1*/*ACS2* genes from *Colletotrichum higginsianum* |
| **87** | Sequence of the second arm of the *CclA*/*ACS1*/*ACS2* dsRNA used to concomitantly target the *CclA*/*ACS1*/*ACS2* genes from *Colletotrichum higginsianum* |
| **88** | Sequence of the first arm of the *ELP1*/*ELP2*/*MOB2* dsRNA used to concomitantly target the *ELP1*/*ELP2*/*MOB2* genes from *Colletotrichum higginsianum* |
| **89** | Sequence of the second arm of the *ELP1*/*ELP2*/*MOB2* dsRNA used to concomitantly target the *ELP1*/*ELP2*/*MOB2* genes from *Colletotrichum higginsianum* |
| **90** | Sequence of the first arm of the *CFA6*/*HRPL* dsRNA used to concomitantly target the *HrpL* and *Cfa6* genes of *Pto DC3000* |
| **91** | Sequence of the second arm of the *CFA6*/*HRPL* dsRNA used to concomitantly target the *HrpL* |
| | and *Cfa6* genes of *Pto DC3000* |
| **92** | Sequence of the first arm of the *HRPL* dsRNA used to target the *HrpL* gene of *Pto DC3000* |
| **93** | Sequence of the second arm of the *HRPL* dsRNA used to target the *HrpL* gene of *Pto DC3000* |
| **94** | Sequence of the first arm of the *AvrPto*/*AvrPtoB* dsRNA used to target the *AvrPto* and *AvrPtoB* genes of *Pto DC3000* |
| **95** | Sequence of the second arm of the *AvrPto*/*AvrPtoB* dsRNA used to target the *AvrPto* and *AvrPtoB* genes of *Pto DC3000* |
| **96** | Sequence of the first arm of the *CYP51* dsRNA used to target the *FgCYP51A, FgCYP51B* and *FgCYP51C* genes of *Fusarium graminearum* |
| **97** | Sequence of the second arm of the *CYP51* dsRNA used to target the *FgCYP51A, FgCYP51B* and *FgCYP51C* genes of *Fusarium graminearum* |
| **98** | Sequence of the first arm of the *HRPG*/*HRPB*/*HRCC* dsRNA used to target concomitantly the *HrpG*, *HrpB* and *HrcC* genes of *Ralstonia solanacearum* |
| **99** | Sequence of the second arm of the *HRPG*/*HRPB*/*HRCC* dsRNA used to target concomitantly the *HrpG*, *HrpB* and *HrcC* genes of *Ralstonia solanacearum* |
| **100** | Sequence of the first arm of the *HRPB*/*HRCC*/*TssB*/*XpsR* dsRNA used to target concomitantly the *HrpB, HrcC, XpsR* and *TssB* genes of Ralstonia solanacearum |
| **101** | Sequence of the second arm of the *HRPB*/*HRCC*/*TssB*/*XpsR* dsRNA used to target concomitantly the *HrpB*, *HrcC*, *XpsR* and *TssB* genes of *Ralstonia solanacearum* |
| **102** | Sequence of the first arm of the *HRPG*/*HRPX*/*RsmA* dsRNA used to target concomitantly the *HrpG*, *HrpX*, and *RsmA* genes of *Xanthomonas campestris* pv. *campestris* |
| **103** | Sequence of the second arm of the *HRPG*/*HRPX*/*RsmA* dsRNA used to target concomitantly the *HrpG*, *HrpX*, and *RsmA* genes of *Xanthomonas campestris* pv. *campestris* |
| **104** | Sequence of the first arm of the *NadHb*/*NadHd*/*NadHe* dsRNA used to target concomitantly the *NadHb, NadHd* and *NadHe* genes of *Xanthomonas campestris* pv. *campestris* (and likely other Xanthomonas species) |
| **105** | Sequence of the second arm of the *NadHb*/*NadHd*/*NadHe* dsRNA used to target concomitantly the *NadHb*, *NadHd* and *NadHe* genes of *Xanthomonas campestris* pv. *campestris* (and likely other Xanthomonas species) |
| **106** | Sequence of the first arm of the *DnaA*/*DnaE1*/*DnaE2* dsRNA used to target concomitantly the *DnaA*, *DnaE1* and *DnaE2* genes of *Xanthomonas campestris* pv. *campestris* (and likely other species) |
| **107** | Sequence of the second arm of the *DnaA*/*DnaE1*/*DnaE2* dsRNA used to target concomitantly the *DnaA*, *DnaE1* and *DnaE2* genes of *Xanthomonas campestris* pv. *campestris* (and likely other species) |
| **108** | Sequence of the first arm of the *Br1A2*/*stuA*/*F1bc*/*pks1*/*PPT1* dsRNA used to concomitantly target the *Br1A2*/*stuA*/*Flbc*/*pks1*/*PPT1* genes from *Zymoseptoria tritici* |
| **109** | Sequence of the second arm of the *Br1A2*/*stuA*/*F1bc*/*pks1*/*PPT1* dsRNA used to concomitantly target the *Br1A2*/*stuA*/*Flbc*/*pks1*/*PPT1* genes from *Zymoseptoria tritici* |
| **110** | Sequence of the first arm of the *Br1A2*/*stuA*/*F1bc* dsRNA used to concomitantly target the *Br1A2*/*stuA*/*F1bc* genes from *Zymoseptoria tritici* |
| **111** | Sequence of the second arm of the *Br1A2*/*stuA*/*F1bc* dsRNA used to concomitantly target the *Br1A2*/*stuA*/*F1bc* genes from *Zymoseptoria tritici* |
| **112** | Sequence of the first arm of the *stuA*/*pks1*/*PPT1* dsRNA used to concomitantly target the *stuA*/*pks1*/*PPT1* genes from *Zymoseptoria tritici* |
| **113** | Sequence of the second arm of the *stuA*/*pks1*/*PPT1* dsRNA used to concomitantly target the *stuA*/*pks1*/*PPT1* genes from *Zymoseptoria tritici* |
| **114** | Sequence of the first arm of the chitinase/elicitin/glucanase inhibitor, transglutaminase elicitor/ secretory protein dsRNA used to concomitantly target genes producing the protein defined by PITG_17947, PITG_10772, PITG_13671, PITG 16956, PITG_00891 from *Plasmopora viticola* |
| **115** | Sequence of the second arm of the chitinase/ elicitin/glucanase inhibitor, transglutaminase elicitor/ secretory protein dsRNA used to concomitantly target genes producing the protein |
| | defined by PITG 17947, PITG_10772, PITG 13671, PITG 16956, PITG 00891 from *Plasmopora viticola* |
| **116** | Sequence of the first arm of the chitinase/elicitin/glucanase inhibitor dsRNA used to concomitantly target genes producing the protein defined by PITG 17947, PITG 10772, PITG 13671 from *Plasmopora viticola* |
| **117** | Sequence of the second arm of the chitinase/elicitin/glucanase inhibitor dsRNA used to concomitantly target genes producing the protein defined by PITG 17947, PITG 10772, PITG 13671 from *Plasmopora viticola* |
| **118** | Sequence of the first arm of the glucanase inhibitor, transglutaminase elicitor/ secretory protein dsRNA used to concomitantly target genes producing the protein defined by PITG 13671, PITG 16956, PITG 00891 from *Plasmopora viticola* |
| **119** | Sequence of the second arm of the glucanase inhibitor, transglutaminase elicitor/ secretory protein dsRNA used to concomitantly target genes producing the protein defined by PITG 13671, PITG 16956, PITG 00891 from *Plasmopora viticola* |
| **120** | Sequence of the first arm of the *GyrB*/*FusA*/*MreB*/*HrpG*/*PhoP*/*FhaB* dsRNA used to concomitantly target the *GyrB*, *FusA*, *MreB*, *HrpG*, *PhoP*, *FhaB* genes from *Xanthomonas citri pv fuscan* |
| **121** | Sequence of the second arm of the *GyrB*/*FusA*/*MreB*/*HrpG*/*PhoP*/*FhaB* dsRNA used to concomitantly target the *GyrB, FusA*, *MreB*, *HrpG*, *PhoP*, *FhaB* genes from *Xanthomonas citri pv fuscan* |
| **122** | Sequence of the first arm of the *GyrB*/*RbfA*/*MreB* dsRNA used to concomitantly target the *GyrB, RbfA*, *MreB* genes from *Xanthomonas citri pv fuscan* |
| **123** | Sequence of the second arm of the *GyrB*/*RbfA*/*MreB* dsRNA used to concomitantly target the *GyrB, RbfA*, *MreB* genes from *Xanthomonas citri pv fuscan* |
| **124** | Sequence of the first arm of the *HrpG*/*PhoP*/*FtsZ* dsRNA used to concomitantly target the *HrpG*, *PhoP*, *FtsZ* genes from *Xanthomonas citri pv fuscan* |
| **125** | Sequence of the second arm of the *HrpG*/*PhoP*/*FtsZ* dsRNA used to concomitantly target the *HrpG*, *PhoP*, *FtsZ* genes from *Xanthomonas citri pv fuscan* |
| **126** | Sequence of the first arm of the *FhaB*/*FusA*/*MreB* dsRNA used to concomitantly target the *HrpG*, *PhoP*, *FtsZ* genes from *Xanthomonas citri pv fuscan* |
| **127** | Sequence of the second arm of the *FhaB*/*FusA*/*MreB* dsRNA used to concomitantly target the *HrpG*, *PhoP*, *FtsZ* genes from *Xanthomonas citri pv fuscan* |
| **128** | Sequence of the first arm of the *GyrB*/*FusA*/*ZipA* dsRNA used to concomitantly target *GyrB, FusA, ZipA* genes from *Xanthomonas hortorum pv vitians* |
| **129** | Sequence of the second arm of the *GyrB*/*FusA*/*ZipA* dsRNA used to concomitantly target *GyrB*, *FusA, ZipA* genes from *Xanthomonas hortorum pv vitians* |
| **130** | Sequence of the first arm of the *AvrPto*/*AvrPtoB*/*HopT1-1* dsRNA used to concomitantly target the *AvrPto*, *AvrPtoB*, *HopT1-1* genes from *Pseudomonas syringae pv. tomato* strain *DC3000* |
| **131** | Sequence of the second arm of the *AvrPto*/*AvrPtoB*/*HopT1-1* dsRNA used to concomitantly target the *AvrPto*, *AvrPtoB*, *HopT1-1* genes from *Pseudomonas syringae pv. tomato* strain *DC3000* |
| **132** | Sequence of the first arm of the *rpoB*/*rpoC*/*fusA* dsRNA used to concomitantly target the *rpoB, RpoC* and *fusA* genes from *Pto DC3000* |
| **133** | Sequence of the second arm of the *rpoB*/*rpoC*/*fusA* dsRNA used to concomitantly target the *rpoB, RpoC* and *fusA* genes from *Pto DC3000* |
| **134** | Sequence of the first arm of the *secE*/*rpoA*/*rplQ* dsRNA used to concomitantly target the *secE*, *RpoA* and *rplQ* genes from *Pto DC3000* |
| **135** | Sequence of the second arm of the *secE*/*rpoA*/*rplQ* dsRNA used to concomitantly target the *secE*, *RpoA* and *rplQ* genes from *Pto DC3000* |
| **136** | Sequence of the first arm of the *fusA* dsRNA used to target the *fusA* gene from *Pto DC3000* |
| **137** | Sequence of the second arm of the *fusA* dsRNA used to target the *fusA* gene from *Pto DC3000* |
| **138** | Sequence of the first arm of the *gyrB* dsRNA used to target the *gyrB* gene from *Pto DC3000* |
| **139** | Sequence of the second arm of the *gyrB* dsRNA used to target the *gyrB* gene from *Pto DC3000* |
| **140** | Sequence of the first arm of the *fusA*/*gyrB* dsRNA used to concomitantly target the *fusA* and *zvrb* genes from *Pto DC3000* |
| **141** | Sequence of the second arm of the *fusA*/*gyrB* dsRNA used to concomitantly target the *fusA* and *gyrB* genes from *Pto DC3000* |
| **142** | Sequence of the first arm of the *secE* dsRNA used to target the *secE* gene from *Pto DC3000* |
| **143** | Sequence of the second arm of the *secE* dsRNA used to target the *secE* gene from *Pto DC3000* |
| **144** | Sequence of the first arm of the *gyrB*/*hrpL* dsRNA used to concomitantly target the *gyrB* and *hrpL* genes from *Pto DC3000* |
| **145** | Sequence of the second arm of the *gyrB*/*hrpL* dsRNA used to concomitantly target the *gyrB* and *hrpL* genes from *Pto DC3000* |
| **146** | Sequence of the first arm of the *dnaA*/*dnaN*/*gyrB* dsRNA used to concomitantly target the *dnaA*, *dnaN* and *gyrB* genes from *Pto DC3000* |
| **147** | Sequence of the second arm of the *dnaA*/*dnaN*/*gyrB* dsRNA used to concomitantly target the *dnaA*, *dnaN* and *gyrB* genes from *Pto DC3000* |
| **148** | Sequence of the second arm of the *HrpR*/ *GyrB*/*RpoD* dsRNA used to concomitantly target the *HrpR**,** GyrB*, *RpoD* genes from *Pseudomonas syringae pv. actinidiae* |
| **149** | Sequence of a specific intron sequence from the *Petunia Chalcone* synthase gene *CHSA* |

### FIGURE LEGENDS

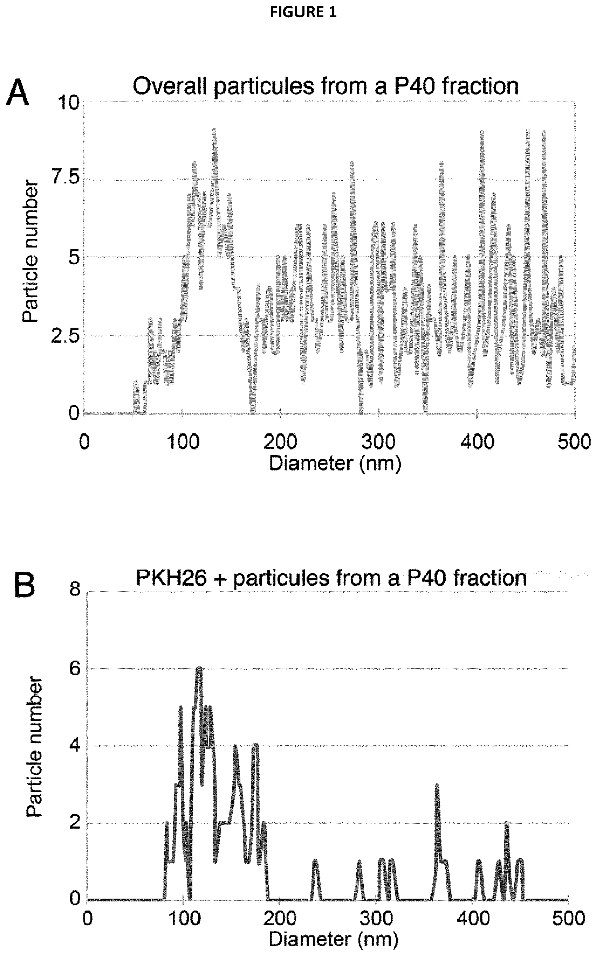

**Figure 1****. Scattering and fluorescence NTA analyses of *Chlorella* EVs**
   A) Size distribution of overall *Chlorella* particles from P40 fractions, determined through scattering analysis (using the Particle Metrix ZetaView system).
   B) Size distribution of PKH26-labeled *Chlorella* particles from P40 fractions (using the Particle Metrix ZetaView system). The measurements were performed using the 488 nm laser.
**Figure 2****. Phylogenetic analysis of *C*. *variabilis* AGO and DCL proteins and stomatal reopening assay using total RNAs from transgenic *C*. *vulgaris* lines**
   A) NJ trees (1000 bootstraps) including 111 AGO and 77 DCL sequences, respectively, from plants, animals, fungi and algae. The position of the C. *variabilis* AGO and DCL proteins are shown. The trees are midpoint rooted.
   B) Schematic representation of the IT20 construct designed to express siRNAs targeting the *Pto* DC3000 *cfa6* and *hrpL* mRNAs under the control of the constitutive Cauliflower Mosaic Virus (CaMV) 35S promoter. The chimeric 504 bp region targeting the two virulence genes has been cloned in direct (B module) and antiparallel (D module) orientations using the Green Gate assembly strategy.
   C) Stomatal reopening assay at 3 hpi on leaf sections incubated with water (Mock) or total RNAs (20 µg) from *Arabidopsis thaliana* (*At*) or *Chlorella* transgenic and wild type (*Pto* wt) lines. Leaves were incubated with wt or mutant *Pto* DC3000 (of note, *Pto Δcor*, is deleted of the *cfa6* gene and thus cannot re-open the stomata). N, total number of analyzed stomata. Statistical analyses were performed using ANOVA and compared with the mock condition (p-value: ns >0.05; ***<0.001; ****<0.0001).
**Figure 3****. Stomatal reopening assay using concentrated media (CM) and P40 fractions from transgenic *C*. *vulgaris* lines**
   A) Stomatal reopening assay at 3 hpi on leaf sections incubated with water (Mock), total RNA (20 µg), CM or Supernatant (SN) from *Arabidopsis thaliana* (*At*) or *Chlorella* transgenic (IT20-3 and IT20-5) and wild type (wt) lines. Leaves were incubated with *Pto* DC3000 wt or mutant (*Pto Δcor*, is deleted of the *cfa6* gene and thus cannot re-open the stomata). SN corresponds to the flow-through medium after CM preparation and contains 100x less particles compared to the CM (not shown). N, total number of analyzed stomata. Statistical analyses were performed using ANOVA and compared with the mock condition (p-value: ns >0.05; ***<0.001; ****<0.0001).
   B) as in A) except that P40 fractions from the line IT20-3 was used for this assay. RNA extracts from the same *Chlorella* transgenic line was also used as positive control. N, total number of analyzed stomata. Statistical analyses were performed using ANOVA and compared with the mock condition (p-value: ns >0.05; ***<0.001; ****<0.0001).
**Figure 4****. Stomatal reopening assay using Mnase-treated P40 fractions from the IT20-3 *C*. *vulgaris* reference transgenic line**
   Stomatal reopening assay at 3 hpi on leaf sections incubated with water, total RNAs (20 µg) from *Arabidopsis thaliana* (*At*) or *Chlorella* transgenic (IT20-3) and wild type (wt) lines. The P40 fraction was obtained from the *Chlorella* IT20-3 and wt lines. Leaves were incubated with *Pto* DC3000 wt or mutant (*Pto Δcor*, is deleted of the *cfa6* gene and thus cannot re-open the stomata). P40 fractions were incubated for 30' at 37°C in the presence or absence of 300U/ml of Mnase. The digestion reaction was blocked by adding EGTA at a final concentration of 20 mM. N, total number of analyzed stomata. Statistical analyses were performed using ANOVA and compared with the control condition (p-value: ns >0.05; ***<0.001; ****<0.0001).
**Figure 5****. Schematic representation of the reporter systems that will be used to monitor EV-embedded siRNA activity in bacterial cells**
   A) Schematic representation of the first dual reporter family cassette designed to detect a specific decrease in *GFP* expression upon treatment with a given EV-embedded siRNA population. The dual reporter cassette is composed of a first *DsRed* reporter construct driven by a constitutive promoter (e.g. *the Rpsm* promoter sequence depicted here as an example) and a downstream terminator sequence (e.g. the *TonB* terminator sequence depicted here as an example); a second construct composed of a destabilized *GFP* reporter version (e.g. the *GFPsmf2* sequence carrying a degradation tag in its downstream region or the intermediate stability *GFP* variant *gpf-aav* sequence (Campbell-Valois *et al*, 2014; Elowitz & Leibler, 2000) that contains the siRNA target sequence region of interest cloned in its downstream region (or upstream, not shown), driven by a constitutive promoter (e.g. the *NPTII* promoter sequence depicted here as an example), with a downstream terminator sequence (e.g. the *SoxR* terminator sequence depicted here as an example).
   B) Schematic representation of the second tripartite cassette designed to detect a specific gain *of GFP* expression upon treatment with a given EV-embedded siRNA population. The tripartite cassette is composed of a first construct that includes a short-lived variant of a transcriptional repressor, such as the depicted *TetR-lite,* which contains a siRNA target region of interest in its downstream region (or eventually in its upstream region, not shown), driven by a constitutive promoter (e.g. the *NPTII* promoter sequence depicted here as an example) and a downstream terminator sequence (e.g. the *SoxR* terminator sequence depicted here as an example); a second construct that includes a destabilized *GFP* reporter sequence (e.g. the *GFPsmf2* sequence carrying a degradation tag in its downstream region or the intermediate stability *GFP* variant *gpf-aav* sequence (Campbell-Valois *et al,* 2014; Elowitz & Leibler, 2000), whose transcriptional activity is controlled by the *tetO2* operator, and composed of a downstream terminator sequence (e.g. the *soxR* or *tonB* terminator sequences depicted here as an example); a third construct that includes a *DsRed* reporter sequence driven by a constitutive promoter (e.g. the *Rpsm* promoter sequence depicted here as an example) and a downstream terminator sequence (e.g. the *TonB* terminator sequence depicted here as an example).
   C) Schematic representation of the third bipartite cassette designed to detect a specific gain of a reporter gene expression upon treatment with a given EV-embedded siRNA population. The bipartite cassette is composed of a first construct that includes a short-lived variant of a transcriptional repressor, such as the depicted *TetR-lite,* which contains a siRNA target region of interest in its downstream region (or eventually in its upstream region, not shown), driven by a constitutive promoter (e.g. the *NPTII*promoter sequence depicted here as an example), and a downstream terminator sequence (e.g. the *SoxR* terminator sequence depicted here as an example); a second construct that includes a destabilized *GFP* reporter sequence (e.g. the *GFPsmf2* sequence carrying a degradation tag in its downstream region or the intermediate stability GFP variant *gpf-aav* sequence (Campbell-Valois *et al*, 2014; Elowitz & Leibler, 2000) or a bioluminescence reporter (e.g. the the *Photorhabdus luminescens* operon *luxCDABE* (Meighen, 1991), whose transcriptional activity is controlled by the *tetO2* operator, and composed of a downstream terminator sequence (e.g. the *soxR* or *tonB* terminator sequence depicted here as an example).

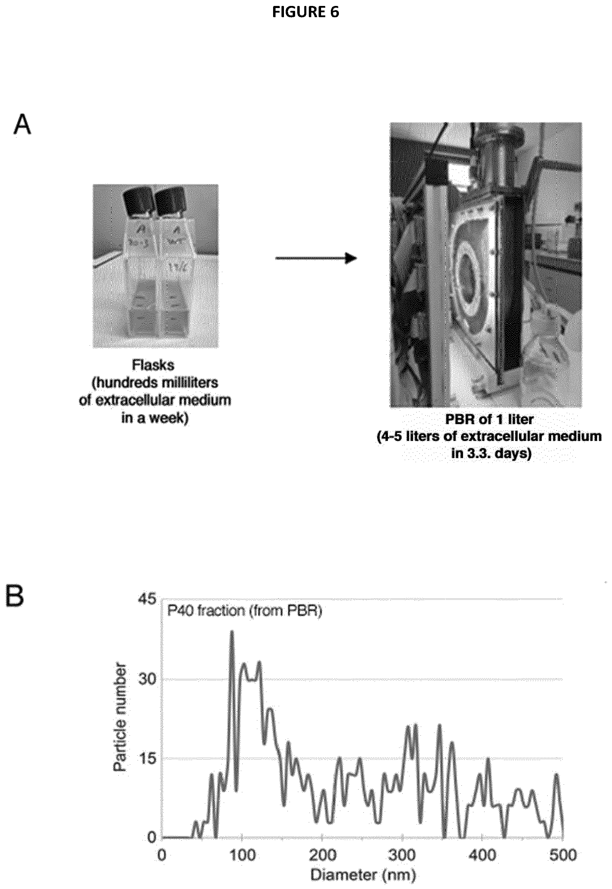

**Figure 6****. The cultivation of a reference** *Chlorella* **line in photobioreactors does not affect the quality and functionality of the corresponding P40 fractions**
   A) *Chlorella* can be easily cultivated in flask (100s ml scale) or in photobioreactor (PBR, liters scale).
   B) Size distribution, in the range 0-500 nm, of *Chlorella* particles from P40 factions obtained from PBR cultures determined through scattering analysis (using the Particle Metrix ZetaView system).

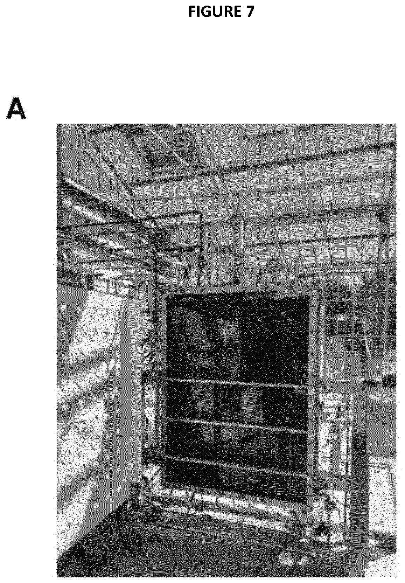

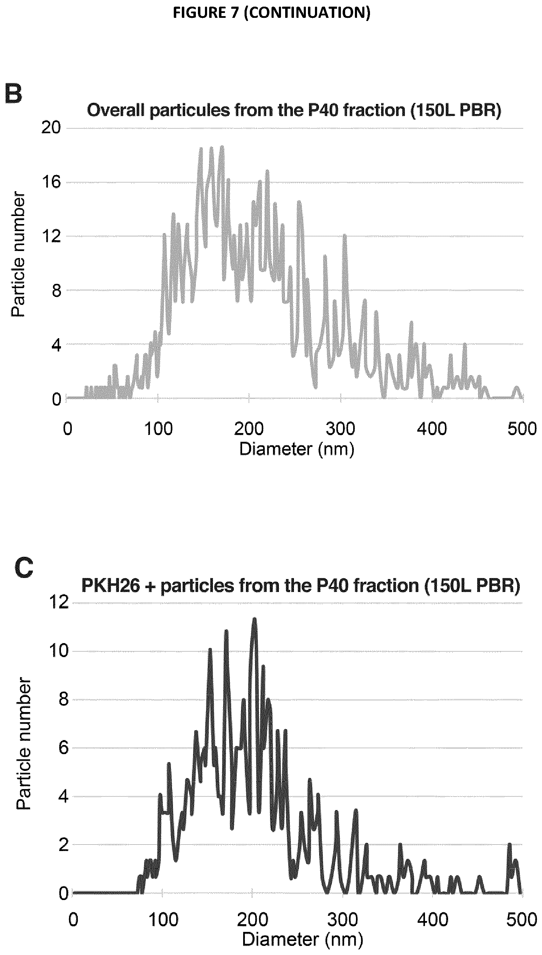

**Figure 7****. *Chlorella* EV-embedded *anti-cfa6* and *anti-hrpL* siRNAs produced from 150 L PBR retain a full integrity and functionality**
   A) Picture of the 150 L PBR (AlgoSolis, Saint Nazaire, France) used for the production of the *Chlorella* IR-*CFA6*/*HRPL* reference line
   (B) Size distribution of overall *Chlorella* particles from P40 fractions obtained in the 150L PBR, determined through scattering analysis (using the Particle Metrix ZetaView system).
   C) Size distribution of PKH26-labeled *Chlorella* particles from P40 fractions obtained in the 150L PBR (using the Particle Metrix ZetaView system). The measurements were performed using the 488 nm laser.

### EXAMPLES

### EXAMPLE 1: Materials and methods

### Chlorella vulgaris material and growth conditions

The wild type C. *vulgaris* strain UTEX263 was kept in BG11, 1% agar plates and grown in autotrophic conditions in a Sanyo MLR-351 growth chamber. Environmental conditions were kept at 25 °C, 14/10 photoperiod and about 100 µmol/m²/s of light intensity. Transgenic *Chlorella* lines were kept in the same condition using plates containing 20 µg/ml of Hygromycin. Liquid culture was started by inoculating a single colony in BG11 (pH 7) in aerated 25 cm² plastic flasks with no agitation. Culture density was assessed by using a Malassez chamber. To assess culture axenicity, routine contamination tests were performed by adding 1 ml of culture to BG11 supplemented with peptone. The mixture was kept in the dark for 3 weeks and bacterial growth followed by microscopic observation. *Chlorella* production in the 150 L PBR was carried out under continuous light cycle regime, with a light intensity increasing from 150 to 400 µmol/m²/s of white light to cope with the growing cell density in the PBR, a mean temperature of 22,9±6°C and a fixed pH at 8. In those standard growth conditions, the transgenic *Chlorella* cells reached a maximum culture density of about 1.1 g/L after 8 days. Cell-free medium collection was performed by two successive rounds of centrifugation at 3600g, for a gross cell precipitation, and 4000g to remove all the remaining cells.

### Bioinformatic searches, sequence conservation and phylogenetic analyses

To identify proteins belonging to the vesicle and extracellular vesicle biogenesis or functions, candidate human and plant proteins were used as query for BLASTP analyses on the NCBI and JGI (*Chlorella variabilis*) databases. The first 10 hits were retained and used for local alignments with the query sequence. The best candidates (*i.e*. the ones with the higher sequence similarity) were also analyzed on the Pfam and SMART databases in order to compare the protein domain composition with the query. The *Chlorella* proteins showing high sequence similarity and a conserved domain composition were considered as "potential orthologs". For *Chlorella* AGO and DCL protein phylogenetic analysis, the protein sequences of AGO and DCL *of Homo sapiens* and *Arabidopsis thaliana* were used as queries for BLASTP analyses against the C. *variabilis* genome (JGI). The protein sequences for plant, animal and fungal AGOs (Murphy et al., 2008) and DCL (Mukherjee et al., 2013; Gao et al., 2014) were obtained from the literature. A total of 111 AGO and 77 DCL proteins were retained after preliminary alignments to eliminate the divergent sequences. Protein domain architecture was analyzed to unambiguously identify AGO and DCL proteins similar to the canonical ones. The sequence alignments were manually trimmed to keep only the most conserved regions for the analysis corresponding to 288 aa for AGO and 436 aa for DCL. MEGA X software was used to perform the NJ phylogenies and the trees edited using FigTree 1.4.

### Generation of constructs for small RNAs production in Chlorella

Inverted repeat constructs producing artificial siRNAs targeting specific regions of virulence and essential genes from various bacterial plant pathogens were generated using the Green Gate assembly strategy. The gene specific or chimeric targeted regions were cloned as "B" (sense) and "D" (antisense) modules and assembled in expression *constructs.* All the generated hairpins contain a specific intron sequence from the *Petunia Chalcone* synthase gene *CHSA* (SEQ ID NO: 149) and were under the control of *Cauliflower Mosaic Virus* (*CaMV*) *35S* promoter, including a Hygromycin resistance cassette. The chimeric *cfa6-hrpL* construct (IT20) has been previously described (Singla-Rastogi *et al*., 2019; Singla-Rastogi & Navarro, PCT/EP2019/072169, PCT/EP2019/072170).

All the chimeric constructs were obtained through simultaneous ligations of the different DNA fragment into a "B" Green Gate module and specific oligonucleotides were used to generate and clone the antiparallel strand as a "D" module. All the plasmids were verified by restriction analysis, Sanger sequencing and then introduced into the *Agrobacterium tumefaciens* strains C58C1 by electroporation.

### Generation of C. vulgaris transgenic lines

*C. vulgaris* genetic transformation was performed using a disarmed *A. tumefaciens* strain. In more detail, 5 x 10⁸ total cells from an exponentially growing culture were plated on BG11 agar plates and grown under normal light irradiance for 5 days. *A. tumefaciens* carrying the appropriate inverted repeat construct was pre-inoculated the day before the transformation either from glycerol stock or from a LB plate. The day of the transformation, the *A. tumefaciens* pre-inoculum was used to seed 50 ml of LB and grown up to OD₆₀₀ = 0,8-1,2. At the right optical density, the bacteria were collected, washed and resuspended in induction medium (BG11, pH 5,6, acetosyringone 100 µM) at OD₆₀₀ = 0,5. *Chlorella* cells were gently scraped form the plates, resuspended in 200 µl of bacteria and co-cultivated for 2 days in induction medium agar plates in the dark at 25°C. After the co-cultivation, the cells were harvested, put in 7 ml of BG11 supplemented with 50 µg/L of TIM or Cefotaxime and left in the dark for 2 days at 25°C. Finally, the cells were collected and plated onto BG11 agar plates supplemented with 20 µg/ml of Hygromycin and 50 µg/ml of TIM or Cefotaxime. After 2 days in the dark, the plates were exposed to the light. After 2-3 weeks, 20-30 colonies were plated on fresh BG11 agar plates with 20 µg/ml of Hygromycin.

### Selection and identification of C. vulgaris transgenic lines

To identify the clones carrying the expression construct, gDNA from the transformant colonies was collected as follows. A few *Chlorella* cells were scraped with a sterile plastic tip from the colony growing on agar plates and put in 10 µl of HotShot5 lysis buffer (150 mM NaOH, 0,1 mM EDTA, 1% Triton X-100). The mix was incubated for 10' at RT and boiled for 15' at 95°C. The lysate was then diluted by adding 100 µl of H₂O and 1-5 µl used as template for a PCR reaction using IT-specific oligonucleotides. The wild type strain was included as negative control and the corresponding IT plasmid (5 ng per reaction) as positive control.

### Total RNA extraction (Chlorella)

50-100 ml of liquid *Chlorella* culture (5 x10⁶ - 1 x 10⁷ cells/ml) were harvested by centrifugation (Beckman rotor JS5.3, 5000g, 15', 18°C), the pellet washed in 1X PBS and flash frozen in LN. The frozen pellet was ground to a fine powder in liquid nitrogen, using a mortar and pestle. Total RNA extraction was performed using Tri-Reagent (Sigma, St. Louis, MO) according to manufacturer's instructions using about 100 mg of powder.

### P40 fraction purification

The BG11 collected after cell separation was further centrifuged (Beckman rotor JS5.3, 5000g, 15', 18°C) to eliminate all residual cells. The supernatant was then filtered using Pall Macrosep 100K devices (MAP100C37) according to manufacturer's instructions. The recovered concentrated medium (CM) was then passed through 0.45 µm filters and stored at 4°C. Starting from the CM, the P40 fraction was obtained by ultracentrifugation at 40,000g for 1 hour at 4°C in a Sorvall WX 80 Ultracentrifuge (ThermoFischer). After centrifugation, the supernatant was discarded and the P40 pellet resuspended in 1 ml of filtered 1X PBS. For sample quality analysis, 1/10 of the P40 sample was processed using a Nanoparticle Tracking system (ParticleMetrix ZetaView). To estimate the amount of exosome-like EVs in the sample, the particles were labeled using the PKH26 dye.

To recover the P40 fraction from the cell-free extracellular medium of 150L PBR a modified protocol of ultrafiltration and ultracentrifugation was employed. At first, two rounds of vacuum filtration on Millipore Glass Fiber Prefilters AP25 (2 µm) were performed. Then, the sample was centrifuged at 5000g (10', 4°C) followed by a second vacuum filtration on MF-Millipore 0,65 µm filters, required to eliminate the suspended organic matter still present in the cell-free medium. The clarified medium was then processed as described above to purify the P40 fraction by ultrafiltration and ultracentrifugation.

### Stomatal reopening assay

Plants were kept under light (100 µE/m²/s) for at least 3 hours before subjecting them to any treatment to ensure full expansion of stomata. Intact young leaf sections, at least 6 per condition, were immersed in water or bacterial suspension (at a concentration of 10⁸ cfu/ml). After 3 hours of infection with the bacteria, unpeeled leaf surface was observed under SP5 laser scanning confocal microscope and 10-15 pictures were taken from different leaf surface regions. From the pictures, at least 60 stomata per condition were manually measured using ImageJ 1.53c to obtain their width and length. The width/length ratio was calculated using excel and statistical analysis performed using the ANOVA test using the mock condition as reference. For RNA and P40 treatments, the leaf sections were incubated with total RNAs for one hour before inoculation with the different *Pto* DC3000 strains.

For Mnase protection assay, before incubation with the leaf sections, the samples were treated incubating them for 30' at 37°C in presence or absence of 300U/ml of Mnase. The reaction was stopped by adding EGTA to a final concentration of 20 mM before using the samples for the stomatal reopening assay.

### EXAMPLE 2: The Chlorella variabilis genome is composed of both highly conserved EV biogenesis factors as well as plant-related EV factors

To determine whether *Chlorella* could be exploited as scaffold for EV-embedded siRNA production, we have first investigated whether core components required for EV biogenesis and functions could be present in its genome. For this end, we have conducted an *in silico* comparative genomic analysis using available genomes of *Chlorella variabilis, Saccharomyces cerevisiae, Homo sapiens* and *Arabidopsis thaliana.* Results from this analysis revealed that *Chlorella variabilis* encodes putative orthologs of the ESCRT-I, ESCRT-II and ESCRT-III complexes and of the plant FREE1/FYVE1-like protein, a plant-specific ESCRT essential for intracellular vesicle biogenesis (Table 1, Kolb *et al,* 2015).

**Table 1: Comparison of the EV-related factors and other microvesicle-related proteins in Yeast, Human, Plant and Chlorella**

| **Regulators** | **Yeast (Sc)** | **Human (Hs)** | **Plant (At)** | ***C. variabilis*** |
|---|---|---|---|---|
| ESCRT-0 and Equivalent | VPS27 | HRS | - | - |
| | Hse1 | STAM1,2 | - | - |
| | - | - | FREE1/FYVE1 | XP_005846966 |
| | - | - | PROS | - |
| Proteins with functional analogies to ESCRT-0 | GGA 1, 2 | GGA 1, 2, 3 | - | - |
| | - | TOM1 | TOL1 | - |
| | - | TOM1L1 | TOL2 | - |
| | - | TOM1L2 | TOL3 | - |
| | - | TOM1L3 | TOL4 | - |
| | - | - | TOL5 | - |
| | - | - | TOL6 | - |
| | - | - | TOL7 | - |
| | - | - | TOL8 | - |
| | - | - | TOL9 | - |
| ESCRT -I | VPS23 | TSG101 | VPS23A/ELC | XP_005843934 |
| | - | - | VPS23B | XP_005843933 |
| | VPS28 | hVPS28 | VPS28-1 | XP_005846911 |
| | - | - | VPS28-2 | - |
| | VPS37 | VPS37 A, B, C, D | VPS37-1 | XP_005846305 |
| | - | - | VPS37-2 | - |
| | MVB12 | hMVB12 A, B | | - |
| | UBAP1 | - | | - |
| ESCRT-II | VPS22 | EAP30 | VPS22 | XP_005846063 |
| | VPS25 | EAP20 | VPS25 | XP_005851443 |
| | VPS36 | EAP45 | VPS36 | XP_005844559 |
| ESCRT-111 | VPS20 | CHMP6 | VPS20-1 | XP_005848234 |
| | SNF7 (VPS32) | CHMP4 A, B, C | SNF7-1 | XP_005850888 |
| | - | - | SN F7-2 | |
| | VPS24 | CHMP3 | VPS24-1 | - |
| | - | - | VPS24-2 | |
| | VPS2 | CHMP2 A, B | VPS2-1 | XP_005849915 |
| | | - | VPS2-2 | XP_005851546 |
| | - | - | VPS2-3 | - |
| | Did2 | CHMP1.5, | CHMP1A | - |
| | - | CHMP1A, CHMP1B | CHMP1B | - |
| | VPS60 | CHMP5 | VPS60-1 | XP_005849964 |
| | - | - | VPS60-2 | - |
| | IST1 | - | ISTL1 | XP_005842806 |
| | Cmp7 | CHMP7 | CHMP7 | - |
| Other ESCRT-related proteins | BR01 | ALIX | BRO1/AUX | XP_005850236 |
| | - | - | Bro1L1 | - |
| | - | - | Bro1L2 | - |
| | Doa4 | AMSH | AMSH1 | XP_005850797 |
| | - | - | AMSH2 | XP_005851620 |
| | - | - | AMSH3 | XP_005845954 |
| ESCRT Independent | NMASE2 | - | - | - |
| | Rab27A | - | - | X_005846307 |
| | Rab27B | - | - | XP_005851611 |
| | - | - | EXO70E2 | ChlNC64A_1 150911 |
| VPS4 and accessory proteins | VPS4 | VPS4/SKD1 | VPS4/SKD1 | XP_005847253 |
| | - | - | - | XP_005845860 |
| | VTA1 | LIP5 | LIP5 | XP_005852259 |
| Microvesicles-related | ARF1 | - | - | ChlNC64A_1\|49011 |
| | - | - | - | ChlNC64A_1\|20429 |
| | - | - | - | ChlNC64A_1\|24653 |
| | ARF6 | - | - | ChlNC64A_1\|35570 |
| | RAC1 | - | - | ChlNC64A_1\|36145 |
| | RHOA | - | - | ChlNC64A_1\|29716 |
| | RAB22 | - | - | ChlNC64A_1\|33302 |
| Marker of Plant Evs | - | - | Syn121/PEN1 | ChlNC64A_1\|53691 |

Surprisingly, we did not identify canonical ESCRT-0-related proteins (*e.g*. human STAM1/2), suggesting that the *Chlorella* ESCRT-0 complex is more likely composed of different and yet-unknown factors. Another intriguing observation is the apparent absence of tetraspanin in *Chlorella variabilis* genome: searches using specific candidates (*e.g.* human CD63) or by using the tetraspanin domain (PF00335) against pfam or JGI protein domain databases failed to identify such factors (which are known to be present in both plants and mammals). By contrast, ESCRT-independent EV biogenesis and microvesicles factors, like Rab GTPases (*e*.*g*. orthologs of human Rab27a and Rab27b, which control different steps of exosome secretion (Ostrowski *et al*., 2010), were recovered in the *Chlorella variabilis* genome. Furthermore, we retrieved putative orthologs of the syntaxin PENETRATION1 (PEN1), which has recently been characterized as an exosome marker in both *Arabidopsis* and *Nicotiana benthamiana* (Rutter and Innes, 2017; Zhang *et al*., 2020). Overall, our data indicate that the genome of C. *variabilis* is composed of conserved EV-related factors, shared between humans, yeast and plants, but also of EV-related factors that have so far been exclusively recovered from plant genomes. They also suggest that the mechanisms of *Chlorella* EV biogenesis and functions is more closely related to the ones from plants than from yeasts or humans.

### EXAMPLE 3: The extracellular medium of Chlorella vulgaris grown in flasks contains exosome-like EVs that are in a size range between 50 and 200 nm

To experimentally verify that *Chlorella* can produce EVs, and to characterize these lipid-based vesicles, we next decided to adapt protocols that have been previously used for the isolation and purification of *Arabidopsis* leaf apoplastic EVs (Rutter and Innes, 2017; Singla-Rastogi *et al*., 2019; Singla-Rastogi & Navarro, PCT/EP2019/072169, PCT/EP2019/072170). Briefly, cell-free culture medium from *Chlorella* grown in flasks was first concentrated using 100k MWCO Pall membranes by centrifugation, in order to obtain a concentrated medium (30-50x) to be used for EVs purification. The resulting concentrated medium (CM) was further subjected to ultracentrifugation at a centrifugation speed of 40,000g, to separate *Chlorella* EVs from the secreted proteins/polysaccharides, as previously reported in *Arabidopsis* (Rutter and Innes, 2017). The latter purification step leads to the recovery of a fraction referred to as the "P40 fraction". Nanoparticle tracking analysis (NTA) of this fraction revealed the presence of particles populations with a size range between 50 and 500 nm, and with a more discrete and abundant population centered around ∼120 nm (Figure 1A). Further labeling of the P40 fraction with the lipophilic dye PKH26, which uses aliphatic tails to anchor into lipid bilayer (Fick et al., 1995; Askenasy et al., 2002), and that is classically used to label mammalian and plant exosomes for fluorescence imaging (Chuo *et al*., 2018), revealed that the vast majority of particles that are above 200 nm in size are not exosome-like EV particles (Figure 1B). Based on this fluorescence imaging coupled with NTA analysis, we conclude that *Chlorella* exosome-like EVs, which are in a size range between 50 and 200 nm, can be recovered from the cell-free culture medium of flasks. To date, these results provide the first evidence supporting the presence of *Chlorella* exosome-like EVs in a cell-free culture medium.

### EXAMPLE 4: Chlorella can be engineered to produce small RNAs with antimicrobial activity

Previous studies demonstrated that the exogenous administration of small RNAs and/or long dsRNAs can be effective against eukaryotic pathogenic/parasitic (micro)organisms including fungi, oomycetes, insects and nematodes (Ivashuta *et al*., 2015; Wang *et al*., 2016; Koch *et al*., 2016; Wang & Jin., 2017; Wang *et al*., 2017; Singla-Rastogi *et al*., 2019; Singla-Rastogi & Navarro, PCT/EP2019/072169, PCT/EP2019/072170). Furthermore, a recent work showed that environmental RNAi against bacteria specifically relies on small RNA entities rather than on long dsRNAs (Singla-Rastogi *et al*., 2019; Singla-Rastogi & Navarro, PCT/EP2019/072169, PCT/EP2019/072170). Therefore, beyond the importance of producing EVs and being adapted for scalable production, and in order to efficiently target prokaryotic cells, it was essential to choose a biological system capable of processing long dsRNAs into small RNAs. This was potentially the case of *Chlorella,* because its genome contains a simple RNAi machinery composed of single DCL and AGO proteins (Cerrutti *et al.* 2011), which we found phylogenetically related to their plant counterparts (Figure 2A). To test whether *Chlorella* could be used for the production of antimicrobial small RNAs, we transformed *Chlorella vulgaris* with an inverted repeat (IR) transgene carrying sequence homology with two major virulence factors *of Pto* DC3000, a Gram negative *Pseudomonas syringae* pathogen which was previously shown to be sensitive to environmental RNAi (Figure 2B; Singla-Rastogi *et al*., 2019; Singla-Rastogi & Navarro, PCT/EP2019/072169, PCT/EP2019/072170). The first targeted virulence factor is the coronafacic acid polyketide synthase I (*cfa6*) gene, which encodes a major structural component of the phytotoxin coronatine (COR) (Brooks *et al*., 2004). The second one is *hrpL*, which encodes an alternative sigma factor that is known to directly control the expression of type III-secretion system associated genes, and to indirectly regulates the expression of COR biosynthesis genes (Fouts *et al*., 2002; Sreedharan *et al*., 2006). Interestingly, when stably expressed in *Arabidopsis,* the IR-*CFA6*/*HRPL* inverted repeat is efficiently processed by endogenous plant DCLs into anti*-Cfa6* and *anti-HrpL* siRNAs, which in turn trigger the repression of *Cfa6* and *hrpL* genes in *Pto* DC3000, along with the dampening of its pathogenicity during infection (Singla-Rastogi *et al*., 2019; Singla-Rastogi & Navarro, PCT/EP2019/072169, PCT/EP2019/072170). Importantly, some of these phenotypes are fully recapitulated upon exogenous administration of total RNAs from these transgenic plants, which contain effective anti*-Cfa6* and *anti-HrpL* siRNAs (Singla-Rastogi *et al*., 2019; Singla-Rastogi & Navarro, PCT/EP2019/072169, PCT/EP2019/072170). In particular, the exogenous application of these RNA extracts suppresses the ability of *Pto* DC3000 to trigger stomatal aperture, a major virulence response employed by this bacterium to enter through stomata and colonize inner leaf tissues (Melotto *et al*., 2006; Singla-Rastogi *et al*., 2019; Singla-Rastogi & Navarro, PCT/EP2019/072169, PCT/EP2019/072170). By using the same phenotypical readout, which is highly sensitive to *anti-Cfa6* and *anti-HrpL* siRNAs, we found that RNA extracts derived from the five independent *Chlorella* IR-*CFA6*/*HRPL* lines tested, suppressed stomatal reopening events, to the same extent as RNA extracts from *Arabidopsis* IR-*CFA6*/*HRPL* plants, thereby mimicking the impaired stomatal reopening phenotype observed in response to a *Pto* DC3000 mutant strain unable to produce COR (Figure 2C). Because this phenotype is known to be dependent on anti*-Cfa6* and *anti-HrpL* siRNAs, but not on unprocessed IR-*CFA6*/*HRPL* transcripts (Singla-Rastogi *et al*., 2019; Singla-Rastogi & Navarro, PCT/EP2019/072169, PCT/EP2019/072170), our data indicate that *Chlorella* IR-*CFA6*/*HRPL* lines are likely competent for antibacterial siRNAs production. These results therefore indicate that *Chlorella* can be engineered to produce small RNAs with antimicrobial activity.

### EXAMPLE 5: EVs from Chlorella IR-CFA6/HRPL transgenic lines presumably deliver anti-Cfa6 and anti-HrpL siRNAs in Pto DC3000, resulting in the dampening of its pathogenicity

Previous studies have reported that plant EVs can deliver biologically active antimicrobial small RNAs in fungal, oomycetal and bacterial cells (Cai *et al*., 2018; Teng *et al*., 2018; Hou *et al.* 2019; Singla-Rastogi *et al*., 2019; Singla-Rastogi & Navarro, PCT/EP2019/072169, PCT/EP2019/072170), thereby reducing their pathogenicity. To investigate whether this phenomenon holds also true for antimicrobial small RNAs embedded into *Chlorella* EVs, we first collected the cell-free medium from two independent *Chlorella* IR-*CFA6*/*HRPL* lines, and further used an ultracentrifugation method designed to retain particles that are above 30-90 nm. The resulting concentrated medium (CM), corresponding to a 30-50 time concentrate of the original *Chlorella* medium, was additionally filtered using a 0.45 µm sterilized filter to eliminate possible bacterial contaminants derived from the ultrafiltration process. In parallel, the flow through supernatant (SN), mostly deprived of EV (data not shown), was collected as control. The antibacterial activities of the CM and SN were further analyzed using a stomatal reopening assay, and RNA extracts from the *Chlorella* IR-*CFA6*/*HRPL* lines were included in the assay as positive controls. Interestingly, the CM from *Chlorella* IR-*CFA6*/*HRPL* transgenic lines suppressed stomatal reopening events, to the same extent as RNA extracts derived from the same producing cells (Figure 3A). Conversely, the SN from these transgenic lines were inactive in this process, such as the CM and RNA extracts from the wild type *Chlorella* strain used as negative controls (Figure 3A). These data indicate that *Chlorella* IR-*CFA6*/*HRPL* lines unlikely release biologically active EV-free siRNAs or, if they do so, the corresponding RNA entities are likely rapidly degraded in the extracellular medium. Instead, these results suggest that *Chlorella* IR-*CFA6*/*HRPL* lines must produce extracellular EVs -bigger than 30-90 nm- containing effective antibacterial siRNAs. Consistent with this idea, we found that the P40 fraction from the *Chlorella* IR*-CFA6*/*HRPL#3* reference line was fully competent to suppress *Pto* DC3000-induced stomatal reopening, to the same extent as total RNAs from this transgenic line (Figure 3B). We conclude that EVs from *Chlorella* IR-*CFA6*/*HRPL* lines are likely loaded with anti*-Cfa6* and anti *HrpL* siRNAs, which must be delivered in *Pto* DC3000 cells to trigger the detected antibacterial effect.

### EXAMPLE 6: EVs from Chlorella IR-CFA6/HRPL transgenic lines protect anti-Cfa6 and anti-HrpL siRNAs from digestion mediated by the Micrococcal nuclease

We have previously shown that plant EVs protect antibacterial small RNAs from digestion mediated by the non-specific Micrococcal nuclease (Mnase) (Singla-Rastogi & Navarro, PCT/EP2019/072169, PCT/EP2019/072170). Here, to determine whether *Chlorella* EVs could share similar feature, we treated the P40 fraction from the *IR-CFA6*/*HRPL#3* reference line with Mnase and further used it in a stomatal reopening assay. In more detail, the samples were incubated for 30' at 37°C in the presence or absence of 300U/ml of Mnase. At the end of the incubation, EGTA, at a final concentration of 20 mM, was added in order to inhibit Mnase activity, and the samples were further used for stomatal reopening assay. Significantly, we found that the P40 fraction treated with Mnase remained fully capable of suppressing *Pto* DC3000-induced stomatal reopening, such as the untreated P40 fraction used as control (Figure 4). These data indicate that anti*-Cfa6* and *anti-HrpL* siRNAs are protected from ribonuclease-mediated digestion when embedded into *Chlorella* EVs. The results presented in EXAMPLES 5 and 6 therefore highlight the strong potential of these nanovesicles as vehicles of antimicrobial small RNAs for agricultural applications.

### EXAMPLE 7: Generation of stable Chlorella lines expressing inverted repeat transgenes targeting essential and/or virulence factors from agriculturally relevant phytopathogens

To produce *Chlorella* EV-embedded small RNAs that might be ultimately used as RNA-based biocontrol agents against agriculturally relevant phytopathogens, we are generating inverted repeat constructs targeting essential genes and/or key virulence factors from the bacterial phytopathogens *Xylella fastidiosa, Candidatus Liberibacter asiaticus, Pseudomonas syringae pv. actinidiae, Pseudomonas syringae pv. tomato, Erwinia carotovora, Rastonia solanacearum, Xanthomonas campestris pv. campestris, Xanthomonas horturum* pv. *vitians, Xanthomonas citri pv. fucan, Acidovorax valerianella, Acidovorax citrulli;* the fungal phytopathogens *Fusarium graminearum, Botrytis cinerea, Colletotrichum* species, *Zymoseptoria tritici;* the oomycetal phytopathogens; *Phytophthora infestans, Plasmopara viticola* and the viral phytopathogen *Plum Pox Virus* (*PPV*).

The following IR constructs target *Xylella fastidiosa* genes (they contain the intron of SEQ ID NO: 149, apart from the target sequences):
- IR*-cheA*/*gaCA*/*tolC*/*pglA*/*engXCA*/*lengXCA2,* SEQ ID NO:1-2;
- IR*-cheA*/*GumH*/*GumD*/*XpsE*/*LesA*/*HolC,* SEQ ID NO:3-4;
- IR-*LesAIgumHlgumD,* SEQ ID NO:5-6;
- IR*-XpsE*/*HolC*/*LesA,* SEQ ID NO:7-8;
- IR*-cheAlpglAlLesA,* SEQ ID NO:9-10;
- IR*-cheA*/*engXCA1*/*engXCA2,* SEQ ID NO:11-12;
- IR*-gacA*/*pglAlengXCA2,* SEQ ID NO:13-14;
- IR*-cheA*/*tolC*/*engXCA1,* SEQ ID NO: 15-16.

The following IR constructs target *Candidatus Liberibacter asiaticus* genes (they contain the intron of SEQ ID NO: 149, apart from the target sequences):
- IR*-wp015452784*/*WP012778510*/*wp015452939*/*act56857*/
   *wp012778668*, SEQ ID NO:55-56;
- IR-*wp012778355*/*wp015452784*/*WP012778510,* SEQ ID NO:57-58;
- IR*-wp015452939*/*act56857*/*wp012778668* NO, SEQ ID NO:59-60.

The following IR constructs target *Erwinia carotovora* genes (they contain the intron of SEQ ID NO: 149, apart from the target sequences):
- IR*-GyrB*/*MreB*/*rbfA*/*RsgA*/*FliA*/*QseC,* SEQ ID NO:47-48;
- IR*-GyrB*/*rbfA*/*QseC,* SEQ ID NO:49-50;
- IR*-fliA*/*MreB*/*QseC* NO, SEQ ID NO: 51-52;
- IR*-GyrB*/*MreB*/*RsgA* NO, SEQ ID NO:53-54.

The following IR constructs target *Pseudomonas syringae* pv. *actinidiae* genes (they contain the intron of SEQ ID NO: 149, apart from the target sequences):
- IR*-GyrB*/*Hfq*/*HrpRIHRPSIA4reBIRpoD,* SEQ ID NO:61-62;
- *TR-GyrB*/*Hfq*/*MreB,* SEQ ID NO:63-64;
- IR*-HrpR*/*HrpS*/*Hfq,* SEQ ID NO:65-66;
- IR-*HrpR*/*GyrB*/*RpoD*, SEQ ID NO:67-/148.

The following IR constructs target genes from *Pseudomonas syringae* pv. *tomato* strain *DC3000* (they contain the intron of SEQ ID NO: 149, apart from the target sequences):
- IR*-CFA6*/*HRPL*, SEQ ID NO:90-91;
- IR*-HRPL*, SEQ ID NO:92-93;
- IR*-FusA*, SEQ ID NO:136-137;
- *IR-GyrB,* SEQ ID NO:138-139;
- IR*-FusA*/*GyrB,* SEQ ID NO:140-141;
- IR*-AvrPtolAvrPtoB,* SEQ ID NO:94-95;
- IR*-AvrPto*/*AvrPtoB*/*HopT1-1:* SEQ ID NO: 130-131
- IR-*rpoB*/*rpoC*/*FusA,* SEQ ID NO: 132-133;
- IR- *secE*/*rpoA*/*rplQ,* SEQ ID NO: 134-135;
- IR- *secE,* SEQ ID NO: 142-143;
- IR- *GyrB*/*hrpL,* SEQ ID NO: 144-145;
- IR- *dnaA*/*dnaN*/*gyrB,* SEQ ID NO: 146-147.

The following IR constructs target genes from *Ralstonia solanacearum* (they contain the intron of SEQ ID NO: 149, apart from the target sequences):
- IR*-HRPG*/*HRPB*/*HRCC,* SEQ ID NO:98-99;
- IR*-HRPB*/*HRCC*/*TssB*/*XpsR,* SEQ ID NO: 100-101.

The following IR constructs target genes from *Xanthomonas campestris pv. campestris* (they contain the intron of SEQ ID NO: 149, apart from the target sequences):
- IR-*HRPG*/*HRPX*/*RsmA,* SEQ ID NO: 102-103;
- IR*-NadHb*/*NadHd*/*NadHe,* SEQ ID NO: 104-105;
- IR*-DnaA*/*DnaE1*/*DnaE2,* SEQ ID NO: 106-107.

The following IR constructs target genes from *Xanthomonas hortorum pv. vitians* (they contain the intron of SEQ ID NO: 149, apart from the target sequences):
- IR*-GyrB,* SEQ ID NO:17-18;
- IR*-FusA*, SEQ ID NO: 19-20;
- IR*-ZipA,* SEQ ID NO:21-22;
- IR*-GyrB*/*FusA*/*ZipA,* SEQ ID NO:128-129.

The following IR constructs target genes from *Xanthomonas citri pv. fucan* (they contain the intron of SEQ ID NO: 149, apart from the target sequences):
- IR*-GyrB*/*FusA*/*MreB*/*HrpG*/*PhoP*/*FhaB,* SEQ ID NO:120-121;
- IR*-GyrB*/*RbfA*/*MreB,* SEQ ID NO: 122-123;
- IR*-HrpG*/*PhoP*/*FtsZ,* SEQ ID NO: 124-125;
- IR*-FhaB*/*FusA*/*MreB,* SEQ ID NO: 126-127;
- IR*-GyrB*/*FusA*/*ZipA,* SEQ ID NO:128-129.

The following IR constructs target genes from *Acidovorax valerianella* (they contain the intron of SEQ ID NO: 149, apart from the target sequences):
- IR*-rimM*/*rsgA*/*rbfA*/*MreB*/*gyrB*/*FtsZ,* SEQ ID NO:23-24;
- IR*-rimM*/*rbfA*/*FtsZ,* SEQ ID NO:25-26;
- IR*-RsgA*/*gyrB*/*MreB,* SEQ ID NO:27-28;
- IR*-RsgA*/*rbfA*/*FtsZ,* SEQ ID NO:29-30.

The following IR constructs target genes from *Acidovorax citrulli* (they contain the intron of SEQ ID NO: 149, apart from the target sequences):
- IR*-MreB*/*ybeY*/*rbfA*/*gyrB*/*FtsZ*/*rsgA*: SEQ ID NO:31-32;
- IR*-MreB*/*ybeY*/*rbfA*: SEQ ID NO:33-34;
- IR*-rsgA*/*gyrB*/*MreB*: SEQ ID NO:35-36;
- IR*-YbeY*/*RsgA*/*MreB*: SEQ ID NO:37-38.

The following IR constructs target genes from *Fusarium graminearum* (they contain the intron of SEQ ID NO: 149, apart from the target sequences):
- IR*-CYP51A*/*CYP51B*/*CYP51C*: SEQ ID NO:96-97.

The following IR constructs target genes from *Botrytis cinerea* (they contain the intron of SEQ ID NO: 149, apart from the target sequences):
- IR- *TOR*/*CGF1*/*DCL1*/*DCL2*/*LTF1*/*HBF1,* SEQ ID NO:76-77;
- IR*-TOR*/*DCL1*/*DCL2,* SEQ ID NO:78-79;
- IR*-CGF1*/*HBF1*/*LTF1,* SEQ ID NO:80-81;
- IR*-DCL1*/*HBF1*/*TOR,* SEQ ID NO:82-83.

The following IR constructs target genes from *Colletotrichum species* (they contain the intron of SEQ ID NO: 149, apart from the target sequences):
- IR*-CclA*/*ACS1*/*ACS2*/*ELP1*/*ELP2*/*MOB2,* SEQ ID NO:84-85;
- IR*-CclA*/*ACS1*/*ACS2,* SEQ ID NO:86-87;
- IR*-ELP1*/*ELP2*/*MOB2,* SEQ ID NO:88-89.

The following IR constructs target genes from *Zymoseptoria tritici* (they contain the intron of SEQ ID NO: 149, apart from the target sequences):
- IR*-Br1A2*/*StuA*/*F1bc*/*PKS1*/*PPT1,* SEQ ID NO: 108-109;
- IR*-Br1A2*/*StuA*/*F1bc*, SEQ ID NO:110-111;
- IR*-StuA*/*PKS*/*PPT1,* SEQ ID NO: 112-113.

The following IR constructs target genes from *Phytophthora infestans* (they contain the intron of SEQ ID NO: 149, apart from the target sequences):
- IR*-NPP1*/*INF1*/*GK4*/*piacwp1-1*/*piacwp1-2*/*piacwpl-3,* SEQ ID NO:68-69;
- IR*-piacwp1-1*/*piacwp1-2piacwp1-3,* SEQ ID NO:70-71;
- IR*-GK4*/*INF1*/*NPP1,* SEQ ID NO:72-73;
- IR-*GK4*/*INF1*/*piacwp1-1,* SEQ ID NO:74-75.

The following IR constructs target genes from *Plasmopara viticola* (they contain the intron of SEQ ID NO: 149, apart from the target sequences):
- IR*-PITG_17947*/ *PITG_10772*/ *PITG_13671*/ *PITG_16956*/ *PITG_00891,* SEQ ID NO:114-115;
- IR*-PITG_17947*/*PITG_10772*/*PITG_13671,* SEQ ID NO: 116-117;
- IR*-PITG_13671*/*PITG_16956*/*PITG_00891,* SEQ ID NO: 118-119.

The following IR constructs target genes from *Plum Pox Virus (PPV)* (they contain the intron of SEQ ID NO: 149, apart from the target sequences):
- IR*-P1*/*HC-Pro*/*CP,* SEQ ID NO: 39-40;
- IR-*P1,* SEQ ID NO:41-42;
- IR*-HC-Pro,* SEQ ID NO:43-44;
- IR*-CP,* SEQ ID NO:45-46.

### EXAMPLE 8: Design and generation of bacterial siRNA reporters to validate the biological activity of P40fraction batches produced from the Chlorella reference lines

Before proceeding with product manufacturing, it is important to generate a system allowing the rapid validation of the biological activity of each P40 fraction batch produced from the *Chlorella* reference lines. For this end, we engineer bacteria (including the *Escherichia coli* K12 strain) to express dual reporter systems that exhibit a differential siRNA targeted reporter gene expression when EV-embedded siRNAs are internalized in bacterial cells. More specifically, a first reporter system family relies on the plasmid-based expression of a cassette composed of a first construct constitutively expressing a non-targeted *DsRed* reporter that is used as an internal control for normalization, and a second construct carrying a destabilized *GFP* reporter, containing in its downstream region the antimicrobial siRNA target region of interest (Figure 5). When expressed in bacteria (e.g. *E. coli*)*,* this system is predicted to result in a specific decrease in *GFP* expression and fluorescence signal upon internalization of a given EV-embedded siRNA population. A second dual reporter system family is based on the plasmid expression of a tripartite cassette composed of a first construct expressing a short-lived variant of the transcriptional repressor, namely TetR-lite, carrying in its downstream region the antimicrobial siRNA target region of interest, a second construct composed of an intermediate stability variant of the *GFP* (Andersen *et al*., 1998; Elowitz & Leibler, 2000), whose transcriptional activity is controlled by the tetO2 (or tetO1) operator, and a third construct expressing a non-targeted *DsRed* reporter, which serves as internal control for normalization (Figure 5). In the absence of EV-embedded small RNA, TetR-lite proteins should be constitutively produced in bacteria and in turn shut-down the expression of the *GFP,* resulting in an absence of GFP fluorescence signal (only the fluorescence of the DsRed reporter should be detected). By contrast, when a given siRNA population is internalized and active in bacterial cells, the silencing of *TetR-lite* results in the derepression of the *GFP* expression, leading to the detection of GFP fluorescence signal. A third family of reporter system, relies on the plasmid-based expression of a bipartite cassette composed of a first construct expressing a short-lived variant of the transcriptional repressor, namely TetR-lite, carrying in its downstream region the antimicrobial siRNA target region of interest and a second construct composed of an intermediate stability variant of the *GFP* (Andersen *et al*., 1998; Elowitz & Leibler, 2000), or a bioluminescence reporter (*e.g.* the *Photorhabdus luminescens* operon *luxCDABE* (Meighen, 1991), whose transcriptional activity is controlled by the tetO2 (or tetO1) operator. When a given siRNA population is internalized and active in bacterial cells, the silencing of *TetR-lite* results in the derepression of the *GFP* or *luxCDABE* operon expression, leading to the detection of GFP fluorescence or bioluminescence signals. Of note, other systems than TetR-tetO2 could also be used for the same purpose, such as the *lacI-lite*/*lacO* or *cl-lite*/*λPR* systems.

### EXAMPLE 9: Chlorella IR-CFA6/HRPL EVs produced in a one liter photobioreactor maintain a normal size distribution and production rate

A prerequisite for the pre-industrialization of any MIGS-derived product, is to verify that *Chlorella* EVs maintain their integrity when produced in photobioreactors (PBRs). To address this issue, a reference *Chlorella* transgenic line expressing the IR-*CFA6*/*HRPL* transgene was grown under continuous light conditions (270 µmol/m²/s) in a PBR of one liter for 3.3 days (Figure 6A). It is noteworthy that the growth rate of this line was comparable to the one achieved with a wild type *Chlorella vulgaris* strain grown in the same PBR conditions, indicating that the expression of the inverted repeat transgene does not alter the fitness of this microalgae (data not shown). This is an important distinction from mammalian cell lines that trigger a potent inflammatory response upon sensing of long dsRNAs by RIG-I-like receptors (RLRs) (Fan & Jin, 2019). The extracellular medium from the above IR-*CFA6*/*HRPL Chlorella* culture was further collected and separated from microalgae cells using a low speed centrifugation method (two rounds of centrifugation at 3,000 to 4,000g for 10 to 15 min). *Chlorella* EVs were further purified using the ultrafiltration and ultracentrifugation methods described in the EXAMPLE 3, and the resulting P40 fractions were analyzed by NTA. We found that the size distribution of EVs was similar to the one retrieved from the same *Chlorella* line grown in flask conditions (Figures 1A, 6B). Furthermore, when comparable volumes of cell-free media recovered from flask and PBR conditions were analyzed, we detected a similar number of PKH26-positive exosome-like particles, ranging from 3.7^{∗}10⁷ to 3.8^{∗}10⁸ particles per ml from 1 liter of collected extracellular medium. These data indicate that we can maintain a normal size distribution and production rate of *Chlorella* EVs when produced in a 1 L PBR.

### EXAMPLE 10. Chlorella IR-CFA6/HRPL-derived EVs produced in a 150L PBR exhibit a normal size distribution and a ∼20-fold enhanced production rate.

To further explore the pre-industrial potential of *Chlorella-derived* EVs, it was mandatory to verify that the scaling-up from a few liters production (laboratory) up to a few m³ (pre-industrial) did not interfere with the integrity of those lipid-based particles. Moreover, it was important to ensure that the EVs yield obtained in those settings would be sufficient to further envisage the establishment a larger pre-industrial scale production. To address those issues, we grew a reference *Chlorella* IR-*CFA6*/*HRPL* line in a 150L PBR, as detailed in EXAMPLE 1. The NTA analysis revealed that, despite the presence in the original sample of suspended organic matter, the EV size distribution was well centered around 150 nm diameter, with ∼70% of the sample being in a size range between 100 and 200 nm and the rest were above 200 nm (Figure 7A). It is noteworthy that we observed much less particles above 200 nm in those conditions, compared to P40 fractions recovered from flasks or 1L PBRs (Figures 1A, 6B and 7B). Further PKH26 labeling of the P40 fractions recovered from the 150L PBR, followed by a NTA analysis in a fluorescence mode, exhibited a size distribution very similar to the one observed when a NTA analysis was conducted from unlabeled P40 fractions in a scattering mode (Figure 7B/C). This result suggests that the detected particles from unlabeled P40 fractions are lipid-based EVs, which are likely composed of exosome-like particles. Interestingly, the particle concentration obtained from 5L of cell-free medium was quite high: 3.3^{∗}10¹⁰ particles/ml, corresponding to ∼60 pM in ∼2,5 ml. In comparison, the EVs production obtained from several flasks (∼800 ml of cell-free medium), usually yield ∼0, 5 pM (3^{∗}10⁸ particles/ml) in 1 ml. Therefore, *Chlorella* EVs production in a 150 L PBR is ∼20 times more productive than in flasks. Collectively, these data indicate that *Chlorella* EVs recovered from a 150L PBR exhibit a normal size distribution. They also show that a ∼20-fold increase in EVs yield can be obtained in such PBRs, even when standard growth conditions were used. It is therefore anticipated that the EVs yield will be relatively easily enhanced by optimizing the growth conditions and subsequent pre-infiltration, filtration and purification steps.

### BIBLIOGRAPHIC REFERENCES

- Andersen, J. B., Sternberg, C., Poulsen, L. K., Bjorn, S. P., Givskov, M., & Molin, S. (1998). New unstable variants of green fluorescent protein for studies of transient gene expression in bacteria. Applied and environmental microbiology, 64(6), 2240-2246.
- Askenasy, N., & Farkas, D. L. (2002). Optical imaging of PKH-labeled hematopoietic cells in recipient bone marrow in vivo. Stem cells (Dayton, Ohio), 20(6), 501-513.
- Bai, L. L., Yin, W. B., Chen, Y. H., Niu, L. L., Sun, Y. R., Zhao, S. M., Yang, F. Q., Wang, R. R., Wu, Q., Zhang, X. Q., & Hu, Z. M. (2013). A new strategy to produce a defensin: stable production of mutated NP-1 in nitrate reductase-deficient Chlorella ellipsoidea. PloS one, 8(1), e54966.
- Baulcombe DC. (2015). VIGS, HIGS and FIGS: small RNA silencing in the interactions of viruses or filamentous organisms with their plant hosts. Curr Opin Plant Biol. 26, 141-25 6.
- Blanc, G., Duncan, G., Agarkova, I., Borodovsky, M., Gurnon, J., Kuo, A., Lindquist, E., Lucas, S., Pangilinan, J., Polle, J., Salamov, A., Terry, A., Yamada, T., Dunigan, D. D., Grigoriev, I. V., Claverie, J. M., & Van Etten, J. L. (2010). The Chlorella variabilis NC64A genome reveals adaptation to photosymbiosis, coevolution with viruses, and cryptic sex. The Plant cell, 22(9), 2943-2955.
- Bolognesi, R., Ramaseshadri, P., Anderson, J., Bachman, P., Clinton, W., Flannagan, R., Ilagan, O., Lawrence, C., Levine, S., Moar, W., Mueller, G., Tan, J., Uffman, J., Wiggins, E., Heck, G., & Segers, G. (2012). Characterizing the mechanism of action of double-stranded RNA activity against western corn rootworm (Diabrotica virgifera virgifera LeConte). PloS one, 7(10), e47534.
- Broglie, K.E., Cerf, D.C., Herrmann, R., Lu, A.L., McGonigle, B. and Presnail, J.K. (2011). Composition and Methods for Insecticidial Control of Stinkbugs. CA 2 799 453, WO 2011/153418
- Brooks, D. M., Hernández-Guzmán, G., Kloek, A. P., Alarcón-Chaidez, F., Sreedharan, A., Rangaswamy, V., Peñaloza-Vázquez, A., Bender, C. L., & Kunkel, B. N. (2004). Identification and characterization of a well-defined series of coronatine biosynthetic mutants of Pseudomonas syringae pv. tomato DC3000. Molecular plant-microbe interactions : MPMI, 17(2), 162-174.
- Cai, Q., Qiao, L., Wang, M., He, B., Lin, F. M., Palmquist, J., Huang, S. D., & Jin, H. (2018). Plants send small RNAs in extracellular vesicles to fungal pathogen to silence virulence genes. Science (New York, N.Y.), 360(6393), 1126-1129.
- Campbell-Valois, F. X., Schnupf, P., Nigro, G., Sachse, M., Sansonetti, P. J., & Parsot, C. (2014). A fluorescent reporter reveals on/off regulation of the Shigella type III secretion apparatus during entry and cell-to-cell spread. Cell host & microbe, 15(2), 177-189.
- Cerutti, H., Ma, X., Msanne, J., & Repas, T. (2011). RNA-mediated silencing in Algae: biological roles and tools for analysis of gene function. Eukaryotic cell, 10(9), 1164-1172.
- Cha, T. S., Yee, W., & Aziz, A. (2012). Assessment of factors affecting Agrobacterium-mediated genetic transformation of the unicellular green alga, Chlorella vulgaris. World journal of microbiology & biotechnology, 28(4), 1771-1779.
- Chien, L.F., Kuo, T.T., Liu, B.H., Lin, H.D., Feng, T.Y., Huang, C.C. (2012). Solar-to-bioH(2) production enhanced by homologous overexpression of hydrogenase in green alga Chlorella sp. DT, Int. J. Hydrog. Energy 37 (2012) 17738-17748.
- Colao, I. L., Corteling, R., Bracewell, D., & Wall, I. (2018). Manufacturing Exosomes: A Promising Therapeutic Platform. Trends in molecular medicine, 24(3), 242-256. Dalakouras A, Jarausch W, Buchholz G, Bassler A, Braun M, Manthey T, Krczal G, Wassenegger M (2018) Delivery of hairpin RNAs and small RNAs into woody and herbaceous plants by trunk injection and petiole absorption. Front Plant Sci 9: 1253.
- de Andrade, C. J., de Andrade, L. M. (2017). An overview on the application of genus Chlorella in bio¬technological processes. J Adv Res Biotech 2(1): 1-9. DOI:
- Elowitz, M. B., & Leibler, S. (2000). A synthetic oscillatory network of transcriptional regulators. Nature, 403(6767), 335-338.
- Escobar, M.A., Civerolo, E.L., Summerfelt, K.R., Dandekar, A.M. (2001). RNAi-mediated oncogene silencing confers resistance to crown gall tumorogenesis. Proc. Natl. Acad. Sci. 98: 13437-13442.
- Fan, X., & Jin, T. (2019). Structures of RIG-I-Like Receptors and Insights into Viral RNA Sensing. Advances in experimental medicine and biology, 1172, 157-188.
- Fick, J., Barker, F. G., 2nd, Dazin, P., Westphale, E. M., Beyer, E. C., & Israel, M. A. (1995). The extent of heterocellular communication mediated by gap junctions is predictive of bystander tumor cytotoxicity in vitro. Proceedings of the National Academy of Sciences of the United States of America, 92(24), 11071-11075.
- Fouts, D. E., Abramovitch, R. B., Alfano, J. R., Baldo, A. M., Buell, C. R., Cartinhour, S., Chatterjee, A. K., D'Ascenzo, M., Gwinn, M. L., Lazarowitz, S. G., Lin, N. C., Martin, G. B., Rehm, A. H., Schneider, D. J., van Dijk, K., Tang, X., & Collmer, A. (2002). Genomewide identification of Pseudomonas syringae pv. tomato DC3000 promoters controlled by the HrpL alternative sigma factor. Proceedings of the National Academy of Sciences of the United States of America, 99(4), 2275-2280.
- Gao, Z., Wang, M., Blair, D., Zheng, Y., & Dou, Y. (2014). Phylogenetic analysis of the endoribonuclease Dicer family. PloS one, 9(4), e95350.
- Guo, Z., Li, Y., & Ding, S. W. (2019). Small RNA-based antimicrobial immunity. Nature reviews. Immunology, 19(1), 31-44.
- Herrmann, R., Lassner, M., Lu, A.L., Nelson, M., Presnail, J.K. and Rice, J.A. (2012). Compositions and methods for the suppression of target polynucleotides from the family Aphididae. EP 2 405 013 A
- Hiruma, K., Gerlach, N., Sacristan, S., Nakano, R. T., Hacquard, S., Kracher, B., Neumann, U., Ramirez, D., Bucher, M., O'Connell, R. J., & Schulze-Lefert, P. (2016). Root Endophyte Colletotrichum tofieldiae Confers Plant Fitness Benefits that Are Phosphate Status Dependent. Cell, 165(2), 464-474.
- Hou, Y., Zhai, Y., Feng, L., Karimi, H.Z., Rutter, B.D., Zeng, L., Choi, D.S.,Zhang, B., Gu, W., Chen, X., et al. (2019). A Phytophthora Effector SuppressesTrans-Kingdom RNAi to Promote Disease Susceptibility. Cell Host Microbe25,153-165.e5
- Ivashuta, S., Zhang, Y., Wiggins, B. E., Ramaseshadri, P., Segers, G. C., Johnson, S., Meyer, S. E., Kerstetter, R. A., McNulty, B. C., Bolognesi, R., & Heck, G. R. (2015). Environmental RNAi in herbivorous insects. RNA (New York, N.Y.), 21(5), 840-850.
- Jones, JD., Vance, RE., Dangl, JL. (2016). Intracellular innate immune surveillance devices in plants and animals. Science. 354, DOI: 10.1126/science.aaf6395.
- Kim, D. H., Kim, Y. T., Cho, J. J., Bae, J. H., Hur, S. B., Hwang, I., & Choi, T. J. (2002). Stable integration and functional expression of flounder growth hormone gene in transformed microalga, Chlorella ellipsoidea. Marine biotechnology (New York, N.Y.), 4(1), 63-73. Koch A., Kumar N., Weber L., Keller, H., Imam J., Kogel, KH. (2013) Host-induced gene silencing of cytochrome P450 lanosterol C14 α-demethylase-encoding genes confers strong resistance to Fusarium species. Proc Natl Acad Sci U S A. 110: 19324-9.
- Koch, A., Biedenkopf, D., Furch, A., Weber, L., Rossbach, O., Abdellatef, E., Linicus, L., Johannsmeier, J., Jelonek, L., Goesmann, A., Cardoza, V., McMillan, J., Mentzel, T., Kogel, KH. (2016). An RNAi-based control of Fusarium graminearum infections through spraying of long dsRNAs involves a plant passage and is controlled by the fungal silencing machinery. PLoS Pathog. 12: e1005901.
- Koch, A., Kogel, KH. (2014). New wind in the sails: improving the agronomic value of crop plants through RNAi-mediated gene silencing. Plant Biotechnol J. 12, 821-31.30
- Kolb, C., Nagel, M. K., Kalinowska, K., Hagmann, J., Ichikawa, M., Anzenberger, F., Alkofer, A., Sato, M. H., Braun, P., & Isono, E. (2015). FYVE1 is essential for vacuole biogenesis and intracellular trafficking in Arabidopsis. Plant physiology, 167(4), 1361-1373.
- Lacombe, S., Rougon-Cardoso, A., Sherwood, E., Peeters, N., Dahlbeck, D., van Esse, HP., Smoker, M., Rallapalli, G., Thomma, BP., Staskawicz, B., Jones, JD., Zipfel, C. (2010). Interfamily transfer of a plant pattern-recognition receptor confers broad-spectrum bacterial resistance. Nat. Biotechnol. 28:365-9.
- Lin, H. D., Liu, B. H., Kuo, T. T., Tsai, H. C., Feng, T. Y., Huang, C. C., & Chien, L. F. (2013). Knockdown of PsbO leads to induction of HydA and production of photobiological H2 in the green alga Chlorella sp. DT. Bioresource technology, 143, 154-162.
- Mansfield, J., Genin, S., Magori, S., Citovsky, V., Sriariyanum, M., Ronald, P., Dow, M., Verdier, V., Beer, SV., Machado, M.A., Toth, I., Salmond, G., Foster, G.D. (2012). Top 10 plant pathogenic bacteria in molecular plant pathology. Mol Plant Pathol. 13:614-29.69
- Meighen E. A. (1991). Molecular biology of bacterial bioluminescence. Microbiological reviews, 55(1), 123-142.
- Melnyk, CW., Molnar, A., Baulcombe, DC. (2011). Intercellular and systemic movement of RNA silencing signals. EMBO J. 30, 3553-63.
- Melotto M, Underwood W, Koczan J, Nomura K, He SY. Plant stomata function in innate immunity against bacterial invasion. Cell. 2006;126(5): 969-980.
- Micali, CO., Neumann, U., Grunewald, D., Panstruga, R., O'Connell. (2011). Biogenesis of a specialized plant-fungal interface during host cell internalization of Golovinomyces orontii haustoria. Cell Microbiol. 13, 210-226.
- Michelmore, R. and Govindarajulu, M. (2012). Host-Induced gene silencing in vegetable species to provide durable disease resistance. WO2012/155109
- Michelmore, R., Dubcovsky, J., Govindarajulu and M., Cantu, D. (2012). Disease-Resistance in cereals mediated by host-Induced gene silencing. WO2012/155112
- Mukherjee, K., Campos, H., & Kolaczkowski, B. (2013). Evolution of animal and plant dicers: early parallel duplications and recurrent adaptation of antiviral RNA binding in plants. Molecular biology and evolution, 30(3), 627-641.
- Murphy D., Dancis B., Brown J. R. (2008). The evolution of core proteins involved in microRNA biogenesis. BMC Evol. Biol. 8:92. 10.1186/1471-2148-8-92
- Navarro, L. and Singla-Rastogi, M. (2020). RNA-Based Biocontrol Methods to Protect Plants Against Pathogenic Bacteria and / or Promote Beneficial Effects of Symbiotic and Commensal Bacteria. WO/2020/035619, PCT/EP2019/072169
- Navarro, L. and Singla-Rastogi, M. (2020). RNA-Based Therapeutic Methods to Protect Animals Against Pathogenic Bacteria and / or Promote Beneficial Effects of Symbiotic and Commensal Bacteria. WO/2020/035620, PCT/EP2019/072170
- Niu, Y. F., Zhang, M. H., Xie, W. H., Li, J. N., Gao, Y. F., Yang, W. D., Liu, J. S., & Li, H. Y. (2011). A new inducible expression system in a transformed green alga, Chlorella vulgaris. Genetics and molecular research : GMR, 10(4), 3427-3434.
- O'Brien, K., Breyne, K., Ughetto, S., Laurent, L. C., & Breakefield, X. O. (2020). RNA delivery by extracellular vesicles in mammalian cells and its applications. Nature reviews. Molecular cell biology, 1-22. Advance online publication.
- Ostrowski, M., Carmo, N. B., Krumeich, S., Fanget, I., Raposo, G., Savina, A., Moita, C. F., Schauer, K., Hume, A. N., Freitas, R. P., Goud, B., Benaroch, P., Hacohen, N., Fukuda, M., Desnos, C., Seabra, M. C., Darchen, F., Amigorena, S., Moita, L. F., & Thery, C. (2010). Rab27a and Rab27b control different steps of the exosome secretion pathway. Nature cell biology, 12(1), 19-13.
- Run, C., Fang, L., Fan, J., Fan, C., Luo, Y., Hu, Z., Li, Y. (2016). Stable nuclear transformation of the industrial alga Chlorella pyrenoidosa, Algal Research 17 (2016) 196-201
- Rutter, B. D., & Innes, R. W. (2017). Extracellular Vesicles Isolated from the Leaf Apoplast Carry Stress-Response Proteins. Plant physiology, 173(1), 728-741.
- Safi, C., Zebib, B., Merah, O., Pontalier, P.-Y., Vaca-Garcia, C. (2014). Morphology, composition, production, processing and applications of Chlorella vulgaris: A review. Renewable and Sustainable Energy Reviews, Volume 35, July 2014, Pages 265-278.
- Singla-Rastogi, M., Charvin, M., Thiebeauld, O., Perez-Quintero, A.L., Ravet, A., Fortunato, A.E., Mendu, V., Navarro, L. (2019). Plant Small RNA Species Direct Gene Silencing in Pathogenic Bacteria as well as Disease Protection. bioRxiv 863902;
- Sreedharan, A., Penaloza-Vazquez, A., Kunkel, B. N., & Bender, C. L. (2006). CorR regulates multiple components of virulence in Pseudomonas syringae pv. tomato DC3000. Molecular plant-microbe interactions : MPMI, 19(7), 768-779.
- Teng, Y., Ren, Y., Sayed, M., Hu, X., Lei, C., Kumar, A., Hutchins, E., Mu, J.,Deng, Z., Luo, C., et al. (2018). Plant-Derived Exosomal MicroRNAs Shape the Gut Microbiota. Cell Host Microbe24, 637-652.e8.
- Wang, M., Thomas, N., Jin, H. (2017). Cross-kingdom RNA trafficking and environmental RNAi for powerful innovative pre- and post-harvest plant protection. Curr Opin Plant Biol. 38:133-141.
- Wang, M., Weiberg, Arne., Lin FM., Thomma, BPH., Huang, HD., Jin, H. (2016). Bidirectional cross-kingdom RNAi and fungal uptake of external RNAs confer plant protection. Nature plants. 16151.
- Wang, Q., Zhuang, X., Mu, J., Deng, Z. B., Jiang, H., Zhang, L., Xiang, X., Wang, B., Yan, J., Miller, D., & Zhang, H. G. (2013). Delivery of therapeutic agents by nanoparticles made of grapefruit-derived lipids. Nature communications, 4, 1867.
- Weiberg, A., Bellinger, M., Jin H. (2015). Conversations between kingdoms: small RNAs. Curr Opin Biotechnol. 32, 207-15.
- Weiberg, A., Wang, M., Lin, FM., Zhao, H., Zhang, Z., Kaloshian, I., Huang, HD., Jin, H. 5 (2013). Fungal small RNAs suppress plant immunity by hijacking host RNA interference pathways. Science. 342, 118-23.
- Whangbo, J. S., & Hunter, C. P. (2008). Environmental RNA interference. Trends in genetics: TIG, 24(6), 297-305.
- Yang, B., Liu, J., Liu, B., Sun, P., Ma, X., Jiang, Y., Wei, D., Chen, F. (2015). Development of a stable genetic system for Chlorella vulgaris-a promising green alga for CO2 biomitigation, Algal Res. 12 (2015) 134-141.
- Zhang, J., Qiu, Y., & Xu, K. (2020). Characterization of GFP-AtPEN1 as a marker protein for extracellular vesicles isolated from Nicotiana benthamiana leaves. Plant signaling & behavior, 15(9), 1791519.
- Zhang, M., Viennois, E., Xu, C., & Merlin, D. (2016). Plant derived edible nanoparticles as a new therapeutic approach against diseases. Tissue barriers, 4(2), e1134415.
- Zhang, R., Lin, Y. (2009). DEG 5.0, a database of essential genes in both prokaryotes and eukaryotes. Nucleic Acids Res., 37: D455-D458.
- Zhang, T., Zhao, YL., Zhao, JH., Wang, S., Jin, Y., Chen, ZQ., Fang, YY., Hua, CL., Ding, SW., Guo, HS. (2016). Cotton plants export microRNAs to inhibit virulence gene expression in a fungal pathogen. Nature Plants.
- Zuñiga, C., Li, C. T., Huelsman, T., Levering, J., Zielinski, D. C., McConnell, B. O., Long, C. P., Knoshaug, E. P., Guarnieri, M. T., Antoniewicz, M. R., Betenbaugh, M. J., & Zengler, K. (2016). Genome-Scale Metabolic Model for the Green Alga Chlorella vulgaris UTEX 395 Accurately Predicts Phenotypes under Autotrophic, Heterotrophic, and Mixotrophic Growth Conditions. Plant physiology, 172(1), 589-602.

## Claims

1. A method for producing functional interfering small RNAs, said method comprising at least the steps of:
a) transforming *Chlorella* cells with a siRNA or miRNA precursor comprising at least one fragment of at least one target gene, and
b) cultivating said *Chlorella* cells in appropriate conditions so that they express said precursor and release extracellular vesicles (EV)-embedded functional small iRNAs targeting said at least one gene fragment.

2. The method of claim 1, wherein said siRNA or miRNA precursor is a long single- or double-stranded RNA molecule, preferably a long double-stranded RNA molecule.

3. The method of claims 1-2, wherein said gene fragment comprises between 50 and 3000 bp.

4. The method of claims 1-3, further comprising the step of recovering said small iRNAs from said *Chlorella* cells.

5. The method of claims 1-3, further comprising the step of recovering the Extracellular Vesicles (EV) released by said *Chlorella* cells in the extracellular medium.

6. The method of claims 1-5, wherein said target gene is an oomycete gene, a viral gene, a bacterial gene, or a fungus gene.

7. *Chlorella-derived* EVs obtained by the method of claim 5, said EV containing a population of functional small iRNAs targeting one or several region(s) in said at least one target gene(s).

8. *Chlorella-derived* EVs of claim 7, wherein said population of functional small iRNAs targets one or several region(s) in at least one viral gene.

9. *Chlorella-derived* EVs of claim 7, wherein said population of functional small iRNAs targets one or several region(s) in at least one bacterial gene.

10. A method for treating a parasitic infection and/or infectious disease in plants, said method comprising the use of the *Chlorella-derived* EV as defined in claims 7-9.

11. A phytotherapeutic composition containing an effective amount of *Chlorella-derived* EV as defined in claims 7 - 10 and an agronomical acceptable vehicle.

12. Recombinant *Chlorella* cells expressing a siRNA or miRNA precursor comprising at least one fragment of at least one target gene, said *Chlorella* cells releasing EV-embedded functional small iRNAs targeting said at least one gene fragment.

13. The method of claim 1-6, or the recombinant *Chlorella* cells of claim 12, wherein said siRNA precursor has a sequence chosen among SEQ ID NO:1-148.

14. Use of any of the siRNA precursor of SEQ ID NO:1-148 to produce a population of functional small iRNAs in *Chlorella* cells.

15. A versatile platform for producing high-throughput amount of EV-embedded functional interfering small RNAs, said platform using the recombinant *Chlorella* cells as defined in claim 12.
